# EUROPEAN PATENT APPLICATION

(11) **EP 4 786 459 A1**
(43) Date of publication of application: **05.08.2026**
(21) Application number: 24871008.9
(22) Date of filing: 27.09.2024
(51) Int. Cl.: C07D 333/20, C07C 275/28, C07C 275/04, C07C 275/26, A61K 31/395, A61K 31/38, A61K 31/41, A61P 25/04

(54) **BENZENE RING-CONTAINING COMPOUND HAVING ANALGESIC EFFECT, PREPARATION METHOD THEREFOR, AND USE THEREOF**

(30) Priority: 28.09.2023 CN 202311275167
(71) Applicant: West China Hospital, Sichuan University, Chengdu, Sichuan 610041 (CN)
(72) Inventor: ZHANG, Wensheng, Chengdu, Sichuan 610041 (CN); DENG, Chaoyi, Chengdu, Sichuan 610041 (CN); LUO, Xudong, Chengdu, Sichuan 610041 (CN); DENG, Yu, Chengdu, Sichuan 610041 (CN); LIU, Jin, Chengdu, Sichuan 610041 (CN); LI, Chenyang, Chengdu, Sichuan 610041 (CN); LU, Junyu, Chengdu, Sichuan 610041 (CN); ZHU, Tao, Chengdu, Sichuan 610041 (CN); LUO, Fengming, Chengdu, Sichuan 610041 (CN)
(74) Representative: Altmann Stößel Dick Patentanwälte PartG mbB
(86) International application number: PCT/CN2024/121886
(87) International publication number: WO 2025/067473

(57) **Abstract**

A benzene ring-containing compound having an analgesic effect, a preparation method therefor, and the use thereof, which relate to the field of pharmaceutical chemistry. The compound is a compound represented by formula I, or a stereoisomer thereof, or a pharmaceutically acceptable salt thereof, or a solvate thereof, or a crystal form thereof, or a prodrug thereof, or a metabolite thereof, or a deuterated derivative thereof. The compound has a uniquely advantageous analgesic effect, is highly safe when used, has only mild toxic side effects, and no dependence develops during use. Therefore, the compound has wide application prospects in the preparation of analgesic pharmaceuticals, and provides a new option when clinically preparing a drug with an analgesic effect.

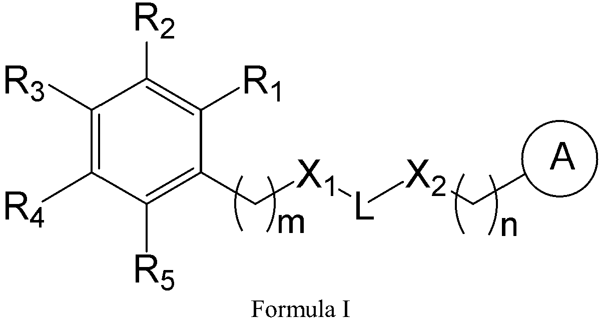

## Description

### Field of the invention

The present invention relates to the field of medicinal chemistry, and specifically to a compound containing a benzene ring with analgesic effect, as well as a preparation method therefor and uses thereof.

### Background of the invention

The Global Burden of Disease Study reports that pain and pain-related conditions are the primary causes of disability and disease burden globally. The report indicates that over 80% of patients who undergo surgery experience acute postoperative pain, and less than half of these patients receive adequate relief from postoperative pain. Among them, nearly 80% have moderate to severe pain scores, and 10% to 50% of patients develop chronic postoperative pain. Therefore, there remains a significant demand for analgesic drugs in clinical practice. In the field of pain treatment, traditional opioids remain the most effective and commonly used analgesic drugs for treating moderate to severe pain. Opioids play their analgesic effects by acting on opioid receptors within the G protein-coupled receptor family, primarily activating the downstream Gi/o protein pathway.

However, while opioids exert potent analgesic effects, they are also accompanied by numerous adverse reactions. Common adverse reactions include respiratory depression, deep sedation, nausea, vomiting, and constipation. Long-term use can lead to tolerance, decreased pain sensation, and even drug abuse and addiction, posing serious social hazards. Opioids that act on µ receptors can produce dose-dependent respiratory depression by direct effects on the respiratory center of the brainstem. It has been shown that the average addiction rate of opioids is between 8% and 12%. Patients with physical dependence or addiction to opioids often abuse opioids to avoid withdrawal symptoms.

Although researchers have successively developed many new opioid analgesics and even non-opioid analgesics over the past century, no particularly significant progress has been made. For example, Oliceridine (TRV130), a targeted µ-receptor analgesic designed based on the concept of G protein bias, was approved for marketing by the U.S. Food and Drug Administration (FDA) in 2020 for the treatment of moderate to severe pain, but it still carries a "black box warning" emphasizing its potential opioid-related side effects. Many known analgesics based on other non-opioid receptor targets have not achieved analgesic effects comparable to morphine or remifentanil due to limitations in analgesic targets or pain models, or have failed to produce consistent results in multiple clinical studies compared to animal experiments.

In summary, although traditional opioids used clinically are the most effective drugs for treating moderate to severe pain, the accompanying adverse reactions and drug dependency limit the effectiveness of pain treatment, and while reducing safety, they also give rise to serious social issues such as drug abuse. Therefore, designing a novel compound that retains the analgesic efficacy of opioids while avoiding their severe adverse reactions, as a new type of non-opioid analgesic drug, holds significant clinical importance and broad market prospects.

### Summary of the invention

The objective of the present invention is to provide a compound containing a benzene ring with analgesic efficacy, as well as a preparation method therefor and uses thereof.

The present invention provides compounds represented by formula I, or stereoisomers thereof, or pharmaceutically acceptable salts thereof, or solvates thereof, or crystal forms thereof, or prodrugs thereof, or metabolites thereof, or deuterated derivatives thereof: 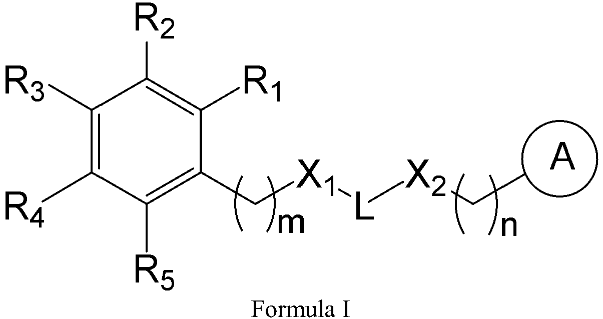 wherein,
R₁ and R₄ are each independently selected from the group consisting of H, NR₆R₇, (3-8)-membered cycloalkyl, (3-8)-membered heterocycloalkyl, substituted or unsubstituted C₁-C₆ alkyl, OR₁ₐ, and R₁ₐ is selected from the group consisting of C₁-C₆ alkyl, (5-8)-membered aryl, and (5-8)-membered heteroaryl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0 to 5, and R_{1b} is selected from the group consisting of C₁-C₆ alkyl, (3-8)-membered cycloalkyl, and (3-8)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0 to 5; n is selected from integers of 0 to 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1 to 5;
provided that ring A is 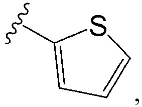 X₁ and X₂ are both NH, L is C=O, m is 0, and n is 1, 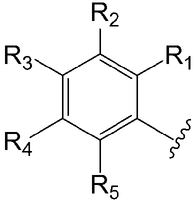 is not 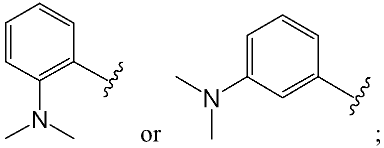
provided that ring A is 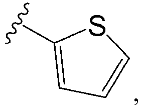 X₁ and X₂ are both NH, L is C=O, m and n are 0, 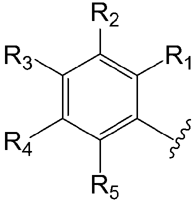 is not 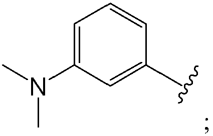
provided that ring A is 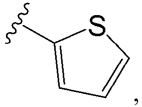 X₁ and X₂ are both NH, L is C=NH, m is 1, n is 2, 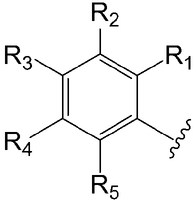 is not

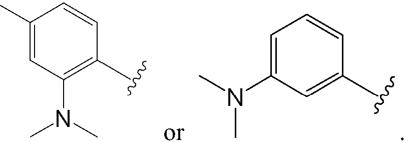

Further, the compound is represented by formula IIa or formula IIb: 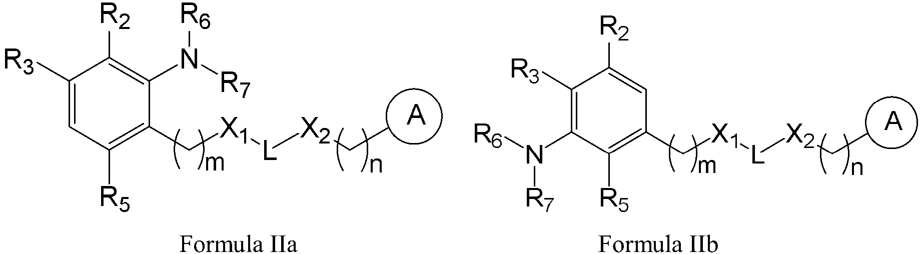 wherein,
R₂ and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0 to 5, and R_{1b} is selected from C₁-C₆ alkyl, (3-6)-membered cycloalkyl, and (3-6)-membered heterocycloalkyl;
R₃ is selected from H, substituted or unsubstituted C₁-C₆ alkyl, halogen, and hydroxyl;
R₆ and R₇ are each independently selected from the group consisting of substituted or unsubstituted C₁-C₆ alkyl, and substituted or unsubstituted C₁-C₆ alkoxy;
m is selected from 0, 1, 2, 3, 4, or 5; n is selected from 0, 1, 2, 3, 4, or 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1, 2, 3, 4, or 5;

Further, the compound is represented by formula IIIa or formula IIIb: 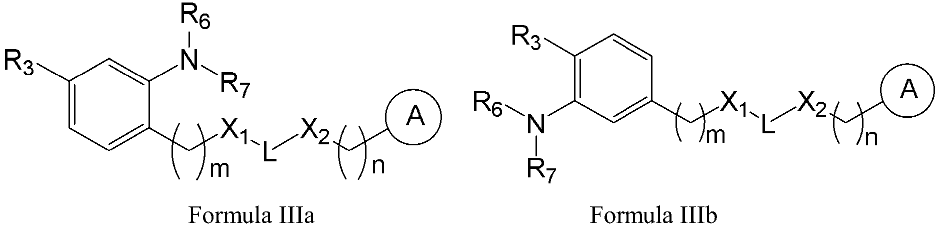 wherein,
R₃ is selected from H, substituted or unsubstituted C₁-C₆ alkyl, halogen, and hydroxyl;
R₆ and R₇ are each independently selected from the group consisting of substituted or unsubstituted C₁-C₆ alkyl, and substituted or unsubstituted C₁-C₆ alkoxy;
m is selected from 0, 1, 2, 3, 4, or 5; n is selected from 0, 1, 2, 3, 4, or 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1, 2, 3, 4, or 5;

Further,
X₁ and X₂ are each independently selected from the group consisting of O, S, and NH;
and/or, L is selected from the group consisting of 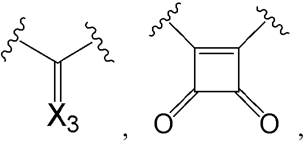 and 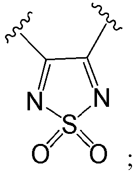
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
the number of substituents for said alkyl and alkoxy is one or two or more, and each substituent is each independently selected from the group consisting of deuterium, halogen, amino, nitro, cyano, carboxyl, hydroxyl, and mercapto.

Further, 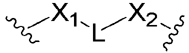 is selected from the group consisting of 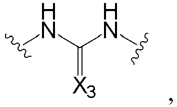 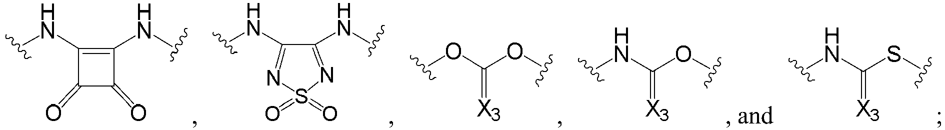
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
the number of substituents for said alkyl and alkoxy is one or two or more, and each substituent is each independently selected from the group consisting of deuterium, halogen, amino, nitro, cyano, carboxyl, hydroxyl, and mercapto.

Further, the compound is represented by formula IV: 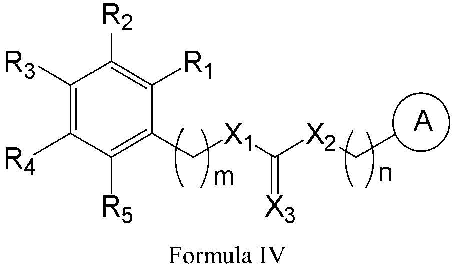 wherein,
R₁ and R₄ are each independently selected from the group consisting of H, NR₆R₇, (3-5)-membered cycloalkyl, (3-5)-membered heterocycloalkyl, substituted or unsubstituted C₁-C₆ alkyl, OR₁ₐ, and R₁ₐ is selected from the group consisting of C₁-C₆ alkyl and phenyl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-5)-membered cycloalkyl, and (3-5)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
X₁ and X₂ are each independently selected from the group consisting of O, S, and NR₈;
each of R₈ is independently selected from the group consisting of H and substituted or unsubstituted C₁-C₆ alkyl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5;

Further, the compound is represented by formula IVa: 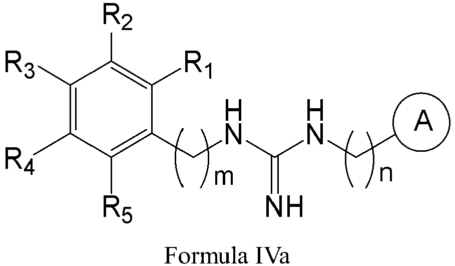 wherein,
R₁ and R₄ are each independently selected from the group consisting of H, NR₆R₇, (3-4)-membered cycloalkyl, (3-4)-membered heterocycloalkyl, substituted or unsubstituted C₁-C₆ alkyl, OR₁ₐ, and R₁ₐ is selected from the group consisting of C₁-C₆ alkyl and phenyl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused ((5-6)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

Further, the compound is represented by formula Va or Vb: 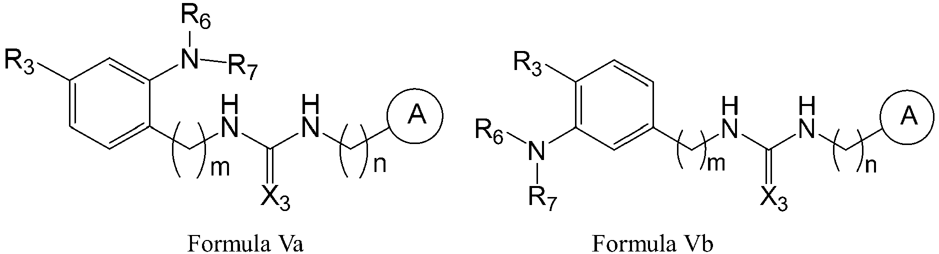 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

Further, the compound is represented by formula VIa or VIb: 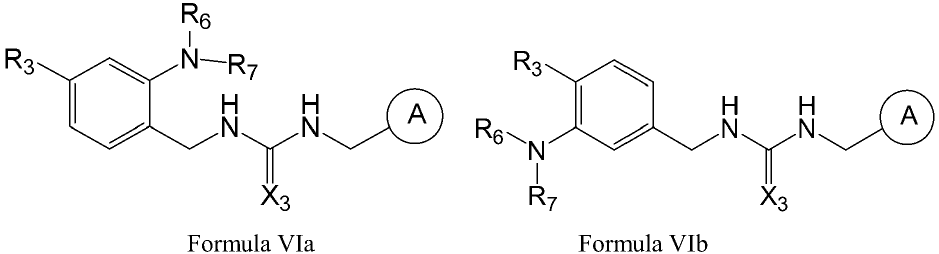 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

Further, the compound is represented by formula VIIa or VIIb: 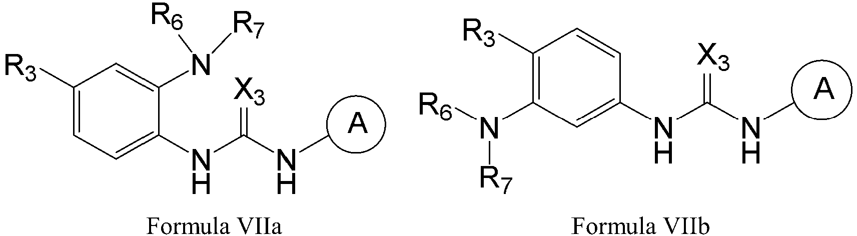 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

Further,
ring A is selected from the group consisting of substituted or unsubstituted following groups:

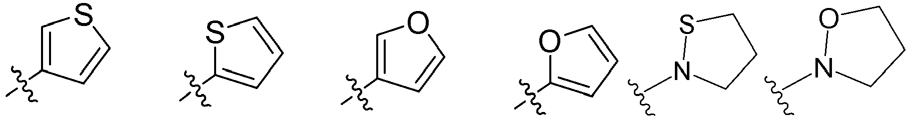

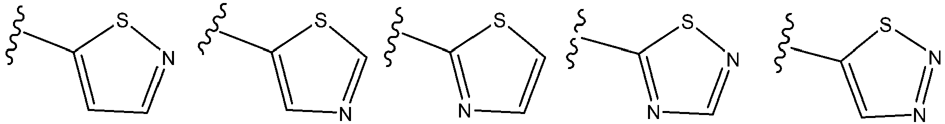

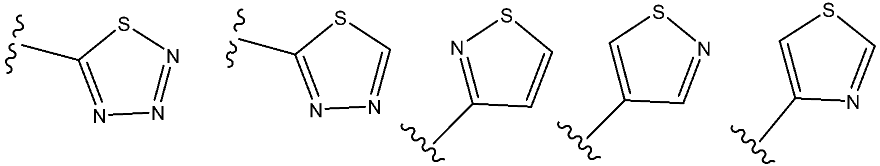

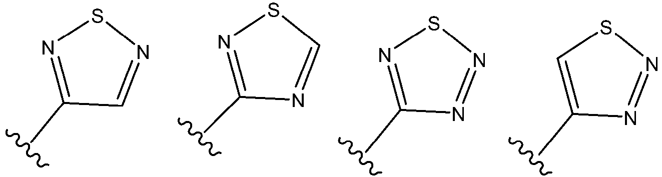

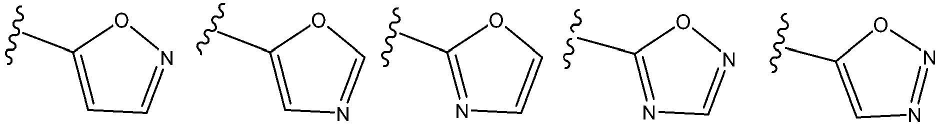

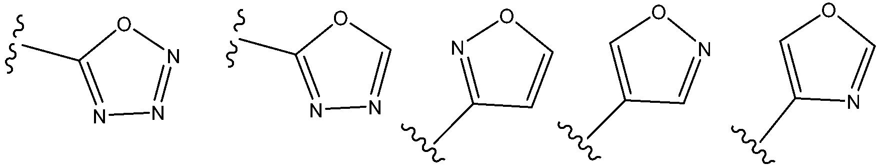

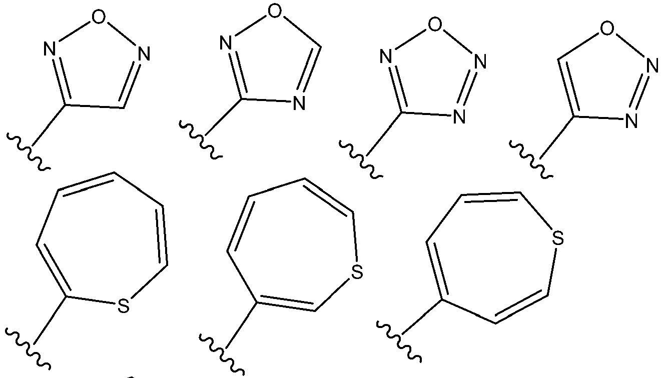

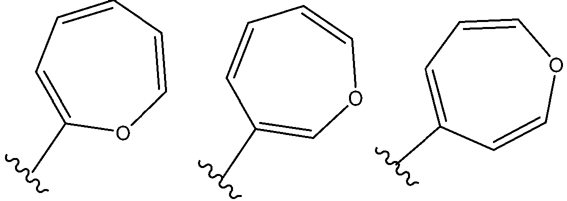

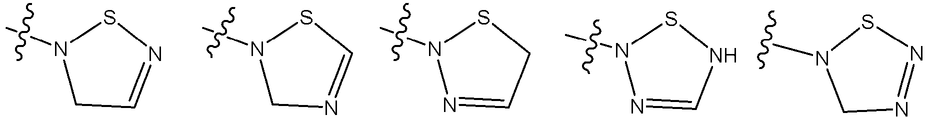

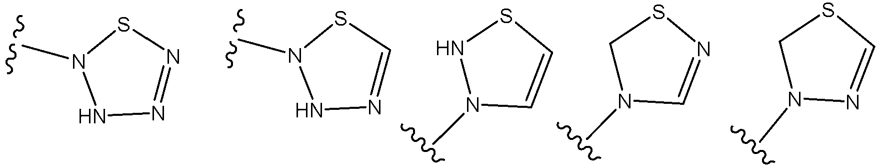

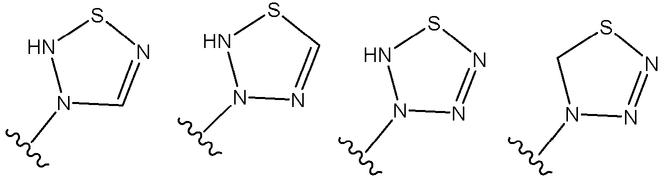

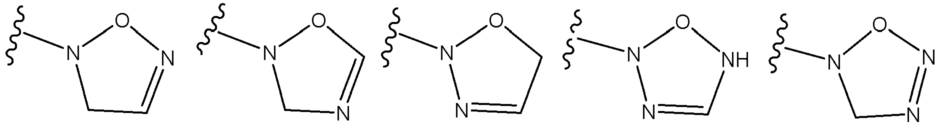

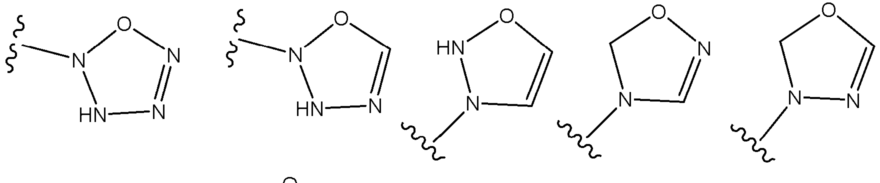

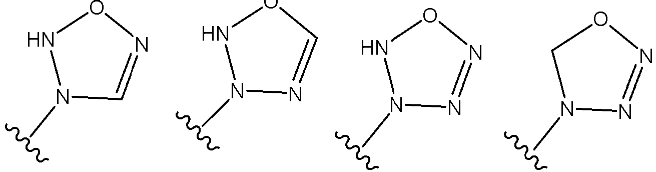

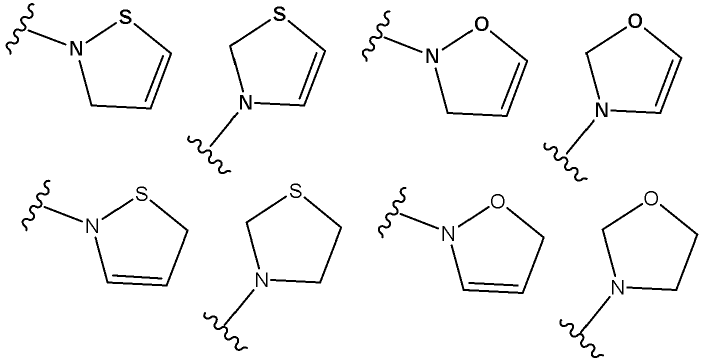

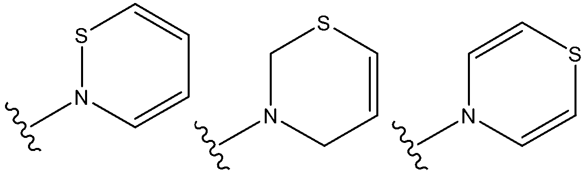

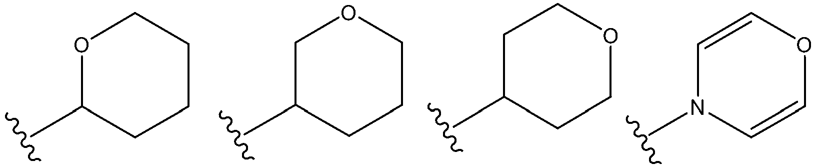

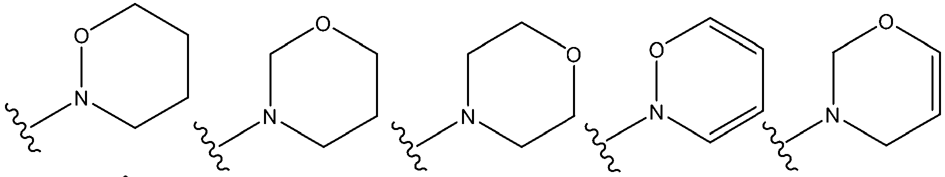

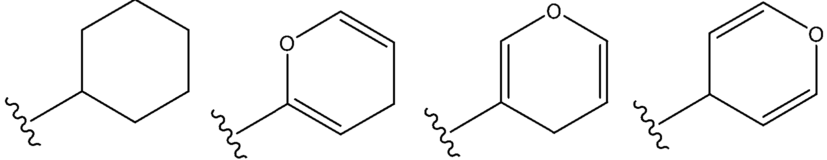

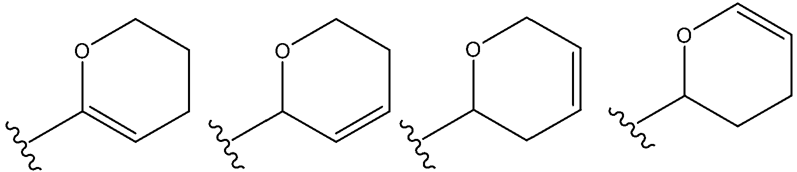

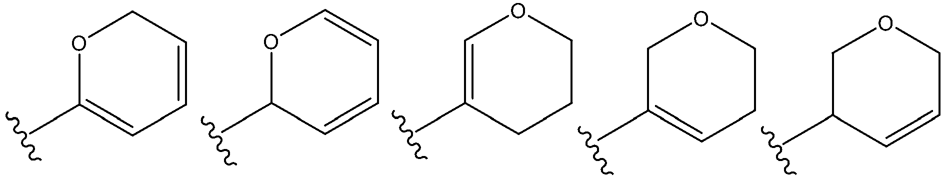

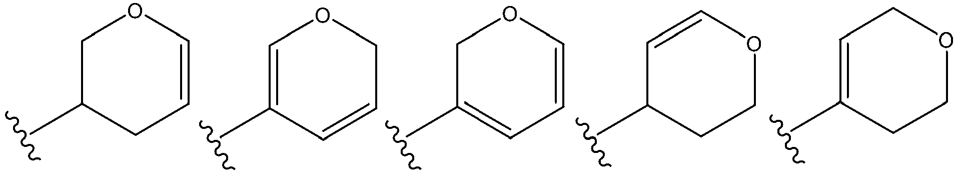

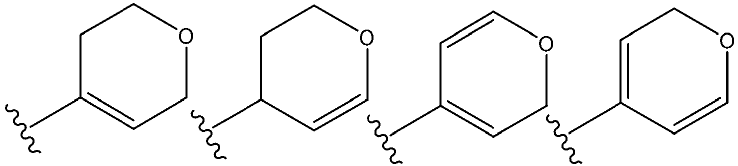

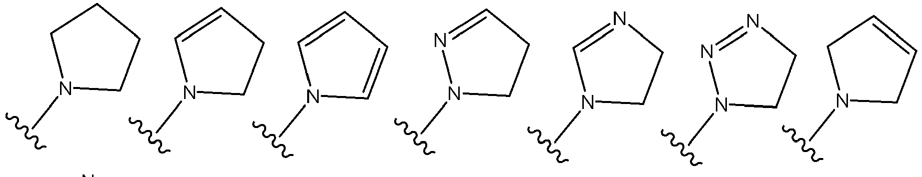

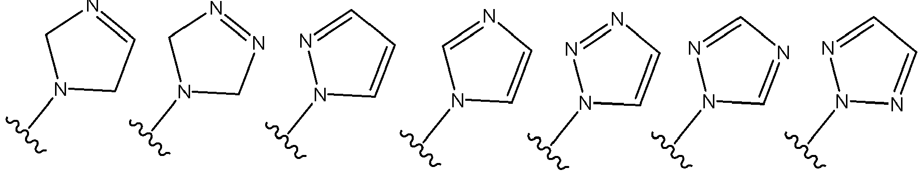

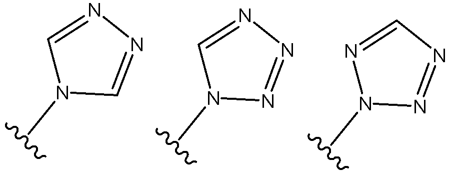

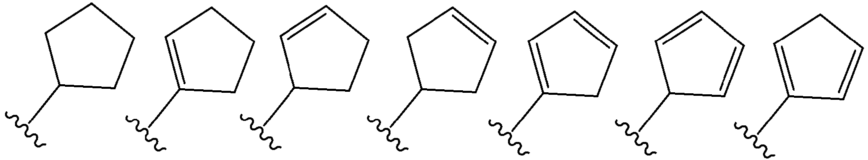

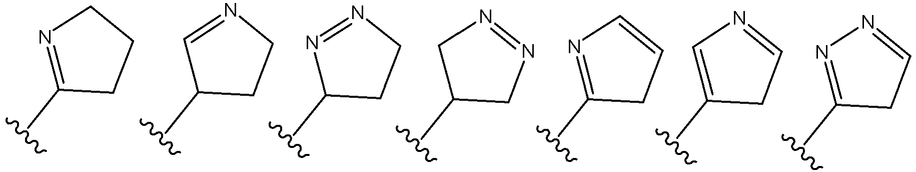

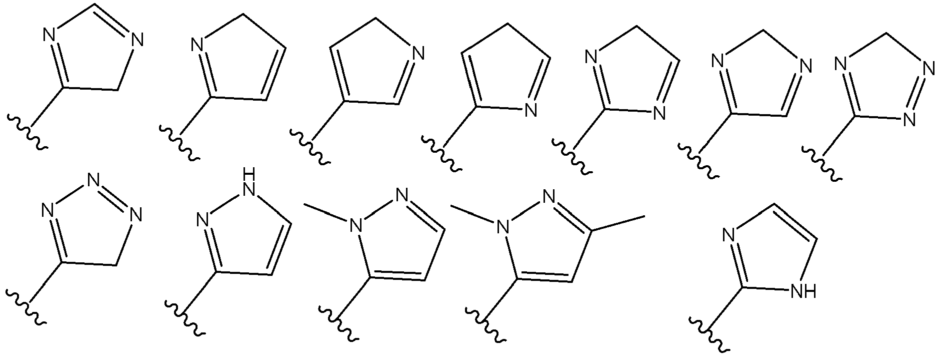

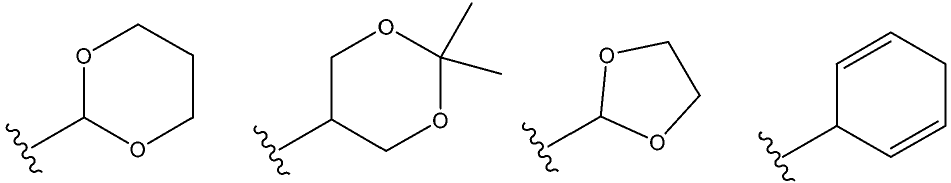

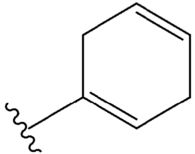

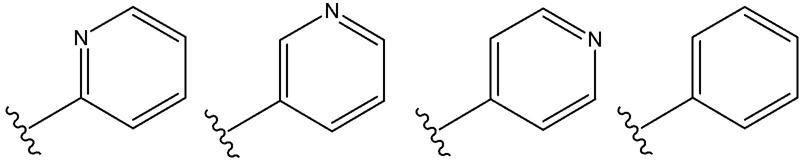

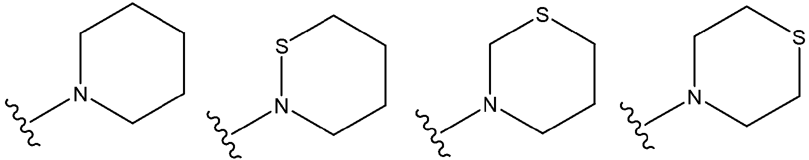

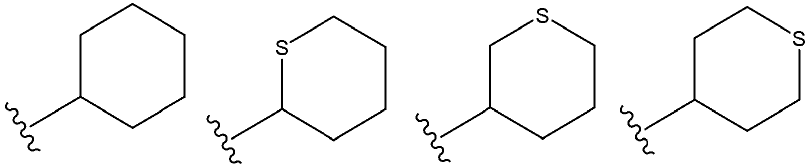

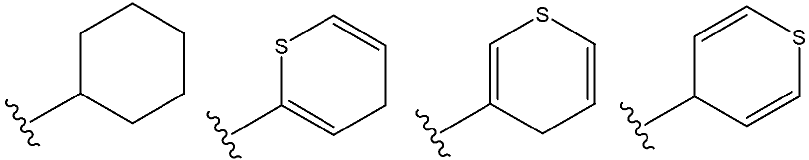

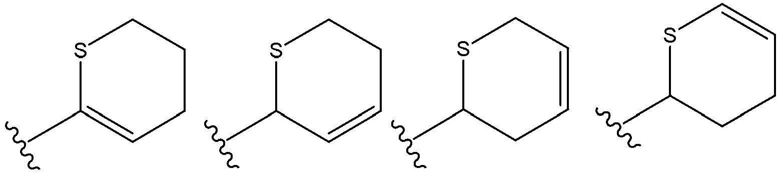

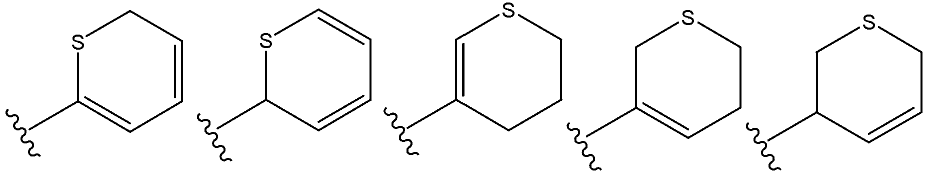

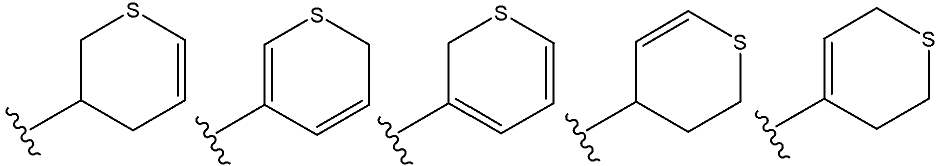

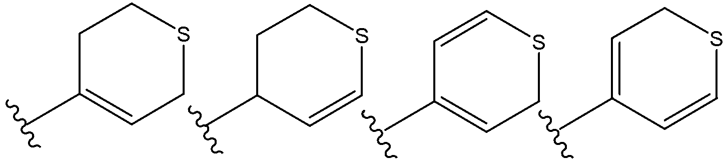

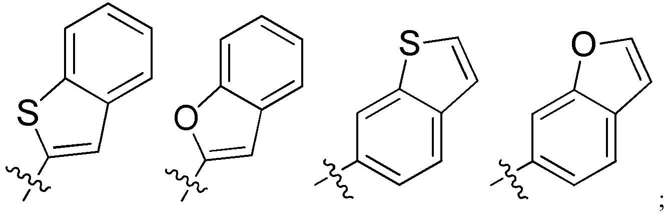

The substituent of ring A is selected from the group consisting of deuterium, halogen, amino, nitro, cyano, carboxyl, hydroxyl, mercapto, C₁-C₆ alkyl, and C₁-C₆ alkoxy;
preferably, the substituent of ring A is selected from the group consisting of deuterium, halogen, amino, nitro, cyano, carboxyl, hydroxyl, mercapto, C₁-C₄ alkyl, and C₁-C₄ alkoxy;

Further, the compound is selected from the group consisting of following compounds:

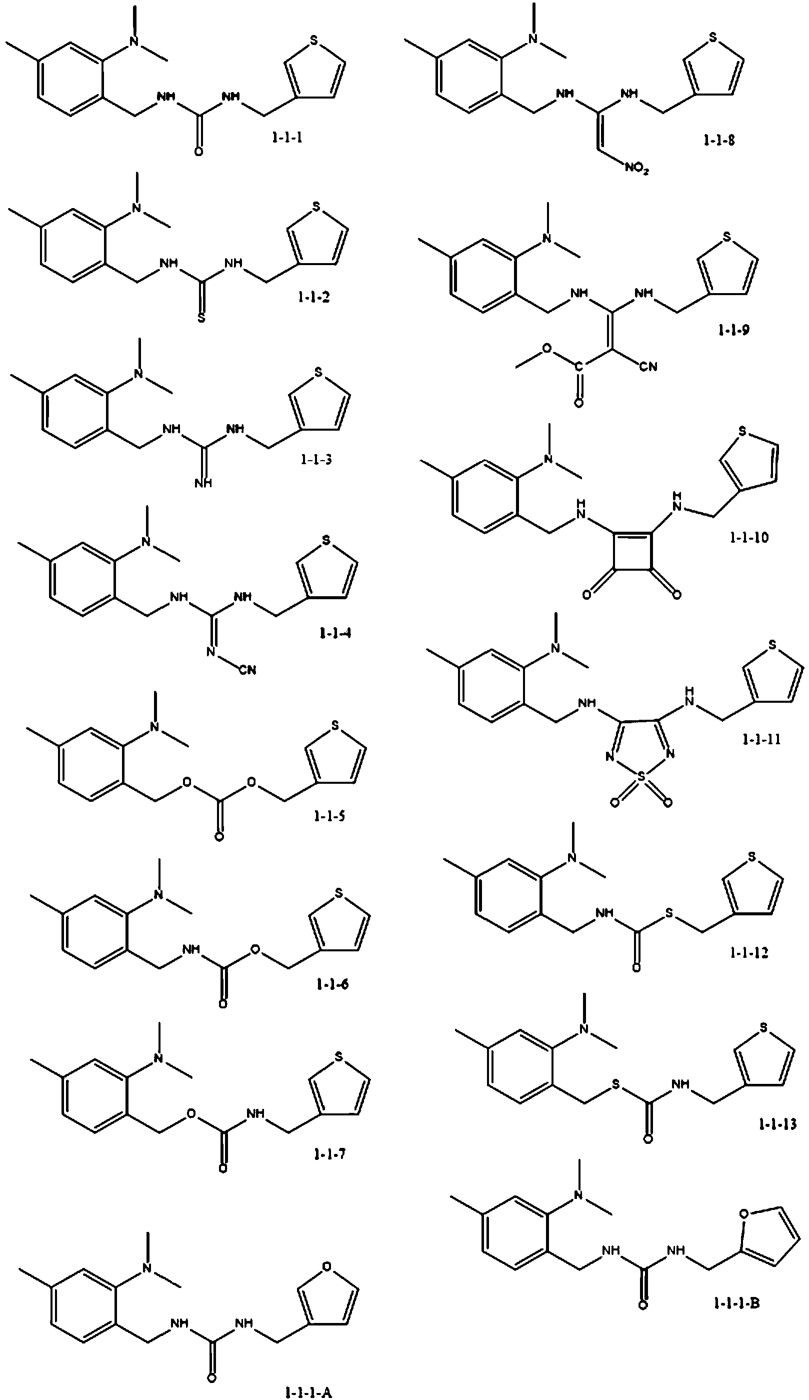

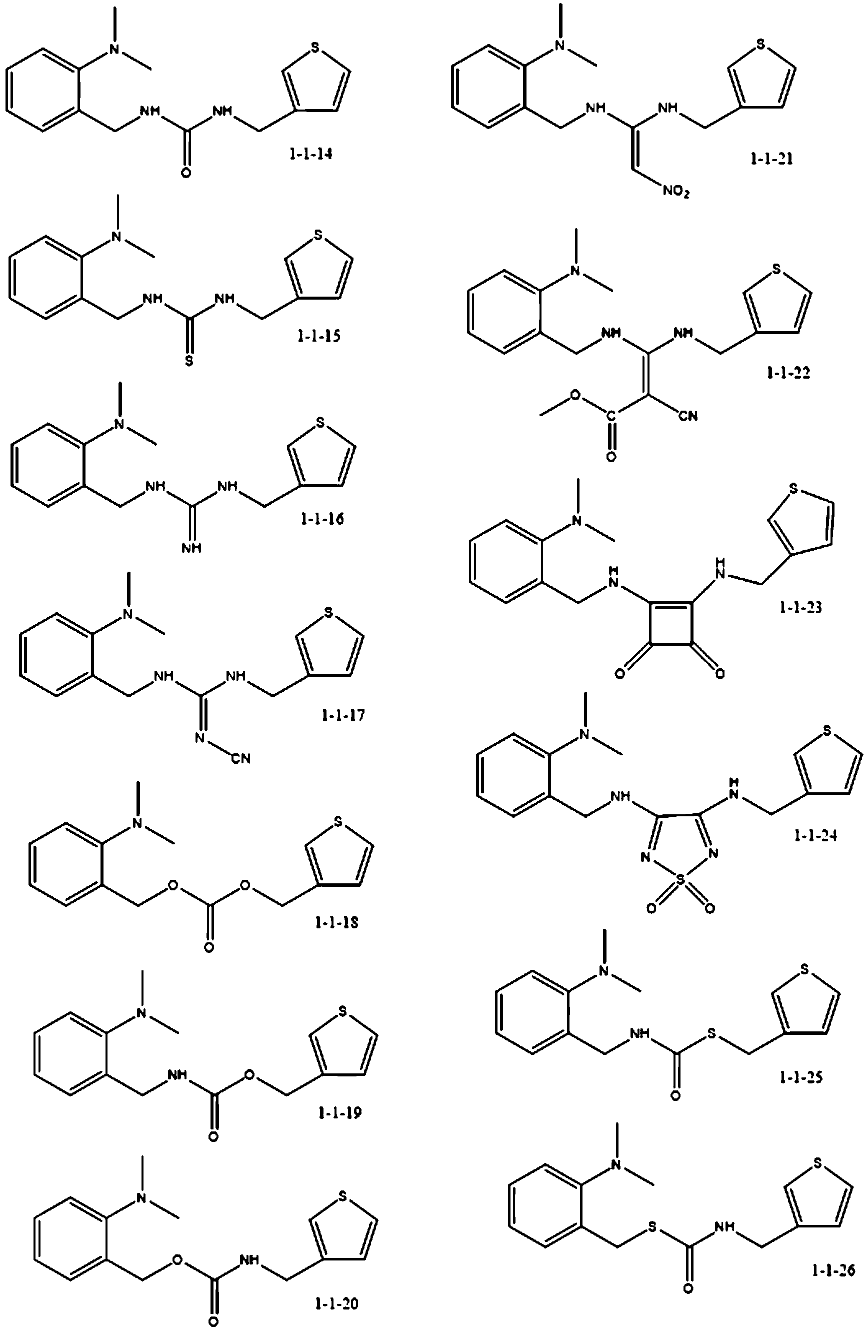

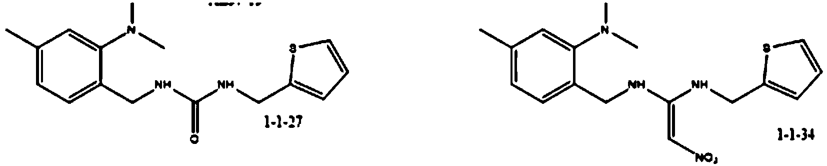

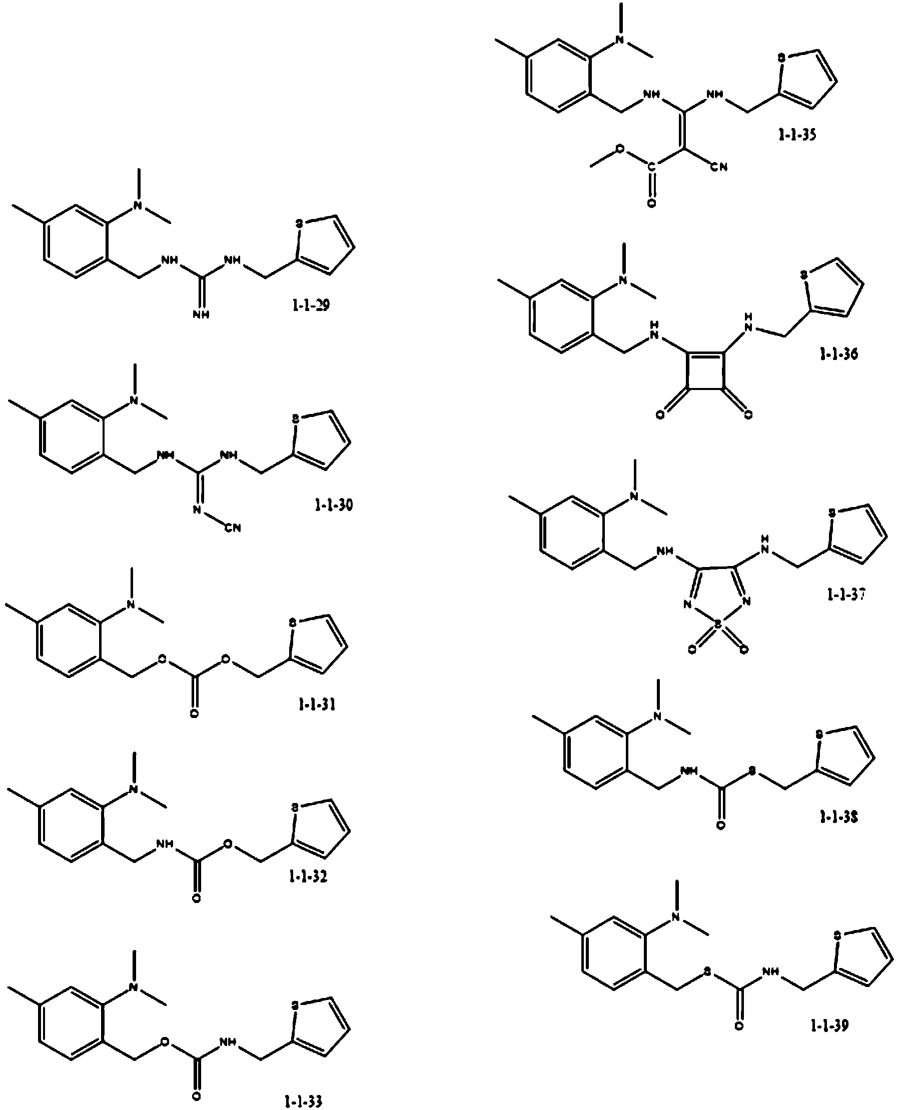

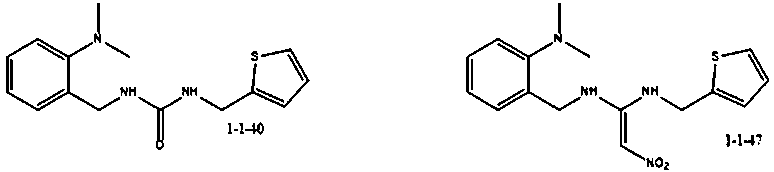

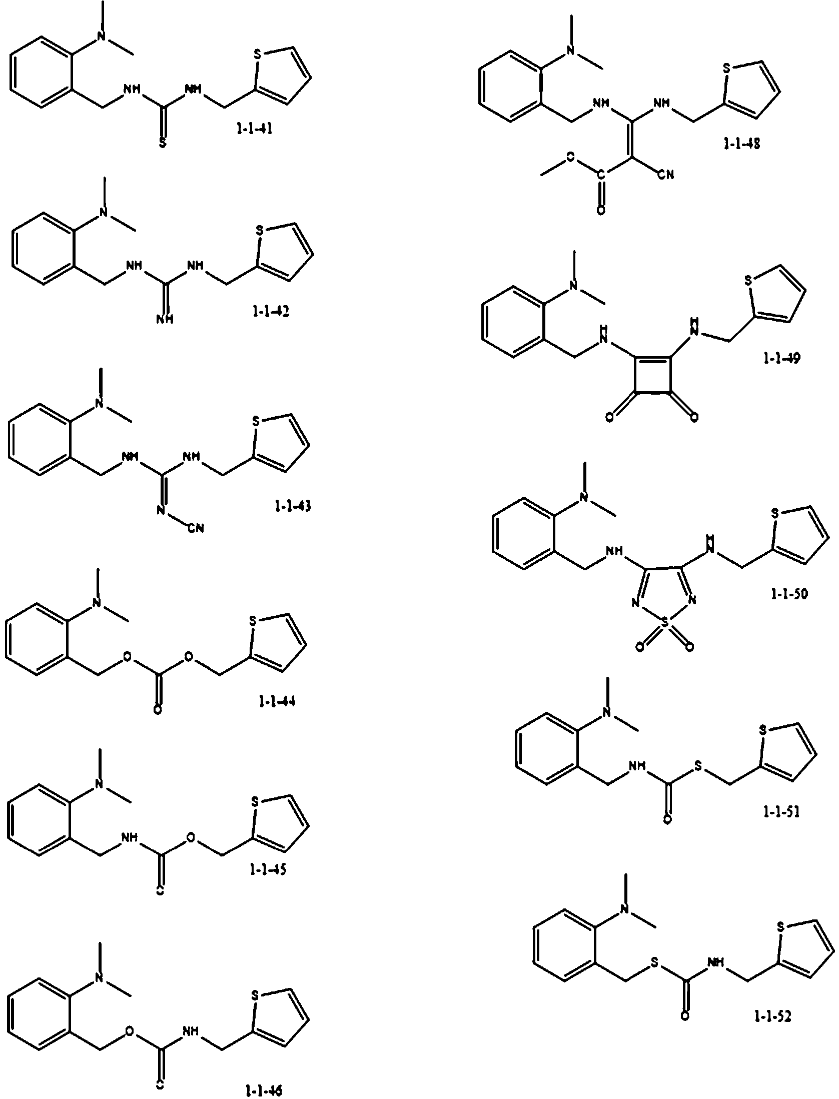

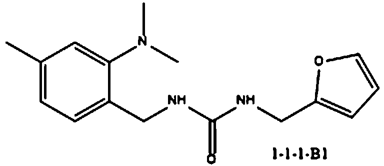

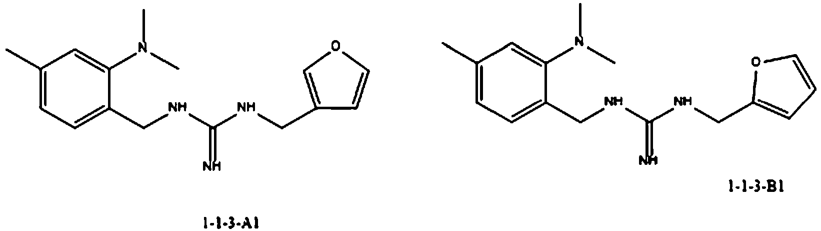

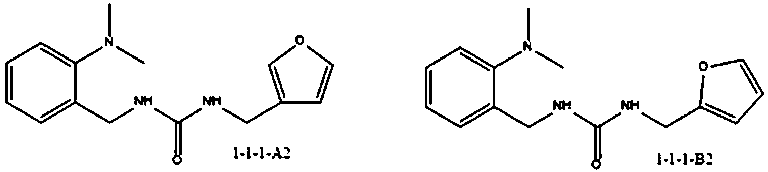

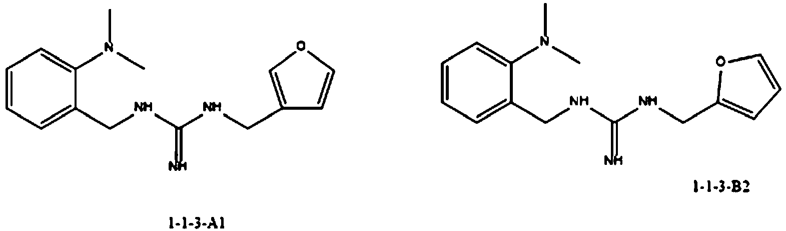

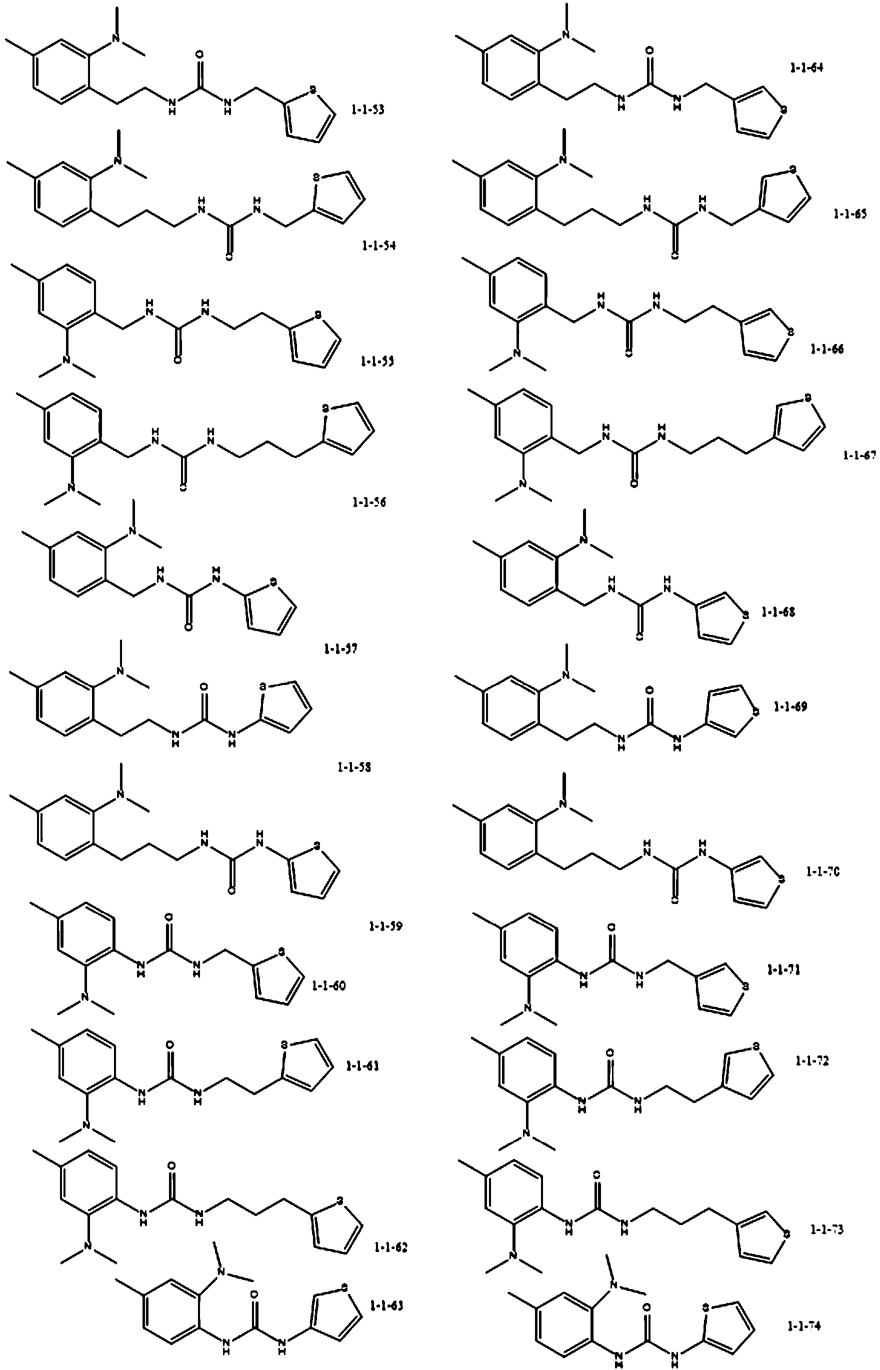

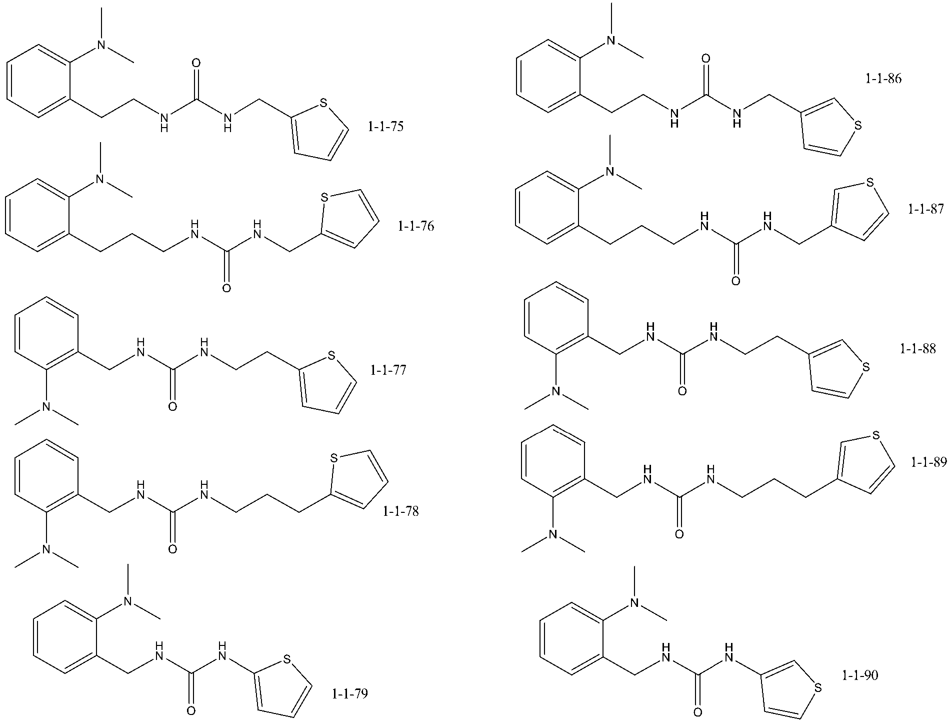

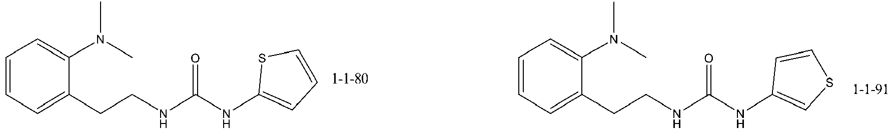

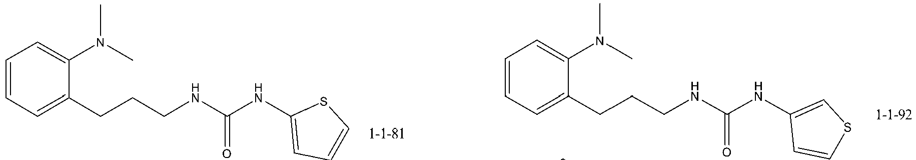

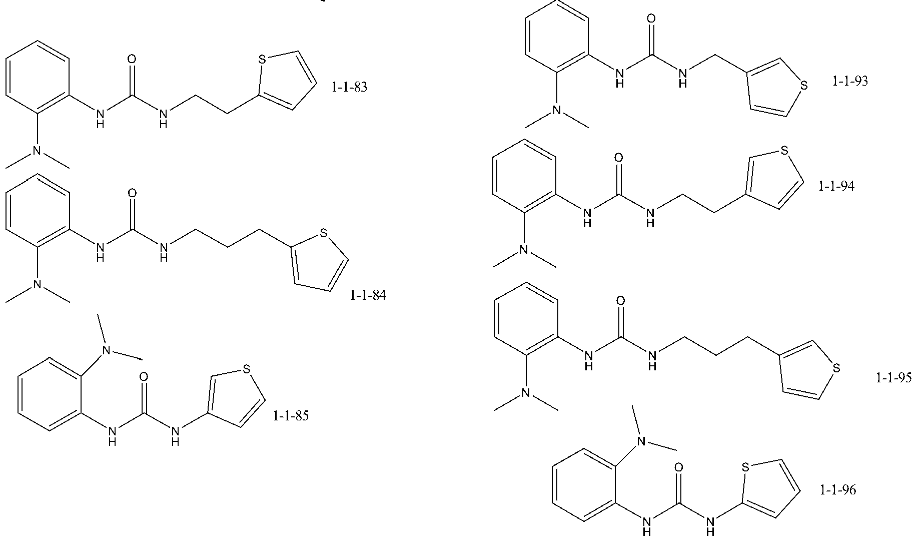

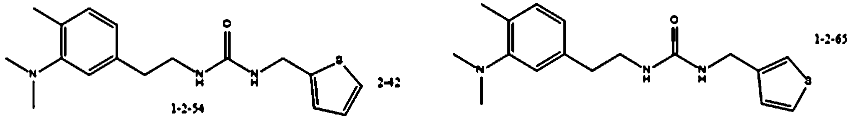

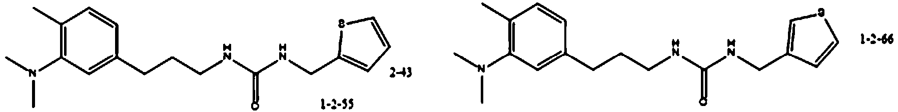

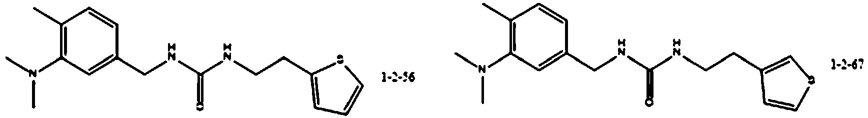

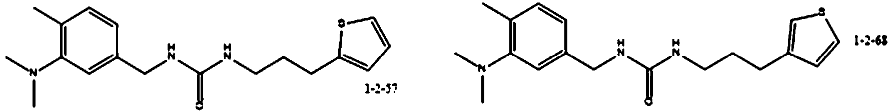

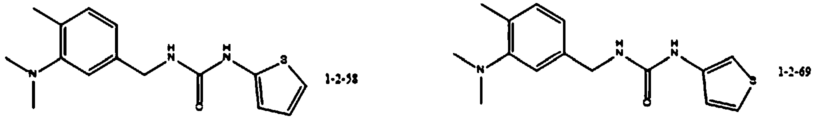

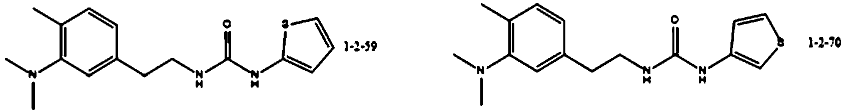

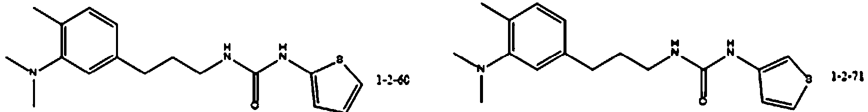

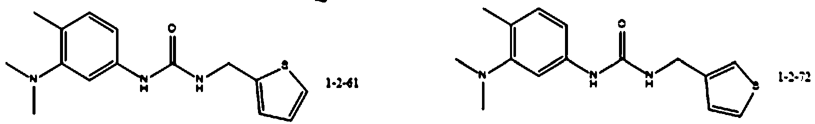

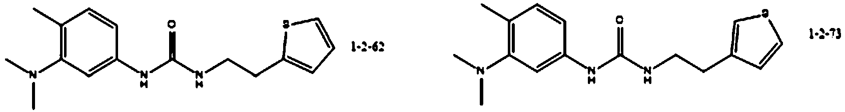

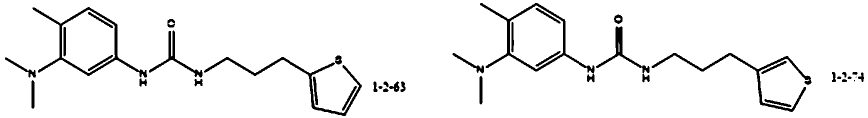

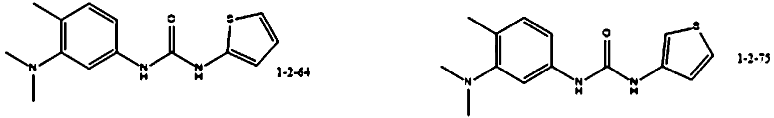

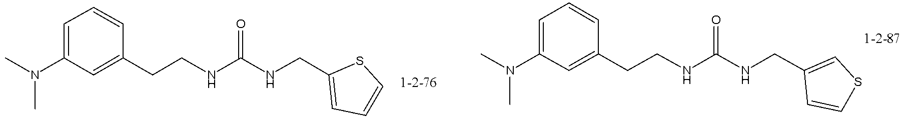

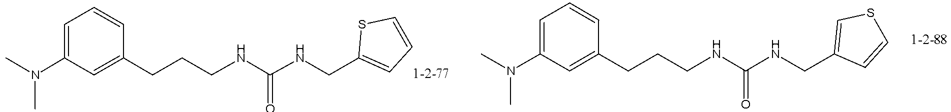

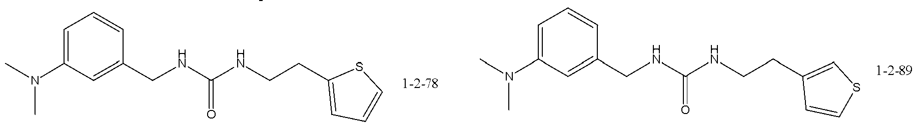

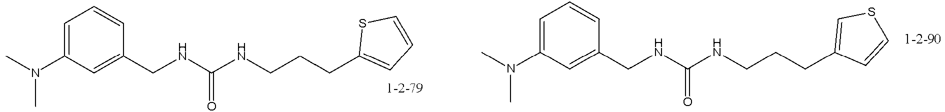

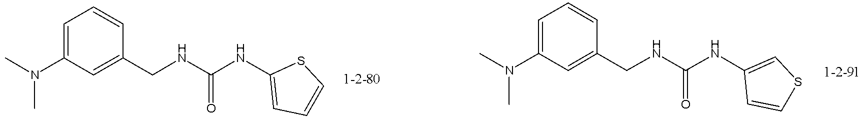

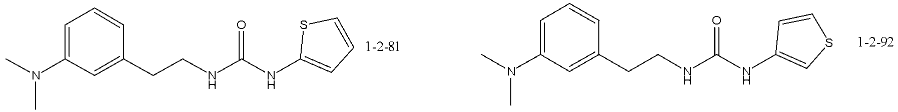

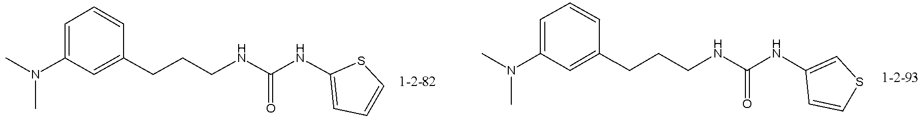

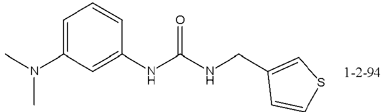

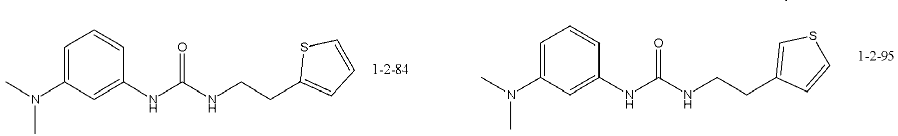

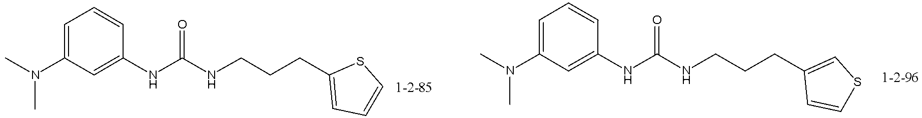

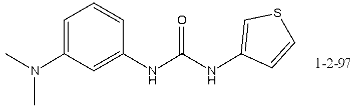

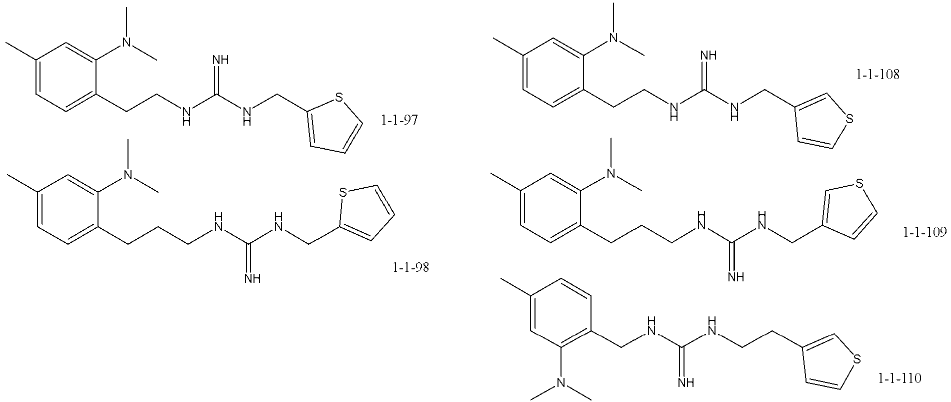

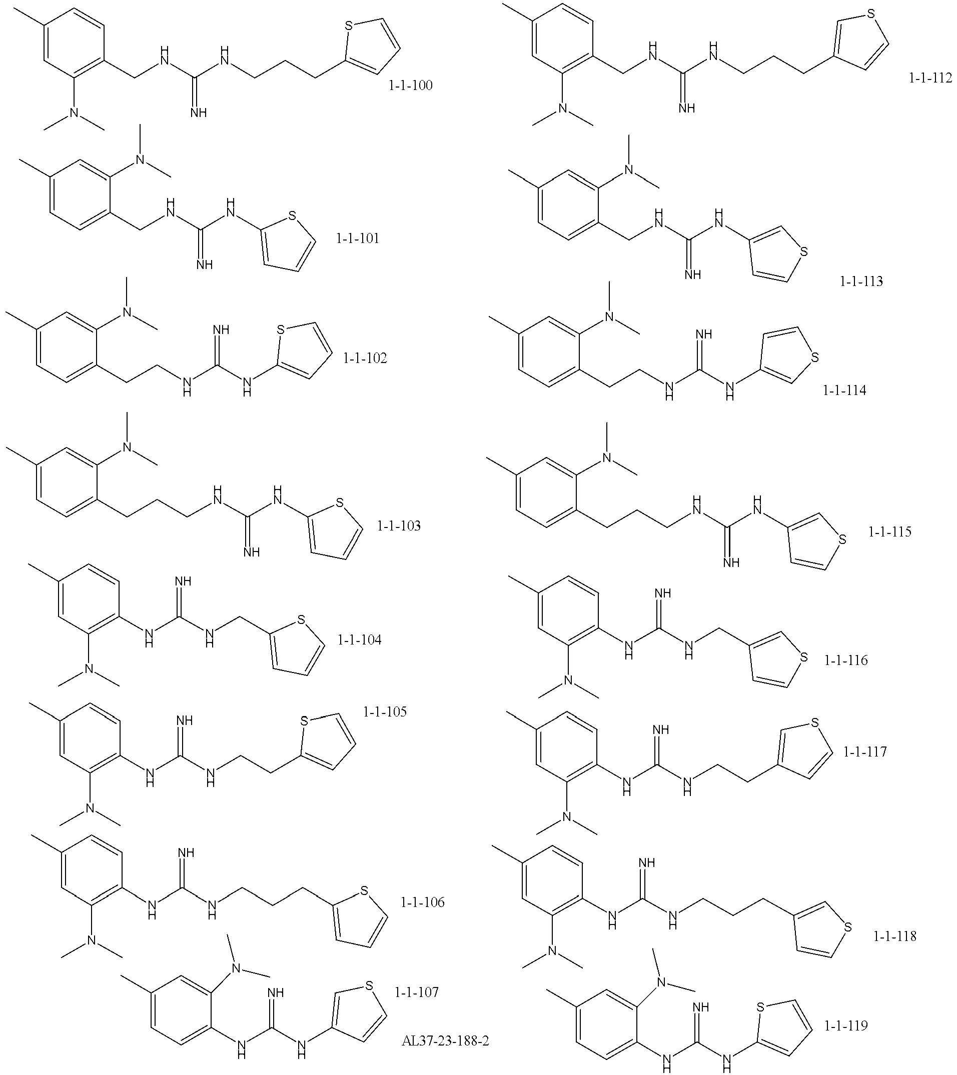

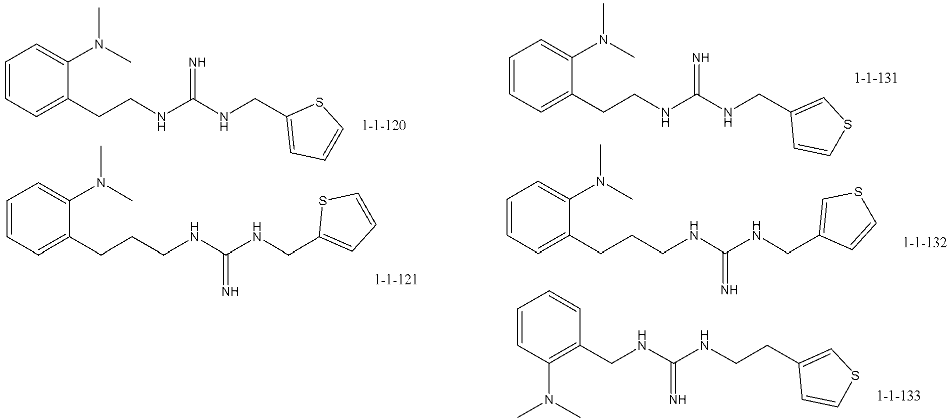

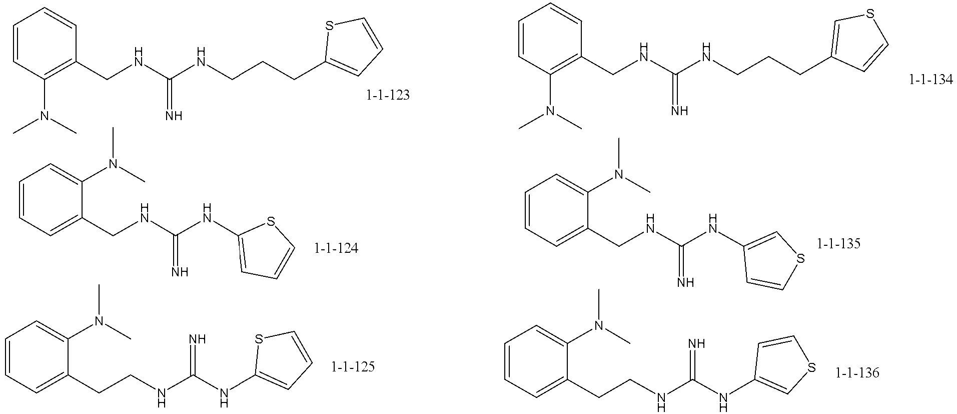

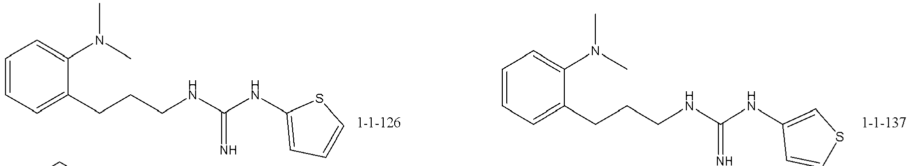

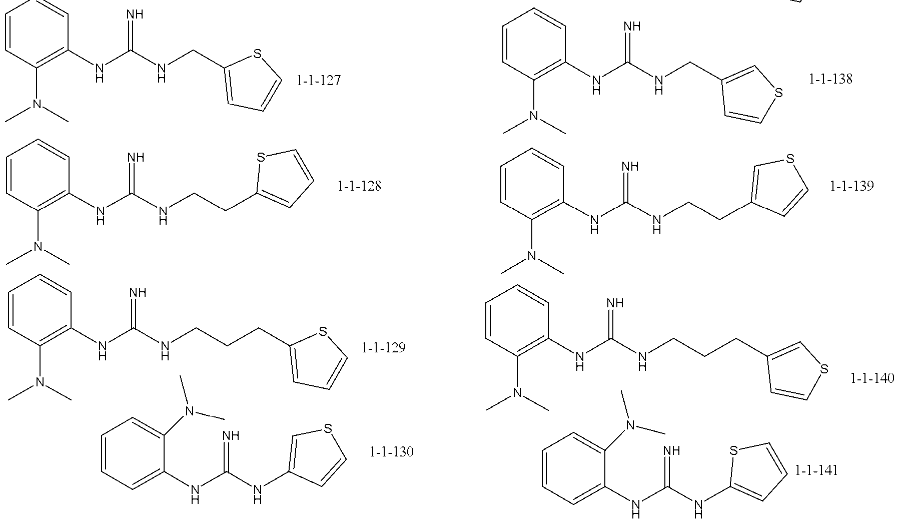

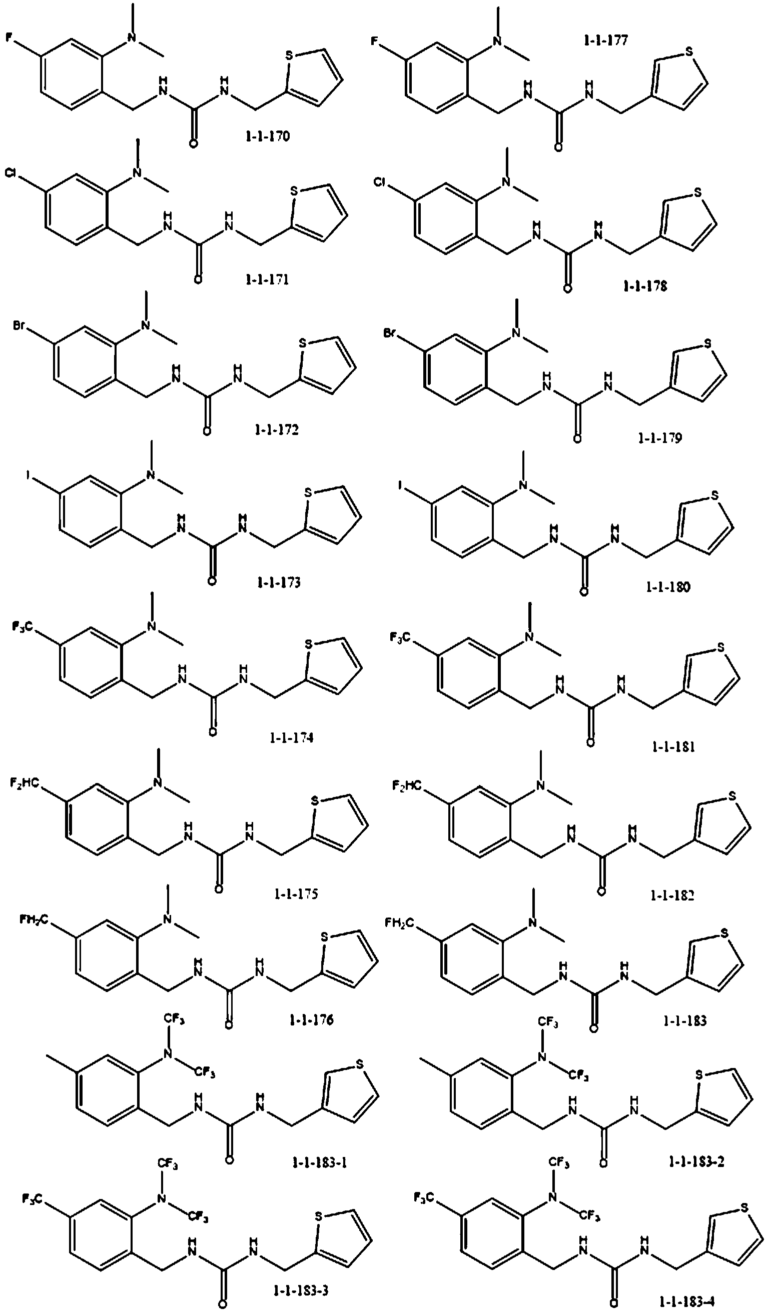

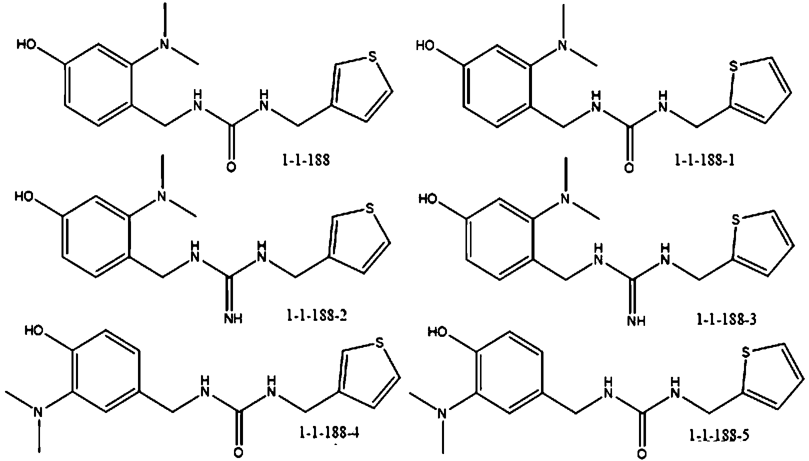

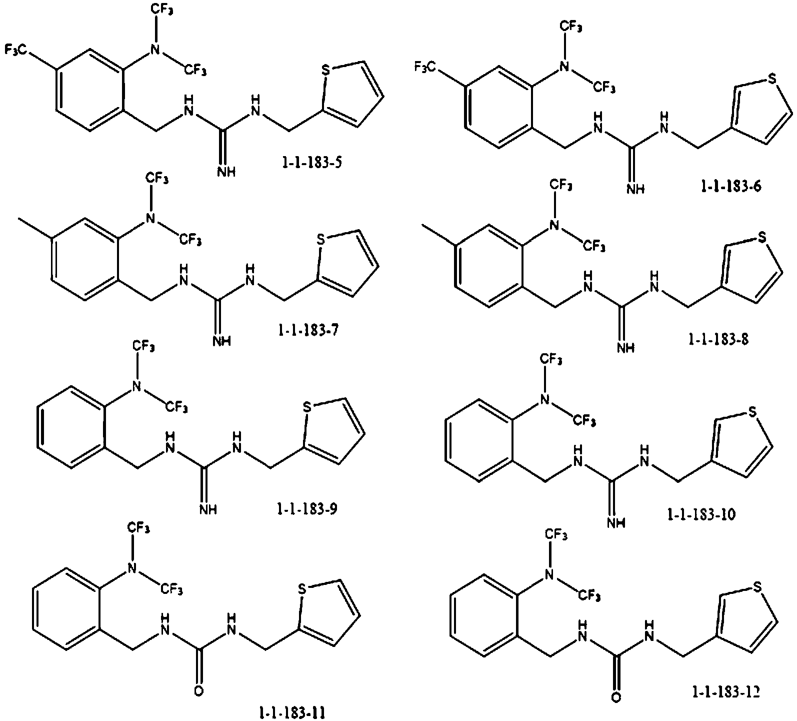

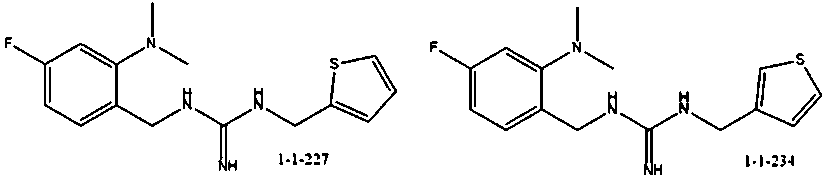

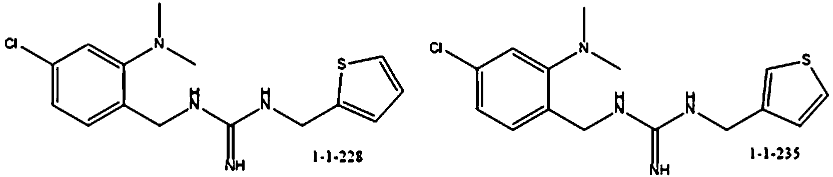

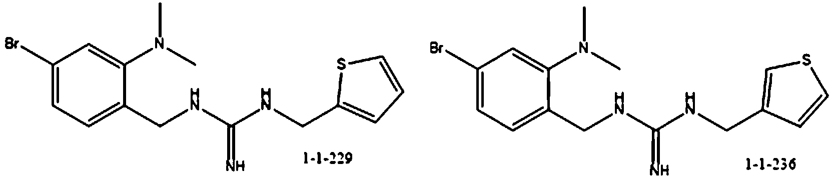

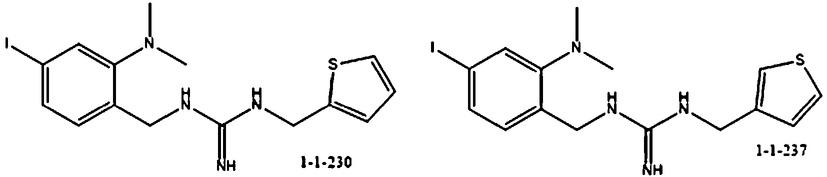

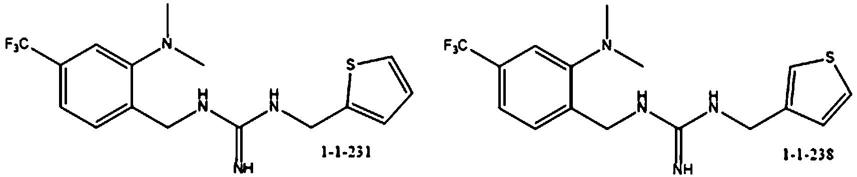

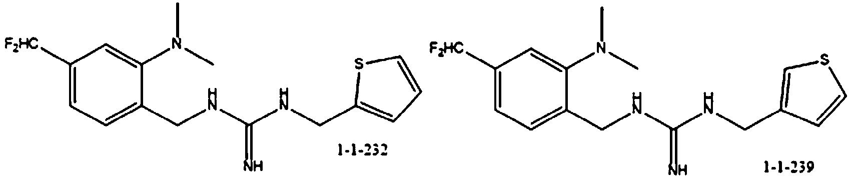

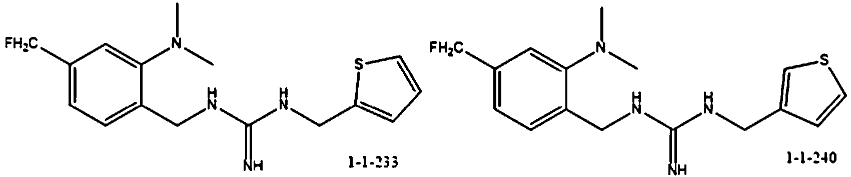

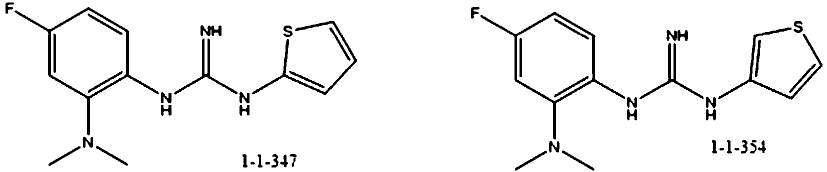

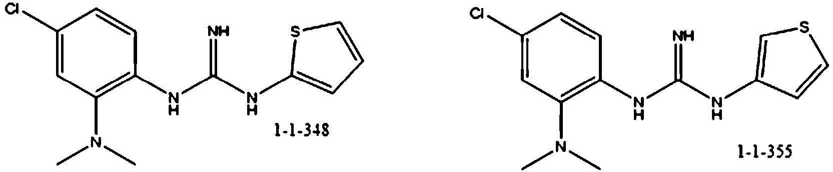

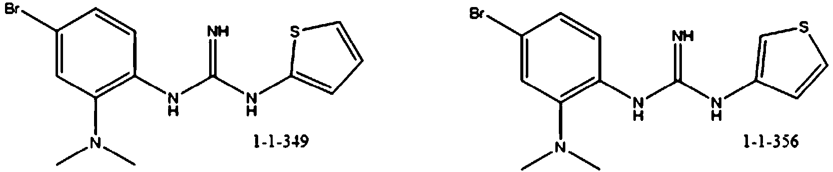

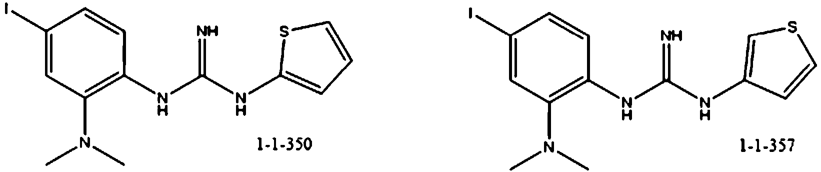

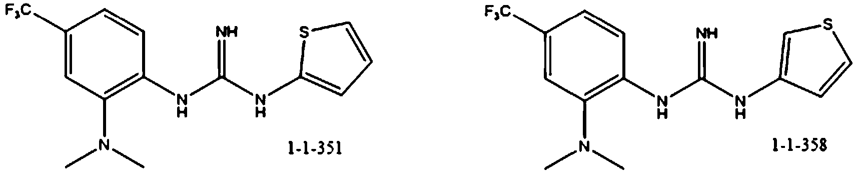

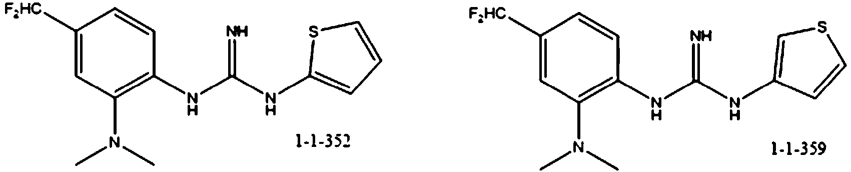

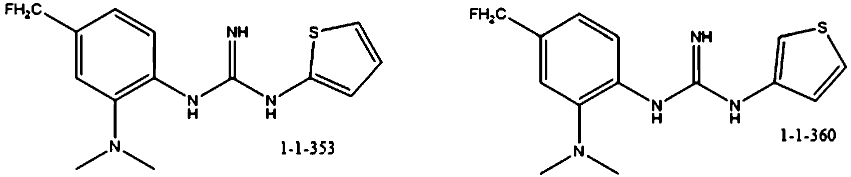

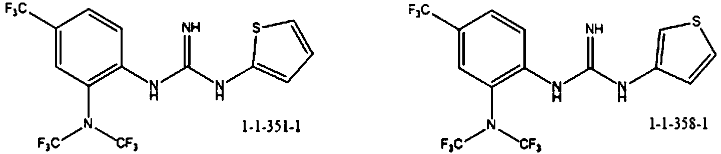

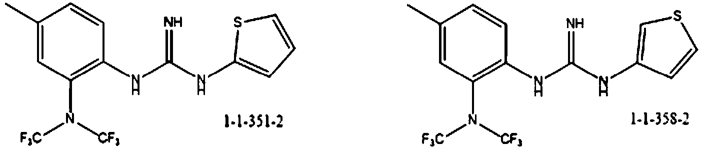

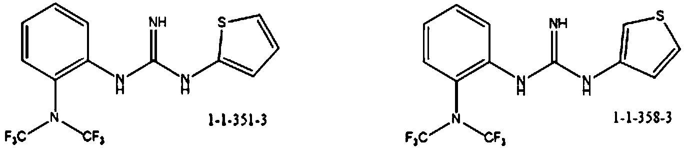

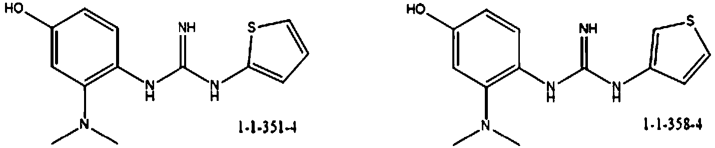

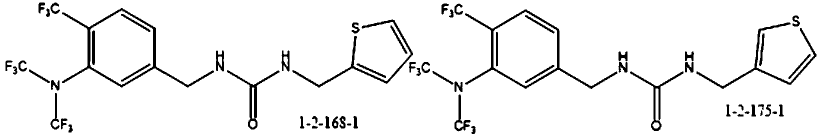

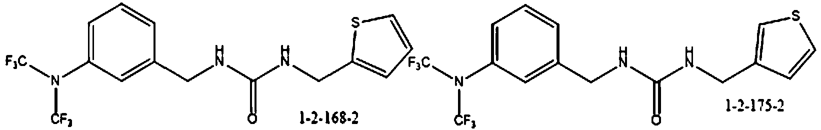

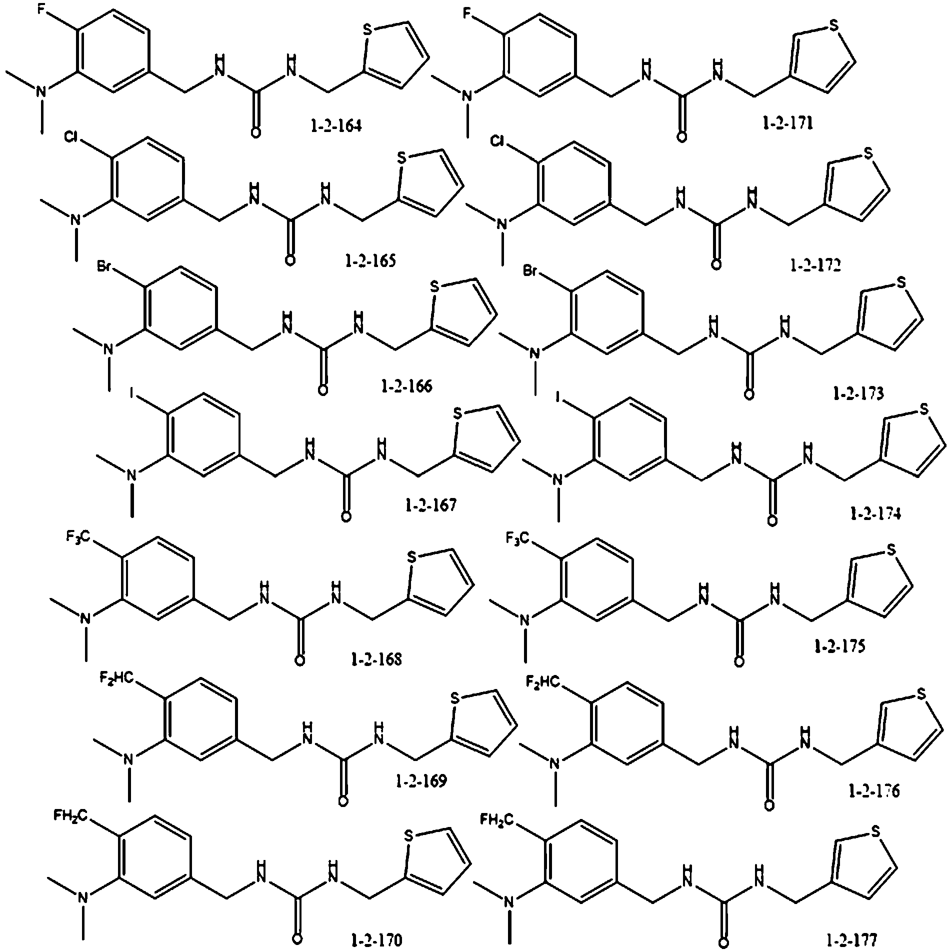

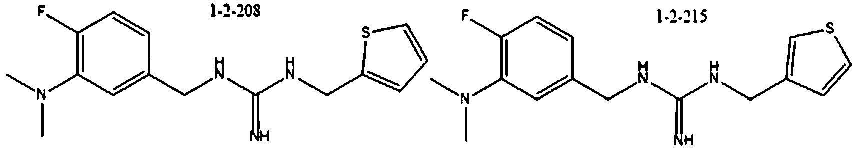

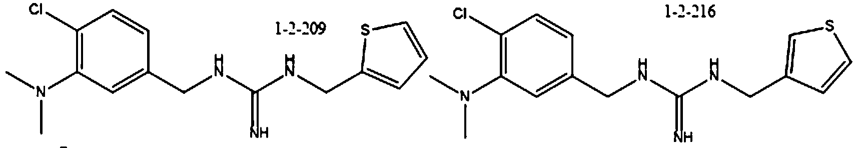

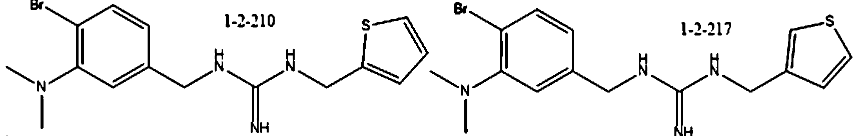

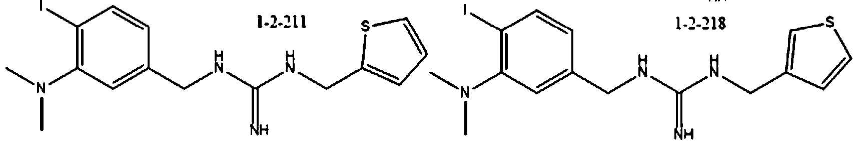

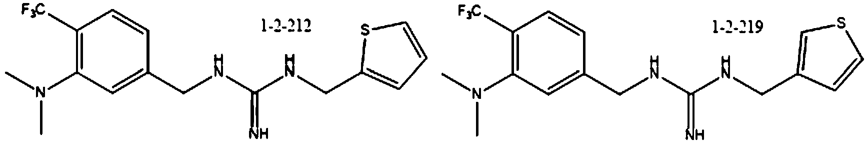

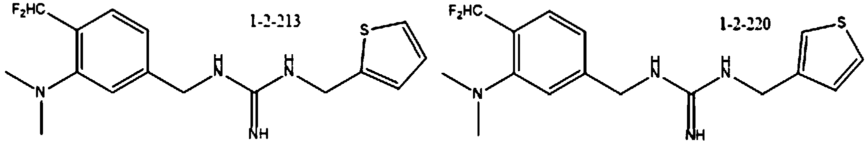

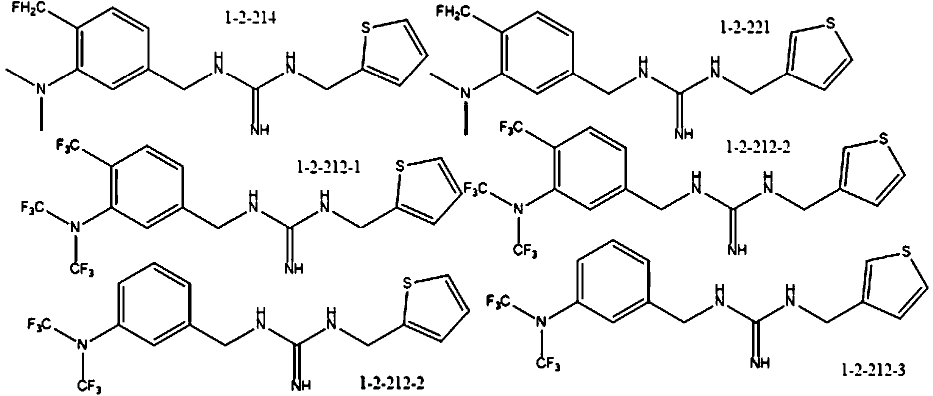

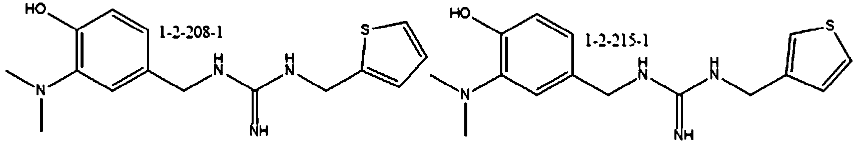

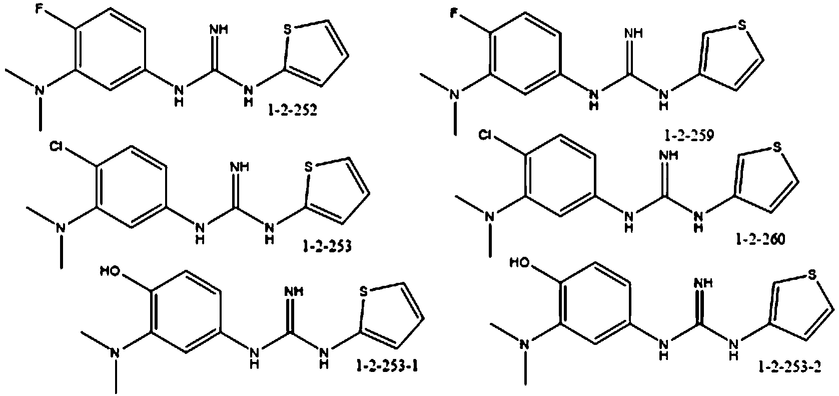

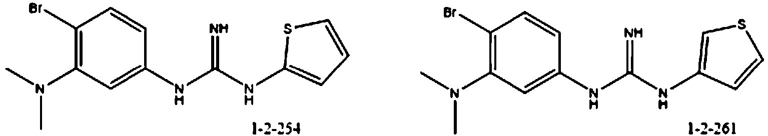

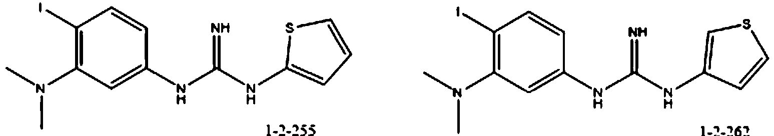

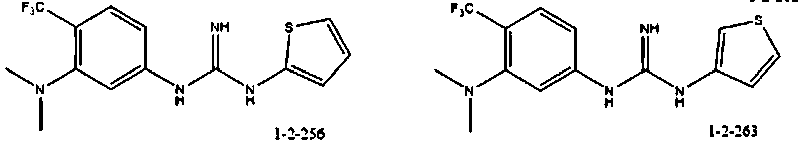

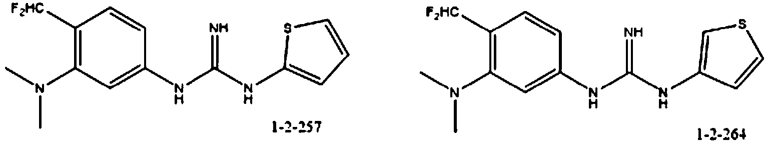

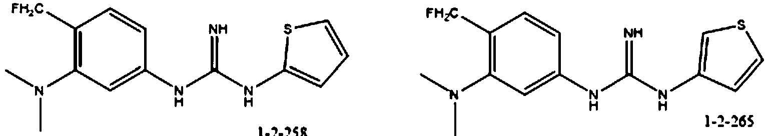

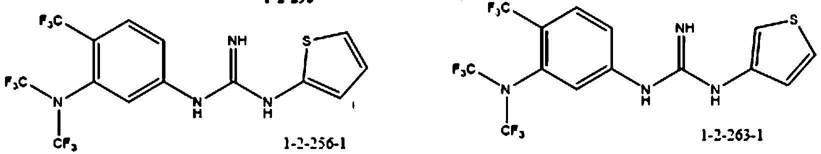

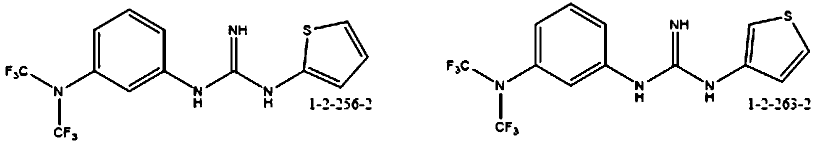

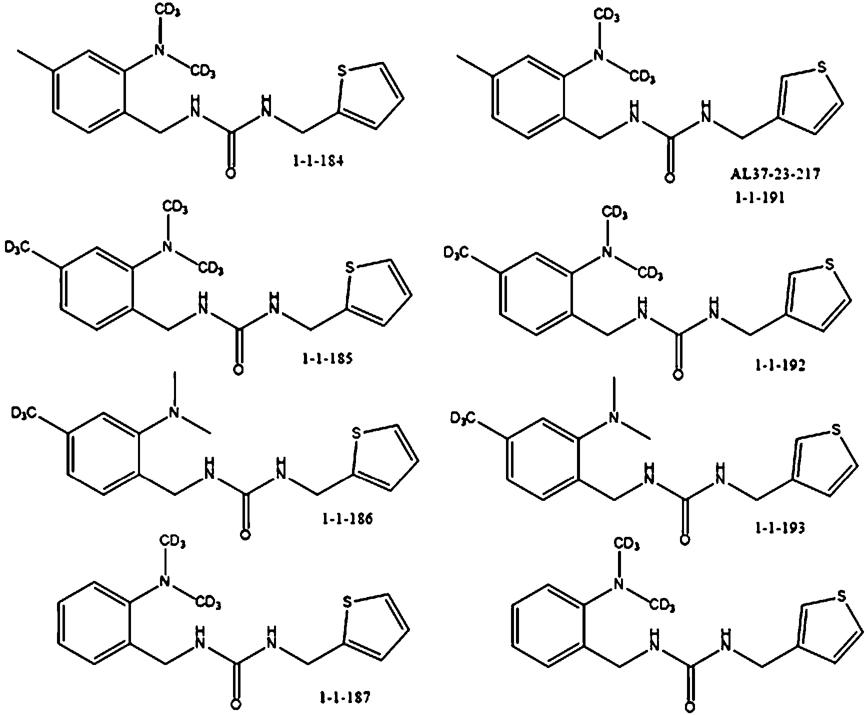

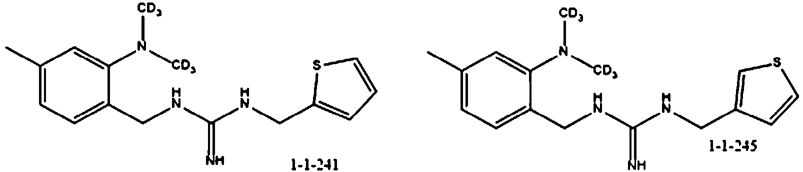

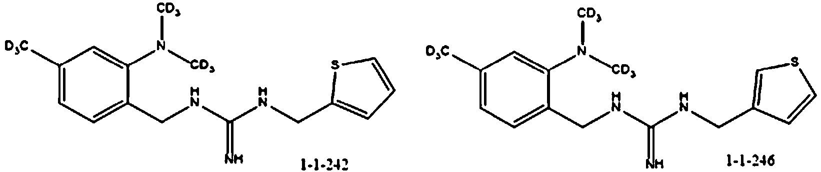

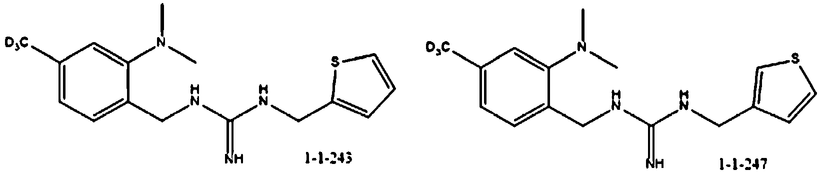

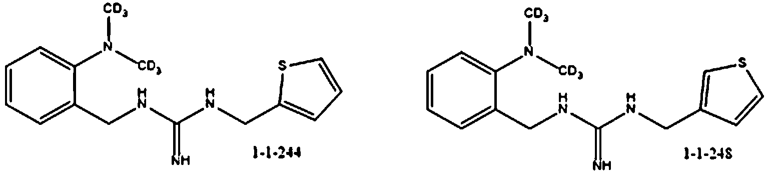

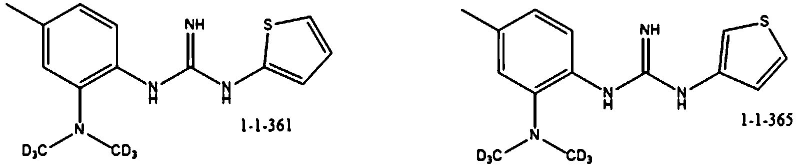

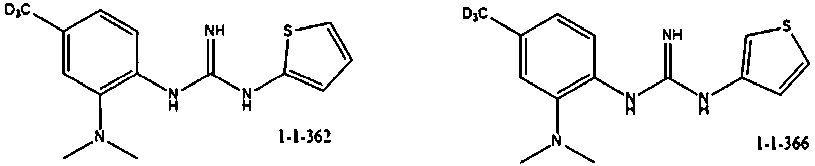

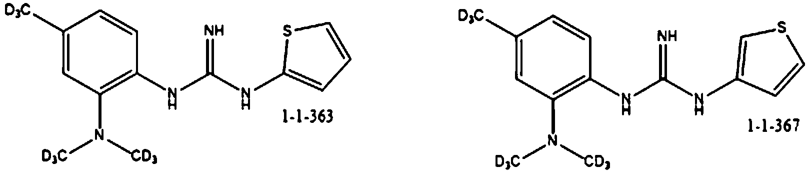

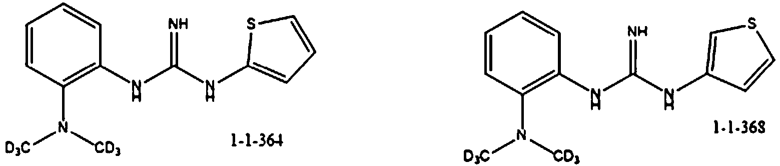

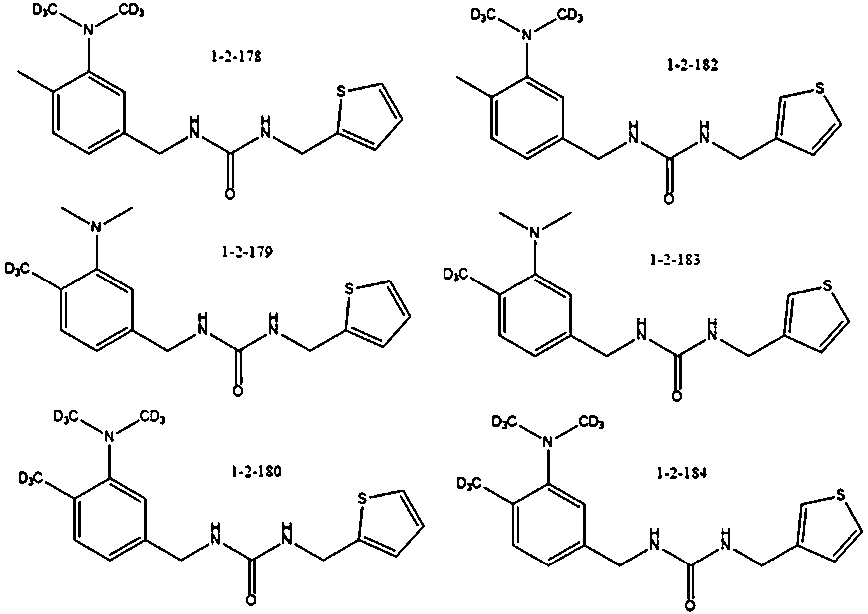

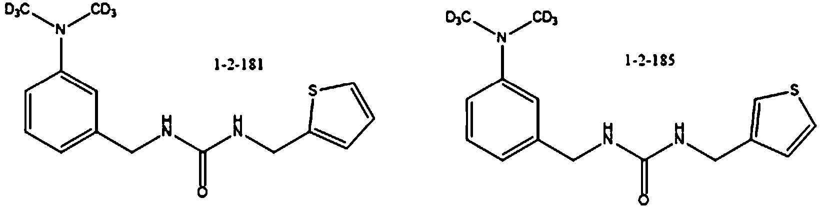

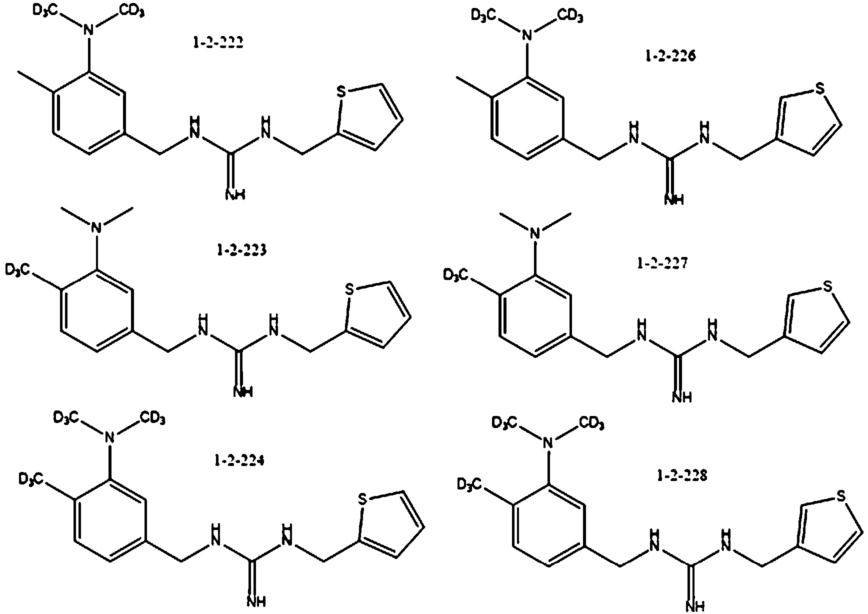

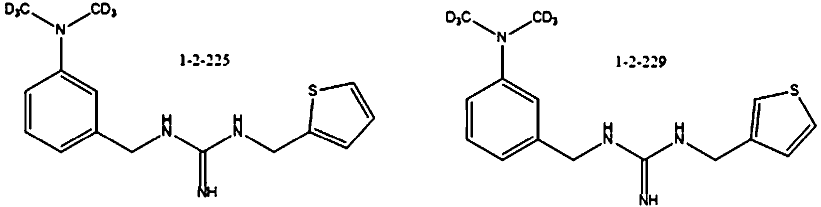

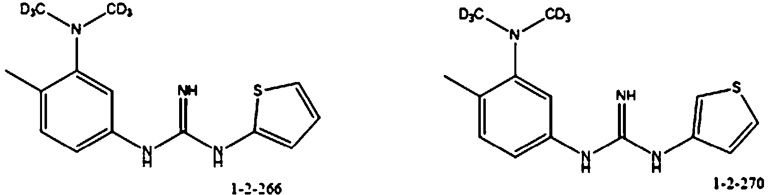

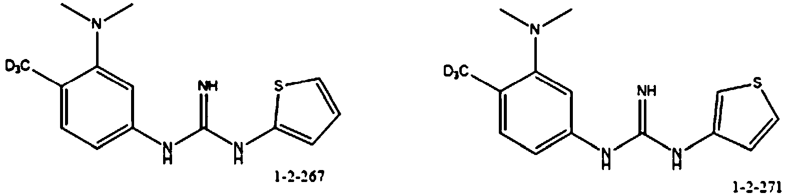

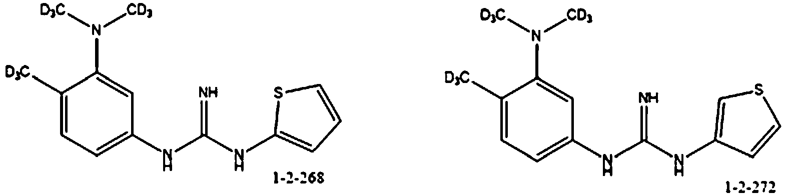

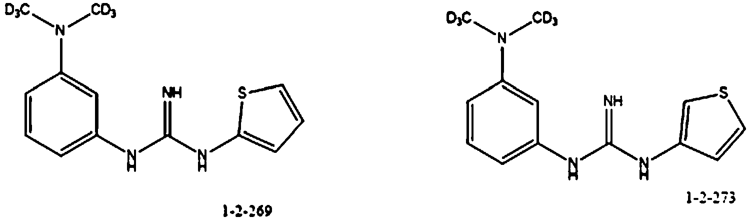

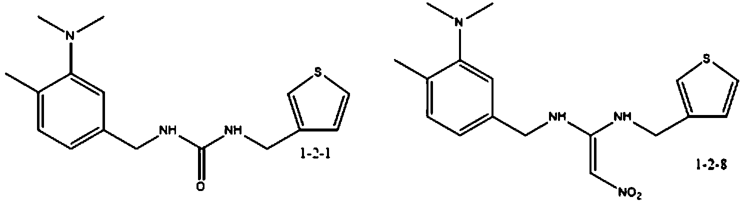

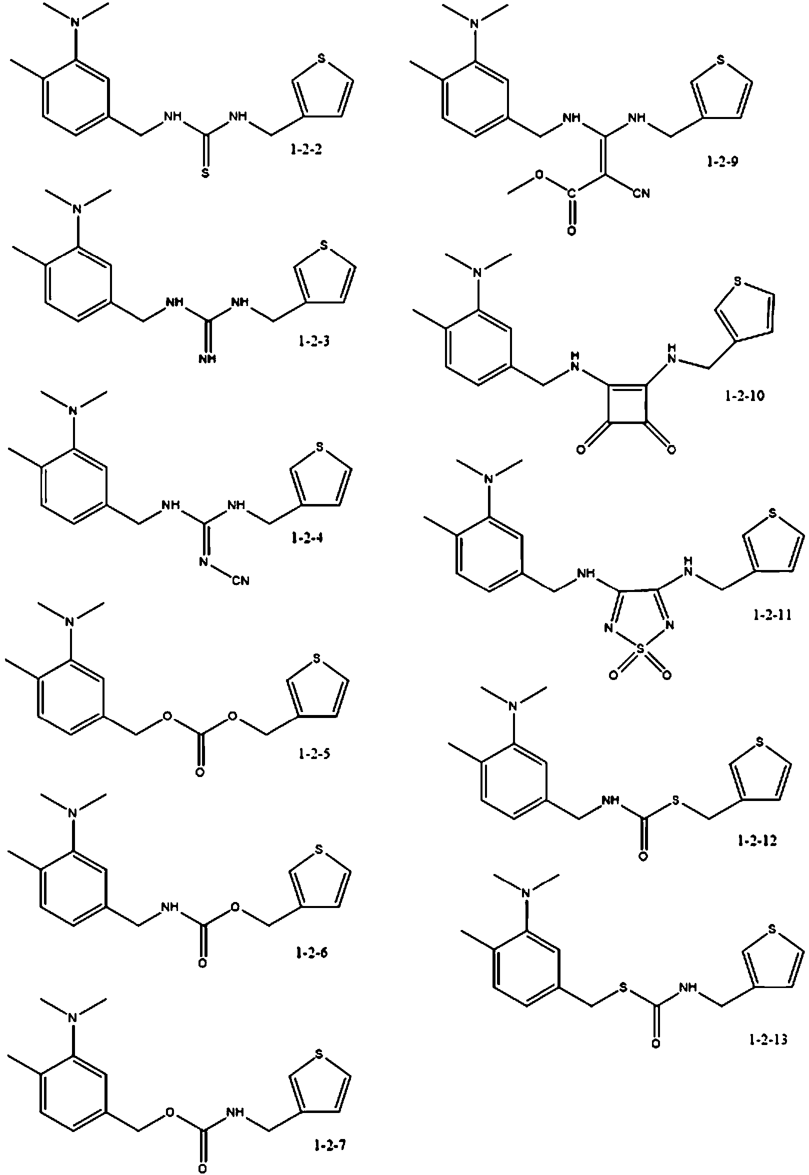

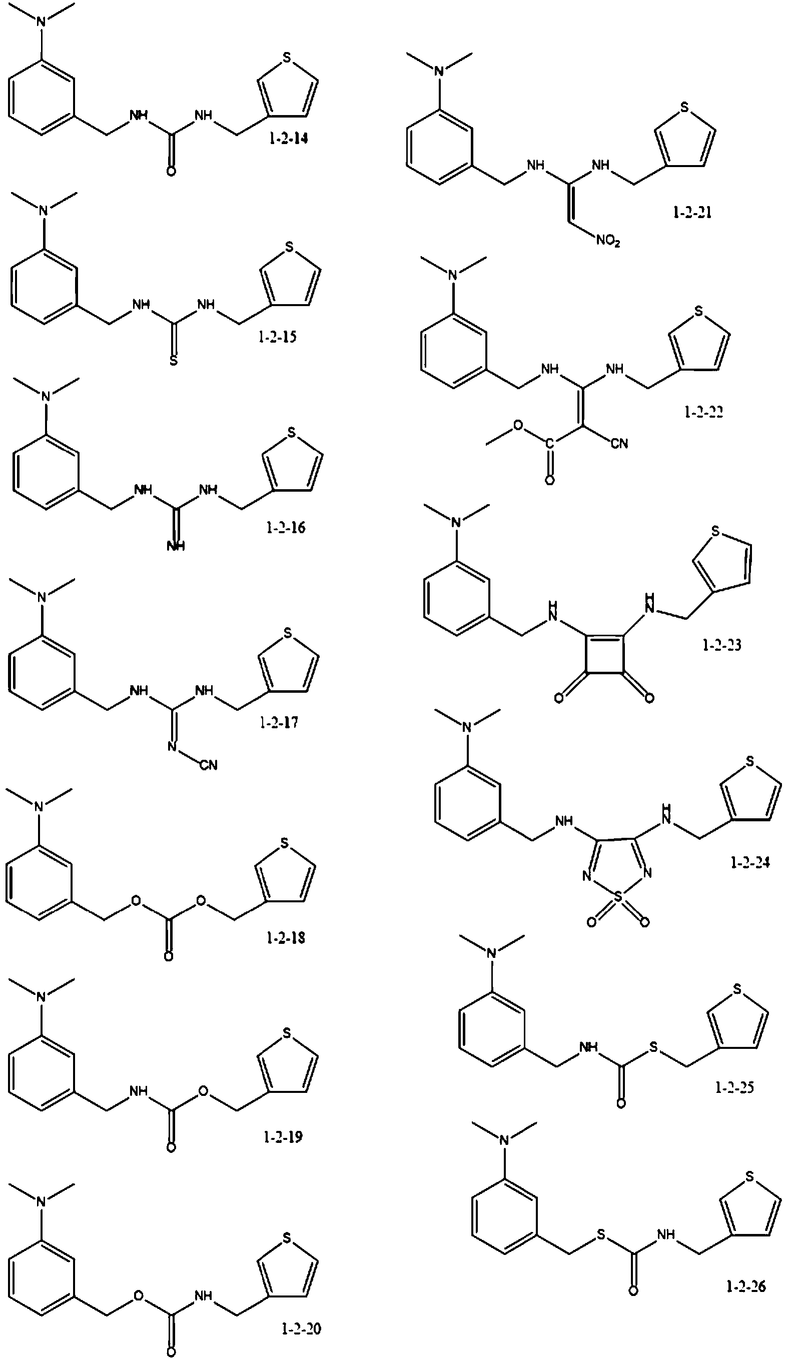

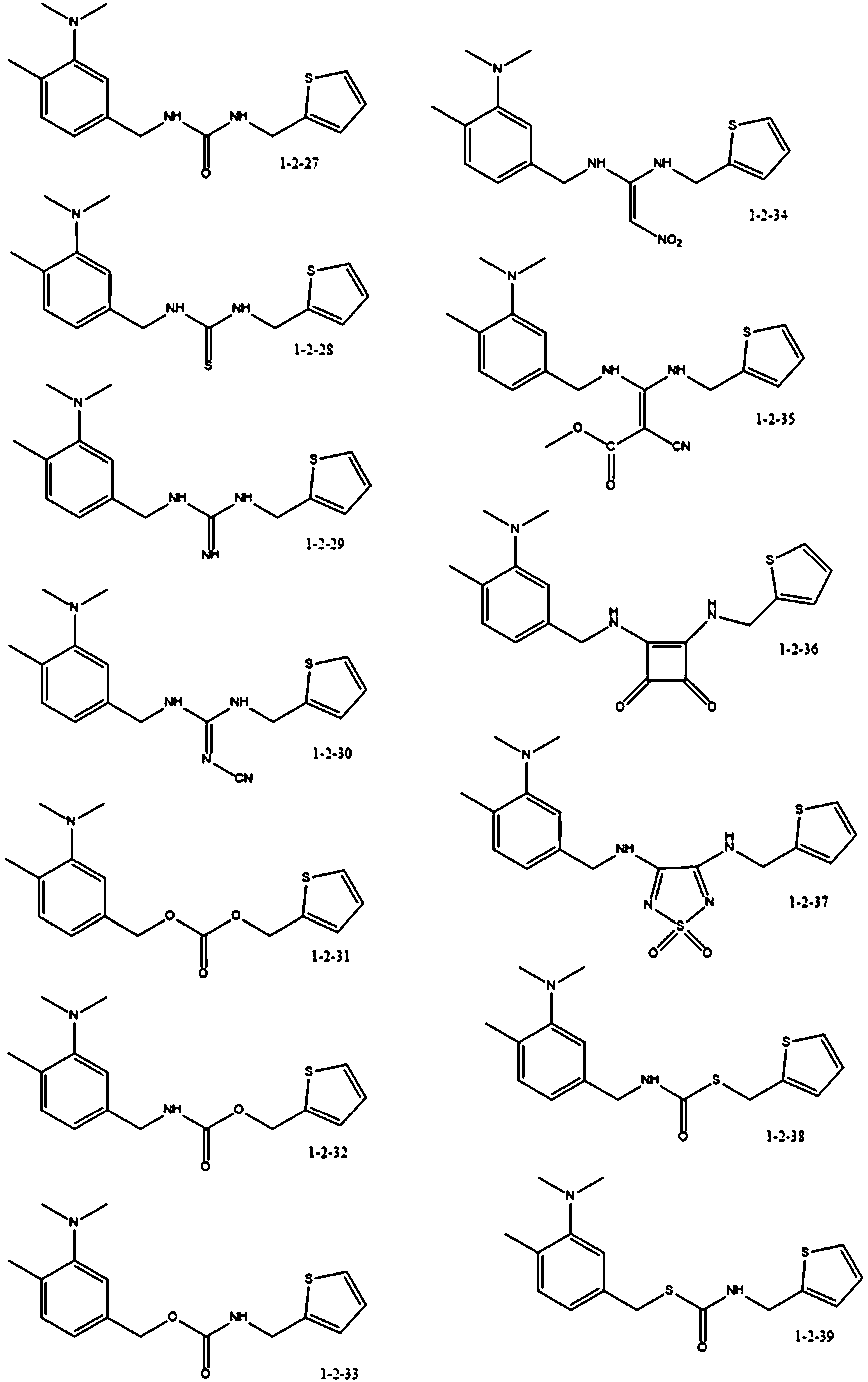

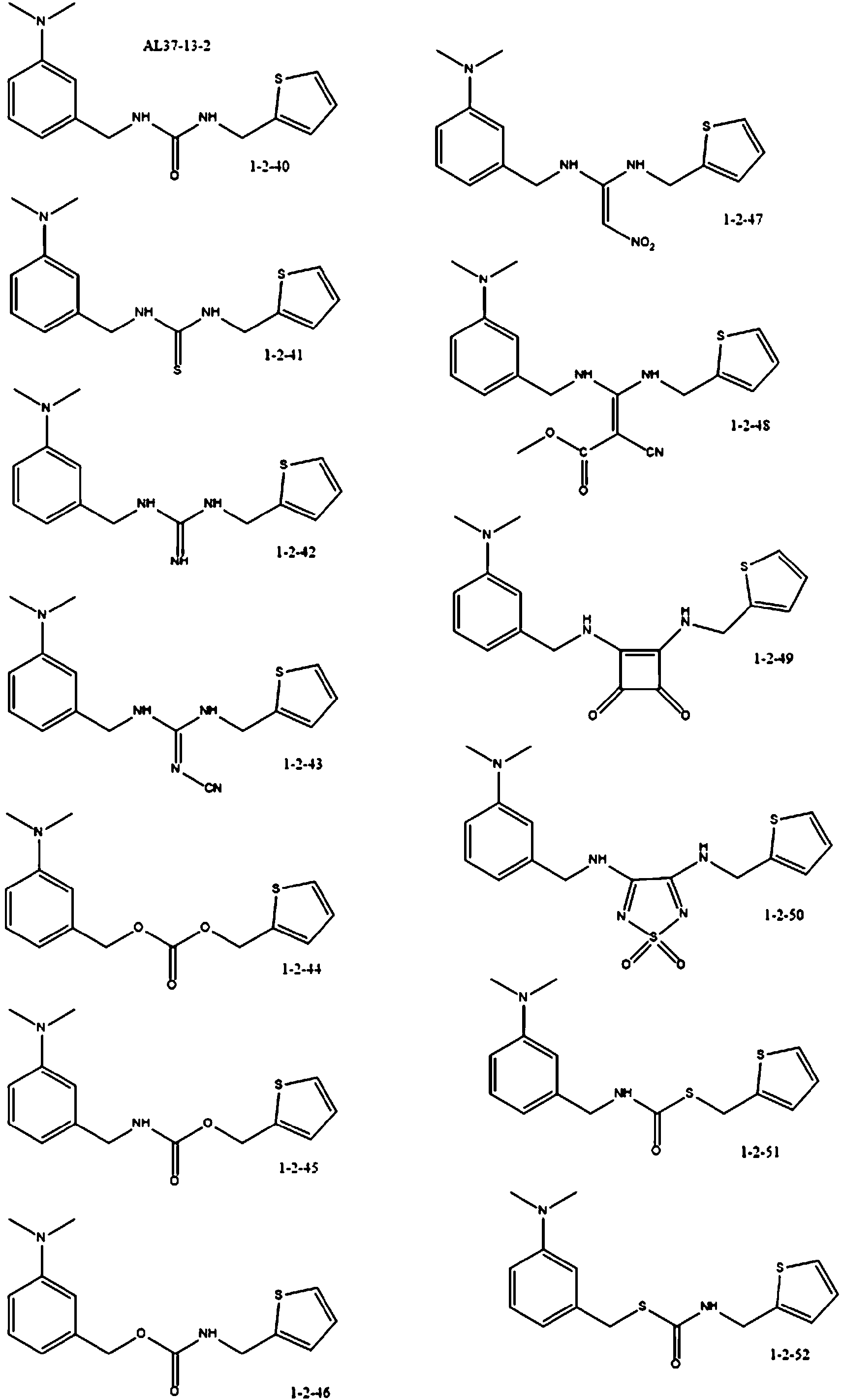

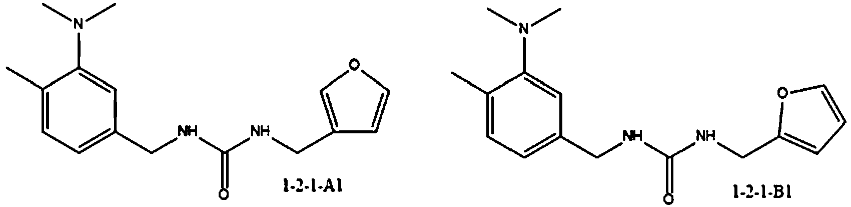

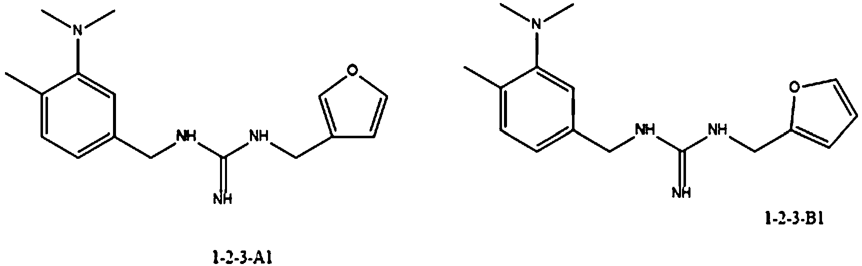

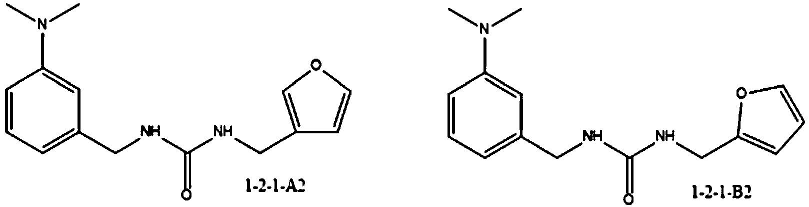

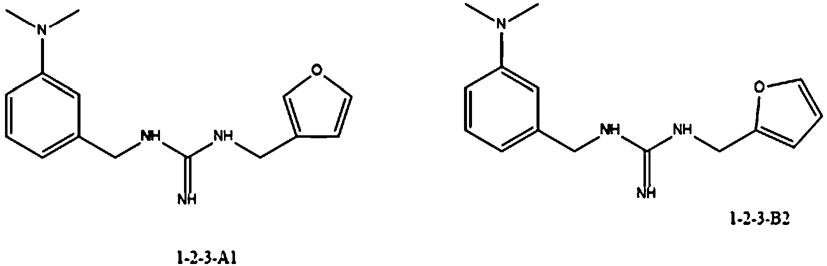

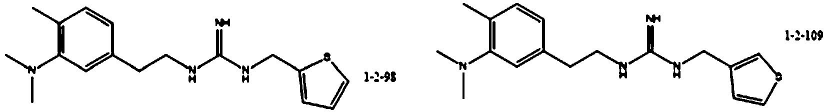

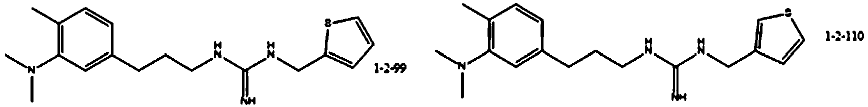

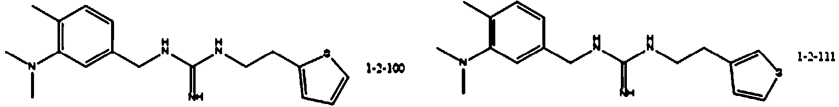

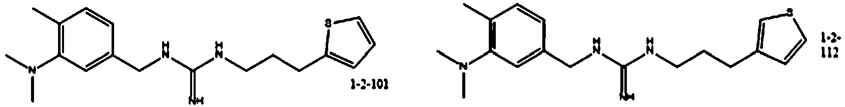

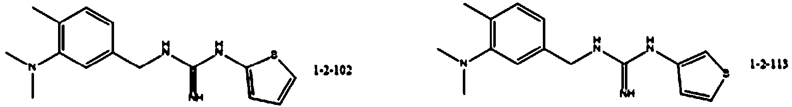

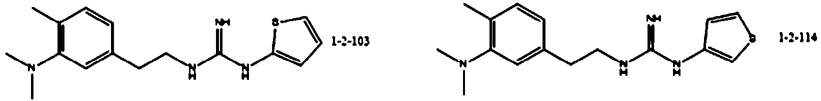

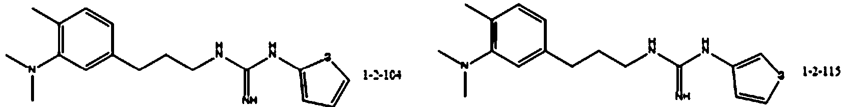

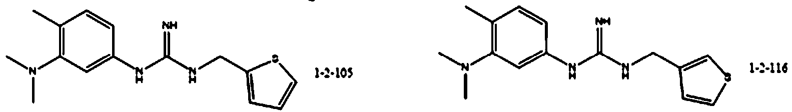

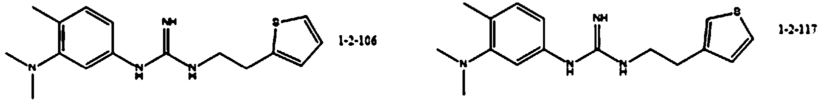

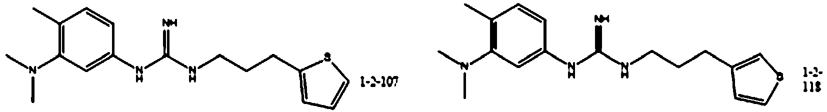

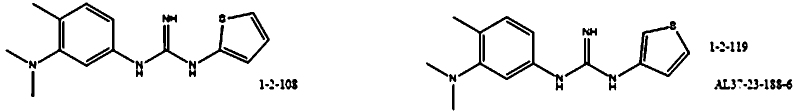

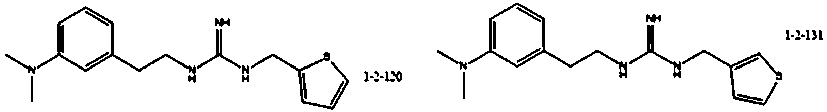

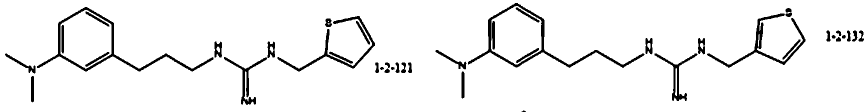

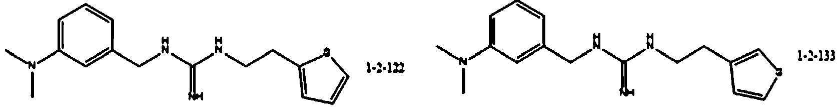

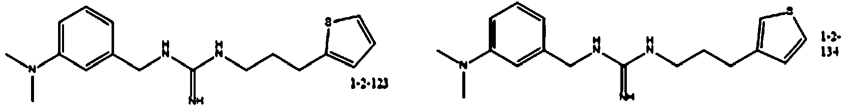

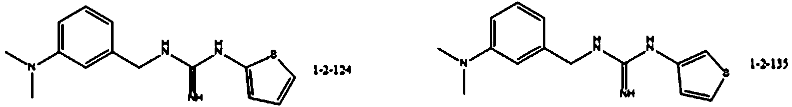

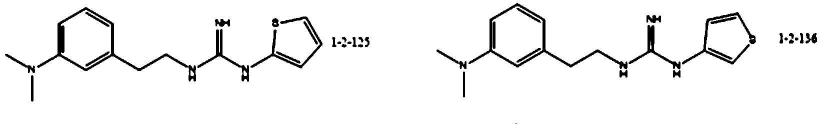

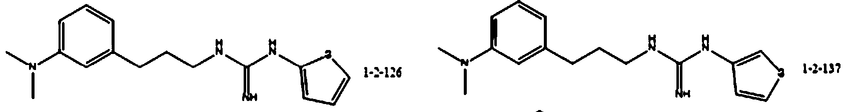

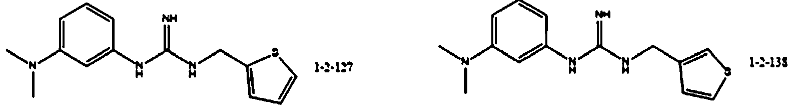

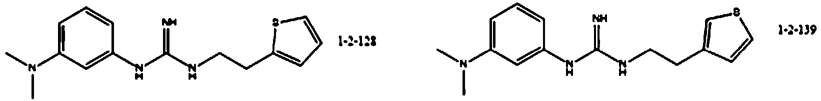

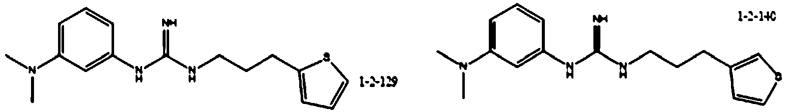

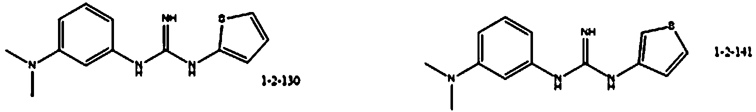

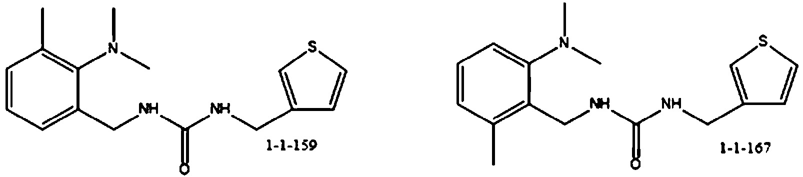

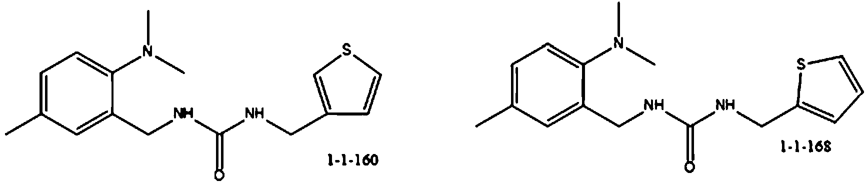

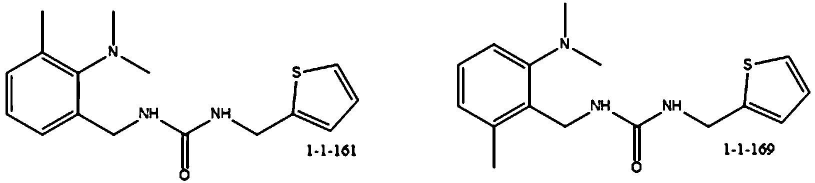

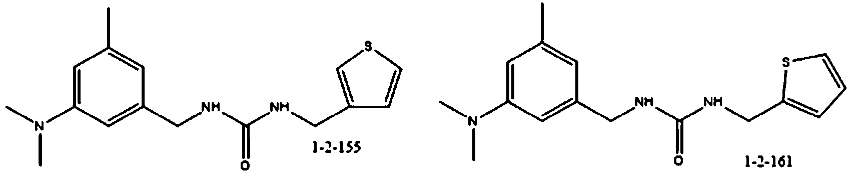

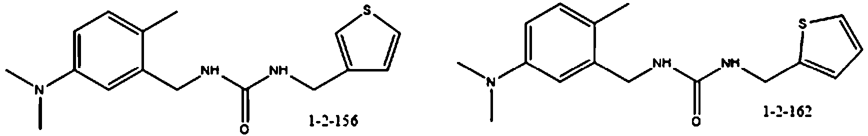

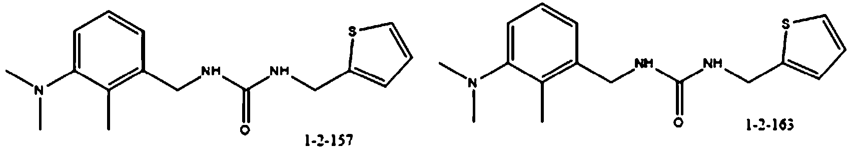

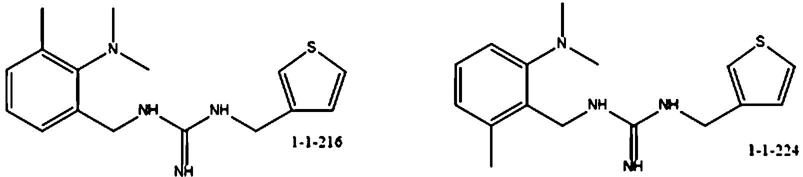

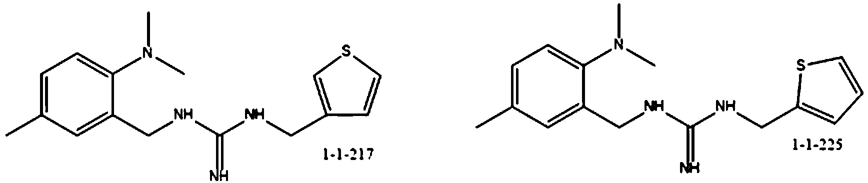

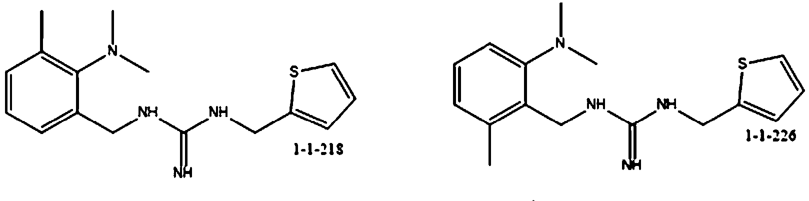

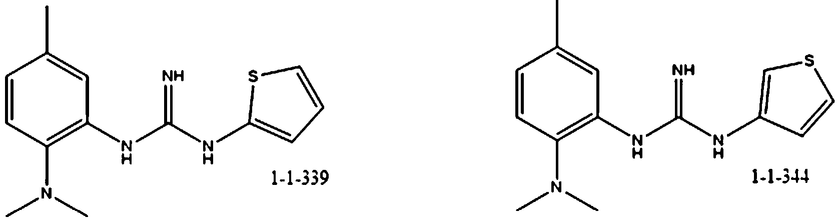

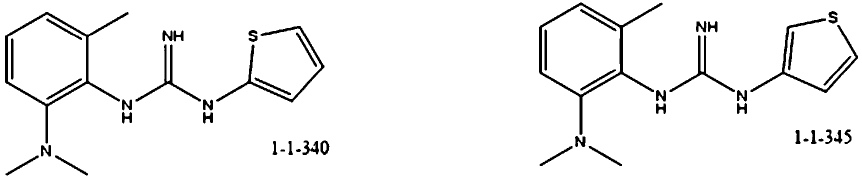

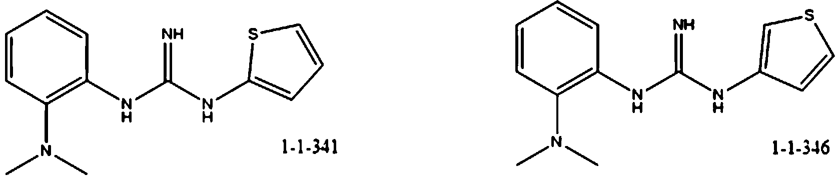

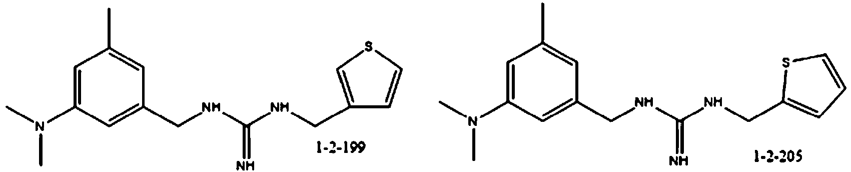

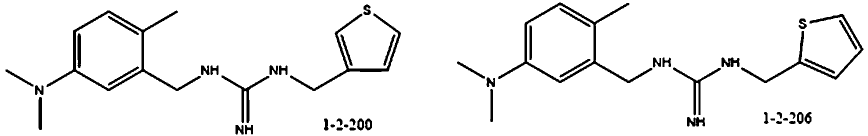

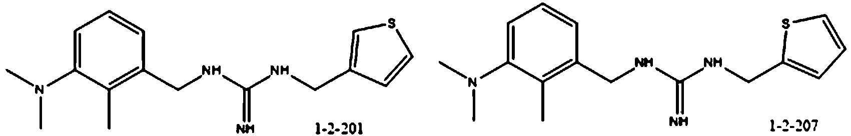

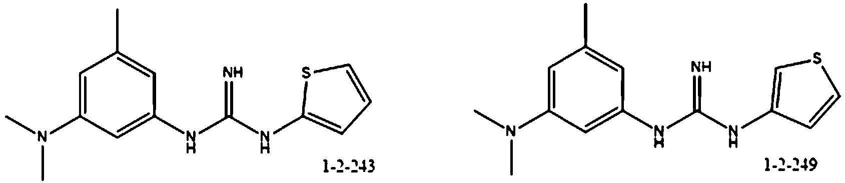

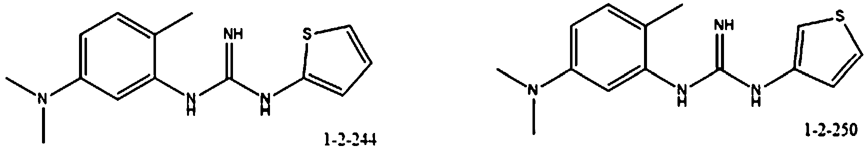

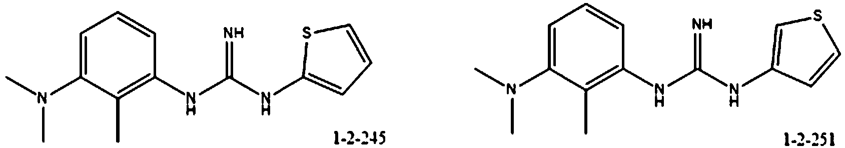

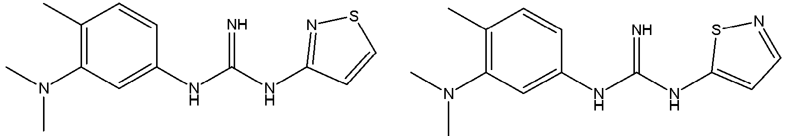

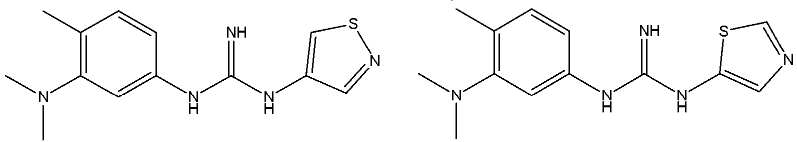

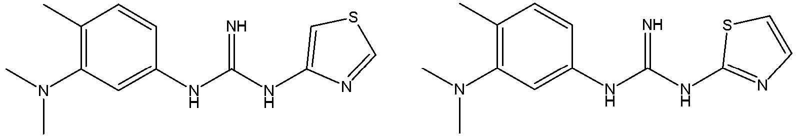

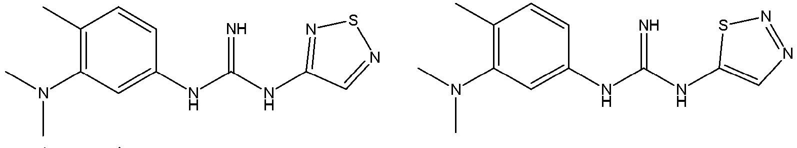

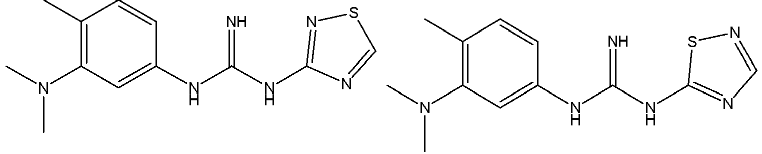

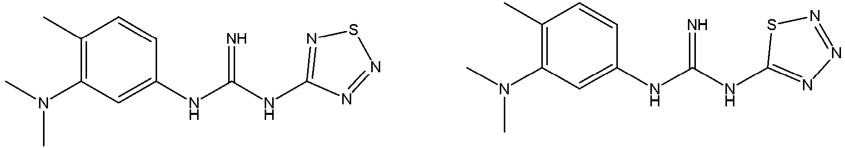

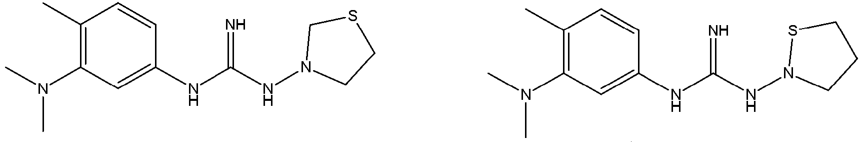

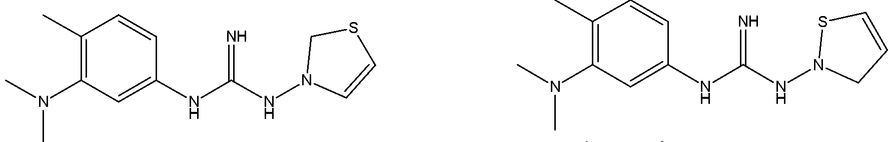

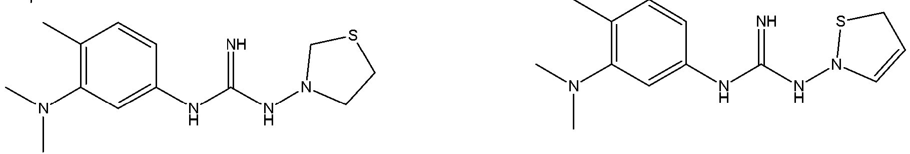

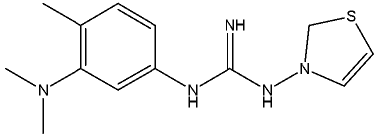

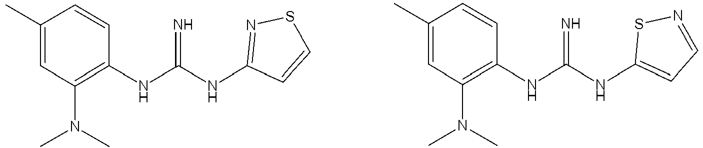

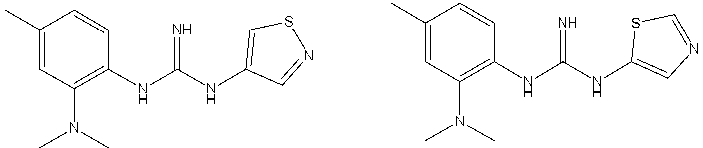

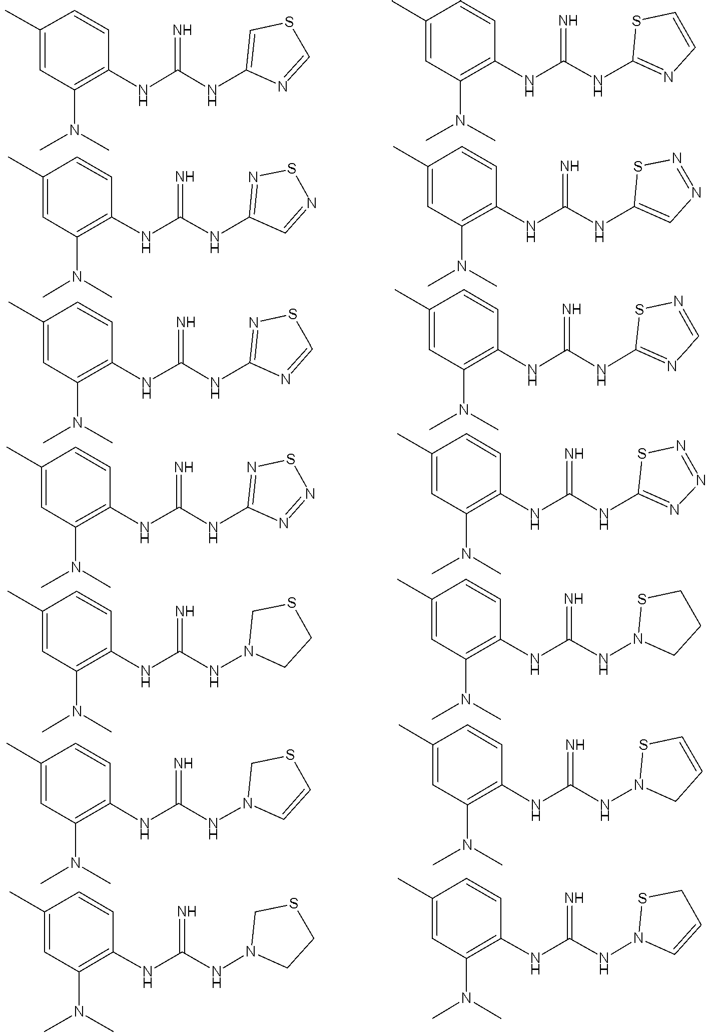

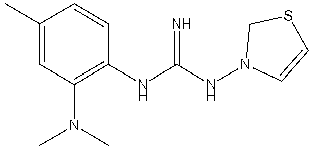

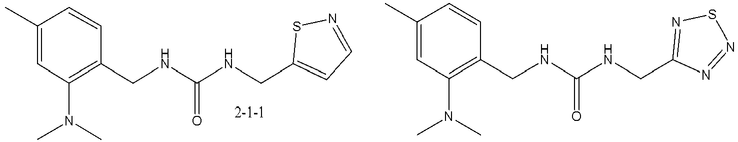

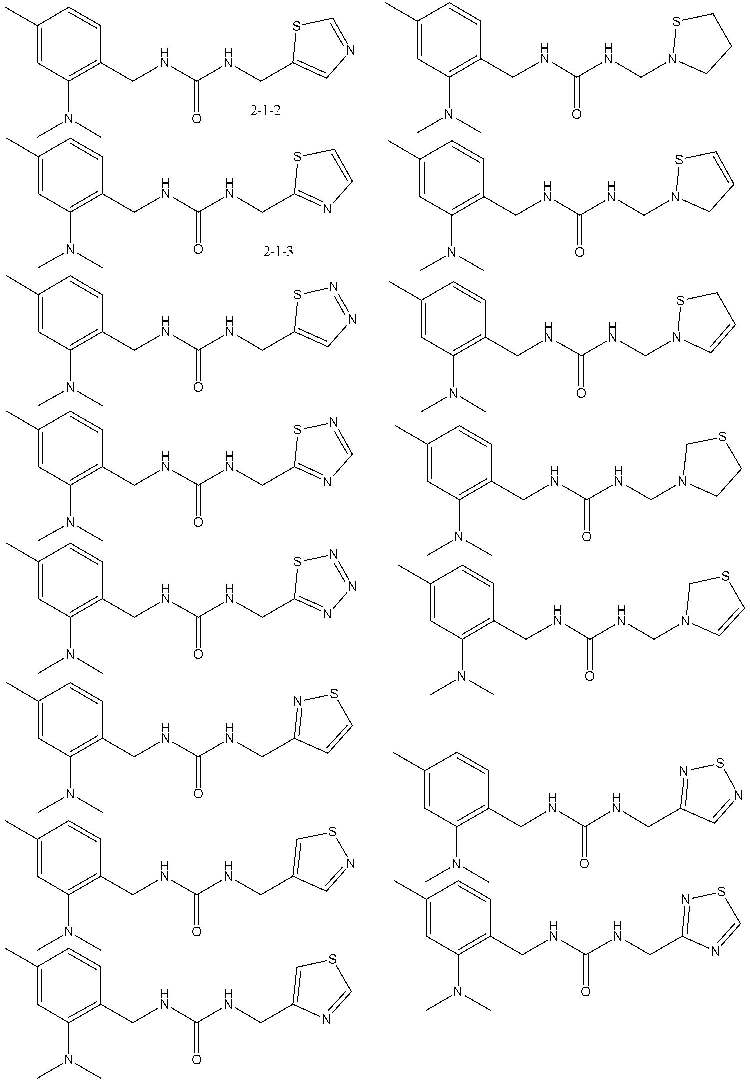

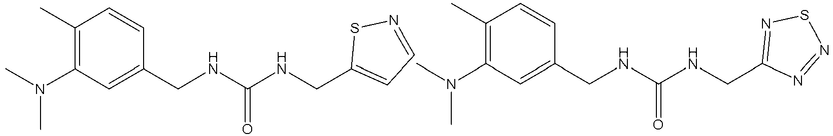

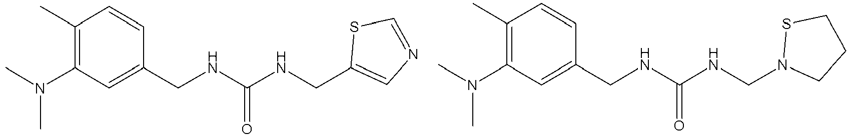

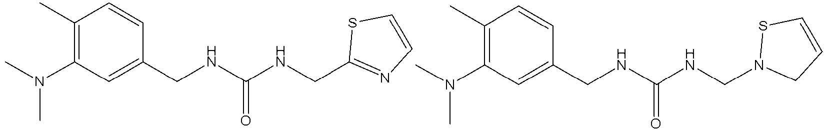

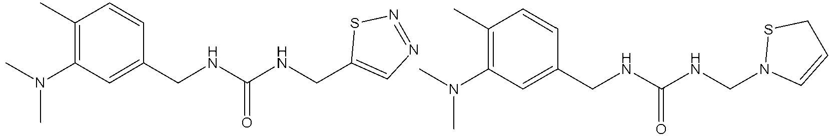

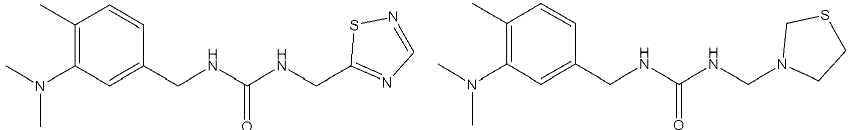

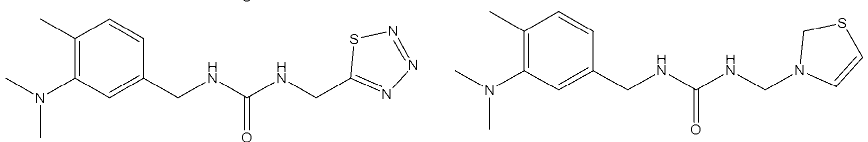

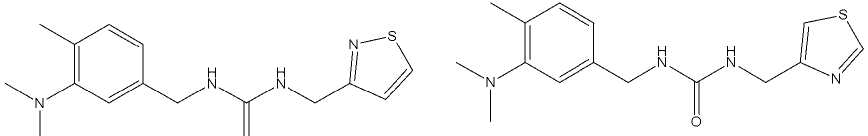

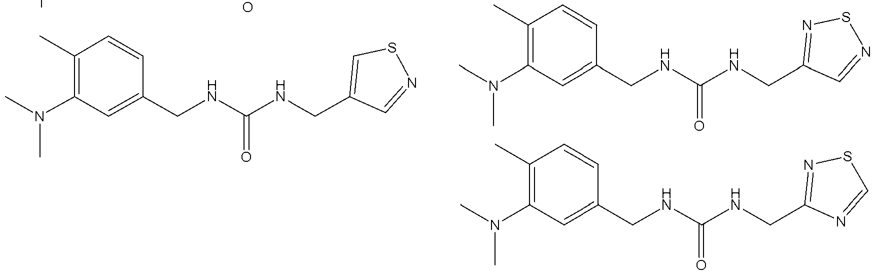

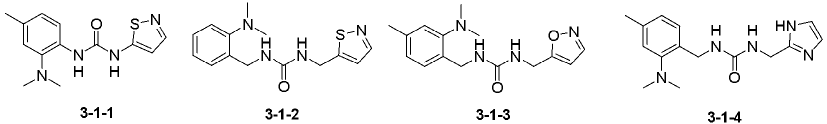

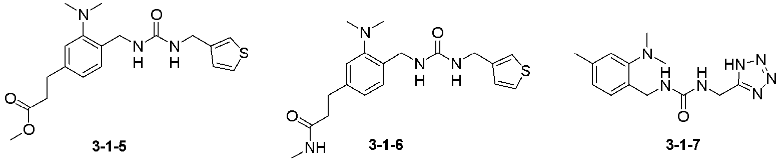

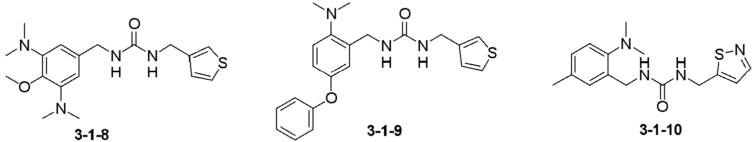

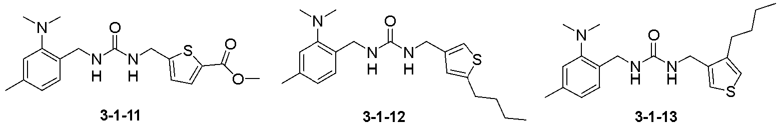

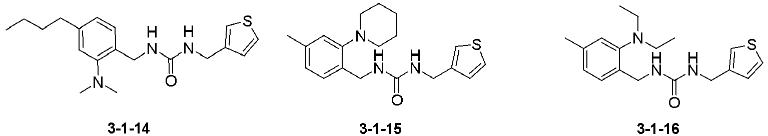

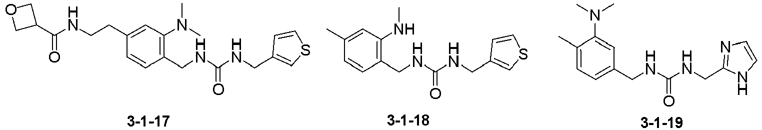

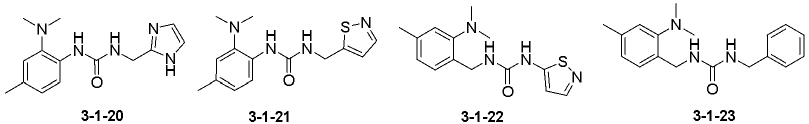

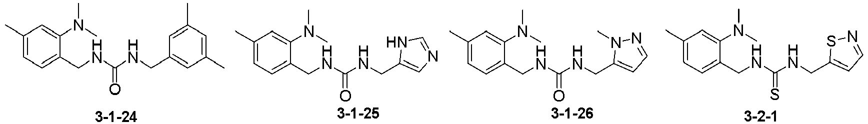

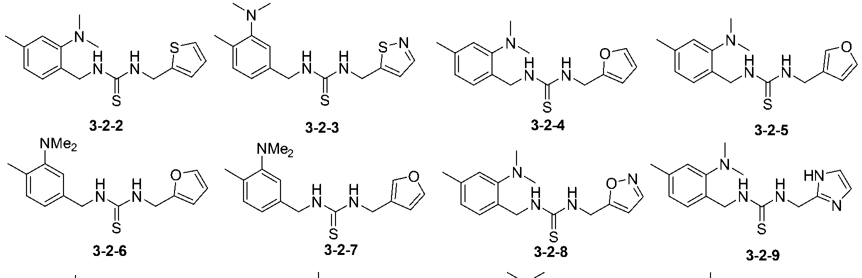

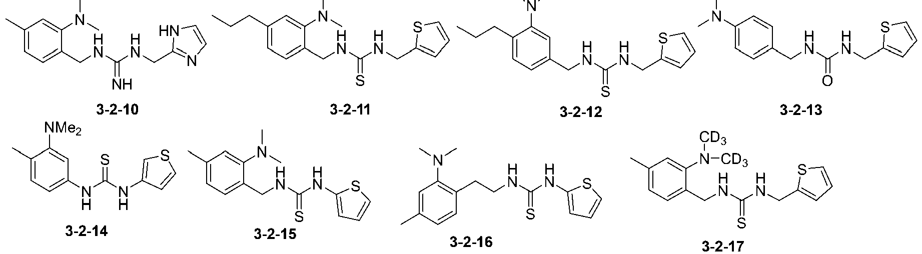

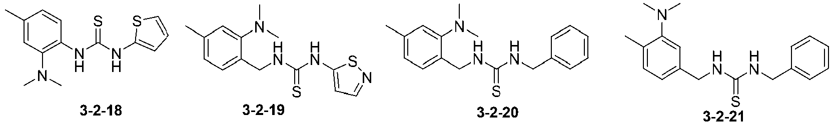

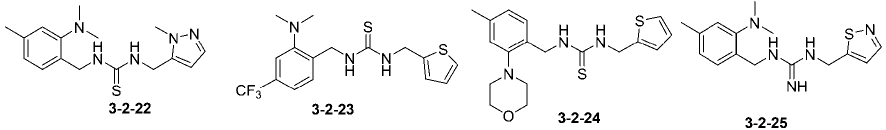

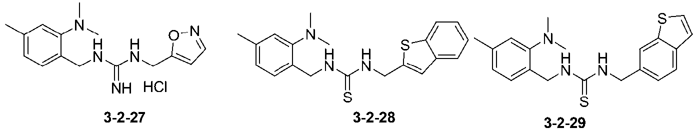

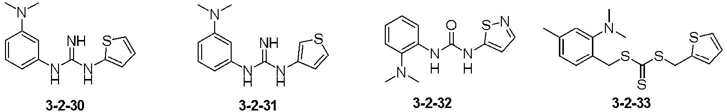

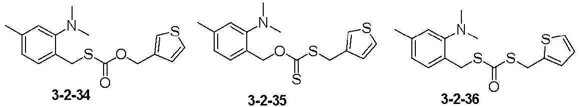

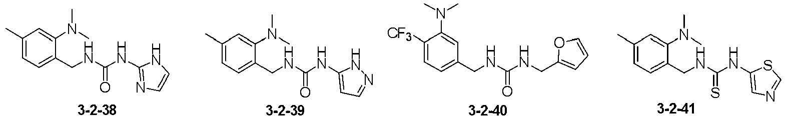

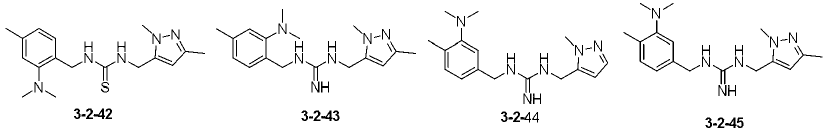

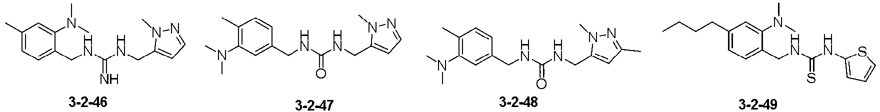

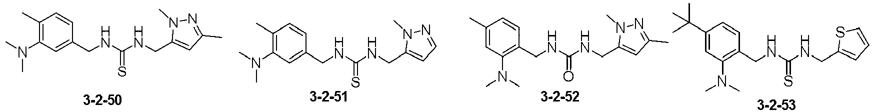

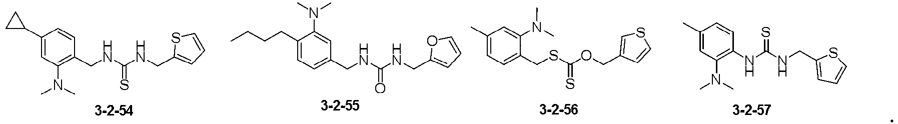

The present invention also provides the method for preparation of the above compound, or a stereoisomer thereof, or a pharmaceutically acceptable salt thereof, or a solvate thereof, or a crystal form thereof, or a prodrug thereof, or a metabolite thereof, or a deuterated derivative thereof, which comprises the following steps:

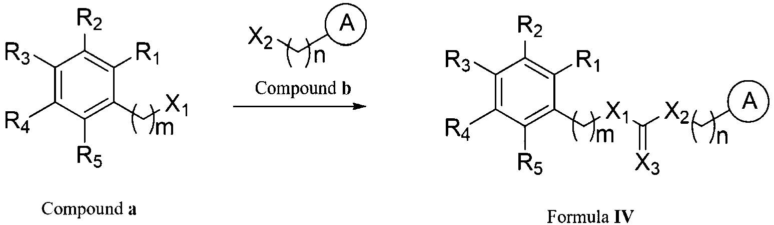

In a solvent, compound a, an organic base, TCDI or CDI, and compound b react to yield the compound represented by formula IV;
R₁, R₂, R₃, R₄, R₅, m, n, X₁, X₂, and ring A are as defined above; X₃ is S or O;
preferably,
the solvent is dichloromethane;
and/or, the organic base is Et₃N;
and/or, the temperature of the reaction is 25-40 °C, and the reaction time is 10-12 h.

The present invention also provides the method for preparation of the above compound, or a stereoisomer thereof, or a pharmaceutically acceptable salt thereof, or a solvate thereof, or a crystal form thereof, or a prodrug thereof, or a metabolite thereof, or a deuterated derivative thereof, which comprises the following steps: 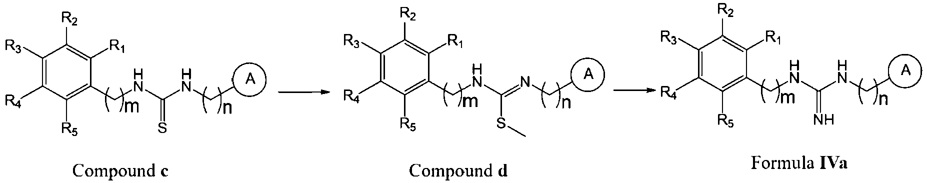
Step 1: In a solvent, compound c reacts with CH₃I to yield compound d;
Step 2: In a solvent, compound d reacts with ammonia water to obtain the compound represented by formula IVa;
R₁, R₂, R₃, R₄, R₅, m, n, and ring A are as defined above;
preferably,
in step 1, the solvent is acetonitrile;
and/or, in step 1, the reaction temperature is 40-60 °C, and the reaction time is 4-10 h;
and/or, in step 2, the solvent is acetonitrile;
and/or, in step 2, the reaction temperature is 80-100 °C, and the reaction time is 10-12 h.

The present invention also provides the use of the above compound, or a stereoisomer thereof, or a pharmaceutically acceptable salt thereof, or a solvate thereof, or a crystal form thereof, or a prodrug thereof, or a metabolite thereof, or a deuterated derivative thereof in the manufacture of medicaments with analgesic effects.

The present invention also provides a medicament, which is formed by using a compound mentioned above, or a stereoisomer thereof, or a pharmaceutically acceptable salt thereof, or a solvate thereof, or a crystalline form thereof, or a prodrug thereof, or a metabolite thereof, or a deuterated derivative thereof as the active ingredient, in combination with a pharmaceutically acceptable excipient.

The present invention also provides a pharmaceutical composition, which comprises a compound mentioned above, or a stereoisomer thereof, or a pharmaceutically acceptable salt thereof, or a solvate thereof, or a crystalline form thereof, or a prodrug thereof, or a metabolite thereof, or a deuterated derivative thereof.

The compounds and derivatives provided in the present invention can be named according to the IUPAC (International Union of Pure and Applied Chemistry) or CAS (Chemical Abstracts Service, Columbus, OH) naming systems.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" refers to the substitution of hydrogen atoms in a molecule with other different atoms or molecules.

The minimum and the maximum for the content of carbon atoms in hydrocarbon groups are represented by prefixes, such as the prefix Cₐ-C_{b} alkyl indicates any alkyl having "a"-"b" carbon atoms. Therefore, for example, "C₁-C₆ alkyl" refers to alkyls comprising 1-6 carbon atoms; "C₁-C₆ alkoxy" refers to alkoxys comprising 1-6 carbon atoms.

"Alkyl" refers to a saturated hydrocarbon chain with a specified number of carbon atoms. For example, C₁-C₆ alkyl refers to alkyl groups with 1-6 carbon atoms, namely 1, 2, 3, 4, 5, or 6 carbon atoms. Alkyl groups can be straight-chain or branched. Representative branched alkyl groups have one, two, or three branches. Alkyl includes methyl, ethyl, propyl (n-propyl and isopropyl), butyl (n-butyl, isobutyl, and tert-butyl), pentyl (n-pentyl, isopentyl, and neopentyl), hexyl, and so on.

"Halogen" refers to fluorine, chlorine, bromine, or iodine.

"Cycloalkyl" refers to a saturated or unsaturated all-carbon monocyclic or polycyclic ring (including fused, spiro, or bridged rings) without a conjugated π-electron system, including but not limited to: 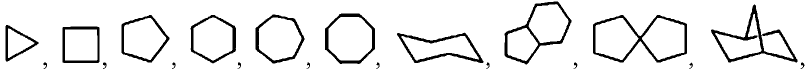 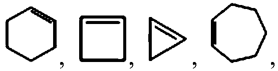 and the same.

"Heterocycloalkyl" refers to a cycloalkyl in which at least one carbon atom in the ring is replaced by a heteroatom, such as O, N, or S, and is a saturated or unsaturated monocyclic or polycyclic ring system (including fused, spiro, or bridged rings) without a conjugated π-electron system. Examples include but are not limited to: 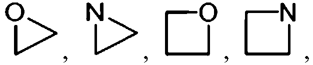 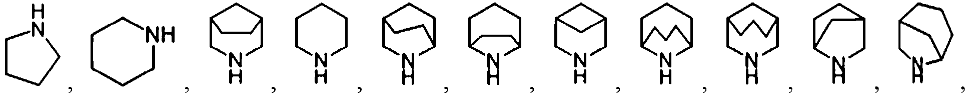 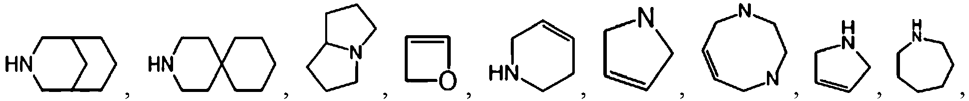 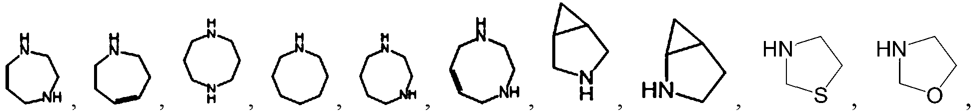 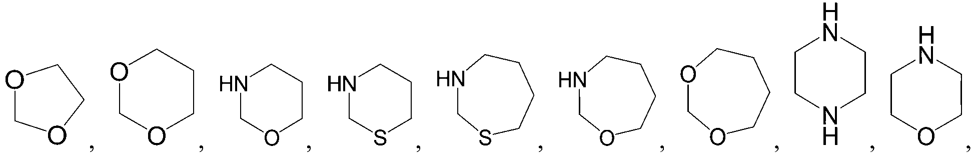 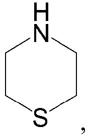 and the same.

"Aryl" refers to an all-carbon monocyclic or polycyclic ring (including fused, spiro, or bridged rings) with a conjugated π-electron system, such as but not limited to: phenyl, naphthyl, phenanthryl, anthryl, fluorenyl, and indenyl. The aromatic ring can be fused with other cyclic groups (including saturated and unsaturated rings), but it must not contain heteroatoms such as O, N, or S. Meanwhile, the point of attachment to the parent structure must be on a carbon atom in the ring with a conjugated π-electron system, such as but not limited to 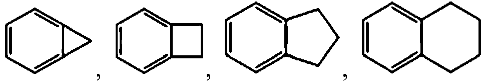 and the same.

"Heteroaryl" refers to an aryl in which at least one carbon atom in the conjugated π-electron system is replaced by a heteroatom, such as O, N, or S. Examples include but are not limited to thiophenyl, furanyl, and isothiazolyl.
"(5-8)-membered aryl" includes 5-, 6-, 7-, and 8-membered aryl.
"(5-8)-membered heteroaryl" includes 5-, 6-, 7-, and 8-membered heteroaryl.
"(5-8)-membered cycloalkyl" includes 5-, 6-, 7-, and 8-membered cycloalkyl.
"(5-8)-membered heterocycloalkyl" includes 5-, 6-, 7-, and 8-membered heterocycloalkyl.

The pharmaceutically acceptable salts in the present invention include acetate, adipate, aspartate, benzoate, benzenesulfonate, bicarbonate, carbonate, bisulfate, sulfate, borate, camphorsulfonate, citrate, cyclohexanesulfamate, ethanedisulfonate, ethanesulfonate, formate, fumarate, glucoheptonate, gluconate, glucuronate, hexafluorophosphate, hydrochloride, hydrobromide, hydroiodide, hydroxyethylsulfonate, lactate, malate, maleate, malonate, methanesulfonate, methylsulfate, naphthoate, naphthalenesulfonate, nicotinate, nitrate, orotate, oxalate, palmitate, pamoate hydrate, phosphate, hydrophosphate, dihydrogen phosphate, pyroglutamate, saccharate, stearate, succinate, tannate, tartrate, toluenesulfonate, trifluoroacetate, xinafoate, methanesulfonate, p-toluenesulfonate, quaternary ammonium salt, or succinate and the same.

The pharmaceutical composition of the present invention comprises a compound of the present invention or a pharmacologically acceptable salt thereof within a safe and effective dosage range, and a pharmacologically acceptable excipient or carrier.

The route of administration for the compounds or pharmaceutical compositions of the present invention include (but are not limited to): intragastric, enteral, parenteral (intravenous, intramuscular, or subcutaneous), oral, and various local administrations.

Compositions for parenteral (intravenous, intramuscular, subcutaneous) injection may comprise physiologically acceptable sterile aqueous or anhydrous solutions, dispersions, suspensions, or lotions, and sterile powders for reconstitution into sterile injectable solutions or dispersions. Suitable aqueous and non-aqueous carriers, diluents, solvents, or excipients include water, ethanol, polyols, and suitable mixtures thereof.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or bulking agents, such as starch, lactose, sucrose, glucose, mannitol, and silicic acid; (b) binders, such as hydroxymethylcellulose, alginates, gelatin, polyvinylpyrrolidone, sucrose, and gum arabic; (c) Humectants, such as glycerin; (d) Disintegrants, such as agar, calcium carbonate, potato starch or cassava starch, alginic acid, certain composite silicates, and sodium carbonate; (e) Delayed-release agents, such as paraffin wax; (f) Absorption enhancers, such as quaternary ammonium compounds; (g) Wetting agents, such as cetyl alcohol and glyceryl monostearate; (h) Adsorbents, such as kaolin; and (i) Lubricants, such as talc, calcium stearate, magnesium stearate, solid polyethylene glycol, sodium lauryl sulfate, or mixtures thereof. In capsules, tablets, and pills, the dosage form may also include buffers.

Liquid dosage forms for oral administration include pharmaceutically acceptable lotions, solutions, suspensions, syrups, or tinctures. In addition to the active compound, the liquid dosage forms may contain inert diluents commonly used in the art, such as water or other solvents, solubilizers, and emulsifiers, for example, ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures thereof.

Solid dosage forms such as tablets, sugar pills, capsules, pills, and granules can be prepared using coating and shell materials, such as enteric coatings and other materials well-known in the art. They can contain opaque agents, and the release of the active compound or compounds in such compositions can be delayed in a specific part of the digestive tract. Examples of embedding components that can be used are polymeric substances and wax-like substances. If necessary, the active compound can also be microencapsulated with one or more of the above excipients.

The dosage forms of the compounds of the present invention for local administration include ointments, powders, patches, sprays, and inhalants. Under sterile conditions, the active ingredient is mixed with a physiologically acceptable carrier, along with any preservatives, buffers, or propellants that may be necessary.

In addition to these inert diluents, the composition may also include auxiliary agents such as wetting agents, emulsifiers and suspending agents, sweeteners, flavoring agents, and fragrances.

In addition to the active compound, the suspension may comprise a suspending agent, such as ethoxylated isostearyl alcohol, polyoxyethylene sorbitol and sorbitan esters, microcrystalline cellulose, aluminum methoxide, and agar, or mixtures thereof.

The compound of the present invention can be administered alone or in combination with other pharmaceutically acceptable compounds.

When using the pharmaceutical composition, a safe and effective amount of the compound of the present invention is administered to a mammal (such as a human) in need of treatment, wherein the dosage at the time of administration is a dose considered safe and effective in pharmacology.

Compared with the prior art, the compounds provided in the present invention achieve the following beneficial effects:
The present invention provides a compound with analgesic effect. The compound of the present invention exhibits excellent analgesic effect, good safety during use, low toxicity and side effects, and no dependency during use. Therefore, the compound of the present invention has broad application prospects in the manufacture of analgesic medicaments, providing a new option for the manufacture of medicaments with analgesic effects in clinical practice.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the examples of the present invention are all known products, obtained by purchasing those commercially available.

The structure of the compound is determined by nuclear magnetic resonance (NMR) and/or mass spectrometry (MS). NMR shifts (δ) are given in units of 10⁻⁶ (ppm). NMR measurements were conducted using a spectrometer (Bruker Avance III 400 NMR), with the solvents being deuterated dimethyl sulfoxide (d₆-DMSO), deuterated chloroform (CDCl₃), deuterated methanol (d₄-MeOH), deuterated water (D₂O), and tetramethylsilane (TMS) as the internal standard.

The LCMS measurement was carried out using an Agilent LCMS 1200-6120 (ESI), with a chromatographic column Waters Xbridge Prep C18 OBD 10 µm, 19*250 mm. The column temperature was maintained at 40°C, and the flow rate was set at 2.0 mL/min. The mobile phase consisted of a gradient from 95% [water + 10 mM ammonium bicarbonate] and 5% [CH₃CN] to 5% [water + 10 mM ammonium bicarbonate] and 95% [CH₃CN] over a period of 1.6 minutes, which was maintained for 1.4 minutes. Subsequently, the gradient was shifted to 95% [water + 10 mM ammonium bicarbonate] and 5% [CH₃CN] over a period of 0.05 minutes, and this condition was maintained for an additional 0.7 minutes.

### (1) Medicinal materials and reagents

The manufacturer of silica gel plates used in thin layer chromatography (TLC) is Qingdao Puke Separation Materials Co., Ltd., with specifications of 50*200 mm and a thickness ranging from 0.2 mm to 0.25 mm.

The silica gel used for column chromatography is 200-300 mesh silica gel from Taiyang Desiccant Co., Ltd. in Rushan City, Weihai, Shandong Province.

### (2) Main instruments

Electronic balance, FA2004, Shanghai Liangping Instrumentation Co., Ltd;
Magnetic stirrer (temperature-controlled and pressure-regulated), TY98-1, Shanghai Sile Instrument Co., Ltd;
Thiple-purpose UV analyzer, ZF-2 model, Shanghai Anting Scientific Instrument Factory;
Rotary evaporator, R201, Zhengzhou Huicheng Electronic Technology Co., Ltd;
Adjustable water bath, R201D, Zhengzhou Huicheng Electronic Technology Co., Ltd;
Circulating water vacuum pump (benchtop), SHB-III, Zhengzhou Huicheng Electronic Technology Co., Ltd.;
Circulating water vacuum pump (mobile), SHB-B95, Zhengzhou Huicheng Electronic Technology Co., Ltd.;
Low-temperature circulating pump, DLSB-5/20, Zhengzhou Huicheng Electronic Technology Co., Ltd;
Rotary vane vacuum pump (oil pump), 2XZ-4, Shanghai Vacuum Pump Factory.

### Example 1. Preparation of compound 1-1-1 of the present invention

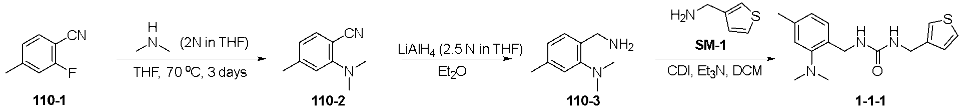

### 1. Synthesis of 110-2

To a 250 mL sealed tube, were added **110-1** (5.0 g, 37 mmol) and a solution of dimethylamine in tetrahydrofuran (2N, 100 mL), and then the reaction was stirred at 70 °C for three days. After completion of the reaction, the solvent was removed by evaporation under reduced pressure to obtain the crude product, which was purified by normal phase column chromatography [petroleum ether: ethyl acetate (20:1-5:1)] to obtain **110-2** as a yellow oil (3.2 g, yield 89.9%). MS Calcd.: 161.1[M+H]⁺; MS Found: 161.4 [M+H]⁺.

### 2. Synthesis of 110-3

To a reaction flask, were added **110-2** (3.2 g, 20 mmol) and diethyl ether (50 mL), and then the system was purged with nitrogen balloon three times, and cooled to 0 °C under nitrogen atmosphere. Then, lithium aluminum hydride (2.5 N in THF, 16 mL) was added, and the reaction was stirred at room temperature overnight. After completion of the reaction, water (3 mL), 15% NaOH solution (3 mL), and water (6 mL) were slowly added, and then the resultant solution was stirred at room temperature for 20 min, dried over anhydrous magnesium sulfate, and filtered. The solvent was removed by evaporation under reduced pressure to obtain the crude product, which was purified by normal phase column chromatography [dichloromethane: methanol (100:1-20:1)] to obtain **110-3** as a yellow oil (2.0 g, yield 61%).

### 3. Synthesis of compound 1-1-1

**110-3** (656 mg, 4.0 mmol) was added to a reaction flask, and dissolved with dichloromethane (30 mL), to which were added triethylamine (2.0 g, 20.0 mmol) and CDI (972 mg, 6.0 mmol). Then, the reaction was stirred at room temperature for 2 h, followed by adding **SM-1** (452 mg, 4.0 mmol) to the system, and the mixture was allowed to react at room temperature overnight. After completion of the reaction, the solvent was removed by evaporation under reduced pressure to obtain the crude product, which was purified by column chromatography [eluent: dichloromethane-methanol (100:1-20:1)] and reversed-phase prep-HPLC to obtain **1-1-1** as white solids (689.35 mg, 57%). MS Calcd.: 304.1[M+H]⁺; MS Found: 304.4 [M + H]⁺.

¹H NMR (400 MHz, DMSO-*d*₆) *δ*: 7.47 (dd, *J =* 4.8, 2.8 Hz, 1H), 7.23 (dd, *J* = 2.4, 0.8 Hz, 1H), 7.08 (d, *J* = 7.6 Hz, 1H), 7.03-7.00 (m, 1H), 6.89 (s, 1H), 6.81 (d, *J* = 8.0 Hz, 1H), 6.37 (t, *J =* 6.0 Hz, 1H), 6.23 (t, *J* = 5.6 Hz, 1H), 4.24-4.20 (m, 4H), 2.60 (s, 6H), 2.25 (s, 3H).

### Example 2. Preparation of compound 1-1-2 of the present invention

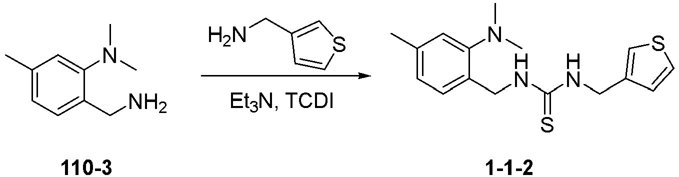

### 1. Preparation of compound 110-3

Compound **110-3** was prepared according to the method described in Example 1.

### 2. Preparation of compound 1-1-2

To a solution of 2-(aminomethyl)-N,N,5-trimethylaniline **(110-3,** 492 mg, 3 mmol) in dichloromethane (20 mL), were added TCDI (1.07 g, 3.6 mmol) and triethylamine (1.25 g, 12 mmol). The mixture was stirred at room temperature for 2 h, followed by addition of thiophene-3-ylmethanamine (339 mg, 3.0 mmol). Finally, the reaction was stirred overnight at room temperature. After completion of the reaction, the reaction solution was concentrated to obtain a crude residue, which was purified by prep-HPLC to yield, 1-(2-(dimethylamino)-4-methylbenzyl)-3-(thiophene-3-ylmethyl)thiourea as yellow solid (compound **1-1-2,** 500 mg, yield 52%). MS Calcd.: 320.1[M+H]⁺; MS Found: 320.2 [M + H]⁺.

¹H NMR (400 MHz, DMSO-*d*₆) *δ*: 8.2-7.87 (m, 2H), 7.74-7.70 (m, 1H), 7.51-7.49 (m, 1H), 7.31 (s, 1H), 7.08 (d, *J=* 4.4 Hz, 2H), 6.92 (s, 1H), 6.84 (s, 1H), 4.65 (s, 4H), 2.57 (s, 6H), 2.26 (s, 3H).

### Example 3. Preparation of compound 1-1-3 of the present invention

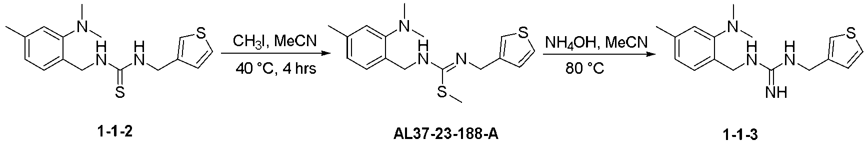

### 1. Preparation of compound 1-1-2

Compound **1-1-2** was prepared according to the method described in Example 2.

### 2. Synthesis of AL-37-23-188-A

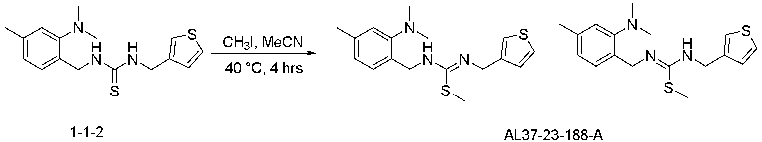

To a solution of **1-1-2** (319 mg, 1 mmol) in acetonitrile (20 mL), was added iodomethane (800 mg, 6 mmol), and the mixture was stirred at 40 °C for 4 h. After completion of the reaction, the reaction solution was concentrated to obtain a crude residue, which was purified by column chromatography (DCM/MeOH=30/1), to yield a mixture of methyl (Z)-N-(2-(dimethylamino)-4-methylbenzyl)-N'-(thiophen-3-ylmethyl)aminothiocarbamate and methyl (Z)-N'-(2-(dimethylamino)-4-methylbenzyl)-N-(thiophen-3-ylmethyl) aminothiocarbamate **(AL-37-23-188-A,** 200 mg, yield 60%).

### 3. Synthesis of compound 1-1-3

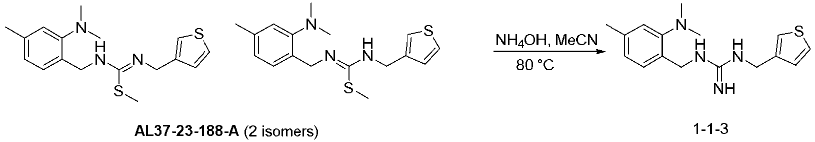

To a solution of a mixture of methyl (Z)-N-(2-(dimethylamino)-4-methylbenzyl)-N'-(thiophen-3-ylmethyl)aminothiocarbamate and methyl (Z)-N'-(2-(dimethylamino)-4-methylbenzyl)-N-(thiophen-3-ylmethyl)aminothiocarbamate **(AL37-23-188-A,** 200 mg, 0.6 mmol) in acetonitrile (10 mL), was added ammonia water (600 mg, 6.0 mmol), and the mixture was stirred overnight at 80 °C. After completion of the reaction, the reaction solution was concentrated to obtain a crude residue, which was purified by prep-HPLC, to yield, 1-(2-(dimethylamino)-4-methylbenzyl)-3-(thiophen-3-ylmethyl)guanidine as white solids (compound **1-1-3,** 30 mg, yield 17%). MS Calcd.: 303.2[M+H]⁺; MS Found: 303.2 [M + H]⁺.

¹H NMR (400 MHz, DMSO-*d*₆) *δ*: 7.50-7.48 (m, 3H), 7.29 (s, 1H), 7.13 (d, *J=* 7.6 Hz, 1H), 7.05-7.03 (m, 1H), 6.93 (m, 1H), 6.84 (d, *J=* 7.6 Hz, 1H), 4.28 (s, 2H), 4.24 (s, 2H), 2.62 (s, 6H), 2.26 (s, 3H).

### Example 4. Preparation of compound 1-1-4 of the present invention

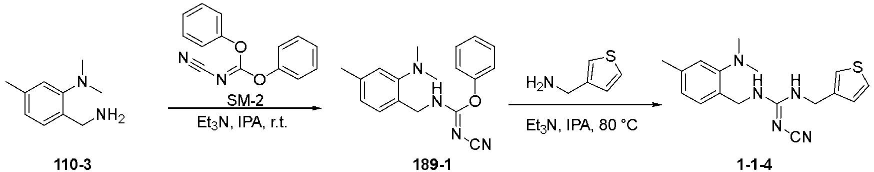

### 1. Preparation of compound 110-3

Compound 110-3 was prepared according to the method described in Example 1.

### 2. Synthesis of 189-1

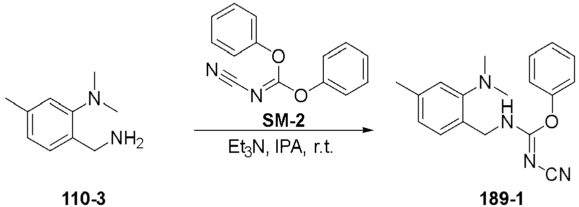

To a solution of 2-(aminomethyl)-N,N,5-trimethylaniline **(110-3,** 492 mg, 3 mmol) and **SM-**2 (714 mg, 3 mmol) in isopropanol (20 mL), was added triethylamine (606 mg, 6 mmol), and the mixture was stirred at room temperature overnight. After completion of the reaction, the reaction solution was concentrated to obtain a crude residue, which was purified by column chromatography (DCM/MeOH=30/1) to yield phenyl (Z)-N'-cyano-N-(2-(dimethylamino)-4-methylbenzyl)carbamate **(189-1,** 500 mg, yield 54%) as a pale yellow oil.

### 3. Synthesis of compound 1-1-4

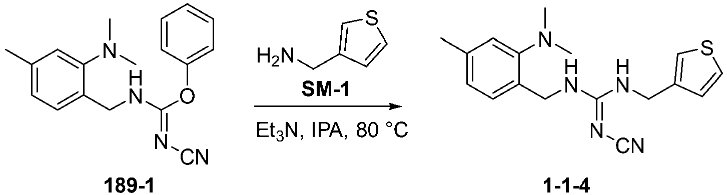

To a solution of **189-1** (308 mg, 1 mmol) and thiophene-3-ylmethylamine **(SM-1,** 113 mg, 1 mmol) in isopropanol (10 mL), was added triethylamine (303 mg, 3 mmol), and then the reaction was stirred overnight at 80°C. After completion of the reaction, the reaction solution was concentrated to obtain a crude residue, which was purified by prep-HPLC to yield phenyl (Z)-N'-cyano-N-(2-(dimethylamino)-4-methylbenzyl)carbamate (compound **1-1-4,** 104.74 mg, yield 32%) as white solids.

¹H NMR (400 MHz, DMSO-*d*₆) *δ*: 7.83 (s, 1H), 7.61 (s, 1H), 7.52-7.50 (m, 1H), 7.25 (m, 1H), 7.05-7.02 (m, 2H), 6.93 (s, 1H), 6.86 (t, *J* = 7.6 Hz, 1H), 4.33 (d, *J=* 5.6 Hz, 2H), 4.30 (d, *J= 6.0* Hz, 2H), 2.52 (s, 6H), 2.26 (s, 3H).

### Example 5. Preparation of compound 1-1-6 of the present invention

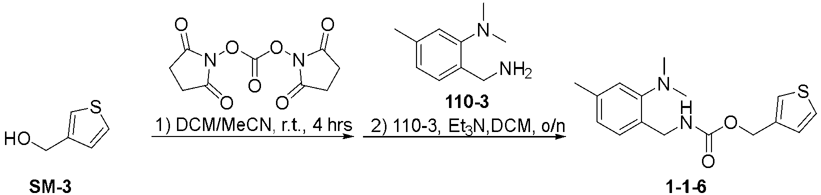

### 1. Preparation of compound 110-3

Compound **110-3** was prepared according to the method described in Example 1.

### 2. Synthesis of compound 1-1-6

At 0 °C, under nitrogen protection, bis(2,5-dioxopyrrolidin-1-yl)carbonate (922 mg, 3.6 mmol) was added to a solution of thiophene-3-ylmethanol **(SM-3,** 342 mg, 3 mmol) in dichloromethane/acetonitrile (10/20 mL). The mixture was stirred at room temperature for 4 h, followed by addition of **110-3** (339 mg, 3 mmol) and triethylamine (909 mg, 9 mmol). The mixture was stirred overnight at room temperature. After completion of the reaction, the reaction solution was concentrated to obtain a crude residue, which was purified by column chromatography and prep-HPLC to yield **1-1-6** as grayish-white solid (98.25 mg, yield 11%).

¹H NMR (400 MHz, DMSO-*d*₆) *δ*: 7.62 (t, *J =* 6.0 Hz, 1H), 7.53 (dd, *J =* 3.2 Hz, 4.8 Hz, 1H), 7.50 (s, 1H), 7.12-7.07 (m, 2H), 6.89 (s, 1H), 6.81 (d, *J=* 7.6 Hz, 1H), 5.03 (s, 2H), 4.23 (d, *J= 6.0* Hz, 2H), 2.59 (s, 6H), 2.25 (s, 3H).

### Example 6. Preparation of compound 1-1-7 of the present invention

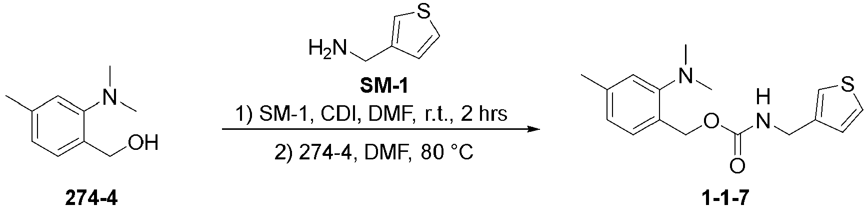

To a solution of thiophene-3-ylmethylamine (SM-1, 226 mg, 2 mmol) in DMF (10 mL), were added CDI (389 mg, 2.4 mmol) and triethylamine (707 mg, 7.0 mmol). The mixture was stirred at room temperature for 2 h, followed by addition of (2-(dimethylamino)-4-methylphenyl)methanol **(274-4,** 330 mg, 2 mmol). The mixture was then stirred at 80 °C for 2 h. After completion of the reaction, the reaction solution was concentrated to obtain a crude residue, which was purified by column chromatography (DCM/MeOH=50/1) to yield 2-(dimethylamino)-4-methylbenzyl(thiophene-3-ylmethyl)carbamate as grayish-white solid (compound 1-1-7, 57.98 mg, yield 10%). MS Calcd.: 305.2[M+H]⁺; MS Found: 305.2 [M + H]⁺

¹H NMR (400 MHz, DMSO-*d*₆) *δ*: 7.73 (t, *J* = 6.0 Hz, 4.8 Hz, 1H), 7.47 (dd, *J=* 3.2 Hz, 4.8 HZ, 1H), 7.25 (s, 1H), 7.20 (d, *J* = 7.6 Hz, 1H), 7.02 (d, *J=* 4.8 Hz, 1H), 6.93 (s, 1H), 6.84 (d, *J* = 7.6 Hz, 1H), 5.06 (s, 2H), 4.19 (d, *J=* 6.0 Hz, 2H), 2.62 (s, 6H), 2.25 (s, 3H).

### Example 7. Preparation of compound 1-1-8 of the present invention

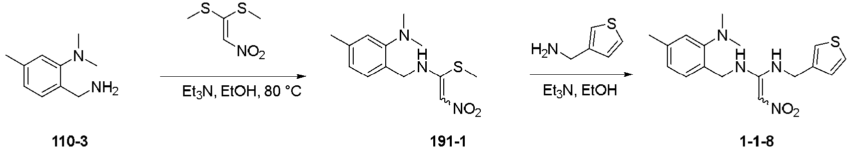

### 1. Preparation of compound 110-3

Compound **110-3** was prepared according to the method described in Example 1.

### 2. Synthesis of compound 191-1

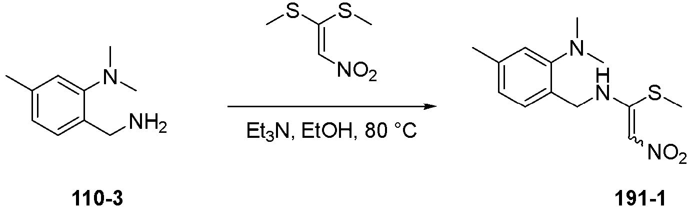

To a solution of 2-(aminomethyl)-N,N,5-trimethylaniline **(110-3,** 492 mg, 3 mmol) and 1,1-bis(methylthio)-2-nitroethylene (495 mg, 3 mmol) in ethanol (20 mL), was added triethylamine (606 mg, 6 mmol), and then the mixture was stirred at 80 °C overnight. After completion of the reaction, the reaction solution was concentrated to obtain a crude residue, which was purified by column chromatography (DCM/MeOH=40/1) to yield N,N,5-trimethyl-2-(((1-(methylthio)-2-nitroethylene)amino)methyl)aniline (191-1, 500 mg, yield 60%) as a light yellow oil.

### 3. Synthesis of 1-1-8

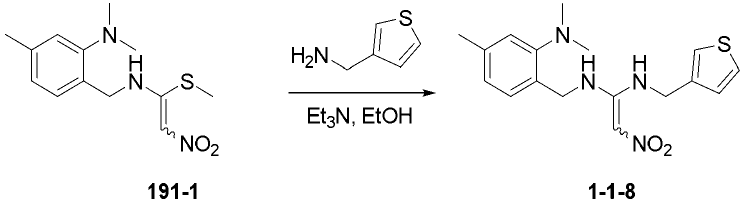

To a solution of **191-1** (281 mg, 1 mmol) and thiophene-3-ylmethylamine (113 mg, 1 mmol) in ethanol (10 mL), was added triethylamine (303 mg, 3 mmol), and the mixture was stirred at 80 °C overnight. After completion of the reaction, the reaction solution was concentrated to obtain a crude residue, which was purified by prep-HPLC to yield **1-1-8** as white solids (87.86 mg, yield 25%).

¹H NMR (400 MHz, DMSO-*d*₆) *δ*: 10.29 (d, *J=* 9.6 Hz, 1H), 8.14-7.99 (m, 1H), 7.56-7.27 (m, 2H), 7.10-6.84 (m, 3H), 6.35 (d, *J =* 8.0 Hz, 1H), 4.55-4.27 (m, 4H), 2.61 (s, 3H), 2.57 (s, 3H), 2.08 (s, 3H).

### Example 8. Preparation of compound 1-1-10 of the present invention

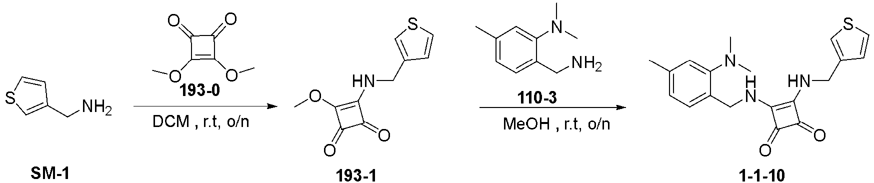

### 1. Synthesis of 193-1

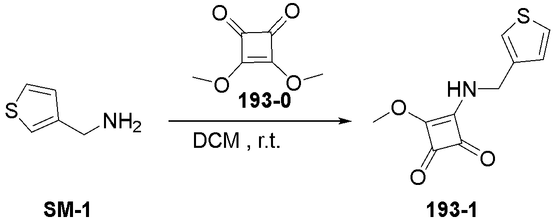

**SM-1** (200 mg, 1.77 mmol) was added to a reaction flask, and then dissolved with dichloromethane (10 mL), to which was added **193-0** (251 mg, 1.77 mmol). The mixture was stirred overnight at room temperature under nitrogen atmosphere. After completion of the reaction, the solvent was removed by evaporation under reduced pressure to obtain the crude product, which was purified by column chromatography [petroleum ether: ethyl acetate (20:1-5:1)] to obtain **193-1** (300 mg, yield 76%) as a yellow oil. MS Calcd.: 224.1 [M+H]⁺; MS Found: 224.3 [M+H]⁺.

### 2. Preparation of compound 110-3

Compound **110-3** was prepared according to the method described in Example 1.

### 3. Synthesis of 1-1-10

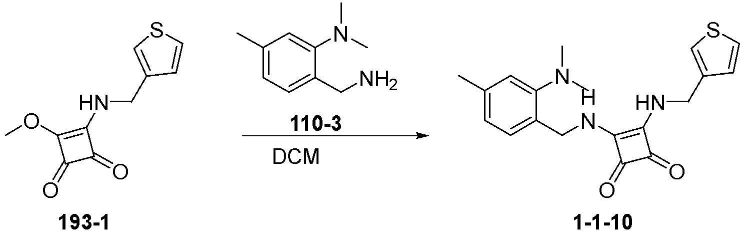

**193-1** (200 mg, 0.90 mmol) was added to a reaction flask, and then dissolved with dichloromethane (5 mL), to which was added **110-3** (147 mg, 0.90 mmol). The mixture was stirred overnight at room temperature under nitrogen atmosphere. After completion of the reaction, the solvent was removed by evaporation under reduced pressure to obtain the crude product, which was purified by normal phase column chromatography [dichloromethane: methanol (100:1-20:1)] to obtain **1-1-10** (110.74 mg, yield 35%) as a yellow oil. MS Calcd.: 356.1 [M+H]⁺; MS Found: 356.4 [M+H]⁺.
¹H NMR (400 MHz, DMSO-_{d*6*}+D₂O) *δ*: 7.54 (dd, *J* = 2.8, 4.8 Hz, 1H), 7.39 (s, 1H), 7.13 (d, *J=* 7.2 Hz, 1H), 7.07 (d, *J=* 4.4 Hz, 1H), 6.99 (s, 1H), 6.89 (d, *J=* 7.6 Hz, 1H), 4.71 (s, 4H), 2.61 (s, 6H), 2.27 (s, 3H)_{∘}

### Example 9. Preparation of compound 1-1-1-A of the present invention

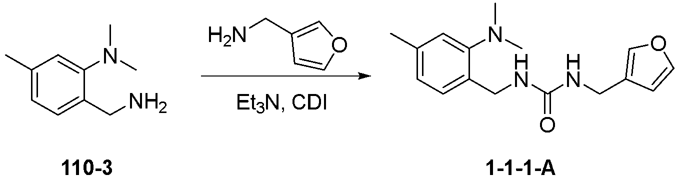

### 1. Preparation of compound 110-3

Compound **110-3** was prepared according to the method described in Example 1.

### 2. Preparation of 1-1-1-A

To a solution of **110-3** (164 mg, 1.0 mmol) in dichloromethane (10 mL), were added CDI (243 mg, 1.5 mmol) and triethylamine (500 mg, 5.0 mmol). The mixture was stirred at room temperature for 2 h, followed by addition of furan-3-ylmethylamine (100 mg, 1.0 mmol). The mixture was stirred overnight at room temperature. After completion of the reaction, the combined organic phases were concentrated under reduced pressure to obtain the crude product, which was purified by prep-HPLC to yield **1-1-1-A** (139.88 mg, yield 49%) as white solids.

¹H NMR (400 MHz, DMSO-*d*₆) *δ*: 7.59 (s, 1H), 7.50 (s, 1H), 7.08 (d, *J* = 7.6 Hz, 1H), 6.89 (s, 1H), 6.81 (d, *J =* 7.6 Hz, 1H), 6.40 (s, 1H), 6.22 (t, *J =* 6.8 Hz, 1H), 4.22 (t, *J* = 6.0 Hz, 2H), 4.04 (t, *J=* 5.6 Hz, 2H), 2.60 (s, 6H), 2.25 (s, 3H).

### Example 10. Preparation of compound 1-1-27 of the present invention

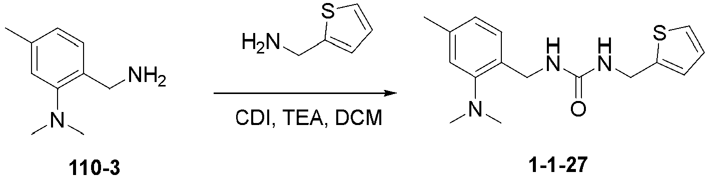

### 1. Preparation of compound 110-3

Compound **110-3** was prepared according to the method described in Example 1.

### 2. Preparation of 1-1-27

**110-3** (328 mg, 2.0 mmol) and triethylamine (606 mg, 6.0 mmol) were dissolved in dichloromethane (20 mL), to which was added N,N'-carbonyldiimidazole (389 mg, 2.4 mmol) at room temperature, and then the mixture was stirred for 2 h at room temperature, followed by addition of 2-thiophenemethylamine (226 mg, 2.0 mmol). The reaction was stirred at room temperature overnight. After completion of the reaction, the reaction solution was evaporated under reduced pressure to remove the solvent and obtain the crude product, which was purified by silica gel column chromatography (DCM/MeOH=40/1) and reversed-phase prep-HPLC to obtain compound 1-1-27 (100 mg, yield 33%).

¹H NMR (400 MHz, DMSO-*d₆*) *δ* 7.36 (dd, *J =* 2.8, 4.8 Hz, 1H), 7.09 (d, *J=* 7.6 Hz, 1H), 6.94 (t, *J =* 3.6 Hz, 1H), 6.89 (s, 1H), 6.81 (d, *J* = 7.6 Hz, 1H), 6.52 (t, *J* = 5.6 Hz, 1H), 6.29 (t, *J* = 6.0 Hz, 1H), 4.38 (d, *J* = 6.0 Hz, 2H), 4.23 (t, *J =* 6.0 Hz, 2H), 2.60 (s, 6H), 2.25 (s, 3H).

### Example 11. Preparation of compound 1-1-64 of the present invention

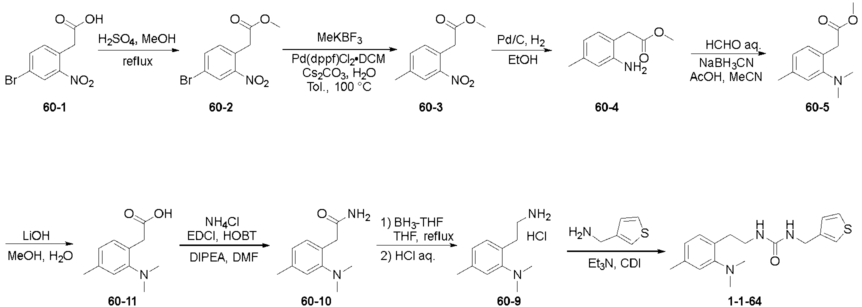

### 1. Synthesis of 60-2

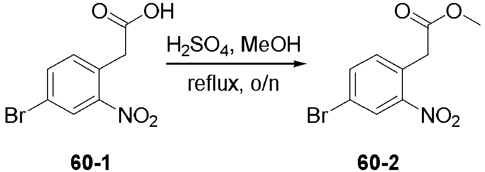

To a solution of 2-(4-bromo-2-nitrophenyl)acetic acid (60-1, 2.59 g, 10 mmol) in methanol (50 mL), was added 5 mL of concentrated sulfuric acid, and the mixture was heated under reflux for 20 h. After completion of the reaction, 100 mL of water was slowly added to the reaction system for quenching, followed by extraction with ethyl acetate three times. The combined organic layers were dried over anhydrous sodium sulfate, filtered, and concentrated to obtain a residue. The crude product was purified by column chromatography (PE/EA=5/1) to yield methyl 2-(4-bromo-2-nitrophenyl)acetate **(60-2,** 2.3 g, 84%) as light yellow solid.

### 2. Synthesis of 60-3

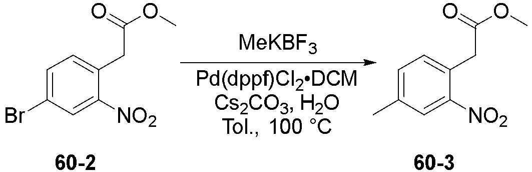

To a solution of **60-2** (2.3 g, 8.4 mmol), MeKBF₃ (4.1 g, 33.6 mmol), and cesium carbonate (8.2 g, 25.2 mmol) in toluene/water mixture (80/8 mL), was added Pd(dppf)Cl₂•DCM (680 mg, 0.84 mmol), and the mixture was stirred at 100 °C for 6 h. After completion of the reaction, 50 mL of 1N aqueous hydrochloric acid was slowly added to the reaction system for quenching, followed by extraction with ethyl acetate three times. The combined organic layers were dried over anhydrous sodium sulfate, filtered, and concentrated to obtain a residue. The crude product was purified by column chromatography (PE/EA=5/1) to yield **60-3** as a light yellow oil (2.3 g, 84%).

### 3. Synthesis of 60-4

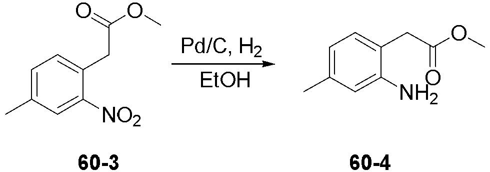

To a solution of **60-3** (1.4 g, 6.7 mmol) in ethanol (100 mL), was added 10% Pd/C (280 mg), and the mixture was stirred under hydrogen atmosphere at room temperature for 6 h. After completion of the reaction, the reaction mixture was filtered and concentrated to obtain a light yellow oily substance **60-4** (1.1 g, 92%).

### 4. Synthesis of 60-5

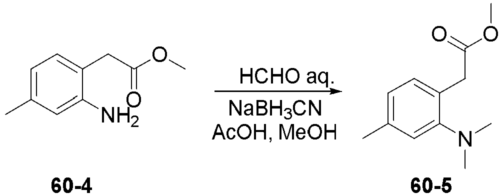

To a solution of **60-4** (1.1 g, 6.15 mmol), 30% formaldehyde (aqueous solution) (2.77 g, 36.9 mmol), and 5 mL acetic acid in methanol (30 mL), was added sodium cyanoborohydride (775 mg, 12.3 mmol), and then the reaction was stirred at room temperature for 4 h. After completion of the reaction, 50 mL of water was slowly added to the reaction system for quenching, followed by extraction with ethyl acetate three times. The combined organic layers were dried over anhydrous sodium sulfate, filtered, and concentrated to obtain a residue. The crude product was purified by column chromatography (PE/EA=1/1) to yield 2-(2-(dimethylamino)-4-methylphenyl)acetate **(60-5,** 700 mg, 55%) as a colorless oil.

¹H NMR (400 MHz, DMSO-*d*₆) *δ*: 7.07 (d, *J=* 7.6 Hz, 1H), 6.96 (s, 1H), 6.83 (dd, *J* = 7.6, 0.8 Hz, 1H), 3.63 (s, 2H), 3.58 (s, 3H), 2.53 (s, 6H), 2.26 (s, 3H).

### 5. Synthesis of 60-11

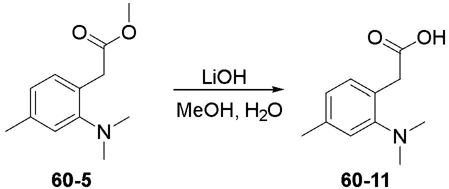

To a solution of **60-5** (700 mg, 3.38 mmol) in methanol/water (12/3 mL), lithium hydroxide (425 mg, 10.14 mmol) was added, and the mixture was stirred at 100 °C for 6 h. After completion of the reaction, 4 N citric acid aqueous solution was slowly added to the reaction system for quenching, followed by concentration to obtain a residue. The crude product was purified by reversed-phase column chromatography (H₂O/MeCN = 3/1) to yield 2-(2-(dimethylamino)-4-methylphenyl)acetic acid **(60-11,** 600 mg, 92%) as a colorless oil.

### 6. Synthesis of 60-10

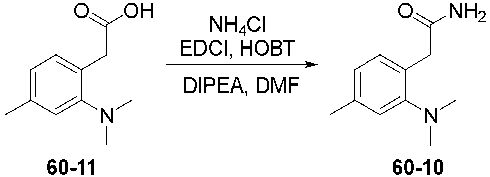

To a solution of **60-11** (600 mg, 3.11 mmol), ammonium chloride (830 mg, 15.55 mmol), and DIPEA (1.7 g, 12.44 mmol) in DMF (30 mL), were added EDCI (886 mg, 4.67 mmol) and HOBT (610 mg, 4.67 mmol), and then the reaction was stirred overnight at room temperature. After completion of the reaction, ethyl acetate and water were added to the reaction system for dilution, followed by extraction with ethyl acetate. The combined organic layers were dried over anhydrous sodium sulfate, filtered, and concentrated *in vacuum* to obtain a residue. The crude product was purified by column chromatography (DCM/MeOH=20/1) to yield 2-(2-(dimethylamino)-4-methylphenyl)acetamide **(60-10,** 450 mg, 75%) as white solids.

¹H NMR (400 MHz, DMSO-*d*₆) *δ*: 7.35 (s, 1H), 7.06 (d, *J =* 7.6 Hz, 1H), 6.91 (s, 1H), 6.87 (s, 1H), 3.63 (s, 2H), 6.79 (dd, *J* = 7.6, 0.8 Hz, 1H), 3.42 (s, 2H), 2.58 (s, 6H), 2.25 (s, 3H).

### 7. Synthesis of 60-9

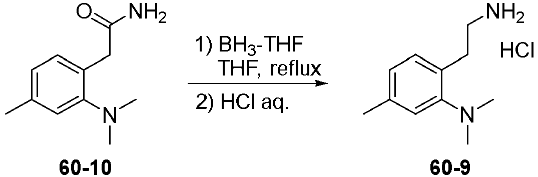

At 0 °C, under nitrogen protection, to a solution of 60-10 (192 mg, 1.0 mmol) in tetrahydrofuran (10 mL), was added BH₃-THF (1.0 N in THF, 4.0 mL), and then the reaction was stirred at 70 °C for 16 h. After completion of the reaction, 2 mL of 1 N hydrochloric acid solution was slowly added to the reaction system for quenching. After concentration, 2-(2-aminoethyl)-N,N,5-trimethylaniline hydrochloride **(60-9,** 250 mg, 100%) was obtained as grayish-white solid.

### 8. Synthesis of compound 1-1-64

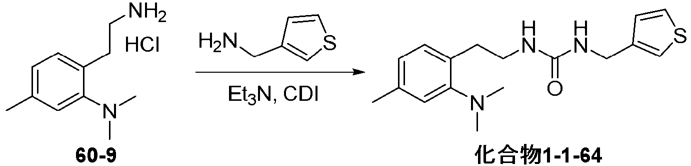

To a solution of **60-9** (108 mg, 0.5 mmol) in DCM (20 mL), were added triethylamine (30 mg, 3.0 mmol), CDI (124.5 mg, 0.75 mmol), and diethyl ether (204 mg, 2.0 mmol), and then the mixture was stirred at room temperature for 2 h, followed by addition of thiophene-3-ylmethylamine (57.5 mg, 0.5 mmol). The mixture was stirred at room temperature overnight. After completion of the reaction, the reaction solution was concentrated *in vacuum* to obtain the crude residue, which was purified by prep-HPLC to yield 1-(2-(dimethylamino)-4-methylphenethyl)-3-(thiophene-3-ylmethyl)urea (1-1-64, 43.60 mg, 27%) as white solids.

¹H NMR (400 MHz, DMSO-*d*₆) *δ*: 7.47-7.45 (m, 1H), 7.20 (d, *J* = 2.0 Hz, 1H), 7.05-6.99 (m, 2H), 6.92 (s, 1H), 6.30 (d, *J= 3.2* Hz, 1H), 6.25 (s, 1H), 5.93 (t, *J* = 5.6 Hz, 1H), 4.17 (t, *J* = 5.2 Hz, 2H), 3.27-3.21 (m, 2H), 2.72-2.69 (m, 2H), 2.58 (s, 6H), 2.25 (s, 3H).

### Example 12. Preparation of compound 1-1-66 of the present invention

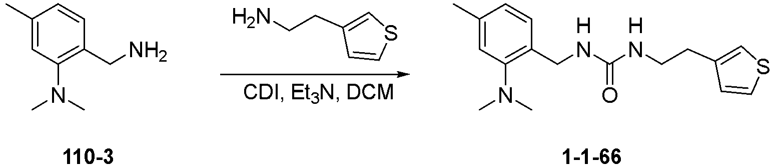

To a solution of 2-(thiophene-3-yl)ethane-1-amine (254 mg, 2.0 mmol) in dichloromethane (20 mL), were added CDI (486 mg, 3.0 mmol) and triethylamine (1.0 g, 10.0 mmol), and then the reaction was stirred at room temperature for 2 h. Then, 2-(aminomethyl)-N,N,5-trimethylaniline **(110-3,** 328 mg, 2.0 mmol) was added, and the mixture was stirred overnight at room temperature. After completion of the reaction, the reaction solution was concentrated *in vacuum* to obtain the crude residue, which was purified by prep-HPLC to yield 1-(2-(dimethylamino)-4-methylbenzyl)-3-(2-(thiophene-3-yl)ethyl)urea (**1-1-66,** 138.87 mg, yield 22%) as white solids.

¹H NMR (400 MHz, DMSO-*d*₆) *δ*: 7.46 (dd, *J* = 4.8, 2.8 Hz, 1H), 7.18-7.17(m, 1H), 7.06 (d, *J* = 8.0 Hz, 1H), 7.01-6.99 (m, 1H), 6.88 (s, 1H), 6.81 (d, *J =* 7.6 Hz, 1H), 6.21 (t, *J* = 5.6 Hz, 1H), 5.97 (t, *J =* 5.6 Hz, 1H), 4.20 (t, *J* = 5.6 Hz, 2H), 3.29-3.24 (m, 2H), 2.70 (t, *J =* 7.2 Hz, 2H), 2.59 (s, 6H), 2.25 (s, 3H).

### Example 13. Preparation of compound 1-1-63 of the present invention

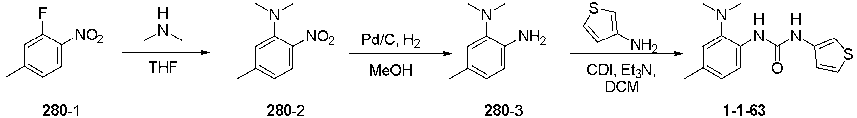

### 1. Synthesis of 280-2

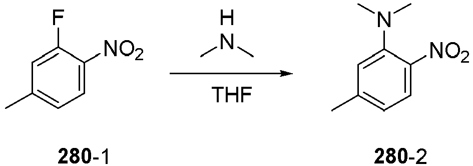

To a 250 mL sealed tube, were added **280-1** (2.2 g, 14.19 mmol) and a solution of dimethylamine in tetrahydrofuran (2N, 60 mL), and then the mixture was allowed to react under reflux for three days. After completion of the reaction, the solvent was removed by evaporation under reduced pressure to obtain the crude product, which was purified by normal phase column chromatography [petroleum ether: ethyl acetate (100:1-10:1)] to obtain **280-2** as a yellow oil (3.2 g, yield 89.9%). MS Calcd.: 181.1 [M+H]⁺; MS Found: 181.4 [M+H] ⁺.

### 2. Synthesis of 280-3

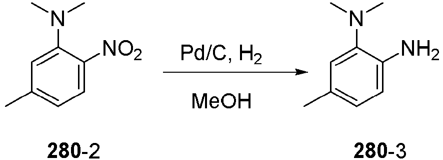

To a reaction flask, was added 280-2 (1.9 g, 10.56 mmol), and then dissolved with methanol (110 mL), to which was added Pd/C (380 mg). The system was purged with hydrogen three times. The reaction was stirred overnight at room temperature under hydrogen atmosphere. After completion of the reaction, the resultant solution was filtered, and the solvent was removed by evaporation under reduced pressure to obtain the crude product, which was purified by normal phase column chromatography [dichloromethane: methanol (100:1-20:1)] to obtain **280-3** as a yellow oil (1.26 g, yield 80%). MS Calcd.: 161.1 [M+H]⁺; MS Found: 161.4 [M+H] ⁺.

### 3. Synthesis of 1-1-63

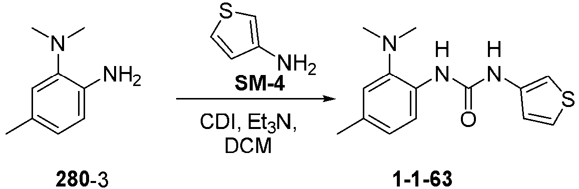

**280-3** (220 mg, 1.08 mmol) was added to a reaction flask, and dissolved with dichloromethane (6 mL), to which were added triethylamine (436 mg, 4.32 mmol) and CDI (262 mg, 1.62 mmol), and then the reaction was stirred at room temperature for 2 h. Then, **SM-4** (122 mg, 1.08 mmol) was added to the system, and the mixture was allowed to react at room temperature overnight. After completion of the reaction, the solvent was removed by evaporation under reduced pressure to obtain the crude product, which was purified by column chromatography [eluent: dichloromethane-methanol (100:1-20:1)] and reversed-phase prep-HPLC to obtain **1-1-63** as white solids (108.79 mg, 34%). MS Calcd.: 276.1[M+H]⁺; MS Found: 276.4 [M + H]⁺.

¹H NMR (400 MHz, DMSO-*d*₆) *δ*: 9.70 (s, 1H), 8.17 (s, 1H), 7.97 (d, *J =* 8.4 Hz, 1H), 7.43 (q, *J =* 13.6 Hz, 1H), 7.27 (q, *J* = 2.0 Hz, 1H), 6.70-6.98 (m, 2H), 6.82 (dd, *J =* 1.2, 8.0 Hz, 1H), 2.59 (s, 6H), 2.23 (s, 3H).

### Example 14. Preparation of compound 1-1-107 of the present invention

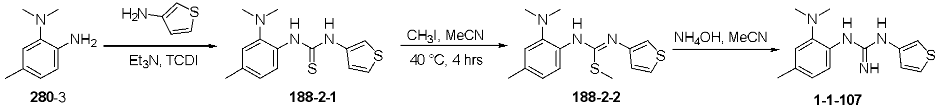

### 1. Synthesis of 188-2-1

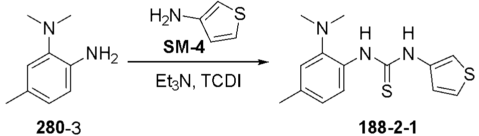

**280-3** (550 mg, 3.67 mmol) was added to a reaction flask, and dissolved with dichloromethane (10 mL), to which were added triethylamine (1.48 g, 14.67 mmol) and TCDI (892 mg, 5.51 mmol), and then the reaction was stirred at room temperature for 2 h. Then, **SM-4** (363 mg, 3.67 mmol) was added to the system, and the mixture was allowed to react at room temperature overnight. After completion of the reaction, the solvent was removed by evaporation under reduced pressure to obtain the crude product, which was purified by column chromatography [eluent: dichloromethane-methanol (100:1-20:1)] and reversed-phase prep-HPLC to obtain **188-2-1** as white solids (710 mg, 66.5%). MS Calcd.: 292.1 [M+H]⁺; MS Found: 292.1 [M + H]⁺.

### 2. Synthesis of 188-2-2

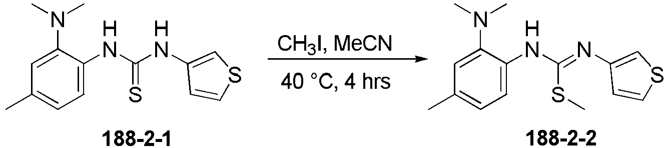

**188-2-1** (570 mg, 1.96 mmol) was added to a sealed tube and dissolved with MeCN (20 mL), to which was then added iodomethane (834 mg, 5.88 mmol). The mixture was stirred at 40 °C for 4 h. After completion of the reaction, the solvent was removed by rotatory evaporation under reduced pressure to obtain the crude product, which was purified by column chromatography [petroleum ether: ethyl acetate (100:1-5:1)] to obtain **188-2-2** as grayish white solid (450 mg, 78%). MS Calcd.: 306.1 [M +H]⁺; MS Found: 306.2 [M +H]⁺.

### 3. Synthesis of 1-1-107

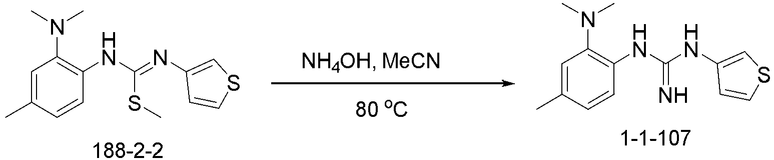

**188-2-2** (430 mg, 1.41 mmol) was added to a sealed tube and dissolved with acetonitrile (15 mL), to which was then added ammonia water (3 mL). The mixture was stirred at 80 °C for 5 h. After completion of the reaction, the solvent was removed by rotatory evaporation under reduced pressure to obtain the crude product, which was purified by column chromatography [dichloromethane: methanol (100:1-5:1)] and prep-HPLC to obtain **1-1-107** as grayish-white solid (112.90 mg, 39.5%). MS Calcd.: 275.1[M+H]⁺; MS Found: 275.4 [M +H]⁺.

¹H NMR (400 MHz, CD3OD) *δ*: 7.38 (dd, *J =* 3.2, 5.2 Hz, 1H), 7.29 (d, *J =* 8.0 Hz, 1H), 6.97 (dd, *J* = 1.6, 2.8 Hz, 2H), 6.93 (dd, *J* = 1.2, 4.8 Hz, 1H), 6.86 (dd, *J* = 1.2, 8.0 Hz, 1H), 2.72 (s, 6H), 2.31 (s, 3H).

### Example 15. Preparation of compound 1-1-188 of the present invention

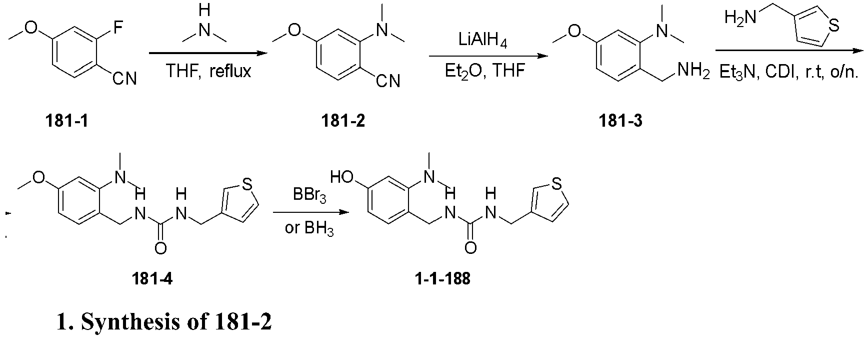

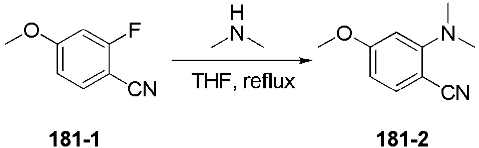

To a 250 mL sealed tube, were added **181-1** (3.0 g, 19.87 mmol) and a solution of dimethylamine in tetrahydrofuran (2N, 100 mL), and then the mixture was stirred at 70 °C for three days. After completion of the reaction, the solvent was removed by rotatory evaporation under reduced pressure to obtain the crude product, which was purified over normal phase column chromatography [petroleum ether: ethyl acetate (20:1-5:1)] to obtain **181-2** as a yellow oil (3.1 g, yield 88.5%). MS Calcd.: 177.1 [M+H]⁺; MS Found: 177.4 [M+H]⁺.

### 2. Synthesis of 181-3

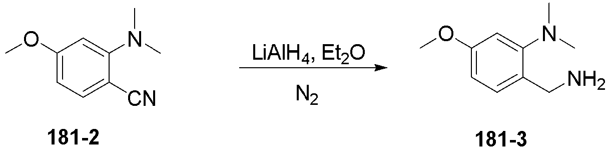

To a reaction flask, were added **181-2** (2.0 g, 20 mmol) and Et₂O (50 mL), and then the system was purged with a nitrogen balloon three times and cooled to 0 °C under nitrogen atmosphere. Lithium aluminum hydride (2.5 N in THF, 16 mL) was then added, and the mixture was stirred at room temperature overnight. After completion of the reaction, water (2 mL), 15% NaOH (2 mL), and water (6 mL) were gradually added, and the resultant solution was stirred at room temperature for 20 min. The reaction solution was then dried over anhydrous magnesium sulfate, and filtered. The solvent was removed by rotatory evaporation under reduced pressure to obtain the crude product, which was purified by normal phase column chromatography [dichloromethane: methanol (100:1-20:1)] to yield **181-3** as a yellow oil (1.6 g, yield 78%). MS Calcd.: 181.1 [M+H]⁺; MS Found: 181.2 [M+H] ⁺.

### 3. Synthesis of 181-4

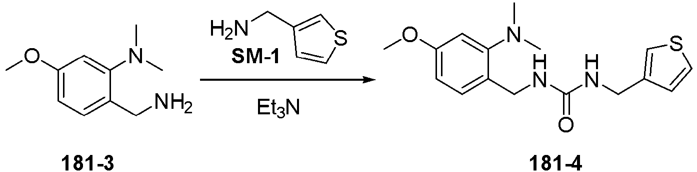

**181-3** (420 mg, 2.32 mmol) was added to a reaction flask, and dissolved with dichloromethane (10 mL), to which were added triethylamine (937 mg, 9.28 mmol) and CDI (564 mg, 3.48 mmol), and then the reaction was stirred at room temperature for 2 h; then, **SM-1** (265 mg, 2.32 mmol) was added to the system, and the mixture was allowed to react at room temperature overnight. After completion of the reaction, the solvent was removed by rotatory evaporation under reduced pressure to obtain the crude product, which was purified by column chromatography [eluent: dichloromethane-methanol (100:1-20:1)] and reversed-phase prep-HPLC to obtain **181-4** as white solids (420 mg, yield 56.5%). MS Calcd.: 320.1[M+H]⁺; MS Found: 320.2 [M + H]⁺.

¹H NMR (400 MHz, DMSO-*d*₆) *δ*: 7.47 (dd, *J* = 2.8, 4.8 Hz, 1H), 7.23 (dd, *J=* 1.2, 3.2 Hz, 1H), 7.11 (q, *J=* 1.4 Hz, 1H), 7.01 (dd, *J=* 1.2, 4.8 Hz, 1H), 6.60-6.58 (m, 2H), 6.36 (t, *J=* 6.0 Hz, 1H), 6.22 (t, *J* = 6.0 Hz, 1H), 4.19 (dd, *J=* 6.0 Hz, 1H), 6.23 (t, *J=* 2.4, 5.6 Hz, 4H), 3.72 (s, 3H), 2.60 (s, 6H).

### 4. Synthesis of 1-1-188

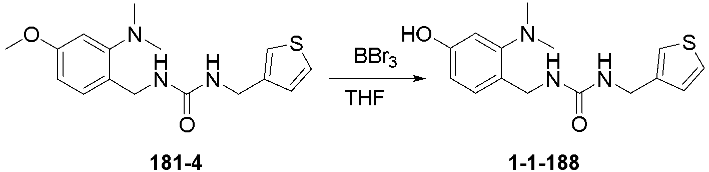

**181-4** (220 mg, 0.69 mmol) was added to a reaction flask, and dissolved with tetrahydrofuran (10 mL). The system was purged with a nitrogen balloon three times, and cool to 0 °C under nitrogen atmosphere. Then, boron tribromide (2.07 mmol, 2.0 N in THF, 1.04 mL) was added, and the resultant solution was stirred at room temperature for 3 h. After completion of the reaction, the reaction solution was slowly poured into water (10 mL). Then, the pH was adjusted to 9 with saturated NaHCO₃ solution. The resultant solution was extracted three times with dichloromethane (20 mL). The organic phase was washed once with saturated saline. The organic phase was separated, dried over anhydrous magnesium sulfate, and filtered. The solvent was removed by evaporation under reduced pressure to obtain the crude product, which was purified by normal phase column chromatography [dichloromethane: methanol (100:1-20:1)] to obtain 1-1-188 as a yellow oil (51.37 mg, yield 24.7%). MS Calcd.: 306.1 [M+H]⁺; MS Found: 306.2 [M+H]⁺.

¹H NMR (400 MHz, DMSO-*d*₆) *δ*: 9.19 (s, 1H), 7.47 (dd, *J* = 4.8, 2.8 Hz, 1H), 7.22 (dd, *J=* 2.4, 0.8 Hz, 1H), 7.02-6.98 (m, 2H), 6.47 (d, *J =* 2.4 Hz, 1H), 6.40 (dd, *J =* 2.4, 8.4 Hz, 1H), 6.33 (t, *J* = 5.6 Hz, 1H), 6.15 (t, *J* = 5.6 Hz, 1H), 4.19 (d, *J* = 6.0 Hz, 2H), 4.15(d, *J* = 6.0 Hz, 2H), 2.56 (s, 6H).

### Example 16. Preparation of compound 1-1-177 of the present invention

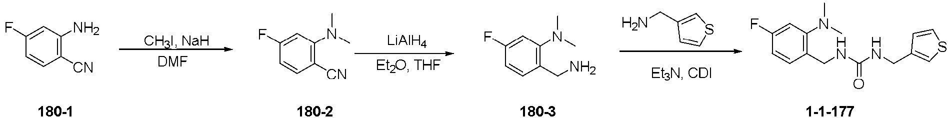

### 1. Synthesis of 180-2

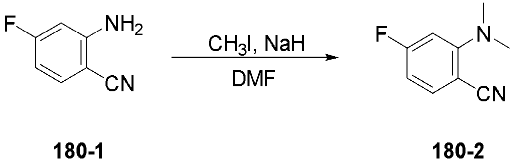

To a 100 mL reaction flask, was added **180-1** (1.0 g, 7.41 mmol), and then dissolved by adding DMF (10 mL). At 0 °C, NaH (1.0 g, 7.41 mmol) was added, and the mixture was allowed to react at room temperature for 2 h. Then, CH₃I (1.0 g, 7.41 mmol) was added, and the reaction was stirred at room temperature overnight. After completion of the reaction, the reaction solution was slowly poured into ice water, and extracted three times with dichloromethane (40 mL). The organic phase was dried over anhydrous magnesium sulfate, and filtered. The solvent was removed by rotatory evaporation under reduced pressure to obtain the crude product, which was purified by normal phase column chromatography [petroleum ether: ethyl acetate (100:1-10:1)] to obtain **180-2** as a brownish yellow oil (1.2 g, yield 81%). MS Calcd.: 165.1 [M+H]⁺; MS Found: 165.4 [M+H]⁺.

### 2. Synthesis of 180-3

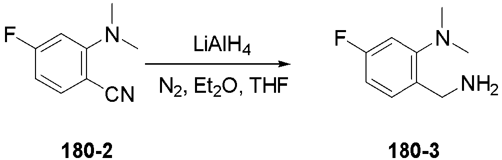

To a reaction flask, were added **180-2** (602 mg, 3.01 mmol) and diethyl ether (20 mL). The system was purged with a nitrogen balloon three times and cooled to 0 °C under nitrogen atmosphere. Then, lithium aluminum hydride (2.5 N in THF, 2.4 mL) was added, and the reaction was stirred at room temperature overnight. After completion of the reaction, water (1 mL), 15% NaOH (3 mL), and water (3 mL) were gradually added. The resultant solution was stirred at room temperature for 20 min, and then dried over anhydrous magnesium sulfate, and filtered. The solvent was removed by rotatory evaporation under reduced pressure to obtain the crude product, which was purified by normal phase column chromatography [dichloromethane: methanol (100:1-20:1)] to yield **180-3** (2.0 g, yield 61%) as a yellow oil.

### 3. Synthesis of 1-1-177

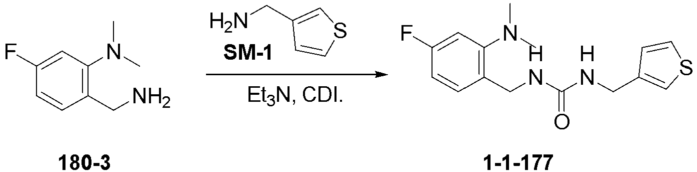

**180-3** (220 mg, 1.08 mmol) was added to a reaction flask, and dissolved with dichloromethane (10 mL), to which were added triethylamine (436 mg, 4.32 mmol) and CDI (262 mg, 1.62 mmol), and then the reaction was stirred at room temperature for 2 h. Then, **SM-1** (122 mg, 1.08 mmol) was added to the system, and the mixture was allowed to react at room temperature overnight. After completion of the reaction, the solvent was removed by rotatory evaporation under reduced pressure to obtain the crude product, which was purified by column chromatography [eluent: dichloromethane-methanol (100:1-20:1)] and reversed-phase prep-HPLC to obtain **1-1-177** as white solids (125.06 mg, yield 34%). MS Calcd.: 344.2 [M+H]⁺; MS Found: 344.4 [M + H]⁺.

¹H NMR (400 MHz, DMSO-d₆) *δ*: 7.45 (dd, *J=* 3.2, 5.2 Hz, 1H), 7.21-7.16 (m, 2H), 7.16 (d, *J* = 7.6 Hz, 1H), 6.99 (dd, *J* = 0.8, 4.8 Hz, 1H), 6.86-6.76 (m, 2H), 6.37 (t, *J* = 5.6 Hz, 1H), 6.31 (t, *J =* 6.0 Hz, 1H), 4.19 (dd, *J* = 6.0, 9.2 Hz, 4H), 2.60 (s, 6H).

### Example 17. Preparation of compound 1-1-191 of the present invention

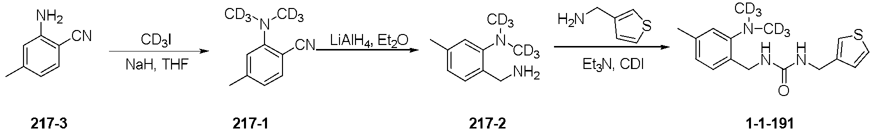

### 1. Synthesis of 217-1

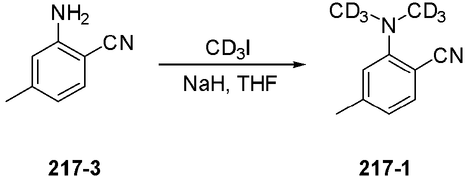

To a solution of 2-amino-4-methylbenzonitrile (**217-3,** 660 mg, 5.0 mmol) in 50 mL of tetrahydrofuran, 60% sodium hydride (440 mg, 11.0 mmol) was added. After stirring at room temperature for half an hour, deuterated iodomethane (1.45 g, 10.0 mmol) was added, and the mixture was stirred overnight at room temperature. After completion of the reaction, water was gradually added to the reaction system for quenching, followed by extraction with ethyl acetate. The combined organic layers were dried over anhydrous sodium sulfate, filtered, and concentrated to obtain a residue. The crude product was purified by column chromatography (DCM/MeOH=3/1) to yield 2-(bis(methyl-d3)amino)-4-methylbenzonitrile (**217-1**, 680 mg, yield 82%) as a yellow oil.

### 2. Synthesis of 217-2

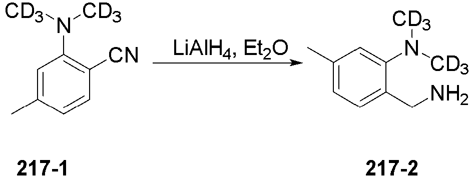

At 0 °C, under nitrogen protection, lithium aluminum hydride (2.5 N in THF, 1.6 mL) was added to a solution of 217-1 (332 mg, 2.0 mmol) in 20 mL of diethyl ether, and the reaction was stirred at room temperature for 16 h. After completion of the reaction, the reaction was quenched by slowly adding 0.1 mL of water, 0.1 mL of 15% NaOH solution, and 0.3 mL of water to the reaction system in sequence. The resultant solution was then filtered and concentrated to obtain a residue. The crude product was purified by flash column chromatography on silica gel (DCM: MeOH = 20:1) to yield 2-(aminomethyl)-5-methyl-N,N-dimethyl(d₃)aniline (217-2, 250 mg, yield 74%) as a yellow oil.

### 3. Synthesis of 1-1-191

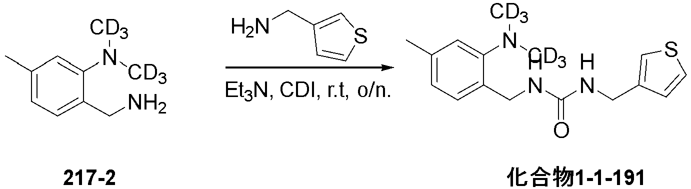

To a solution of 217-2 (340 mg, 2.0 mmol) in 20 mL of dichloromethane, were added CDI (486 mg, 3.0 mmol) and triethylamine (1.0 g, 10.0 mmol), and then the reaction was stirred at room temperature for 2 h. Then, thiophene-3-ylmethylamine (226 mg, 2.0 mmol) was added, and the mixture was stirred at room temperature overnight. After completion of the reaction, the reaction solution was concentrated to obtain a crude residue, which was purified by prep-HPLC to yield 1-((1H-imidazol-2-yl)methyl)-3-(2-(dimethyl(d₃)amino)-4-methylbenzyl)urea as white solids (**1-1-191,** 187 mg, yield 30%).

¹H NMR (400 MHz, DMSO-d₆) *δ*: 7.46-7.45 (dd, *J* = 2.8, 4.8 Hz, 1H), 7.20 (dd, *J* = 2.8, 1.2 Hz, 1H), 7.05 (d, *J=* 8.0 Hz, 1H), 6.99 (dd, *J* = 4.8, 1.2 Hz, 1H), 6.85 (s, 1H), 6.78 (d, *J* = 8.0 Hz, 1H), 6.35 (t, *J=* 5.6 Hz, 1H), 6.22 (t, *J=* 6.0 Hz, 1H), 4.19 (dd, *J =* 6.0, 10.4 Hz, 1H), 2.23 (s, 3H).

### Example 18. Preparation of compound 1-2-1 of the present invention

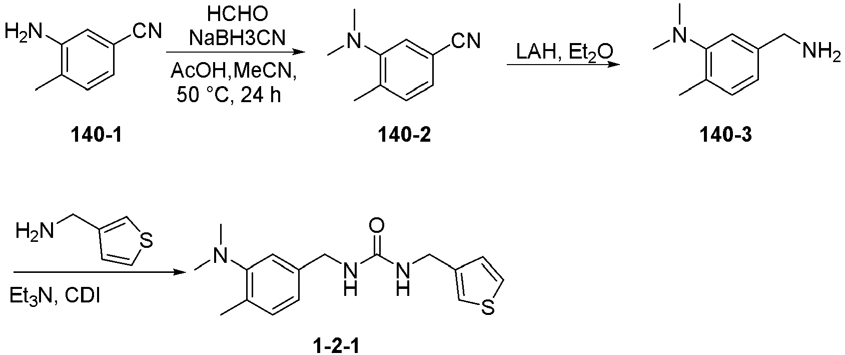

### 1. Synthesis of 140-2

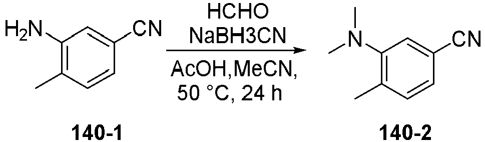

To a reaction flask, were added **140-1** (3.0 g, 22.73 mmol), aqueous formaldehyde solution (8.5 g, 113.65 mmol), glacial acetic acid (5 mL), and methanol (45 mL). Under nitrogen protection, sodium cyanoborohydride (7.2 g, 113.65 mmol) was added, and then the system was purged with a nitrogen balloon three times. The mixture was stirred and reacted at room temperature under nitrogen atmosphere overnight. After completion of the reaction, the reaction solution was poured into water (100 mL), and extracted with dichloromethane (100 mL) three times. The organic phase was washed with saturated saline (50 mL x 2), and then dried over anhydrous sodium sulfate. The solvent was removed by rotatory evaporation under reduced pressure to obtain the crude product, which was purified by reversed-phase column chromatography to obtain **140-2** as a yellow oil (3.2 g, yield 90%). MS Calcd.: 161.1 [M+H]⁺; MS Found: 161.4 [M+H]⁺.

### 2. Synthesis of 140-3

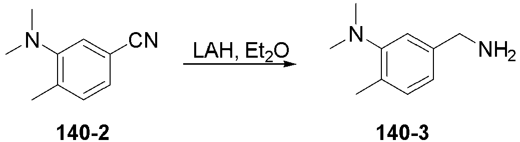

To a reaction flask, were added **140-2** (600 mg, 3.75 mmol) and Et₂O (20 mL). The system was purged with a nitrogen balloon three times and cooled to 0 °C under nitrogen atmosphere. Then, lithium aluminum hydride (4.8 g , 22.0 mmol) was added, and the reaction was stirred at room temperature overnight. After completion of the reaction, water (1 mL), 15% NaOH (3 mL), and water (3 mL) were gradually added. The resultant solution was stirred at room temperature for 20 min, and then dried over anhydrous magnesium sulfate, and filtered. The solvent was removed by rotatory evaporation under reduced pressure to obtain the crude product, which was purified by normal phase column chromatography to yield 140-3 (420 mg, 68%) as a yellow oil. MS Calcd.: 165.1 [M+H]⁺; MS Found: 165.4 [M +H]⁺.

### 3. Synthesis of compound 1-2-1

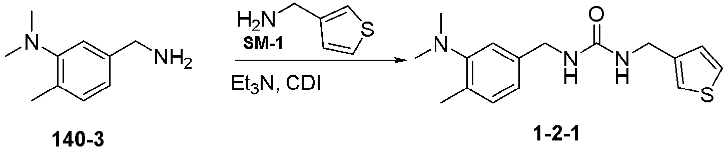

**SM-1** (151 mg, 1.34 mmol) was added to a reaction flask, and dissolved with dichloromethane (10 mL), to which were added triethylamine (271 mg, 2.68 mmol) and CDI (326 mg, 2.01 mmol), and then the reaction was stirred at room temperature for 2 h. Then, **140-3** (220 mg, 1.34 mmol) was added to the system, and the mixture was allowed to react at room temperature overnight. After completion of the reaction, the solvent was removed by rotatory evaporation under reduced pressure to obtain the crude product, which was purified by column chromatography [eluent: dichloromethane-methanol (100:1-20:1)] and reversed-phase prep-HPLC to obtain **1-2-1** as white solids (83.75 mg, 21%). MS Calcd.: 304.1 [M+H]⁺; MS Found: 304.2 [M + H]⁺.

¹H NMR (400 MHz, DMSO-d₆) *δ*: 7.44 (dd, *J* = 2.8, 4.8 Hz, 1H), 7.19 (dd, *J* = 1.2, 2.8 Hz, 1H), 7.04-6.99 (m, 2H), 6.89 (d, *J =* 1.2 Hz, 1H), 6.76 (dd, *J=* 1.6, 7.6 Hz, 1H), 6.29 (d, *J =* 4.8 Hz, 2H), 4.18 (d, *J=* 5.6 Hz, 2H), 4.13 (d, *J=* 6.0 Hz, 2H), 2.57 (s, 6H), 2.19 (s, 3H).

### Example 19. Preparation of compound 1-2-2 of the present invention

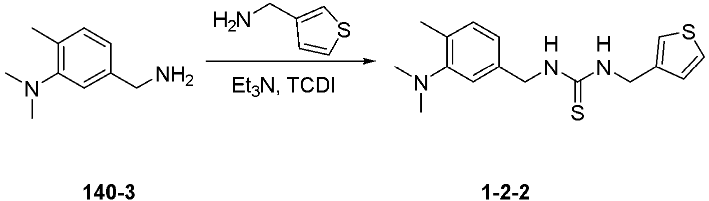

### 1. Synthesis of 140-3

Compound **140-3** was synthesized with reference to Example 18.

### 2. Synthesis of compound 1-2-2

To a solution of 5-(aminomethyl)-N,N,2-trimethylaniline **(140-3,** 492 mg, 3.0 mmol) in 20 mL of dichloromethane, TCDI (1.07 g, 3.6 mmol) and triethylamine (1.25 g, 12 mmol) were added. The reaction was stirred at room temperature for 2 h, followed by addition of thiophene-3-ylmethylamine (339 mg, 3.0 mmol). The mixture was then stirred overnight at room temperature. After completion of the reaction, the reaction solution was concentrated to obtain a crude residue, which was purified by prep-HPLC to yield a grayish-white solid, 1-(3-(dimethylamino)-4-methylbenzyl)-3-(thiophene-3-ylmethyl)thiourea (compound 1-2-2, 500 mg, yield 52%).

### Example 20. Preparation of compound 1-2-3 of the present invention

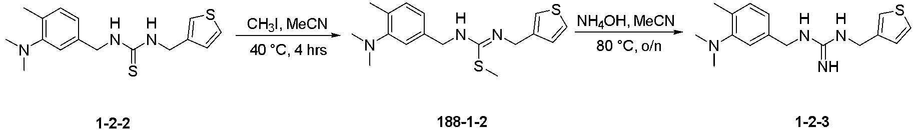

### 1. Synthesis of compound 1-2-2

Compound **1-2-2** was synthesized with reference to Example 19.

### 2. Synthesis of compound 188-1-2

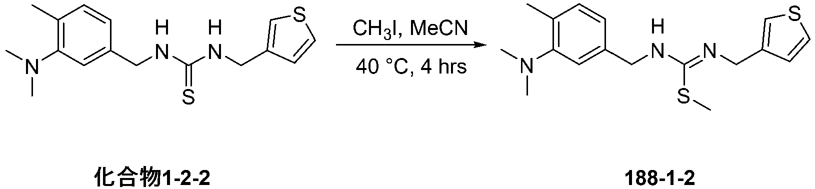

To a solution of compound **1-2-2** (319 mg, 1 mmol) in 20 mL of acetonitrile, was added iodomethane (800 mg, 6 mmol), and then the mixture was stirred at 40 °C for 4 h. After completion of the reaction, the reaction solution was concentrated to obtain a residue. The crude product was purified by column chromatography (DCM/MeOH=30/1) to yield methyl (Z)-N-(3-(dimethylamino)-4-methylbenzyl)-N'-(thiophen-3-ylmethyl)aminothiocarbamate **(188-1-2,** 200 mg, yield 60%) as a pale yellow oil.

### 3. Synthesis of compound 1-2-3

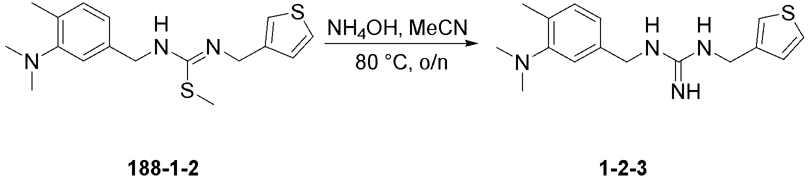

To a solution of **188-1-2** (200 mg, 0.6 mmol) in 10 mL of acetonitrile, was added ammonia water (600 mg, 6.0 mmol), and then the mixture was stirred at 80 °C overnight. After completion of the reaction, the reaction solution was concentrated to obtain a crude residue, which was purified by prep-HPLC to yield1-(3-(dimethylamino)-4-methylbenzyl)-3-(thiophen- 3-ylmethyl)guanidine as white solids (compound **1-2-3,** 54.13 mg, yield 32%). MS Calcd.: 303.2 [M+H]⁺; MS Found: 303.2 [M + H]⁺.

¹H NMR (400 MHz, DMSO-*d*₆) *δ*: 7.50-7.48 (m, 3H), 7.27 (s, 1H), 7.07 (d, *J =* 7.6 Hz, 1H), 7.02 (d, *J* = 4.8 Hz, 1H), 6.96 (s, 1H), 6.81 (d, *J* = 7.6 Hz, 1H), 4.31 (s, 2H), 4.25 (s, 2H), 2.60 (s, 6H), 2.19 (s, 3H).

### Example 21. Preparation of compound 1-2-119 of the present invention

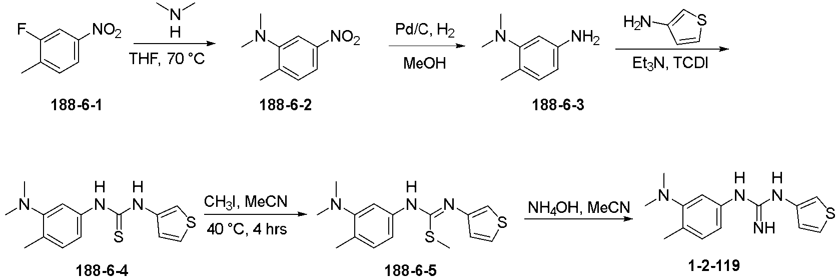

### 1. Synthesis of 188-6-2

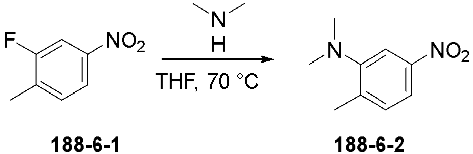

To a 250 mL sealed tube, were add **188-6-1** (3.0 g, 19.35 mmol) and a solution of dimethylamine in tetrahydrofuran (2N, 100 mL), and then the reaction was allowed to react at 70 °C overnight. After completion of the reaction, the solvent was removed by rotatory evaporation under reduced pressure to obtain the crude product, which was purified by normal phase column chromatography [petroleum ether: ethyl acetate (20:1-5:1)] to obtain **188-6-2** as a yellow oil (3.0 g, yield 86%). MS Calcd.: 181.1 [M+H]⁺; MS Found: 181.3 [M+H]⁺.

### 2. Synthesis of 188-6-3

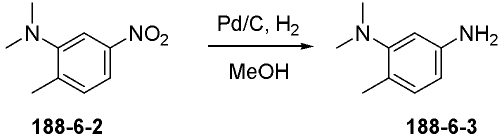

To a reaction flask, was added **188-6-2** (3.0 g, 16.67 mmol), and then dissolved with methanol (170 mL), to which was added Pd/C (600 mg). The system was purged with a hydrogen balloon three times, and stirred at room temperature under hydrogen atmosphere overnight. After completion of the reaction, the reaction solution was filtered. The solvent was removed by rotatory evaporation under reduced pressure to obtain the crude product, which was purified by normal phase column chromatography [dichloromethane: methanol (100:1-20:1)] to obtain 188-6-3 as a yellow oil (2.2 g, yield 88%). MS Calcd.: 151.1 [M+H]⁺; MS Found: 151.4 [M+H]⁺.

### 3. Synthesis of 188-6-4

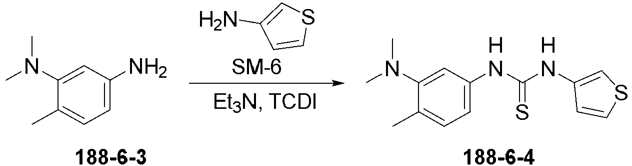

**188-6-3** (450 mg, 3.0 mmol) was added to a reaction flask, and then dissolved with dichloromethane (10 mL), to which were added triethylamine (1.21 g, 12.0 mmol) and TCDI (729 mg, 4.5 mmol), and the reaction was stirred at room temperature for 2 h. Then, **SM-1** (297 mg, 3.0 mmol) was added to the system, and the reaction was allowed to further react at room temperature overnight. After completion of the reaction, the solvent was removed by evaporation under reduced pressure to obtain the crude product, which was purified by column chromatography [eluent: dichloromethane-methanol (100:1-20:1)] and reversed-phase prep-HPLC to obtain **188-6-4** as white solids (610 mg, 69.9%). MS Calcd.: 292.1 [M+H]⁺; MS Found: 292.1 [M + H]⁺.

### 4. Synthesis of 188-6-5

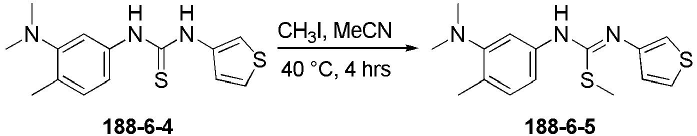

**188-6-4** (450 mg, 1.55 mmol) was added to a sealed tube and dissolved with acetonitrile (10 mL). Then, iodomethane (660 mg, 4.65 mmol) was added, and the mixture was stirred and allowed to react in the sealed tube at 40 °C for 4 h. After completion of the reaction, the solvent was removed by rotatory evaporation under reduced pressure to obtain the crude product, which was subjected to column chromatography [petroleum ether: ethyl acetate (100:1-5:1)] to obtain **188-6-5** as off-white solid (300 mg, 63.6%). MS Calcd.: 306.1 [M+H]⁺; MS Found: 306.1 [M +H]⁺.

### 5. Synthesis of 1-2-119

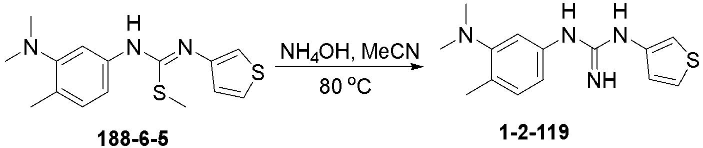

**188-6-5** (350 mg, 1.15 mmol) was added to a sealed tube and dissolved with acetonitrile (12 mL). Then, ammonia water (2 mL) was added, and the reaction was stirred at 80 °C for 5 h. After completion of the reaction, the solvent was removed by rotatory evaporation under reduced pressure to obtain the crude product, which was purified by column chromatography [dichloromethane: methanol (100:1-5:1)] to obtain **1-2-119** as grayish-white solid (76.05 mg, 24%). MS Calcd.: 275.1 [M+H]⁺; MS Found: 275.4 [M +H]⁺.

¹H NMR (400 MHz, DMSO-*d*₆) *δ*: 7.37 (dd, *J =* 3.2, 5.2Hz, 1H), 7.11 (d, *J =* 8.0 Hz, 1H), 7.02 (dd, *J=* 1.2, 2.4 Hz, 1H), 6.97-6.94 (m, 2H), 6.80 (dd, *J* = 2.4, 8.0Hz, 1H), 2.69 (s, 6H), 2.29 (s, 3H).

### Example 22. Preparation of compound 1-1-9 of the present invention

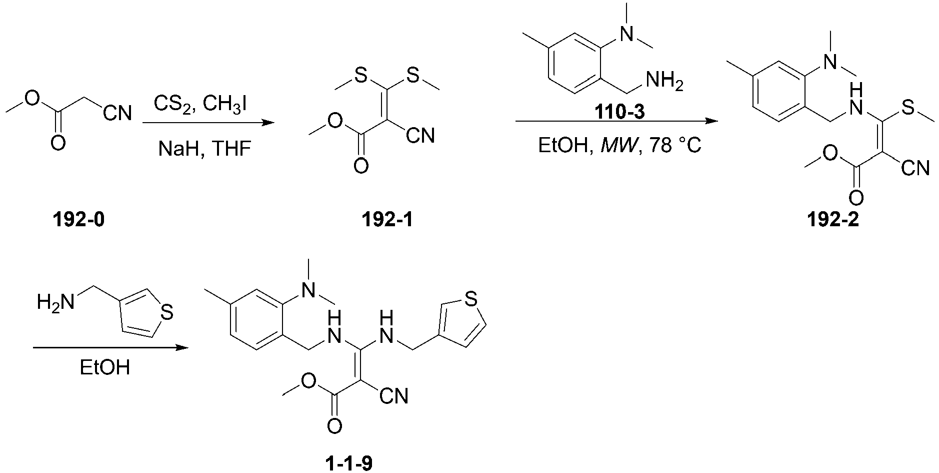

### 1. Synthesis of compound 192-1

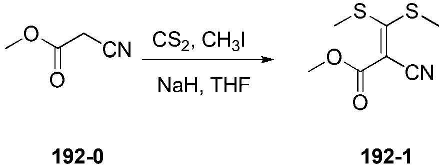

At 0 °C, sodium hydride (2.42 g, 60.60 mmol) was gradually added to tetrahydrofuran (150 mL). Under nitrogen protection, **192-1-4** (3.0 g, 30.30 mmol) was dissolved in tetrahydrofuran (30 mL) and slowly added into the reaction system dropwise. The mixture was stirred at room temperature for 30 min. Then, iodomethane (8.6 g, 60.60 mmol) was gradually added dropwise, and the mixture was allowed to react at room temperature for another 30 min. After completion of the reaction, the reaction solution was slowly poured into ice water, and filtered. The filter cake was washed three times with water (5 mL) to obtain **192-1-2** as brown solid (3.0 g, yield: 48.8%).

### 2. Synthesis of compound 110-3

Compound **110-3** was synthesized by referring to Example 1.

### 3. Synthesis of compound 192-2

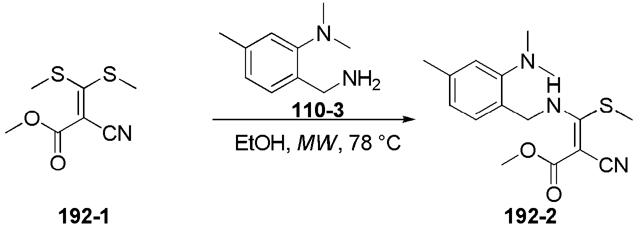

**192-1** (1.0 g, 4.93 mmol) and **110-3** (808 mg, 4.93 mmol) were added to a microwave tube and dissolved with ethanol (20 mL). The mixture was stirred and reacted at 78 °C for 10 min under microwave. After completion of the reaction, the solvent was removed by rotatory evaporation under reduced pressure to obtain the crude product, which was subjected to column chromatography [petroleum ether: ethyl acetate (100:1-5:1)] to obtain 192-2 as grayish-white solid (1.0 g, yield: 63.7%). MS Calcd.: 320.1 [M+H]⁺; MS Found: 320.2 [M +H]⁺.

### 4. Synthesis of compound 1-1-9

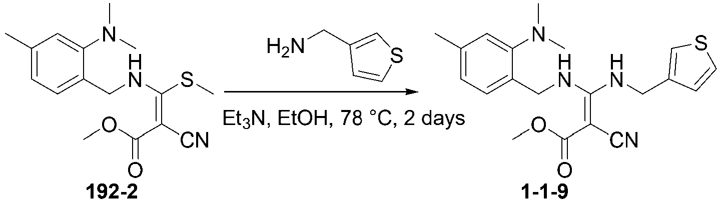

**192-2** (230 mg, 0.72 mmol) and **SM-1** (82 mg, 0.72 mmol) were added to a one-neck flask, and dissolved with ethanol (1 mL), to which was then added triethylamine (218 mg, 2.16 mmol), and the reaction was stirred at 78 °C under nitrogen atmosphere for two days. After completion of the reaction, the solvent was removed by rotatory evaporation under reduced pressure to obtain the crude product, which was subjected to column chromatography [petroleum ether: ethyl acetate (100:1-5:1)] to obtain **1-1-9** as grayish-white solid (420 mg, 68%). MS Calcd.: 385.1 [M+H]⁺; MS Found: 385.4 [M +H]⁺.

¹H NMR (400 MHz, DMSO-*d*₆) *δ*: 8.30-8.22 (m, 2H), 7.56 (dd, *J =* 2.8, 4.8 Hz, 1H), 7.41 (q, *J=* 1.2 Hz, 1H), 7.20 (s, 1H), 7.07 (dd, *J* = 1.6, 5.2 Hz, 1H), 6.97 (s, 1H), 6.88 (d, *J* = 7.6 Hz, 1H), 4.55 (d, *J =* 5.2 Hz, 2H), 4.67 (d, *J =* 4.4 Hz, 2H), 3.73 (s, 3H),6.46 (s, 6H), 2.27 (s, 3H).

### Example 23. Preparation of compound 1-1-1-B of the present invention

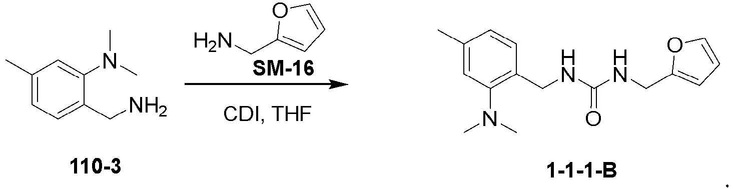

### 1. Synthesis of compound 110-3

Compound **110-3** was synthesized by referring to Example 1.

### 2. Synthesis of compound 1-1-1-B

To a solution of compound **110-3** (300 mg, 1.826 mmol) in tetrahydrofuran (10 mL), was added CDI (296.09 mg, 1.826 mmol), and the reaction was stirred at 25 °C for 0.5 h. Then, **SM-16** (177.39 mg, 1.826 mmol) was added, and the reaction was further stirred at 80 °C for 2.5 h. After completion of the reaction, the reaction solution was concentrated under reduced pressure and purified by triturating in acetonitrile to obtain **1-1-1-B** as off-white solid (157.8 mg, yield 30.07%). MS Calcd.: 288.1 [M+H]⁺; MS Found: 288.1 [M +H]⁺.

¹HNMR (400 MHz, DMSO-*d₆*) *δ* 7.56 (s, 1H), 7.08 (d, *J* = 8.0 Hz, 1H), 6.89 (s, 1H), 6.82 (d, *J= 8.0* Hz, 1H), 6.38 (s, 2H), 6.25 - 6.19 (m, 2H), 4.22 (brs., 4H), 2.60 (s, 6H), 2.25 (s, 3H).

### Example 24. Preparation of compound 1-1-14 of the present invention

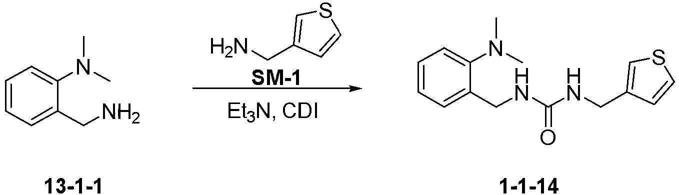

**SM-1** (150 mg, 1.33 mmol) was added to a reaction flask, and dissolved with dichloromethane (10 mL), to which were added triethylamine (537 mg, 5.32 mmol) and CDI (324 mg, 2.00 mmol), and then the reaction was stirred at room temperature for 2 h. Then, **13-1-1** (200 mg, 1.33 mmol) was added to the system, and the mixture was allowed to react at room temperature overnight. After completion of the reaction, the solvent was removed by rotatory evaporation under reduced pressure to obtain the crude product, which was purified by column chromatography [eluent: dichloromethane-methanol (100:1-20:1)] and reversed-phase prep-HPLC to obtain **1-1-14** as white solids (160 mg, yield 41.6%). MS Calcd.: 290.1 [M+H]⁺; MS Found: 290.4 [M + H]⁺.

¹H NMR (400 MHz, DMSO*-d*₆) *δ*: 7.47 (q, *J =* 2.8 Hz, 1H), 7.24-7.17 (m, 3H), 7.08 (d, *J=* 7.2 Hz, 1H), 7.03-6.99 (m, 2H), 6.39 (t, *J=* 5.6 Hz, 1H), 6.29 (t, *J =* 6.0 Hz, 1H), 4.28 (d, *J* = 6.0 Hz, 2H), 4.21 (d, *J=* 6.0 Hz, 2H), 2.61 (s, 6H).

### Example 25. Preparation of compound 1-1-28 of the present invention

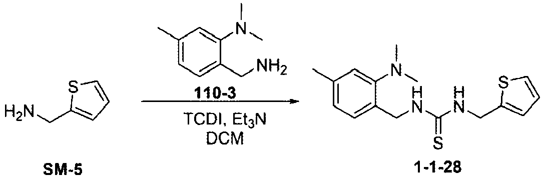

### 1. Synthesis of compound 110-3

Compound **110-3** was synthesized by referring to Example 1.

### 2. Synthesis of compound 1-1-28

**SM-5** (500 mg, 4.39 mmol) was added to a reaction flask, and then dissolved with dichloromethane (20 mL), to which were added triethylamine (1.33 g, 13.16 mmol) and TCDI (781 mg, 4.39 mmol), and the reaction was stirred at room temperature for 2 h. Then, **110-3** (720 mg, 4.39 mmol) was added to the system, and the reaction was stirred and allowed to react at room temperature overnight. After completion of the reaction, the solvent was removed by evaporation under reduced pressure to obtain the crude product, which was purified by silica gel column chromatography [methanol-dichloromethane (0-3%)] to obtain **1-1-28** as light yellow solid (405 mg, 29%). MS Calcd.: 320.1 [M+H]⁺; MS Found: 320.4 [M + H]⁺.

¹H NMR (400 MHz, CD₃OD) *δ* 7.35 (dd, *J=* 0.8, 4.8 Hz, 1H), 7.20 (d, *J=* 7.6 Hz, 1H), 7.06 (s, 1H), 7.02 (d, *J =* 3.2 Hz, 1H), 6.97 (q, *J =* 5.2 Hz, 2H), 4.57 (s, 2H), 4.34 (s, 2H), 2.63 (s, 2H), 2.32 (s, 1H).

### Example 26. Preparation of compound 1-1-29 of the present invention

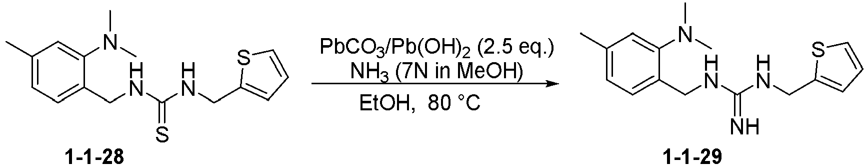

### 1. Synthesis of compound 1-1-28

Compound **110-3** was synthesized by referring to Example 25.

### 2. Synthesis of compound 1-1-29

**1-1-28** (280 mg, 0.88 mmol) was added to the reaction tube, and dissolved with acetonitrile (10 mL), to which were then added 7N ammonia water (5 mL) and lead carbonate (596 mg, 2.19 mmol), and then the mixture was stirred at 80 °C overnight. After completion of the reaction, the solvent was removed by evaporation under reduced pressure to obtain the crude product, which was purified by silica gel column chromatography [methanol/dichloromethane (v/v) = 0-5%] and reversed-phase prep-HPLC to obtain **1-1-29** as white solids (143.92 mg, yield 54%).

¹H NMR (400 MHz, CD₃OD) *δ* 7.35 (dd, *J* = 0.8, 4.8 Hz, 1H), 7.20 (d, *J=* 7.6 Hz, 1H), 7.06 (s, 1H), 7.02 (d, *J=* 3.2 Hz, 1H), 6.97 (q, *J=* 5.2 Hz, 2H), 4.57 (s, 2H), 4.34 (s, 2H), 2.63 (s, 6H), 2.32 (s, 1H).

### Example 27. Preparation of compound 1-1-40 of the present invention

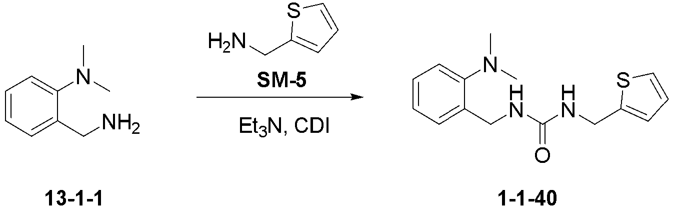

**13-1-1** (300 mg, 2.0 mmol) was added to a reaction flask, and dissolved with dichloromethane (20 mL), to which were added triethylamine (606 mg, 6.0 mmol) and CDI (630 mg, 2.4 mmol), and then the reaction was stirred at room temperature for 2 h. Then, **SM-5** (226 mg, 2.0 mmol) was added to the system, and the mixture was allowed to react at room temperature overnight. After completion of the reaction, the solvent was removed by rotatory evaporation under reduced pressure to obtain the crude product, which was purified by silica gel column chromatography (DCM/MeOH=30/1) and reversed-phase prep-HPLC to obtain **1-1-40** as white solids (156 mg, yield 26%). MS Calcd.: 290.1 [M+H]⁺; MS Found: 290.4 [M + H]⁺.

¹H NMR (400 MHz, DMSO-*d₆*) *δ* 7.36 (dd, *J* = 4.8, 1.2 Hz, 1H), 7.22-7.17 (m, 2H), 7.08 (d, *J* = 7.2 Hz, 1H), 7.02-6.98 (m, 1H), 6.95-6.93 (m, 2H), 6.53 (d, *J* = 6.0 Hz, 1H), 6.34 (d, *J* = 6.0 Hz, 1H), 4.39 (d, *J* = 6.0 Hz, 2H), 4.28 (d, *J* = 5.6 Hz, 2H), 2.61(s, 6H).

### Example 28. Preparation of compound 1-1-41 of the present invention

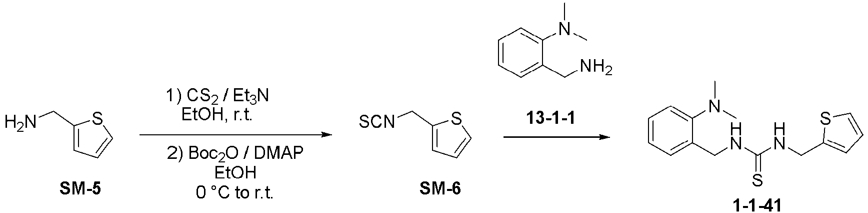

### 1. Synthesis of compound SM-6

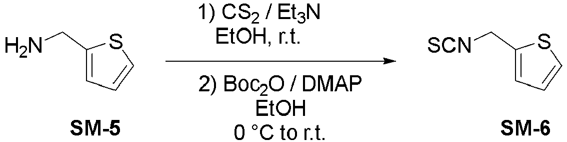

**SM-5** (2.05 mL, 20 mmol, 1.0 equiv.) was dissolved in EtOH (40 mL), to which were then added carbon disulfide (3.61 mL, 60 mmol, 3.0 equiv.) and triethylamine (2.78 mL, 20 mmol, 1.0 equiv.) at room temperature, and the mixture was allowed to react at room temperature for 1 h. Then, DMAP (73 mg, 0.6 mmol, 0.03 equiv.) and Boc₂O (4.6 mL, 20 mmol, 1.0 equiv.) were added at 0 °C, and the reaction solution was warmed to room temperature and reacted overnight. After completion of the reaction, water (50 mL) was added to dilute the solution, and then the resultant solution was extracted with EtOAc (50 mL × 3). The organic phase was dried over anhydrous sodium sulfate, concentrated under reduced pressure, and purified by column chromatography (PE/EtOAc = 40:1) to obtain compound **SM-6** as a yellow oil (2.72 g, yield 88%).

### 2. Synthesis of compound 1-1-41

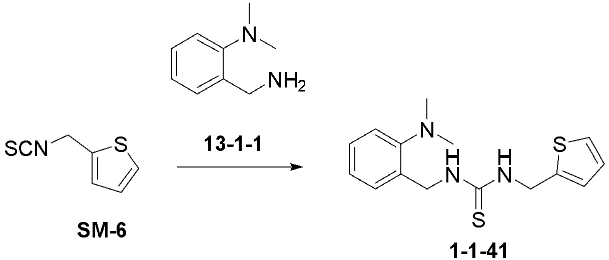

**13-1-1** (180 mg, 1.2 mmol, 1.1 equiv) was added into a 10 mL flask, to which were added toluene (3 mL) and **SM-6** (150 mg, 1.0 mmol, 1.0 equiv), and the reaction was stirred at room temperature for 2 h. The reaction solution was purified by silica gel column chromatography (DCM) to obtain **1-1-41** as white solids (205 mg, 0.67 mmol, yield 66%), HRMS (ESI-TOF) *m*/*z*: [M + H]⁺ Calcd for C₁₅H₁₉N₃S₂H⁺ 306.4655; Found 306.1108.

¹H NMR (400 MHz, CDCl₃) *δ* 7.29 (d, *J* = 7.5 Hz, 1H), 7.26 (d, *J* = 1.9 Hz, 1H), 7.23 (dd, *J* = 4.5, 1.9 Hz, 1H), 7.16-7.05 (m, 2H), 6.95 (d, *J* = 4.6 Hz, 2H), 4.94 (s, 2H), 4.40 (s, 2H), 2.49 (s, 6H).

### Example 29. Preparation of compound 1-1-174 of the present invention

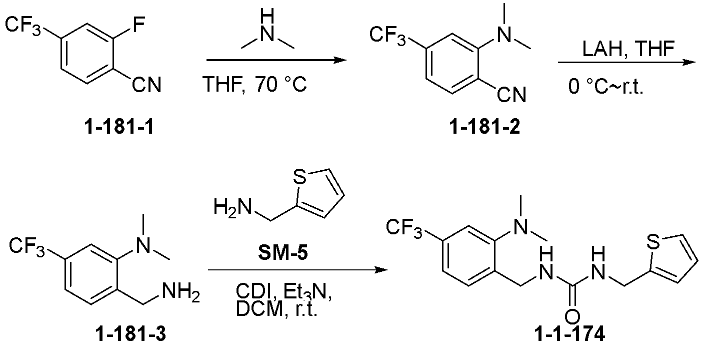

### 1. Synthesis of compound 1-181-2

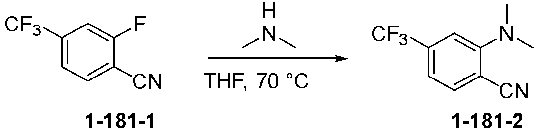

To a 250 mL sealed tube, were added **1-181-1** (2.0 g, 10.58 mmol) and the solution of dimethylamine in tetrahydrofuran (2.0 N in THF, 70 mL), and then the reaction was stirred at 70 °C for three days. After completion of the reaction, the solvent was removed by rotatory evaporation under reduced pressure to obtain the crude product, which was purified by normal phase column chromatography [petroleum ether: ethyl acetate (20:1-5:1)] to obtain **1-181-2** as a yellow oil (2.0 g, yield: 88.0%). MS Calcd.: 215.1 [M+H]⁺; MS Found: 215.3 [M + H]⁺.

### 2. Synthesis of compound 1-181-3

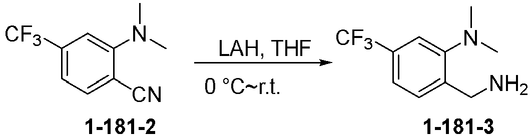

To a reaction flask, were added **1-181-2** (1.0 g, 4.67 mmol) and THF (20 mL). The system was purged with a nitrogen balloon three times and cooled to 0 °C in an ice bath. Then, LAH (2.5 N in THF, 3.74 mL, 9.34 mmol) was slowly added dropwise, and the reaction was stirred at room temperature overnight. After completion of the reaction, the system was cooled to 0 °C, and then water (1 mL), 15% NaOH solution (1 mL), and water (3 mL) were gradually added. The resultant solution was stirred at room temperature for 20 min, and then dried over anhydrous magnesium sulfate, and filtered. The solvent was removed by rotatory evaporation under reduced pressure to obtain the crude product, which was purified by normal phase column chromatography [dichloromethane: methanol (100:1-20:1)] to yield **1-181-3** (700 mg, yiled 70%) as a yellow oil. MS Calcd.: 219.1[M+H]⁺; MS Found: 219.3 [M + H]⁺.

### 3. Synthesis of compound 1-1-174

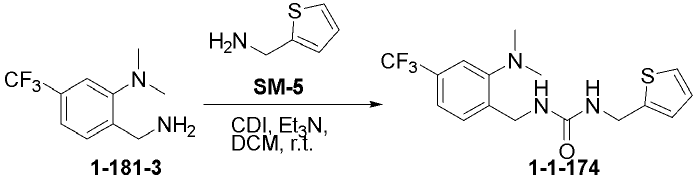

**SM-5** (250 mg, 2.21 mmol) was added to a reaction flask, and dissolved with dichloromethane (10 mL), to which were added triethylamine (670 mg, 6.64 mmol) and CDI (358 mg, 2.21 mmol), and then the reaction was stirred at room temperature for 2 h. Then, **1-181-3** (482 mg, 2.21 mmol) was added to the system, and the mixture was allowed to react at room temperature overnight. After completion of the reaction, the solvent was removed by rotatory evaporation under reduced pressure to obtain the crude product, which was purified by silica gel column chromatography [Eluent: dichloromethane-methanol (100:1-20:1)] and reversed-phase prep-HPLC to obtain **1-1-174** as white solids (166.54 mg, yield 21%). MS Calcd.: 357.1 [M+H]⁺; MS Found: 357.3 [M + H]⁺.

¹H NMR (400 MHz, DMSO-*d₆*) *δ*: 7.42-7.34 (m, 3H), 7.30 (s, 1H), 6.95 (t, *J* = 3.6 Hz, 2H), 6.63 (t, *J* = 6.0 Hz, 1H), 6.52 (t, *J* = 6.0 Hz, 1H), 4.39 (d, *J* = 6.0 Hz, 2H), 4.33 (d, *J* = 5.6 Hz, 1H), 2.68 (s, 6H).

### Example 30. Preparation of compound 1-2-14 of the present invention

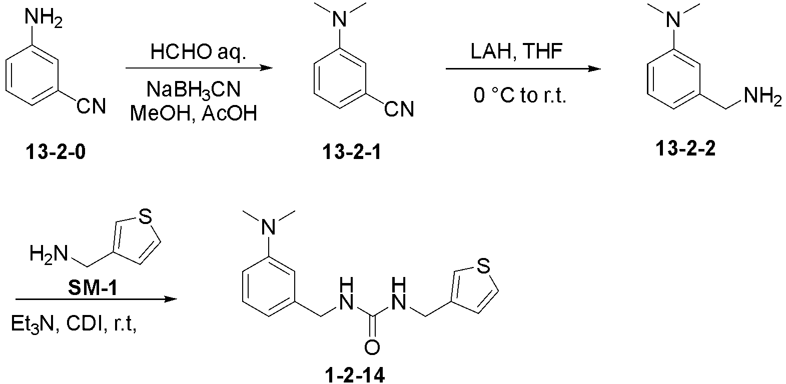

### 1. Synthesis of compound 13-2-1

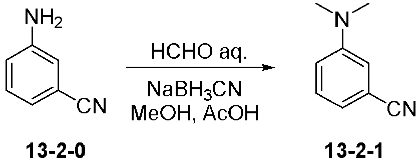

To a solution of **13-2-0** (2.4 g, 20 mmol), formaldehyde aqueous solution (6.0 g, 80 mmol), and acetic acid (5 mL) in methanol (45 mL), was added NaBH₃CN (2.5 g, 40 mmol), and then the reaction solution was stirred at room temperature overnight. After completion of the reaction, 50 mL of saturated saline was added to the reaction system, and the resultant solution was extracted with dichloroethane (3 × 100 mL). The combined organic phase was washed with saturated saline (50 mL), dried over anhydrous sodium sulfate, filtered under suction, and concentrated under reduced pressure to obtain a residue. The crude product was purified by silica gel column chromatography (PE/EA=5/1) to yield compound **13-2-1** as a light yellow oil (1.5 g, yield 51%). MS Calcd.: 147.1 [M+H]⁺; MS Found: 147.2 [M + H]⁺.

### 2. Synthesis of compound 13-2-2

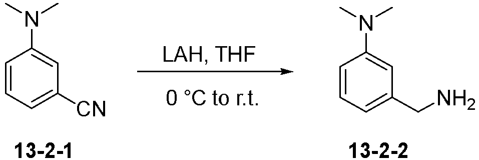

**13-2-1** (1.46 g, 10 mmol) was dissolved in 150 mL of tetrahydrofuran. At 0 °C, 2.5 N lithium aluminum hydride solution (8 mL, 20 mmol) was added dropwise to the reaction system. Then, the reaction solution was warmed to room temperature and stirred overnight. After completion of the reaction, the reaction solution was quenched with water (1.0 mL), 15% aqueous NaOH solution (1.0 mL), and water (3.0 mL). Then, the reaction solution was filtered under suction. The filtrate was concentrated under reduced pressure. The crude product was purified by silica gel column chromatography (DCM/MeOH=20/1) to obtain compound **13-2-2** (900 mg, yield 60%) as a light yellow oil. MS Calcd.: 151.1[M+H]⁺; MS Found: 151.2 [M + H]⁺.

### 3. Synthesis of compound 1-2-14

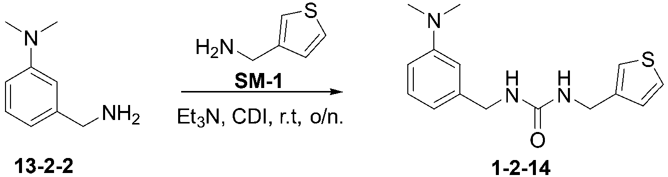

**13-2-2** (300 mg, 2.0 mmol) was added to a reaction flask, and dissolved with dichloromethane (20 mL), to which were added triethylamine (606 mg, 6.0 mmol) and CDI (630 mg, 2.4 mmol), and then the reaction was stirred at room temperature for 2 h. Then, **SM-1** (226 mg, 2.0 mmol) was added to the system, and the mixture was allowed to react at room temperature overnight. After completion of the reaction, the solvent was removed by rotatory evaporation under reduced pressure to obtain the crude product, which was purified by silica gel column chromatography (DCM/MeOH=30/1) and reversed-phase prep-HPLC to obtain **1-2-14** as white solids (159 mg, yield 28%). MS Calcd.: 290.1 [M+H]⁺; MS Found: 290.2 [M + H]⁺.

¹H NMR (400 MHz, DMSO-*d₆*) δ 7.46 (dd, *J* = 4.8, 1.2 Hz, 1H), 7.21 (t, *J* = 3.6 Hz , 1H), 7.10 (t, *J* = 8.0 Hz , 1H), 7.02 (dd, *J* = 5.2, 1.2 Hz, 1H), 6.61-6.55 (m, 3H), 6.33-6.27 (m, 2H), 4.21 (d, *J* = 6.0 Hz, 2H), 4.16 (d, *J* = 5.6 Hz, 2H), 2.86 (s, 6H).

### Example 31. Preparation of compound 1-2-27 of the present invention

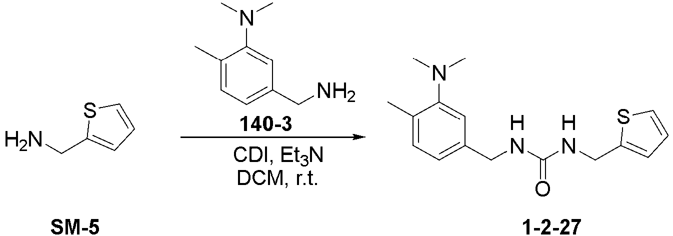

### 1. Synthesis of 140-3

Compound **140-3** was synthesized by referring to Example 18.

### 2. Synthesis of compound 1-2-27

**SM-5** (250 mg, 2.21 mmol) was added to a reaction flask, and dissolved with dichloromethane (20 mL), to which were added triethylamine (670 mg, 6.64 mmol) and CDI (358 mg, 2.21 mmol), and then the reaction was stirred at room temperature for 2 h. Then, **140-3** (362 mg, 2.21 mmol) was added to the system, and the mixture was allowed to react at room temperature overnight. After completion of the reaction, the solvent was removed by rotatory evaporation under reduced pressure to obtain the crude product, which was purified by silica gel column chromatography [Eluent: dichloromethane-methanol (100:1-20:1)] and reversed-phase prep-HPLC to obtain **1-2-27** as white solids (108.79 mg, yield 34%). MS Calcd.: 303.2[M+H]⁺; MS Found: 303.4 [M + H]⁺.

¹H NMR (400 MHz, DMSO-*d*₆) *δ*: 7.36 (q, *J* = 2.0 Hz, 1H), 7.05 (d, *J* = 7.6 Hz, 1H), 6.95-6.92(m, 3H), 6.79 (d, *J* = 7.2 Hz, 1H), 6.46 (t, *J* = 5.6 Hz, 1H), 6.38 (t, *J* = 5.6 Hz, 1H), 4.38 (d, *J* = 6.0 Hz, 2H), 4.15 (d, *J* = 6.0 Hz, 2H), 2.59 (s, 6H), 2.21 (s, 3H).

### Example 32. Preparation of compound 1-2-28 of the present invention

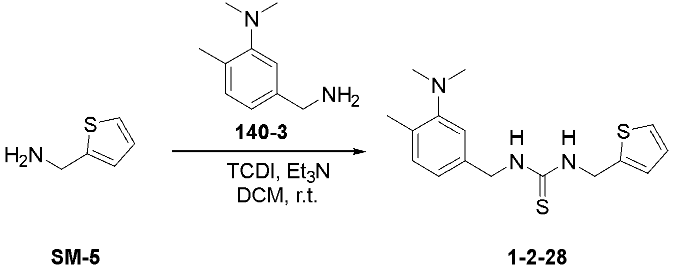

### 1. Synthesis of 140-3

Compound **140-3** was synthesized by referring to Example 18.

### 2. Synthesis of compound 1-2-28

**SM-5** (600 mg, 5.31 mmol) was added to a reaction flask, and dissolved with dichloromethane (30 mL), to which were added triethylamine (1.60 g, 15.93 mmol) and TCDI (945 mg, 5.31 mmol), and then the reaction was stirred at room temperature for 2 h. Then, **140-3** (870 mg, 5.31 mmol) was added to the system, and the mixture was allowed to react at room temperature overnight. After completion of the reaction, the solvent was removed by rotatory evaporation under reduced pressure to obtain the crude product, which was purified by column chromatography [Eluent: dichloromethane-methanol (100:1-20:1)] and reversed-phase prep-HPLC to obtain **1-2-28** as white solids (620 mg, 36%). MS Calcd.: 320.1 [M+H]⁺; MS Found: 320.2 [M + H]⁺.

¹H NMR (400 MHz, DMSO-d₆) *δ*: 7.96-7.73 (m, 2H), 7.40 (dd, *J* = 1.2, 5.2 Hz, 1H), 7.08 (d, *J* = 6.0 Hz, 1H), 7.01 (s, 1H), 6.96 (q, *J* = 3.6 Hz, 1H), 6.92 (s, 1H), 6.84 (s, 1H), 4.86 (s, 2H), 4.68 (s, 2H), 2.59 (s, 6H), 2.26 (s, 3H).

### Example 33. Preparation of compound 1-2-29 of the present invention

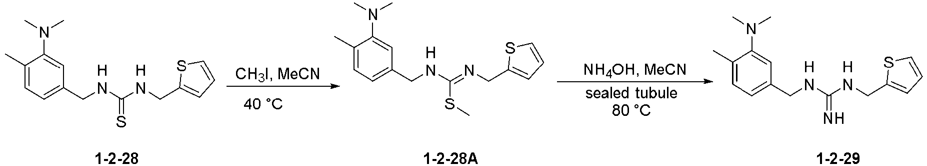

### 1. Synthesis of 1-2-28

Compound **1-2-28** was synthesized by referring to Example 32.

### 2. Synthesis of compound 1-2-28A

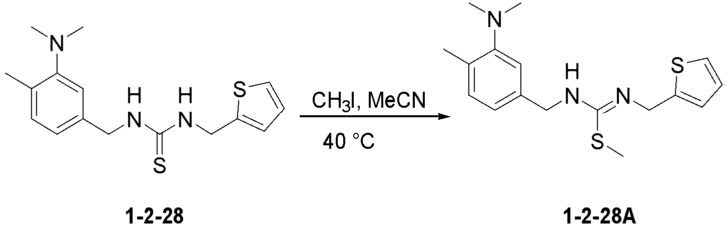

**1-2-28** (320 mg, 1.0 mmol) was added to a sealed tube and dissolved with acetonitrile (20 mL). Then, iodomethane (426 mg, 3.0 mmol) was added, and the mixture was stirred and reacted at 40 °C overnight. After completion of the reaction, the solvent was evaporated under reduced pressure to obtain the crude product, which was subjected to column chromatography [petroleum ether: ethyl acetate (100:1-5:1)] to obtain **1-2-28A** aa gray white solid (300 mg, 90%). MS Calcd.: 334.2 [M+H]⁺; MS Found: 334.4 [M + H]⁺.

### 3. Synthesis of compound 1-2-29

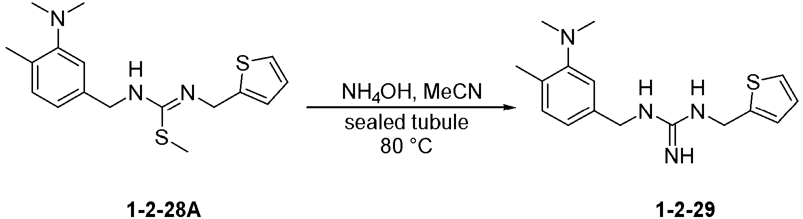

**1-2-28A** (300 mg, 0.9 mmol) was added to a sealed tube and dissolved with acetonitrile (15 mL). Then, ammonia water (5 mL) was added, and the mixture was stirred and reacted at 80 °C overnight. After completion of the reaction, the solvent was removed by rotatory evaporation under reduced pressure to obtain the crude product, which was subjected to column chromatography [dichloromethane: methanol (100:1-5:1)] and prep-HPLC to obtain **1-2-29** as gray white solid (120.28 mg, 44%). MS Calcd.: 303.2 [M+H]⁺; MS Found: 303.4 [M + H]⁺.

¹H NMR (400 MHz, CD₃OD) *δ*: 7.34 (dd, *J* = 1.2, 5.2 Hz, 1H), 7.11 (d, *J* = 7.6 Hz, 1H), 7.02-6.96 (m, 3H), 6.84 (dd, *J* = 1.6, 8.0 Hz, 1H), 4.59 (dd, *J* = 0.8Hz, 2H), 4.34 (s, 2H), 2.66 (s, 6H), 2.28 (s, 3H).

### Example 34. Preparation of compound 1-2-40 of the present invention

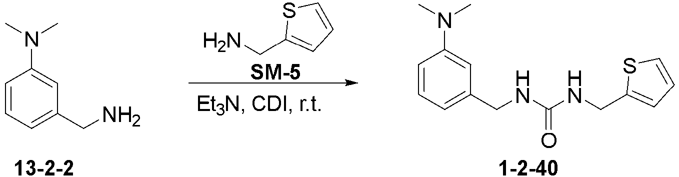

### 1. Synthesis of 13-2-2

Compound **13-2-2** was synthesized by referring to Example 30.

### 2. Synthesis of 1-2-40

**13-2-2** (300 mg, 2.0 mmol) was added to a reaction flask, and dissolved with dichloromethane (20 mL), to which were added triethylamine (606 mg, 6.0 mmol) and CDI (630 mg, 2.4 mmol), and then the reaction was stirred at room temperature for 2 h. Then, **SM-5** (226 mg, 2.0 mmol) was added to the system, and the mixture was allowed to react at room temperature overnight. After completion of the reaction, the solvent was removed by rotatory evaporation under reduced pressure to obtain the crude product, which was purified by silica gel column chromatography (DCM/MeOH = 30/1) and reversed-phase prep-HPLC to obtain **1-2-40** as white solids (163 mg, yield 28%). MS Calcd.: 290.2 [M+H]⁺; MS Found: 290.2 [M + H]⁺.

¹H NMR (400 MHz, DMSO-*d₆*) δ 7.35 (dd, J = 4.8, 1.2 Hz, 1H), 7.09 (t, J = 7.6 Hz , 1H), 6.93 (d, J = 3.6 Hz , 2H), 6.61-6.53 (m, 3H), 6.44(d, J = 6.0 Hz, 1H), 6.34 (d, J = 6.0 Hz, 1H), 4.38 (d, J = 6.0 Hz, 2H), 4.15 (d, J = 5.6 Hz, 2H), 2.86(s, 6H).

### Example 35. Preparation of compound 1-2-1-A1 of the present invention

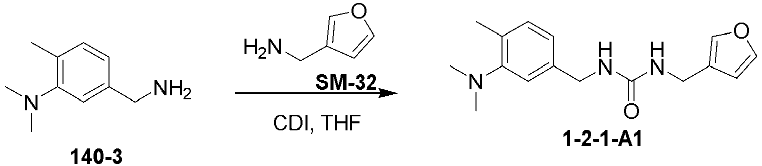

### 1. Synthesis of 140-3

Compound **140-3** was synthesized by referring to Example 18.

### 2. Synthesis of 1-2-1-A1

CDI (295.89 mg, 1.826 mmol) was added to a solution of compound **SM-32** (354.78 mg, 3.653 mmol) in tetrahydrofuran (10 mL), and the reaction was stirred at 25 °C for 1 h. Then, compound **140-3** (300 mg, 1.826 mmol) was added, and the reaction was further stirred and reacted at 80 °C for 1 h. After completion of the reaction, the resultant solution was extracted with ethyl acetate, and the combined organic phases were dried over anhydrous sodium sulfate, and concentrated under reduced pressure. The residue was purified by prep-HPLC to obtain **1-2-1-A1** as white solids (264.2 mg, yield 50%). MS Calcd.: 288.2 [M+H]⁺; MS Found: 288.1 [M + H]⁺.

¹H NMR (400 MHz, DMSO-*d₆*) δ 7.58-7.57 (m, 1H), 7.49-7.48 (m, 1H), 7.06-7.04 (m, 1H), 6.91 (s, 1H), 6.80-6.78 (m, *J* = 1.3 Hz, 1H), 6.40-6.39 (m, 1H), 6.29-6.26 (m, 1H), 6.18-6.15 (m, 1H), 4.15 (d, *J* = 8 Hz, 2H), 4.04 (d, *J* = 4 Hz, 2H), 2.60 (s, 6H), 2.21 (s, 3H).

### Example 36. Preparation of compound 1-2-1-B1 of the present invention

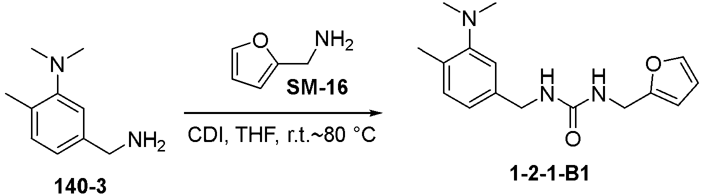

**SM-16** (1.0 g, 10.3 mmol) was added to a flask and dissolved with tetrahydrofuran (50 mL), to which was then added CDI (1.67 g, 10.3 mmol), and the reaction was stirred at room temperature for 1 h. **140-3** (1.69 g, 10.3 mmol) was added to the system and stirred overnight at 80 °C. After completion of the reaction, the reaction solution was poured into 100 mL of water. The resultant solution was extracted with dichloromethane (50 mL × 3). The organic layers were combined, dried over anhydrous sodium sulfate, and filtered. The filtrate was concentrated to obtain the crude product, to which was added a small amount of ethyl acetate to triturate, followed by filtering. The filter cake was dissolved in a small amount of methanol, and freezed dry with deionized water to obtain **1-2-1-B1** as white solids (667.39 mg, yield 22.5%). MS Calcd.: 288.2 [M+H]⁺; MS Found: 288.3 [M + H]⁺.

¹H NMR (400 MHz, DMSO-*d₆*) δ 7.54 (s, 1H), 7.05 (d, *J* = 7.6 Hz, 1H), 6.91 (s, 1H), 6.78 (d, *J* = 7.6 Hz, 1H), 6.37 (br, 1H), 6.35-6.28 (m, 2H), 6.18 (d, *J* = 2.8 Hz, 1H), 4.21 (d, *J* = 6.0 Hz, 2H), 4.14 (d, *J* = 6.0 Hz, 2H), 2.60 (s, 6H), 2.21 (s, 3H).

### Example 37. Preparation of compound 2-1-1 of the present invention

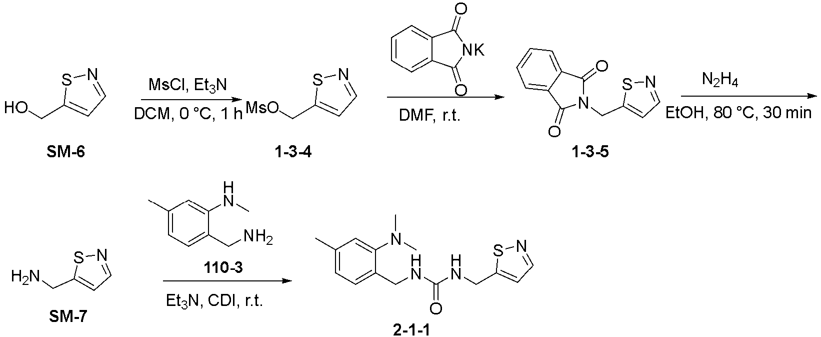

### 1. Synthesis of 1-3-4

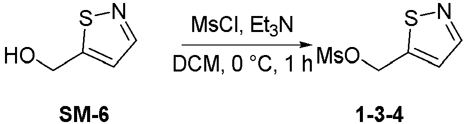

**SM-6** (500 mg, 4.35 mmol) was dissolved in dichloromethane (10 mL), to which was added methylsulfonyl chloride (600 mg, 5.22 mmol) dropwise in an ice bath, and then the reaction was stirred at 0 °C for 1 h. After completion of the reaction, the reaction solution was poured into ice water and quenched. The resultant solution was extracted three times with ethyl acetate. The organic phases were combined, washed with sodium bicarbonate (10 mL), dried over anhydrous sodium sulfate, filtered, and concentrated under reduced pressure to obtain compound **1-3-4** (920 mg, yield: 100.00%). MS Calcd.: 194.0 [M+H]⁺; MS Found: 194.3 [M +H]⁺.

### 2. Synthesis of 1-3-5

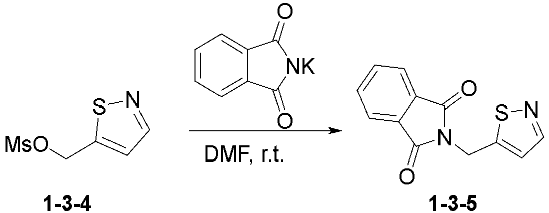

**1-3-4** (920 mg, 4.77 mmol) was dissolved in DMF (10 mL), to which was added potassium phthalimide (1.06 g, 5.72 mmol), and then the mixture was stirred and reacted at room temperature for 1 h. After completion of the reaction, the reaction solution was diluted with ethyl acetate, and then washed with NH₄Cl solution, water, and saturated saline, dried over anhydrous sodium sulfate, and filtered. The solvent was removed by rotatory evaporation under reduced pressure to obtain the crude product, which was purified by silica gel column chromatography (petroleum ether: ethyl acetate = 100/1-2/1) to obtain compound **1-3-5** (930 mg, yield: 80%). MS Calcd.: 245.0 [M+H]⁺; MS Found: 245.3 [M +H]⁺.

### 3. Synthesis of SM-7

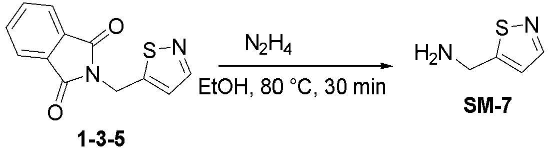

**1-3-5** (930 mg, 3.81 mmol) was dissolved in ethanol (40 mL), to which was added hydrazine hydrate (65 mg, 0.29 mmol) at room temperature, and then the mixture was stirred and reacted at 80 °C for 30 min. After completion of the reaction, the reaction solution was filtered and concentrated under reduced pressure. Then, the residue was diluted with dichloromethane and poured into water. The resultant solution was extracted three times with dichloromethane. The organic phases were combined, dried over anhydrous sodium sulfate, filtered, and concentrated under reduced pressure to obtain the crude product, which was purified by silica gel column chromatography (dichloromethane: methanol = 100/1-2/1) to obtain compound **SM-7** (2.2 g, yield: 83%).

### 4. Synthesis of compound 110-3

Compound **110-3** was synthesized with reference to Example 1.

### 5. Synthesis of 2-1-1

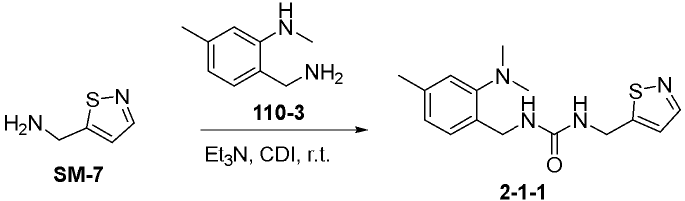

**SM-7** (300 mg, 2.63 mmol) was added to a reaction flask, and dissolved with dichloromethane (10 mL), to which were added triethylamine (1.06 g, 10.53 mmol) and CDI (639 mg, 3.95 mmol), and then the reaction was stirred at room temperature for 2 h. Then, **110-3** (648 mg, 3.95 mmol) was added to the system, and the mixture was allowed to react at room temperature overnight. After completion of the reaction, the solvent was removed by rotatory evaporation under reduced pressure to obtain the crude product, which was purified by column chromatography [Eluent: dichloromethane-methanol (100:1-20:1)] and reversed-phase prep-HPLC to obtain **2-1-1** as white solids (177.87 mg, 22%). MS Calcd.: 305.1 [M+H]⁺; MS Found: 305.1 [M + H]⁺.

¹H NMR (400 MHz, DMSO-*d*₆) *δ*: 8.41 (d, *J* = 1.6 Hz, 1H), 7.21 (t, *J* = 4.0 Hz, 1H), 7.09 (d, *J* = 7.6 Hz, 1H), 6.89 (s, 1H), 6.81 (d, *J* = 7.6 Hz, 1H), 6.73 (t, *J* = 6.0 Hz, 1H), 6.45 (t, *J* = 6.0 Hz, 1H), 4.51 (d, *J* = 6.0 Hz, 2H), 4.24 (d, *J* = 6.0 Hz, 2H), 2.60 (s, 6H), 2.25 (s, 3H).

### Example 38. Preparation of compound 2-1-2 of the present invention

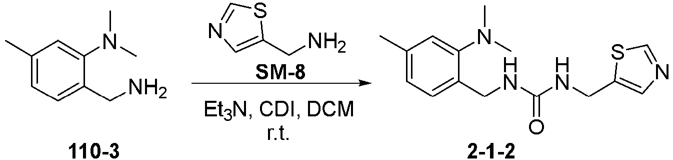

### 1. Synthesis of compound 110-3

Compound **110-3** was synthesized with reference to Example 1.

### 2. Synthesis of 2-1-2

**110-3** (328 mg, 2.0 mmol) was added to a reaction flask, and dissolved with dichloromethane (20 mL), to which were added triethylamine (1.01 g, 10.0 mmol) and CDI (324 mg, 2.0 mmol), and then the reaction was stirred at room temperature for 2 h. Then, **SM-8** (228 mg, 2.0 mmol) was added to the system, and the mixture was stirred and allowed to react at room temperature overnight. After completion of the reaction, the solvent was removed by rotatory evaporation under reduced pressure to obtain the crude product, which was purified by silica gel column chromatography (methanol/dichloromethane (v/v) = 0-3%) and reversed-phase prep-HPLC to obtain **2-1-2** as off-white solid (152.15 mg, yield: 25.6%). MS Calcd.: 305.1 [M+H]⁺; MS Found: 305.1 [M + H]⁺.

¹H NMR (400 MHz, DMSO-d₆) *δ* 8.94 (s, 1H), 7.72 (s, 1H), 7.07 (d, *J* = 7.6 Hz, 1H), 6.89 (s, 1H), 6.80 (d, *J* = 8.0 Hz, 1H), 6.58 (t, *J* = 8.0 Hz, 1H), 6.34 (t, *J* = 5.6 Hz, 1H), 4.43 (d, *J* = 6.0 Hz, 2H), 4.23 (d, *J* = 5.6 Hz, 2H), 2.60 (s, 6H), 2.25 (s, 3H).

### Example 39. Preparation of compound 2-1-3 of the present invention

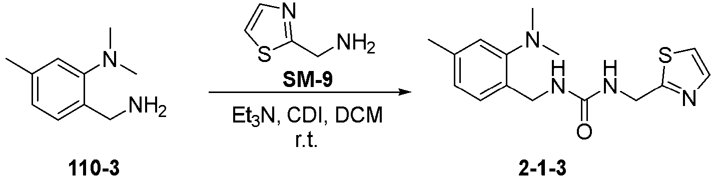

### 1. Synthesis of compound 110-3

Compound **110-3** was synthesized with reference to Example 1.

### 2. Synthesis of 2-1-3

**110-3** (328 mg, 2.0 mmol) was added to a reaction flask, and dissolved with dichloromethane (20 mL), to which were added triethylamine (1.01 g, 10.0 mmol) and CDI (324 mg, 2.0 mmol), and then the reaction was stirred at room temperature for 2 h. Then, **SM-9** (228 mg, 2.0 mmol) was added to the system, and the mixture was stirred and allowed to react at room temperature overnight. After completion of the reaction, the solvent was removed by rotatory evaporation under reduced pressure to obtain the crude product, which was purified by silica gel column chromatography (methanol/dichloromethane (v/v) = 0-3%) and reversed-phase prep-HPLC to obtain **2-1-3** as off-white solid (158.01 mg, yield: 26%). MS Calcd.: 305.1 [M+H]⁺; MS Found: 305.4 [M + H]⁺.

¹H NMR (400 MHz, DMSO-d₆) *δ* 7.70 (d, *J* = 3.6 Hz, 1H), 7.58 (d, *J* = 3.2 Hz, 1H), 7.11 (d, *J* = 7.6 Hz, 1H), 6.90 (s, 1H), 6.83-6.81 (m, 3H), 6.51 (t, *J* = 5.6 Hz, 2H), 4.50 (d, *J* = 6.4 Hz, 2H), 4.25 (d, *J* = 6.0 Hz, 2H), 2.60 (s, 6H), 2.26 (s, 3H).

### Example 40. Preparation of compound 3-1-1 of the present invention

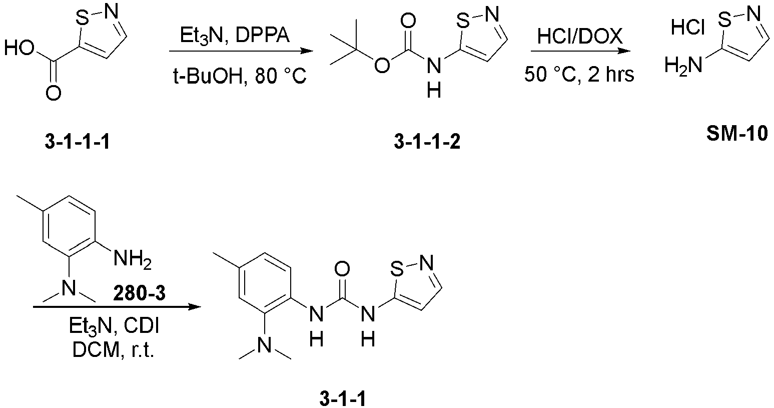

### 1. Synthesis of compound 3-1-1-2

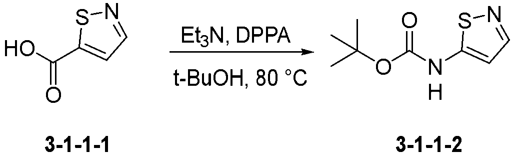

**3-1-1-1** (2.6 g, 20.0 mmol) was added to a reaction flask, and dissolved with tert-butanol (50 mL), to which were added triethylamine (2.02 g, 20.0 mmol) and DPPA (5.5 g, 20.0 mmol), and the reaction solution was stirred at 80 °C overnight. After completion of the reaction, the reaction solution was cooled to room temperature, and then water (50 mL) was added to the reaction system, followed by extraction with ethyl acetate (3×50 mL). The organic phases were combined, dried over anhydrous sodium sulfate, filtered under suction, and concentrated under reduced pressure to obtain the crude product, which was purified by silica gel column chromatography (ethyl acetate/petroleum ether (v/v) = 0-10%) to obtain **13-1-1-2** as light yellow solid (1.8 g, yield: 45%). MS Calcd.: 201.1 [M+H]⁺; MS Found: 202.2 [M + H]⁺.

### 2. Synthesis of compound 3-1-1-3

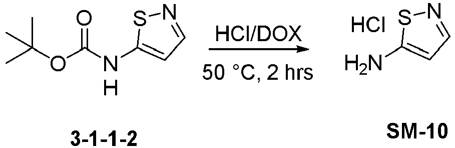

**3-1-1-2** (1.8 g, 9.0 mmol) was added to a reaction flask, and dissolved with 4 N solution of hydrochloric acid in dioxane (50 mL), and then the reaction solution was stirred at 50 °C for 2 h. After completion of the reaction, the solvent was removed by rotatory evaporation under reduced pressure to obtain **SM-10** as white solids (1.2 g, yield: 98%). MS Calcd.: 101.0 [M]⁺; MS Found: 101.3 [M]⁺.

### 3. Synthesis of 280-3

Compound **280-3** was synthesized with reference to Example 13.

### 4. Synthesis of compound 3-1-1

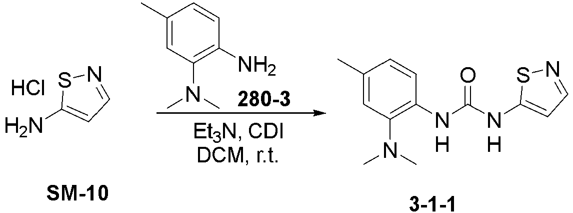

**SM-10** (408 mg, 3.0 mmol) was added to a reaction flask, and dissolved with dichloromethane (20 mL), to which were added triethylamine (1.52 g, 15.0 mmol) and CDI (486 mg, 3.0 mmol), and then the reaction was stirred at room temperature for 4 h. Then, **280-3** (408 mg, 3.0 mmol) was added to the system, and the mixture was stirred and allowed to react at 45 °C overnight. After completion of the reaction, the solvent was removed by rotatory evaporation under reduced pressure to obtain the crude product, which was purified by silica gel column chromatography (methanol/dichloromethane (v/v) = 0-3%) and reversed-phase prep-HPLC to obtain **3-1-1** as off-white solid (131.88 mg, yield: 17.5%).

¹H NMR (400 MHz, DMSO-d₆) *δ* 11.17 (s, 1H), 8.73 (s, 1H), 8.18 (d, *J* = 2.0 Hz, 1H), 8.09 (dd, *J* = 8.0, 1.6 Hz, 1H), 7.23 (dd, *J* = 7.6, 1.6 Hz, 1H), 7.09-6.99 (m, 2H), 6.80 (d, *J* = 1.6 Hz, 1H), 2.62 (s, 6H).

### Example 41. Preparation of compound 3-1-2 of the present invention

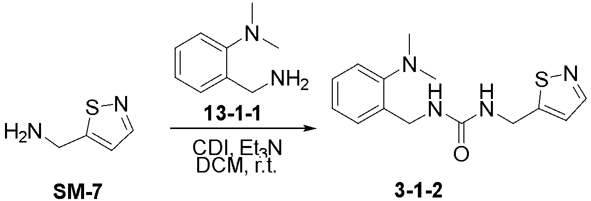

### 1. Synthesis of SM-7

Compound **SM-7** was synthesized with reference to Example 36.

### 2. Synthesis of 3-1-2

**SM-7** (220 mg, 1.93 mmol) was added to a reaction flask, and dissolved with dichloromethane (10 mL), to which were added triethylamine (585 mg, 5.79 mmol) and CDI (313 mg, 1.93 mmol), and then the reaction was stirred at room temperature for 2 h. Then, **13-1-1** (290 mg, 1.93 mmol) was added to the system, and the mixture was stirred and allowed to react at room temperature overnight. After completion of the reaction, the solvent was removed by rotatory evaporation under reduced pressure to obtain the crude product, which was purified by silica gel column chromatography (methanol/dichloromethane (v/v) = 0-3%) and reversed-phase prep-HPLC to obtain **3-1-2** as white solids (158.31 mg, yield: 28%). MS Calcd.: 291.1 [M+H]⁺; MS Found: 291.3 [M+H]⁺.

¹H NMR (400 MHz, CD₃OD) *δ* 8.38 (d, *J* = 2.0 Hz, 1H), 7.29 (d, *J* = 7.2 Hz, 1H), 7.26-7.16 (m, 3H), 7.07-7.03(m, 1H), 4.67 (s, 2H), 4.45 (s, 2H), 2.69 (s, 6H).

### Example 42. Preparation of compound 3-1-3 of the present invention

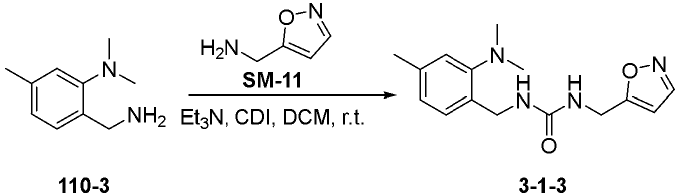

### 1. Synthesis of compound 110-3

Compound **110-3** was synthesized with reference to Example 1.

### 2. Synthesis of 3-1-3

**110-3** (250 mg, 1.52 mmol) was added to a reaction flask, and dissolved with dichloromethane (10 mL), to which were added triethylamine (462 mg,4.57 mmol) and CDI (246 mg, 1.52 mmol), and then the reaction was stirred at room temperature for 2 h. Then, **SM-11** (149 mg, 1.52 mmol) was added to the system, and the mixture was stirred and allowed to react at room temperature overnight. After completion of the reaction, the solvent was removed by rotatory evaporation under reduced pressure to obtain the crude product, which was purified by silica gel column chromatography (methanol/dichloromethane (v/v) = 0-3%) to obtain **3-1-3** as white solids (56.35 mg, yield: 13%). MS Calcd.: 289.2 [M+H]⁺; MS Found: 289.2 [M+H]⁺.

¹H NMR (400 MHz, DMSO-d₆) *δ* 8.45 (d, *J* = 1.2 Hz, 1H), 7.08 (d, *J* = 7.6 Hz, 1H), 6.90 (s, 1H), 6.81 (d, *J* = 7.6 Hz, 1H), 6.61 (t, *J* = 5.2 Hz, 1H), 6.42 (t, *J* = 5.6 Hz, 1H), 6.24 (s, 1H), 4.38 (d, *J* = 6.0 Hz, 2H), 4.23 (d, *J* = 6.0 Hz, 2H), 2.60 (s, 6H), 2.25 (s, 3H).

### Example 43. Preparation of compound 3-1-4 of the present invention

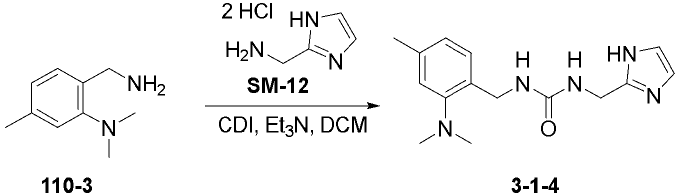

### 1. Synthesis of compound 110-3

Compound **110-3** was synthesized with reference to Example 1.

### 2. Synthesis of 3-1-4

To a solution of **110-3** (328 mg, 2.0 mmol) in dichloromethane (10 mL), were added CDI (486 mg, 3.0 mmol) and triethylamine (1.0 g, 10.0 mmol). The resultant mixture was stirred at room temperature for 2 h. Subsequently, **SM-12** (340 mg, 2.0 mmol) was added to the system, and the mixture was stirred and allowed to react at room temperature overnight. After completion of the reaction, the solvent was removed by rotatory evaporation under reduced pressure to obtain the crude product, which was purified by prep-HPLC to obtain **3-1-4** as white solids (116.92 mg, yield 20%).

¹H NMR (400 MHz, DMSO-*d*₆) *δ*: 11.77 (d, *J* = 6.4 Hz, 1H), 7.09 (d, *J* = 8.0 Hz, 1H), 6.89 (t, *J* = 4.0 Hz, 3H), 6.81 (d, *J* = 7.6 Hz, 1H), 6.39-6.37 (m, 2H), 4.25-4.21 (m, 4H), 2.60 (s, 6H), 2.25 (s, 3H).

### Example 44. Preparation of compound 3-1-5 of the present invention

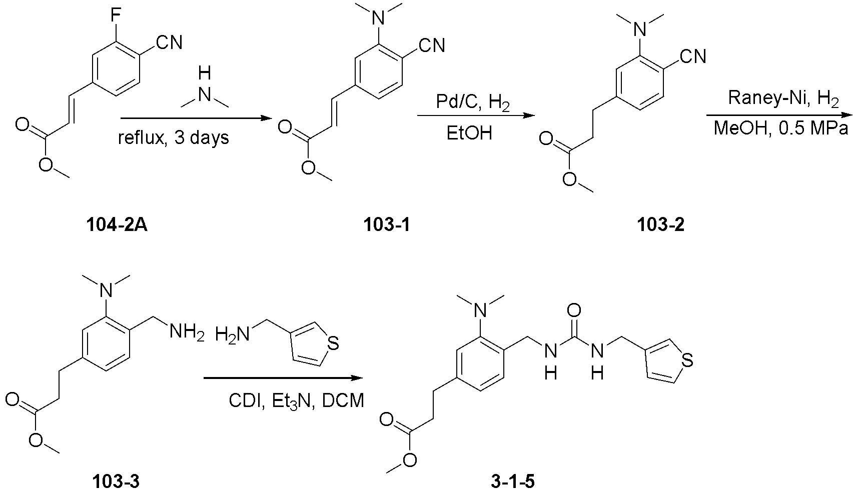

### 1. Synthesis of compound 103-1

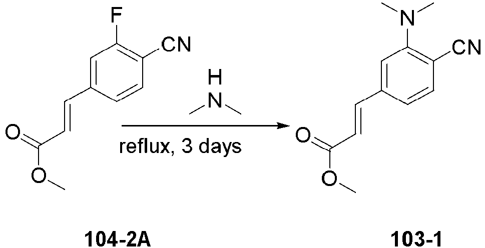

**104-2A** (485 mg, 2.37 mmol) was dissolved in 40 mL of dimethylamine, and the mixture was stirred at 70 °C for 3 days. After completion of the reaction, the solvent was evaporated under reduced pressure to obtain the crude product, which was purified by silica gel chromatography to yield **103-1** (387 mg, yield 80%). MS Calcd.: 231.1 [M + H]⁺; MS Found: 231.3 [M + H]⁺

### 2. Synthesis of compound 103-2

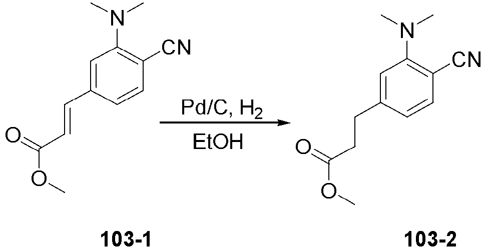

To a solution of **103-1** (387 mg, 1.68 mmol) in methanol (20 mL), was added Pd/C (50 mg), and the mixture was stirred at room temperature under hydrogen atmosphere at 0.5 MPa overnight. After completion of the reaction, the solvent was removed by rotatory evaporation under reduced pressure to obtain the crude product, which was subjected to silica gel column chromatography (methanol/dichloromethane = 0 - 5%) to obtain **103-2** as yellow solid (232 mg, yield 80%). MS Calcd.: 233.1 [M + H]⁺; MS Found: 233.4 [M + H]⁺

### 3. Synthesis of compound 103-3

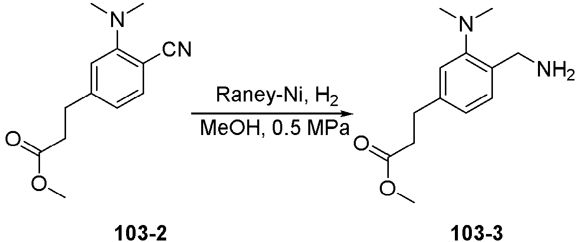

To a solution of **103-2** (232 mg, 1.0 mmol) in methanol (20 mL), was added Riney Ni (50 mg), and the mixture was stirred at room temperature under hydrogen atmosphere at 0.5 MPa overnight. After completion of the reaction, the solvent was removed by rotatory evaporation under reduced pressure to obtain the crude product, which was subjected to silica gel column chromatography (methanol/dichloromethane (v/v) = 0 - 5%) to obtain **103-3** as yellow solid (118 mg, yield 50%). MS Calcd.: 237.2 [M + H]⁺; MS Found: 237.4 [M + H]⁺

### 4. Synthesis of compound 3-1-5

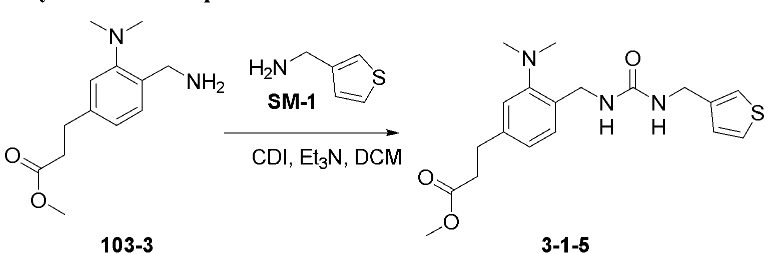

To a solution of **103-3** (170 mg, 0.72 mmol) in dichloromethane (10 mL), were added CDI (178 mg, 1.1 mmol) and triethylamine (363 mg, 3.6 mmol). The resultant mixture was stirred at room temperature for 2 h. Subsequently, **SM-1** (81mg, 0.72 mmol) was added to the system, and the resultant mixture was stirred at room temperature overnight. After completion of the reaction, the solvent was removed by rotatory evaporation under reduced pressure to obtain the crude product, which was purified by prep-HPLC to obtain 3-1-5 as white solids (50.33 mg, yield 48.5%). MS Calcd.: 376.2 [M + H]⁺; MS Found: 376.3 [M + H]⁺

¹H NMR (400 MHz, DMSO-*d*₆) *δ*: 7.47 (dd, *J* = 4.8, 3.2 Hz, 1H), 7.23 (d, *J* = 2.0 Hz, 1H), 7.10 (d, *J* = 7.6 Hz, 1H), 7.01 (dd, *J* = 4.8, 1.2 Hz, 1H), 6.92 (d, *J* = 1.6 Hz, 1H), 6.86-6.84 (m, 1H), 6.37 (t, *J* = 6.0 Hz, 1H), 6.25 (t, *J* = 6.0 Hz, 1H), 4.24-4.20 (m, 4H), 3.58 (s, 3H), 2.82-2.76 (m, 2H), 2.61-2.58 (m, 8H).

### Example 45. Preparation of compound 3-1-6 of the present invention

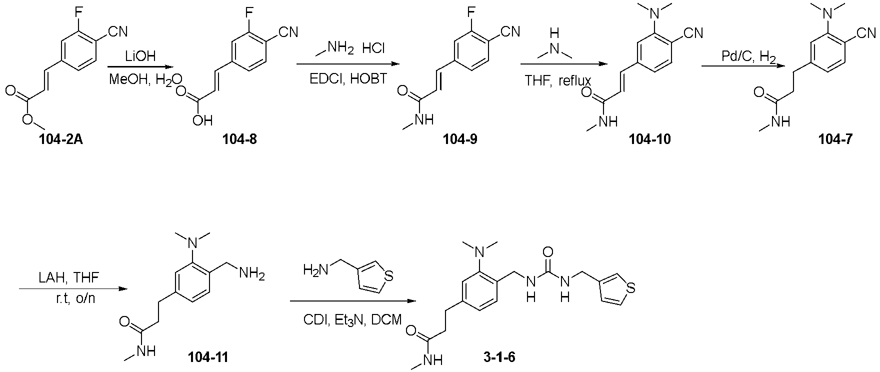

### 1. Synthesis of compound 104-8

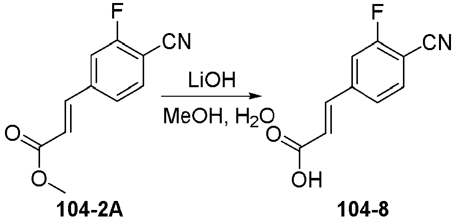

To a solution of **104-2A** (1.0 g, 4.88 mmol) in methanol/water (24/6 mL), lithium hydroxide (615 mg, 14.63 mmol) was added, and the mixture was stirred at room temperature for 2 h. After completion of the reaction, the reaction solution was quenched with water, acidified to pH ~ 6 with citric acid, and filtered to obtain the crude product, which was subjected to silica gel column chromatography to obtain **104-8** as white solids (750 mg, yield 80%).

¹H NMR (400 MHz, DMSO-*d₆*) *δ*: 12.76 (s, 1H), 7.96 (q, *J* = 7.2 Hz, 2H), 7.75 (d, *J* = 8.4 Hz, 1H), 7.63 (d, *J* = 1.6 Hz, 1H), 6.79 (d, *J* = 16 Hz, 1H).

### 2. Synthesis of compound 104-9

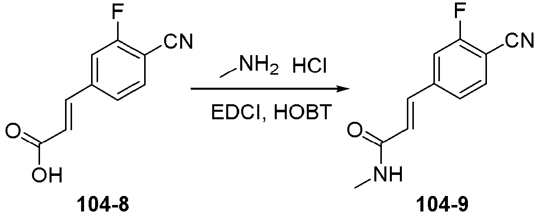

**104-8** (900 mg, 4.71 mmol), methylamine hydrochloride (1.0 g, 4.88 mmol), EDCI (1.36 g, 7.07 mmol), and DIPEA (1.82 g, 14.13 mmol) were dissolved in 20 mL of DMF. The resulting mixture was stirred at room temperature for 30 min, and then HOBT (954 mg, 7.07 mmol) was added, and the reaction was further stirred overnight. After completion of the reaction, the solvent was removed by rotatory evaporation under reduced pressure to obtain the crude product, which was then purified by silica gel column chromatography to yield **104-9** as white solids (480 mg, yield 50%).

### 3. Synthesis of compound 104-10

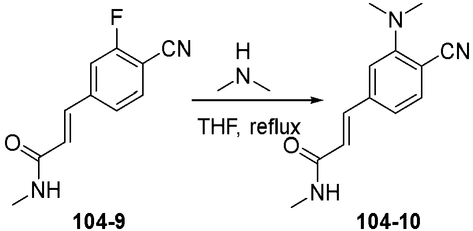

**104-9** (480 mg, 1.68 mmol) was dissolved in dimethylamine (20 mL), and stirred at 70 °C for 48 h. After completion of the reaction, the reaction was purified by silica gel column chromatography (methanol/dichloromethane (v/v) = 0-2.5%) to obtain **104-10** as yellow solid (370 mg, yield 69%). MS Calcd.: 230.1 [M + H]⁺; MS Found: 230.3 [M + H]⁺.

### 4. Synthesis of compound 104-7

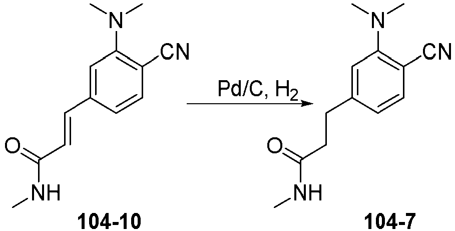

To a solution of **104-10** (340 mg, 1.47 mmol) in ethanol (20 mL), was added Pd/C (68 mg), and the mixture was stirred at room temperature under hydrogen atmosphere overnight. After completion of the reaction, the solvent was removed by rotatory evaporation under reduced pressure to obtain the crude product, which was subjected to silica gel column chromatography (methanol/dichloromethane (v/v) = 0-5%), to obtain **104-7** as yellow solid (300 mg, yield 88%). MS Calcd.: 232.1 [M + H]⁺; MS Found: 232.3 [M + H]⁺.

### 5. Synthesis of compound 104-11

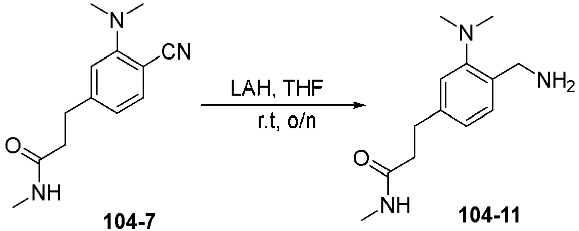

At 0 °C, lithium aluminum hydride (2.6 mmol) was added to a solution of 104-7 (300 mg, 1.30 mmol) in tetrahydrofuran (5 mL), and the mixture was stirred at room temperature overnight. After completion of the reaction, water (0.3 mL), 15% NaOH solution (0.3 mL), and water (0.6 mL) were added sequentially, and the resulting mixture was stirred at room temperature for another 20 min. The mixture was filtered to obtain the crude product, which was then purified by silica gel column chromatography (methanol/dichloromethane (v/v) = 0-5%) to yield yellow solid **104-11** (120 mg, yield 40%). MS Calcd.: 236.1 [M + H]⁺; MS Found: 236.4 [M + H]⁺.

### 6. Synthesis of compound 3-1-6

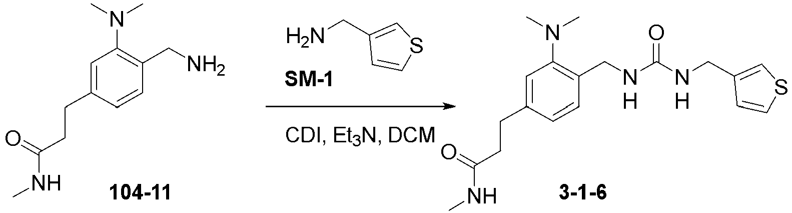

To a solution of **104-11** (169 mg, 0.72 mmol) in dichloromethane (10 mL), were added CDI (178 mg, 1.1 mmol) and triethylamine (363 mg, 3.6 mmol). The resultant mixture was stirred at room temperature for 2 h. Subsequently, **SM-1** (81 mg, 0.72 mmol) was added to the system, and the resultant mixture was stirred at room temperature overnight. After completion of the reaction, the solvent was removed by rotatory evaporation under reduced pressure to obtain the crude product, which was purified by prep-HPLC to obtain **3-1-6** as white solids (88 mg, yield 33%). MS Calcd.: 375.2 [M + H]⁺; MS Found: 375.4 [M + H]⁺

¹H NMR (400 MHz, DMSO-*d*₆) *δ*: 7.75 (d, *J* = 4.4 Hz, 1H), 7.48-7.46 (m, 1H), 7.24-7.22 (m, 1H), 7.09 (d, *J* = 8.0 Hz, 1H), 6.90 (d, *J* = 1.6 Hz, 1H), 6.84-6.82(m, 2H), 6.37 (t, *J* = 6.8 Hz, 1H), 6.25 (t, *J* = 5.6 Hz, 1H), 4.24-4.20 (m, 2H), 2.75 (t, *J* = 7.2 Hz, 2H), 2.59 (s, 6H), 2.55 (t, *J* = 4.8 Hz, 3H), 2.32 (t, *J* = 8.0 Hz, 2H).

### Example 46. Preparation of compound 3-1-7 of the present invention

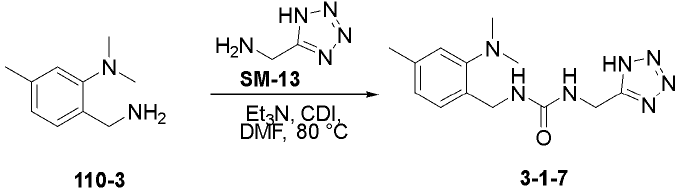

### 1. Synthesis of compound 110-3

Compound **110-3** was synthesized by referring to Example 1.

### 2. Synthesis of compound 3-1-7

To a solution of **110-3** (164 mg, 1.0 mmol) in DMF (10 mL), were added CDI (243 mg, 1.5 mmol) and triethylamine (500 mg, 5.0 mmol). The resultant mixture was stirred at room temperature for 2 h. Subsequently, **SM-13** (99 mg, 1.0 mmol) was added to the system, and the resultant mixture was stirred at 80 °C overnight. After completion of the reaction, the solvent was removed by rotatory evaporation under reduced pressure to obtain the crude product, which was purified by prep-HPLC to obtain **3-1-7** as white solids (117 mg, yield 40%). MS Calcd.: 290.2 [M + H]⁺; MS Found: 290.1 [M + H]⁺.

¹H NMR (400 MHz, DMSO-*d*₆) *δ*: 7.10 (d, *J* = 8.0 Hz, 1H), 6.88 (s, 1H), 6.81 (d, *J* = 8.0 Hz, 1H), 6.45 (s, 1H), 6.32 (s, 1H), 4.34-4.31 (s, 2H), 4.23 (t, *J* = 6.0 Hz, 2H), 2.59 (s, 6H), 2.25 (s, 3H).

### Example 47. Preparation of compound 3-1-8 of the present invention

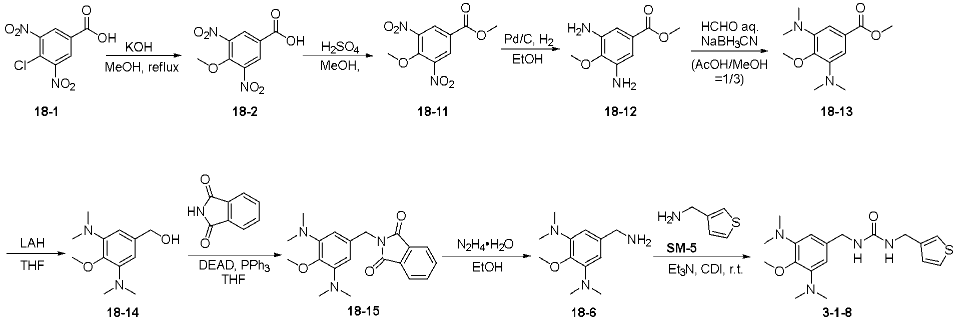

### 1. Synthesis of compound 18-2

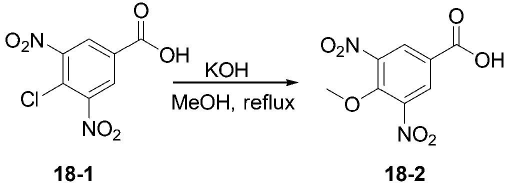

To a solution of 4-chloro-3,5-dinitrobenzoic acid **(18-1,** 9.8 g, 40 mmol) in methanol (500 mL), was added potassium hydroxide (4.48 g, 80 mmol), and the resulting mixture was stirred at 70 °C for 20 h. After the reaction was complete, 500 mL of 3N hydrochloric acid solution was added to quench the reaction. The resultant solution was extracted three times with ethyl acetate. The combined organic phases were dried over anhydrous sodium sulfate and concentrated under reduced pressure to obtain the crude product, which was purified by silica gel column chromatography to obtain **18-2** as light yellow solid (8.2 g, yield 85%).

### 2. Synthesis of compound 18-11

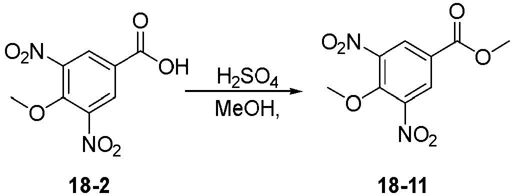

To a solution of 4-methoxy-3,5-dinitrobenzoic acid **(18-2,** 8.2 g, 34 mmol) in methanol (250 mL), concentrated sulfuric acid (10 mL) was added, and the resulting mixture was stirred at 70 °C for 20 h. After completion of the reaction, 200 mL of water was added to quench the reaction. The resultant solution was extracted three times with ethyl acetate. The combined organic phases were dried over anhydrous sodium sulfate and concentrated under reduced pressure to obtain the crude product, which was purified by silica gel column chromatography to yield **18-11** as a light yellow oil (4.8 g, 55% yield).

### 3. Synthesis of compound 18-12

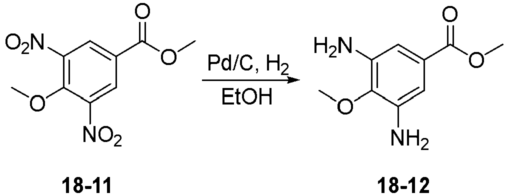

To a solution of methyl 4-methoxy-3,5-dinitrobenzoate **(18-11,** 4.8 g, 18.8 mmol) in ethanol (200 mL), was added Pd/C (960 mg), and the resulting mixture was stirred at room temperature under hydrogen atmosphere for 20 h. After completion of the reaction, **18-12** (8.2 g, yield 85%) was obtained as a light yellow oil after concentration under reduced pressure.

### 4. Synthesis of compound 18-13

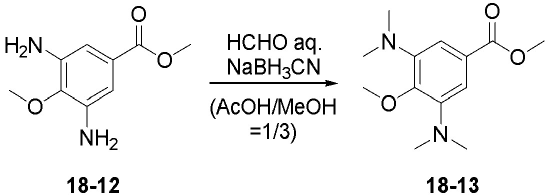

To a solution of methyl 4-methoxy-3,5-diaminobenzoate **(18-12,** 3.0 g, 15.3 mmol), aqueous formaldehyde solution (11.5 g, 153 mmol), and acetic acid (10 mL) in methanol (30 mL), was added NaBH₃CN (5.7 g, 91.8 mmol), and the resulting mixture was stirred at room temperature for 20 h. After completion of the reaction, a saturated NaHCO₃ solution was added to adjust the pH value of the reaction solution to be about 8. The aqueous phase was extracted three times with ethyl acetate, and the combined organic phases were dried over anhydrous sodium sulfate. The crude product was obtained by concentration under reduced pressure and purified by column chromatography to yield **18-13** as a light brown oil (2.5 g, yield 65%).

### 5. Synthesis of compound 18-14

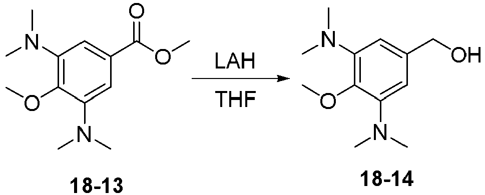

Under nitrogen protection, in an ice-water bath, to a solution of methyl 4-methoxy-3,5-bis(dimethylamino)benzoate **(18-13,** 2.5 g, 9.9 mmol) in ether (100 mL), was added lithium aluminum hydride (2.5 N tetrahydrofuran solution, 8.0 mL), and the resulting mixture was stirred at room temperature for 16 h. After completion of the reaction, water (0.8 mL), 15% NaOH solution (0.8 mL), and water (2.4 mL) were added sequentially. After filtration, the crude product was obtained by concentration under reduced pressure, and purified by column chromatography to obtain **18-14** as a yellow oil (1.7 g, yield 77%).

### 6. Synthesis of compound 18-15

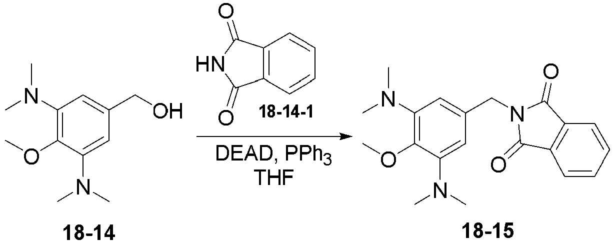

To a solution of **18-14** (1.7 g, 7.6 mmol) and **18-14-1** (1.12 g, 7.6 mmol) in tetrahydrofuran (50 mL), were added DEAD (5.29 g, 30.4 mmol) and PPh₃ (3.0 g, 11.4 mmol), and the resulting mixture was stirred at room temperature for 20 h. After completion of the reaction, the mixture was concentrated under reduced pressure to obtain the crude product, which was purified by column chromatography to yield **18-15** as a yellow oil (1.5 g, yield 56%).

### 7. Synthesis of compound 18-6

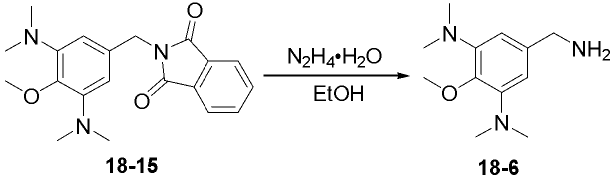

To a solution of **18-15** (1.5 g, 4.2 mmol) in ethanol (30 mL), hydrazine hydrate (353 mg, 8.4 mmol) was added, and the resulting mixture was stirred at room temperature for 20 h. After completion of the reaction, the reaction solution was concentrated under reduced pressure to obtain the crude product, which was purified by column chromatography (DCM/MeOH=20/1) to yield **18-6** as a light brown oil (700 mg, yield 75%).

### 8. Synthesis of compound 3-1-8

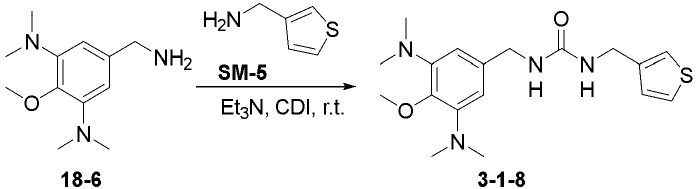

To a solution of **18-6** (200 mg, 0.9 mmol) in dichloromethane (20 mL), were added CDI (220 mg, 1.35 mmol) and triethylamine (455 mg, 4.5 mmol), and the mixture was stirred at room temperature for 2 h. Subsequently, **SM-5** (102 mg, 0.9 mmol) was added, and the resulting mixture was stirred at room temperature overnight. After completion of the reaction, the reaction solution was concentrated under reduced pressure to obtain the crude product, which was purified by prep-HPLC to obtain **3-1-8** as a light brown oil (102 mg, yield 31%). MS Calcd.: 363.2 [M + H]⁺; MS Found: 363.2 [M + H]⁺.

¹H NMR (400 MHz, DMSO-*d*₆) *δ*: 7.43 (dd, *J* = 4.8, 2.8 Hz, 1H), 7.18 (dd, *J* = 3.2, 1.2 Hz, 1H), 6.98 (dd, *J* = 4.8, 1.2 Hz, 1H),6.40 (s, 2H), 6.30-6.26 (m, 2H), 4.18 (d, *J* = 6.0 Hz, 2H), 6.06 (d, *J* = 6.0 Hz, 2H), 3.58 (s, 3H), 2.68 (s, 12H).

### Example 48. Preparation of compound 3-1-9 of the present invention

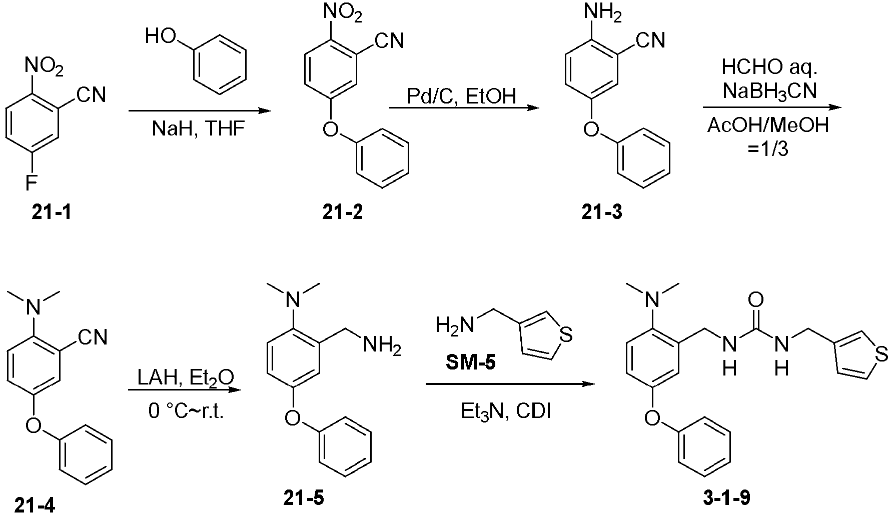

### 1. Synthesis of compound 21-2

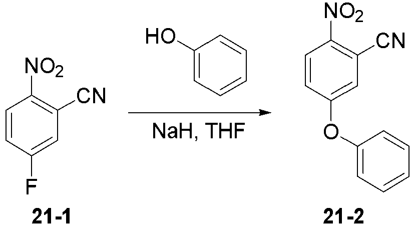

To a solution of **21-1** (1.66 g, 10 mmol) and phenol (1.04 g, 11 mmol) in tetrahydrofuran (50 mL), was added 60% NaOH solution (400 mg, 10 mmol), and then the resulting mixture was stirred at room temperature for 2 h. After completion of the reaction, water was added to quench the reaction. The aqueous phase was extracted with ethyl acetate, and the combined organic phases were dried over anhydrous sodium sulfate. The crude product was obtained by concentration under reduced pressure and purified by column chromatography to obtain **21-2** as a light yellow solid (2.0 g, yield 83%).

### 2. Synthesis of compound 21-3

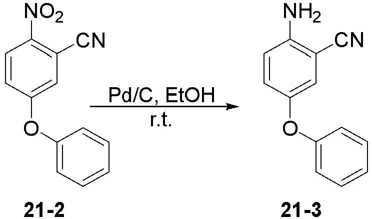

To a solution of **21-2** (480 mg, 2.0 mmol) in ethanol (20 mL), was added Pd/C (96 mg), and the resulting mixture was stirred at room temperature under hydrogen atmosphere for 6 h. After completion of the reaction, the reaction solution was concentrated under reduced pressure to obtain **21-3** as a light yellow oil (400 mg, yield 95%).

### 3. Synthesis of compound 21-4

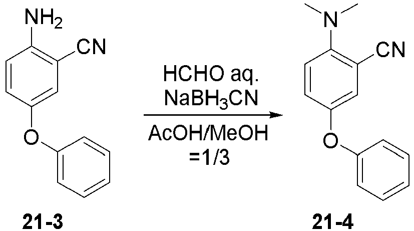

To a solution of **21-3** (400 mg, 1.9 mmol), aqueous formaldehyde solution (2.85g, 38 mmol), and acetic acid (3 mL) in methanol (10 mL), was added NaBH₃CN (1.2 g, 19 mmol), and the resulting mixture was stirred at room temperature for 4 h. After completion of the reaction, a saturated NaHCO₃ solution was added to adjust the pH value of the reaction solution to be about 8. The aqueous phase was extracted three times with ethyl acetate, and the combined organic phases were dried over anhydrous sodium sulfate. The crude product was obtained by concentration under reduced pressure and purified by column chromatography to yield **21-4** as a light brown oil (300 mg, yield 66%).

### 4. Synthesis of compound 21-5

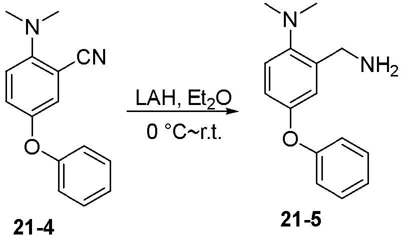

Under nitrogen protection, in an ice-water bath, to a solution of **21-4** (2.5 g, 9.9 mmol) in ether (20 mL), was added lithium aluminum hydride (2.5 N tetrahydrofuran solution, 1.0 mL), and the resulting mixture was stirred at room temperature for 16 h. After completion of the reaction, H₂O (0.2 mL), 15% NaOH solution (0.2 mL), and water (0.6 mL) were added sequentially. After filtration, the crude product was obtained by concentration under reduced pressure, and purified by column chromatography to obtain **21-5** as a yellow oil (200 mg, yield 66%).

### 5. Synthesis of compound 3-1-9

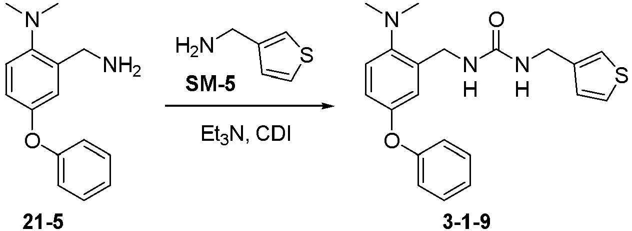

To a solution **of 21-5** (200 mg, 0.83 mmol) in dichloromethane (20 mL), were added CDI (207 mg, 1.25 mmol) and triethylamine (420 mg, 4.15 mmol), and the mixture was stirred at room temperature for 2 h. Subsequently, **SM-5** (94 mg, 0.83 mmol) was added, and the resulting mixture was stirred at room temperature overnight. After completion of the reaction, the reaction solution was concentrated under reduced pressure to obtain the crude product, which was purified by prep-HPLC to obtain **3-1-9** as white solids (190 mg, yield 60%). MS Calcd.: 382.2 [M + H]⁺; MS Found: 382.4 [M + H]⁺.

¹H NMR (400 MHz, DMSO-*d*₆) *δ*: 7.42-7.40 (m, 1H), 7.37-7.35 (m, 1H), 7.17-7.07 (m, 3H), 7.97-6.92 (m, 4H), 6.87-6.84 (m, 1H), 6.41 (t, *J* = 6.8 Hz, 1H), 6.35 (t, *J* = 5.6 Hz, 1H), 4.28 (d, *J* = 6.4 Hz, 2H), 4.17 (d, *J* = 5.6 Hz, 2H), 2.61 (s, 6H).

### Example 49. Preparation of compound 3-1-10 of the present invention

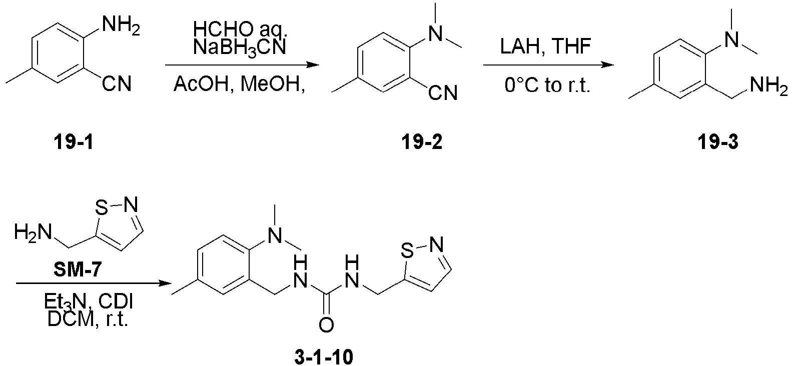

### 1. Synthesis of compound 19-2

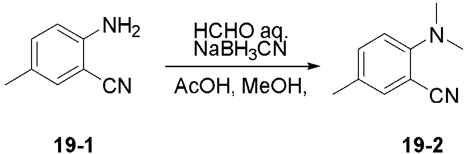

**19-1** (600 mg, 4.55 mmol) was added to a reaction flask, and dissolved with methanol (25 mL), to which were then added formaldehyde aqueous solution (3.69 g, 45.5 mmol), acetic acid (1.37 g, 22.75 mmol), and sodium cyanoborohydride (1.43 g, 22.75 mmol) in sequence, and then the mixture was stirred at room temperature under nitrogen atmosphere overnight. After completion of the reaction, the reaction was quenched by adding water (40 mL). The resultant solution was extracted with dichloromethane (40 mL) three times, dried over anhydrous sodium sulfate, and filtered. The solvent was removed by evaporation under reduced pressure to obtain the crude product, which was purified by normal phase column chromatography [petroleum ether: ethyl acetate (20:1-5:1)] to obtain 19-2 as a yellow oil (560 mg, yield: 77%). MS Calcd.: 161.1 [M + H]⁺; MS Found: 161.4 [M + H]⁺.

### 2. Synthesis of compound 19-3

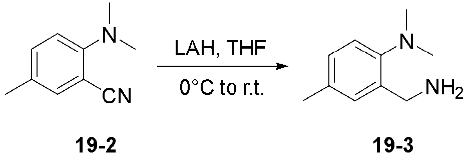

To a reaction flask, were added **19-2** (560 mg, 3.50 mmol) and tetrahydrofuran (15 mL), and then the system was purged with a nitrogen balloon three times, and cooled to 0 °C under nitrogen atmosphere. Then, lithium aluminum hydride (2.5 N in THF, 7.0 mmol, 2.8 mL) was added dropwise, and the reaction was stirred at room temperature overnight. After completion of the reaction, the system was cooled to 0 °C, to which were slowly added water (0.6 mL), 15% aqueous NaOH solution (0.6 mL), and water (1.8 mL). The reaction solution was stirred at room temperature for 20 min, dried over anhydrous magnesium sulfate, and filtered. The solvent was removed by evaporation under reduced pressure to obtain the crude product, which was purified by normal phase column chromatography [dichloromethane: methanol (100:1-20:1)] to obtain **19-3** as a yellow oil (300 mg, yield: 52%). MS Calcd.: 164.2 [M + H]⁺; MS Found: 164.4 [M + H]⁺.

### 3. Synthesis of SM-7

Compound **SM-7** was synthesized with reference to Example 36.

### 4. Synthesis of compound 3-1-10

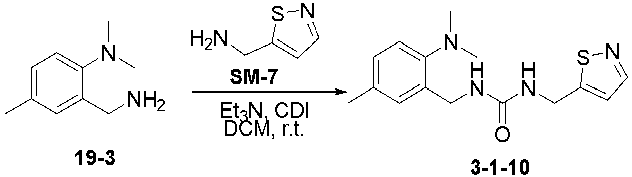

**SM-7** (209 mg, 1.83 mmol) was added to a reaction flask, and then dissolved with dichloromethane (10 mL), to which were added triethylamine (554 mg, 5.49 mmol) and CDI (297 mg, 1.83 mmol), and the mixture was stirred at room temperature for 2 h. Subsequently, **19-3** (300 mg, 1.83 mmol) was added to the system, and the reaction solution was stirred and reacted at room temperature overnight. After completion of the reaction, the solvent was removed by evaporation under reduced pressure to obtain the crude product, which was purified by column chromatography [eluent: dichloromethane-methanol (100:1-20:1)] and reversed-phase column chromatography, to obtain **3-1-10** as white solids (170.65 mg, yield: 31%).

¹H NMR (400 MHz, DMSO-*d₆*) *δ*: 8.42 (d, *J* = 1.6 Hz, 1H), 7.22 (t, *J* = 0.8 Hz, 1H), 7.02 (s, 1H), 6.99 (d, *J* = 1.2 Hz, 2H), 6.76 (t, *J* = 5.6 Hz, 1H), 6.49 (t, *J* = 5.6 Hz, 1H), 4.53 (d, *J* = 6.0 Hz, 2H), 4.26 (d, *J* = 6.0 Hz, 2H), 2.58 (s, 6H), 2.22 (s, 3H).

### Example 50. Preparation of compound 3-1-11 of the present invention

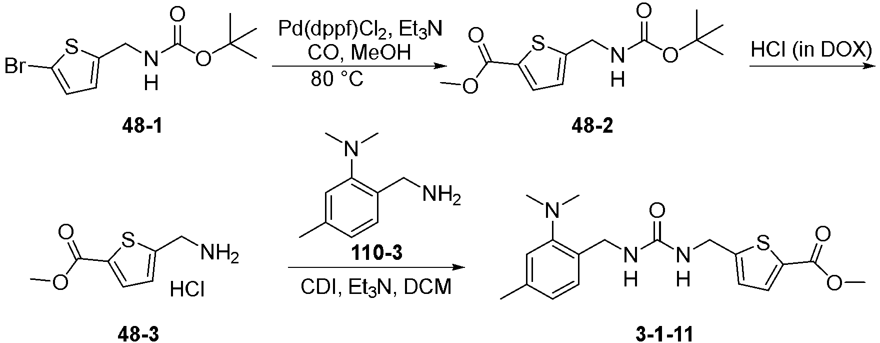

### 1. Synthesis of compound 48-1

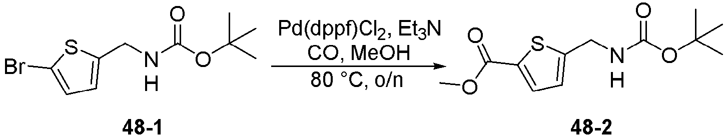

To a solution of **48-1** (290 mg, 1.0 mmol) and triethylamine (404 mg, 4.0 mmol) in methanol (20 mL), was added Pd(dppf)Cl₂ (73 mg, 0.1 mmol), and the resulting mixture was stirred at 80 °C overnight under CO atmosphere. After completion of the reaction, the reaction solution was concentrated under reduced pressure to obtain the crude product, which was purified by prep-HPLC to yield **48-2** as white solids (200 mg, yield 74%).

### 2. Synthesis of compound 48-3

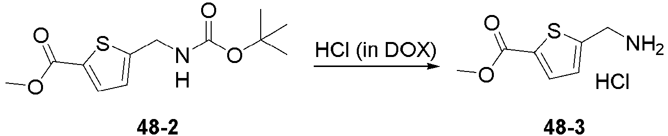

**48-2** (200 mg, 0.74 mmol) was added to 10 mL solution of 4N hydrochloric acid in dioxane, and then stirred at room temperature for 16 h. After completion of the reaction, the reaction solution was concentrated under reduced pressure to obtain **48-3** as yellow solid (150 mg, yield 93%).

### 3. Synthesis of compound 110-3

Compound **110-3** was synthesized with reference to Example 1.

### 4. Synthesis of compound 3-1-11

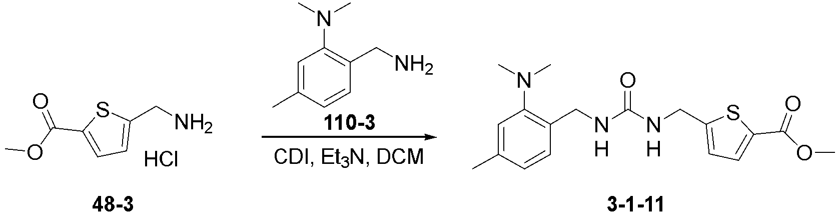

To a solution of **110-3** (118 mg, 0.72 mmol) in dichloromethane (10 mL), were added CDI (178 mg, 1.1 mmol) and triethylamine (363 mg, 3.6 mmol), and the mixture was stirred at room temperature for 2 h. Subsequently, **48-3** (150 mg, 0.72 mmol) was added, and the resulting mixture was stirred at room temperature overnight. After completion of the reaction, the reaction solution was concentrated under reduced pressure to obtain the crude product, which was purified by prep-HPLC to obtain **3-1-11** as white solids (98 mg, yield 52%). MS Calcd.: 362.2 [M + H]⁺; MS Found: 362.4 [M + H]⁺.

¹H NMR (400 MHz, DMSO-*d*₆) *δ*: 7.64 (d, *J* = 3.6 Hz, 1H), 7.09 (d, *J* = 7.6 Hz, 1H), 7.01 (d, *J* = 3.6 Hz, 1H), 6.89 (s, 1H), 6.81 (d, *J* = 7.6 Hz, 1H), 6.67(s, 1H), 6.41 (t, *J* = 5.6 Hz, 1H), 4.41 (d, *J* = 6.0 Hz, 2H), 4.24 (d, *J* = 5.6 Hz, 2H), 3.80 (s, 3H), 2.60 (s, 6H), 2.26 (s, 3H).

### Example 51. Preparation of compound 3-1-12 of the present invention

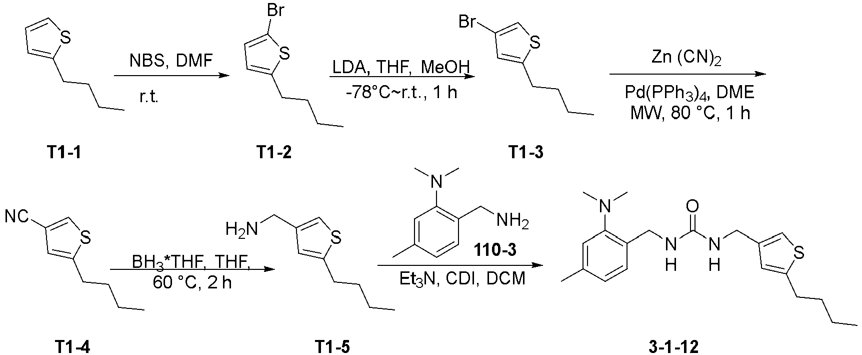

### 1. Synthesis of compound T1-2

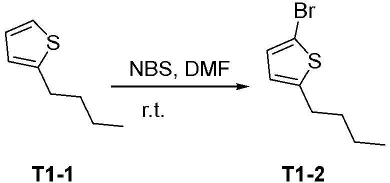

**T1-1** (2.0 g, 14.26 mmol) was added to a reaction flask, and dissolved with DMF (40 mL), to which was then added NBS (2.54 g, 14.26 mmol). Subsequently, the reaction was stirred at room temperature overnight. After completion of the reaction, the reaction solution was diluted with water (50 mL), and then extracted with ethyl acetate (50 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatography (ethyl acetate/petroleum ether (v/v) = 5%) to obtain compound **T1-2** as a colorless oil (3.00 g, yield 96%).

¹H NMR (400 MHz, DMSO-*d₆*) *δ* 6.84 (d, *J* = 3.6 Hz, 1H), 6.55-6.50 (m, 1H), 2.78-2.70 (m, 2H), 1.66-1.58 (m, 2H), 1.45-1.30 (m, 2H), 0.92 (t, *J* = 7.2 Hz, 1H).

### 2. Synthesis of compound T1-3

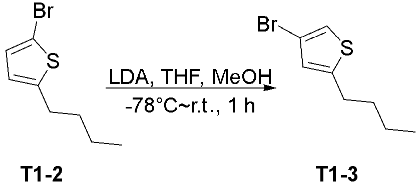

**TI-2** (2.50 g, 11.41 mmol) was added to a reaction flask and dissolved with tetrahydrofuran (40 mL). The reaction system was purged with nitrogen three times, and then cooled to -78 °C, to which was slowly added LDA (1 N, 11 mL, 11.41 mmol) dropwise. Subsequently, the mixture was allowed to react for 1 h, and then methanol (7.5 mL) was added. After completion of the reaction, the reaction was quenched by adding saturated NH₄Cl solution (50 mL). Then, the resultant solution was extracted with ethyl acetate (50 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatography (ethyl acetate/petroleum ether (v/v) = 5%) to obtain compound **T1-3** (2.00 g, yield 80%) as a colorless oil.

¹H NMR (400 MHz, DMSO-*d₆*) *δ* 7.00 (d, *J* = 1.6 Hz, 1H), 6.70 (dd, *J* = 1.2, 2.4 Hz, 1H), 2.83-2.69 (m, 2H), 1.70-1.58 (m, 2H), 1.45-1.32 (m, 2H), 0.93 (t, *J* = 7.6 Hz, 1H).

### 3. Synthesis of compound T1-4

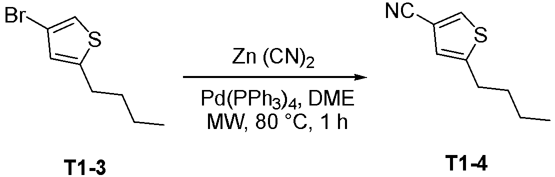

**T1-3** (700 mg, 2.92 mmol) was added to a reaction flask, and dissolved with NMP (10 mL), to which were then added Zn(CN)₂ (343 mg, 2.92 mmol) and Pd(PPh₃)₄ (6.75 mg, 5.84 mmol). The reaction solution was placed in a microwave reactor, and then heated to 80 °C and allowed to react for 1 h. After completion of the reaction, the reaction solution was diluted with water (50 mL), and then extracted with ethyl acetate (50 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatograph (ethyl acetate/petroleum ether (v/v) = 10%) to obtain compound **T1-4** as a colorless oil (350 mg, yield 73%). ESI [M+H]⁺ = 183.2.

### 4. Synthesis of compound T1-5

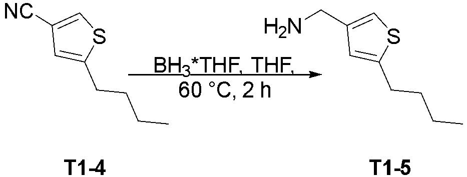

**T1-4** (300 mg, 1.82 mmol) was added to a reaction flask, and dissolved with tetrahydrofuran (10 mL), to which was then added BH3·THF (1 N, 4 mL, 3.63 mmol). Subsequently, the reaction was stirred at 60 °C for 2 h. After completion of the reaction, the reaction solution was diluted with water (40 mL), and then extracted with ethyl acetate (30 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatograph (ethyl acetate/petroleum ether (v/v) = 50%) to obtain compound **T1-5** as a colorless oil (200 mg, yield 65%). ESI [M-16] ⁺ = 153.3.

### 5. Synthesis of compound 110-3

Compound **110-3** was synthesized with reference to Example 1.

### 6. Synthesis of compound 3-1-12

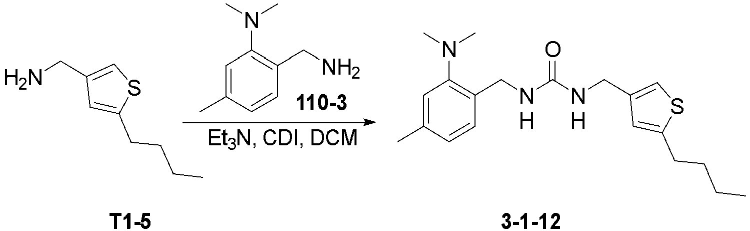

**110-3** (408, 2.48 mmol) was added to a reaction flask, and then dissolved with dichloromethane (40 mL), to which were added triethylamine (1.00 g, 9.92 mmol) and CDI (402 mg, 2.48 mmol), and the mixture was stirred at room temperature for 2 h. Subsequently, **T1-5** (420 mg, 2.48 mmol) was added to the system, and the reaction solution was stirred at room temperature overnight. After completion of the reaction, the solvent was removed by evaporation under reduced pressure to obtain the crude product, which was purified by column chromatography (methanol/dichloromethane (v/v) = 5%] and reversed-phase prep-HPLC, to obtain **3-1-12** as white solids (210 mg, yield: 24%). MS Calcd.: 360.3 [M + H]⁺; MS Found: 359.9 [M + H]⁺.

¹H NMR (400 MHz, DMSO-*d₆*) *δ* 7.08 (d, *J* = 7.6 Hz, 1H), 6.97 (s, 1H), 6.89 (s, 1H), 6.81 (d, *J* = 8.0 Hz, 1H), 6.71 (s, 1H), 6.32 (t, *J* = 6.0 Hz, 1H), 6.22 (t, *J* = 6.0 Hz, 1H), 4.23 (d, *J* = 6.0 Hz, 2H), 4.11 (d, *J* = 6.0 Hz, 2H), 2.73 (t, *J* = 7.6 Hz, 2H), 2.60 (s, 6H), 2.25 (s, 3H), 1.62-1.50 (m, 2H), 1.40-1.27 (m, 2H), 0.89 (t, *J* = 7.6 Hz, 3H).

### Example 52. Preparation of compound 3-1-13 of the present invention

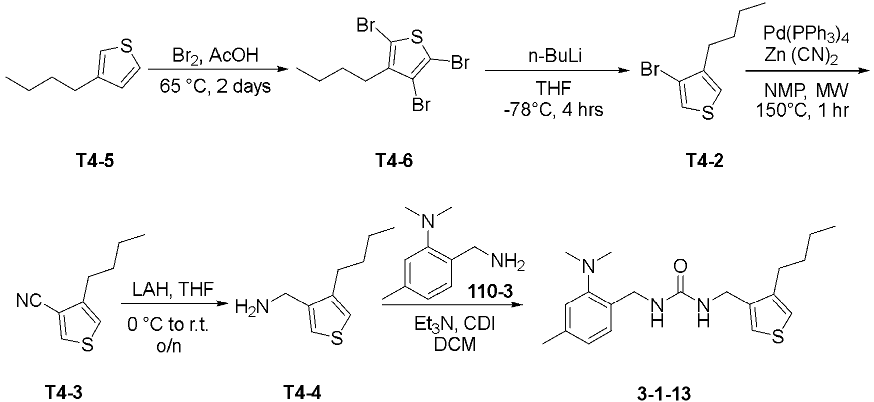

### 1. Synthesis of compound T4-6

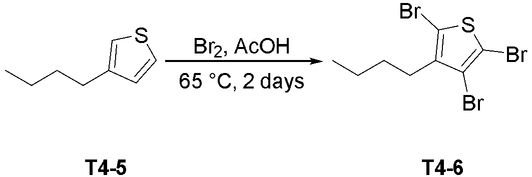

**T4-5** (2.8 g, 20.0 mmol) was added to a reaction flask, and dissolved with acetic acid (100 mL), to which was then slowly added bromine (9.6 g, 60 mmol). Subsequently, the reaction was stirred at 65 °C for 2 days. After completion of the reaction, the reaction solution was diluted with ice water (300 mL), and then the bromine was destroyed with sodium thiosulfate. The resultant solution was extracted with ethyl acetate (200 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatography (petroleum ether) to obtain compound **T4-6** as a colorless oil (5.0 g, yield 66%).

### 2. Synthesis of compound T4-2

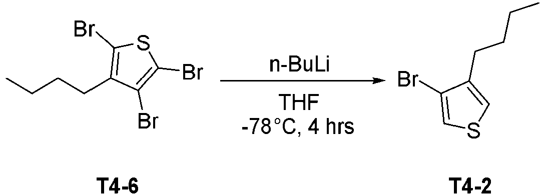

**T4-6** (2.5 g, 6.7 mmol) was added to a reaction flask, and dissolved with THF (100 mL). Then, the reaction system was purged with nitrogen three times, and then cooled to -78°C, to which was slowly added 2.5 N n-butyllithium (5.4 mL, 13.4 mmol) dropwise. Subsequently, the reaction was stirred for 4 h at -78 °C. After completion of the reaction, saturated NH₄Cl solution (100 mL) was added to the reaction system. The resultant solution was extracted with ethyl acetate (3×100 mL). The organic phases were combined, dried over anhydrous sodium sulfate, filtered under suction, and concentrated under reduced pressure to obtain the crude product, which was subjected to silica gel column chromatograph (petroleum ether) to obtain compound T4-2 as a colorless oil (500 mg, yield 34%).

### 3. Synthesis of compound T4-3

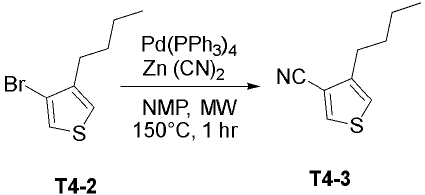

**T4-2** (500 mg, 2.3 mmol) was added to a reaction flask, and dissolved with NMP (10 mL), to which were then added Zn(CN)₂ (538 mg, 4.6 mmol) and Pd(PPh₃)₄ (265 mg, 0.23 mmol). The reaction solution was placed in a microwave reactor, and then heated to 150 °C and allowed to react for 1 h. After completion of the reaction, the reaction solution was diluted with water (50 mL), and then extracted with ethyl acetate (50 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatograph (ethyl acetate/petroleum ether (v/v) = 5%) to obtain compound **T4-3** as a light yellow oil (500 mg, yield 100%).

### 4. Synthesis of compound T4-4

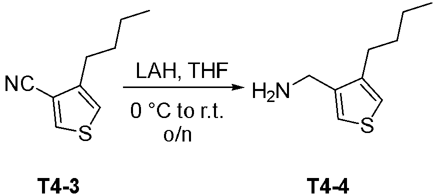

**T4-3** (500 mg, 3.03 mmol) was added to a reaction flask, and dissolved with THF (20 mL), to which was then added 2.5 N lithium aluminum hydride (2.4 mL, 6.06 mmol) at 0 °C. Then, the reaction was slowly warmed to room temperature and stirred overnight. After completion of the reaction, water (1 mL), 15% NaOH solution (1 mL), and water (3 mL) were successively added. Then, the reaction solution was stirred for 30 min, dried over anhydrous magnesium sulfate, and filtered. The filtrate was evaporated under reduced pressure. The crude product was subjected to silica gel column chromatograph (methanol/dichloromethane = 5%) to obtain compound **T4-4** as a light yellow oil (110 mg, yield 21.6%).

### 5. Synthesis of compound 110-3

Compound **110-3** was synthesized with reference to Example 1.

### 6. Synthesis of compound 3-1-13

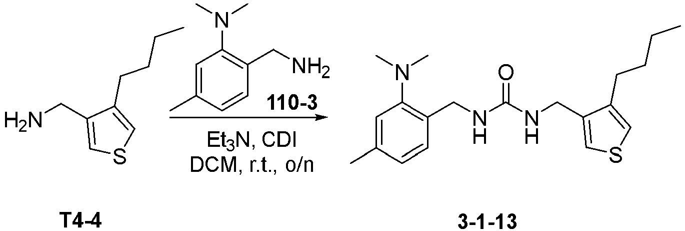

**110-3** (107 mg, 0.65 mmol) was added to a reaction flask, and then dissolved with dichloromethane (10 mL), to which were added triethylamine (197 mg, 1.95 mmol) and CDI (116 mg, 0.72 mmol), and the mixture was stirred at room temperature for 2 h. Subsequently, **T4-4** (110 mg, 0.65 mmol) was added to the system, and the reaction solution was stirred at room temperature overnight. After completion of the reaction, the solvent was removed by evaporation under reduced pressure to obtain the crude product, which was purified by column chromatography (methanol/dichloromethane (v/v) = 5%] and reversed-phase prep-HPLC, to obtain **3-1-13** as white solids (116.36 mg, yield: 50%). MS Calcd.: 360.3 [M + H]⁺; MS Found: 360.2 [M + H]⁺.

¹H NMR (400 MHz, DMSO-*d₆*) *δ* 7.17 (d, *J* = 3.2 Hz, 1H), 7.11 (d, *J* = 4.4 Hz, 1H), 7.09 (s, 1H), 6.89 (s, 1H), 6.81 (d, *J* = 7.6 Hz, 1H), 6.26-6.20 (m, 2H), 4.23 (d, *J* = 6.0 Hz, 2H), 4.15 (d, *J* = 5.6 Hz, 2H), 2.59 (s, 6H), 2.51-2.49 (m, 2H), 2.25 (s, 3H), 1.58-1.50 (m, 2H), 1.36-1.31 (m, 2H), 0.90 (t, *J* = 7.2 Hz, 3H).

### Example 53. Preparation of compound 3-1-14 of the present invention

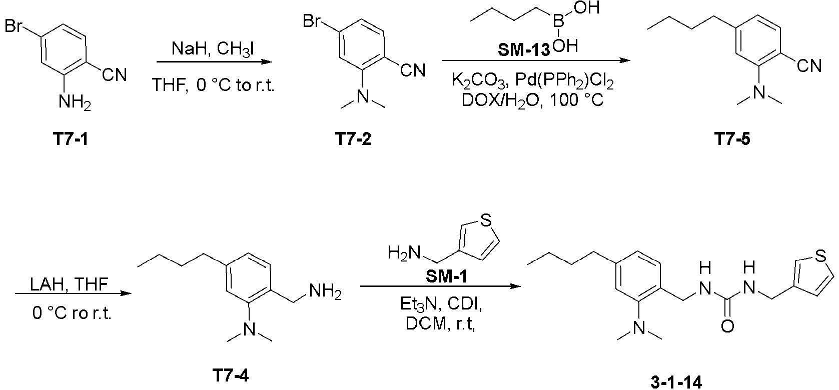

### 1. Synthesis of compound T7-2

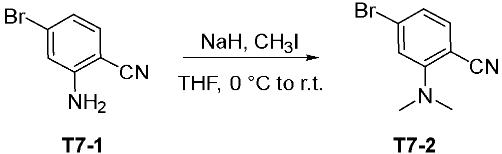

**T7-1** (1.96 g, 10 mmol) was added to a reaction flask, and dissolved with THF (50 mL), to which was added 60% NaH (1.0 g, 25 mmol) in batches at 0 °C, and then the reaction solution was warmed to room temperature and stirred for 3 h. Subsequently, iodomethane (3.55 g, 25 mmol) was added to the reaction solution, and the resultant solution was stirred at room temperature overnight. After completion of the reaction, the reaction solution was diluted with saturated aqueous NH₄Cl solution (100 mL), and then extracted with ethyl acetate (100 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatography (ethyl acetate/petroleum ether (v/v) = 10%) to obtain compound **T7-2** as a light yellow oil (~1.5 g, yield 67%).

### 2. Synthesis of compound T7-5

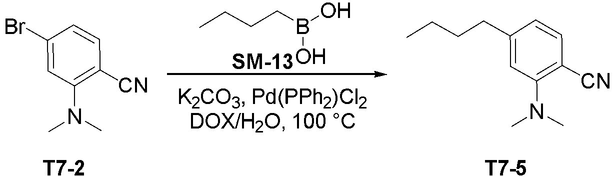

**T7-2** (896 mg, 4 mmol) was added to a reaction flask, and dissolved with DOX/H₂O (50/10 mL), to which were successively added **SM-13** (612 mg, 6 mmol), potassium carbonate (1.1 g, 8 mmol), and Pd(PPh₂)Cl₂ (292 mg, 0.4 mmol). Subsequently, the reaction solution was heated to 100 °C and stirred overnight. After completion of the reaction, water (100 mL) was added, and the resultant solution was extracted with ethyl acetate (100 mL x 3). The organic phases were combined, washed with saturated aqueous NaCl solution (40 mL x 2), and dried over anhydrous sodium sulfate. The solvent was removed by evaporation under reduced pressure. The crude product was subjected to silica gel column chromatograph (ethyl acetate/petroleum ether (v/v) = 10%) to obtain compound **T7-5** as a light yellow oil (~500 mg, yield 62%). MS Calcd.: 203.3 [M + H]⁺; MS Found: 203.2 [M + H]⁺.

### 3. Synthesis of compound T7-4

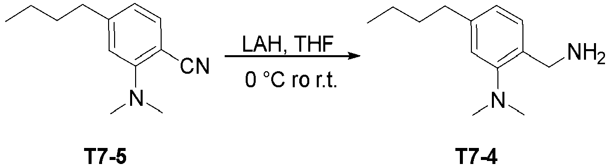

**T7-5** (500 mg, 2.5 mmol) was added to a reaction flask, and dissolved with THF (20 mL), to which was then added 2.5 N lithium aluminum hydride (2.0 mL, 5.0 mmol) at 0 °C. Then, the reaction was slowly warmed to room temperature and stirred overnight. After completion of the reaction, water (1 mL), 15% NaOH solution (1 mL), and water (3 mL) were successively added. Then, the reaction solution was stirred for 30 min, dried over anhydrous magnesium sulfate (20 g), and filtered. The filtrate was evaporated under reduced pressure. The crude product was subjected to silica gel column chromatography (methanol/dichloromethane = 5%) to obtain compound **T7-4** as a light yellow oil (280 mg, yield 53%). MS Calcd.: 207.2 [M + H]⁺; MS Found: 207.3 [M + H]⁺.

### 4. Synthesis of compound 3-1-14

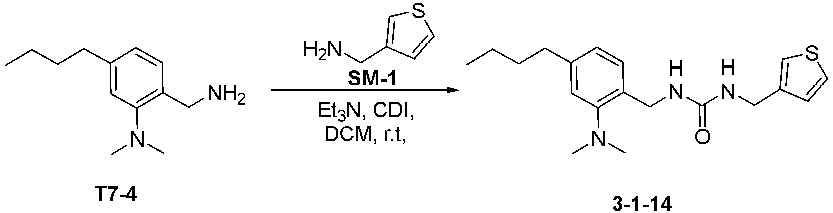

**T7-4** (280 mg, 1.36 mmol) was added to a reaction flask, and then dissolved with dichloromethane (20 mL), to which were added triethylamine (550 mg, 5.44 mmol) and CDI (221 mg, 1.36 mmol), and the mixture was stirred at room temperature for 2 h. Subsequently, **SM-1** (154 mg, 1.36 mmol) was added to the system, and the reaction solution was stirred at room temperature overnight. After completion of the reaction, the solvent was removed by evaporation under reduced pressure to obtain the crude product, which was purified by column chromatography (methanol/dichloromethane (v/v) = 5%) and reversed-phase prep-HPLC, to obtain **3-1-14** as off-white solid (125.06 mg, yield: 27%). MS Calcd.: 346.2 [M + H]⁺; MS Found: 346.2 [M + H]⁺.
¹H NMR (400 MHz, DMSO-*d₆*) δ 7.47 (dd, *J* = 2.8, 5.2 Hz, 1H), 7.23 (s, 1H), 7.10 (d, *J* = 8.0 Hz, 1H), 7.02 (d, *J* = 4.8 Hz, 1H), 6.89 (s, 1H), 6.82 (d, *J* = 7.6 Hz, 1H), 6.36 (t, *J* = 5.6 Hz, 1H), 6.24 (t, *J* = 5.6 Hz, 1H), 4.22 (dd, *J* = 6.0, 12.4 Hz, 4H), 2.60 (s, 6H), 2.54-2.50 (m, 2H), 1.56-1.48 (m, 2H), 1.33-1.27 (m, 2H), 0.89 (t, *J* = 7.2 Hz, 3H)_{∘}

### Example 54. Preparation of compound 3-1-15 of the present invention

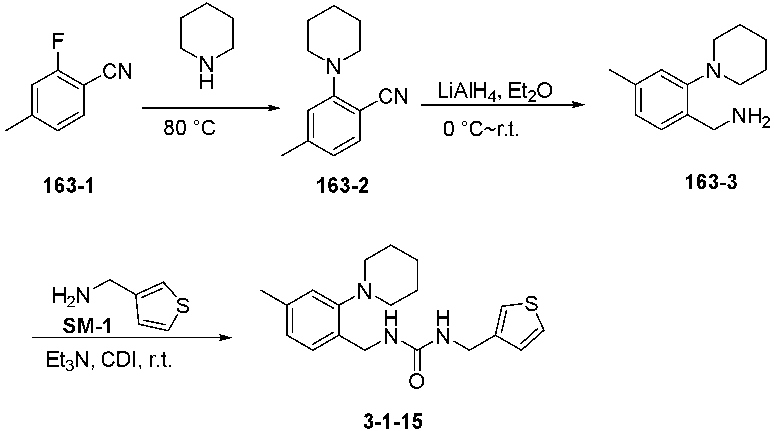

### 1. Synthesis of compound 163-2

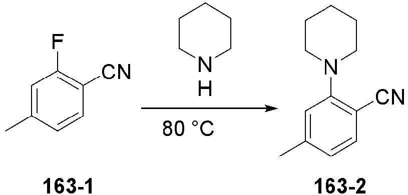

To a 250 mL reaction flask, were added **163-1** (1.0 g, 7.41 mmol) and piperidine (10 mL), and the reaction was stirred at 80 °C overnight. After completion of the reaction, the solvent was removed by evaporation under reduced pressure to obtain the crude product, which was purified by normal phase column chromatography [petroleum ether: ethyl acetate (100:1-10:1)] to obtain **163-2** (1.2 g, yield: 81%) as a brownish yellow oil. MS Calcd.: 201.1 [M+H] ⁺; MS Found: 201.4 [M+H]⁺.

### 2. Synthesis of compound 163-3

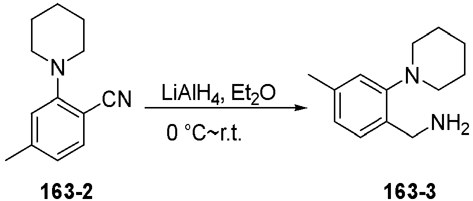

To a reaction flask, were added **163-2** (602 mg, 3.01 mmol) and Et₂O (20 mL). The reaction system was purged with a nitrogen balloon three times and cooled to 0 °C under nitrogen atmosphere. LAH (2.5 N in THF, 2.4 mL) was then added, and the mixture was stirred at room temperature overnight. After completion of the reaction, water (1 mL), 15% NaOH (3 mL), and water (3 mL) were gradually added. The resultant solution was stirred at room temperature for 20 min, dried over anhydrous magnesium sulfate, and filtered. The solvent was removed by evaporation under reduced pressure to obtain the crude product, which was purified by normal phase column chromatography [dichloromethane: methanol 20:1] to yield 163-3 as a yellow oil (2.0 g, yield 61%).

### 3. Synthesis of compound 3-1-15

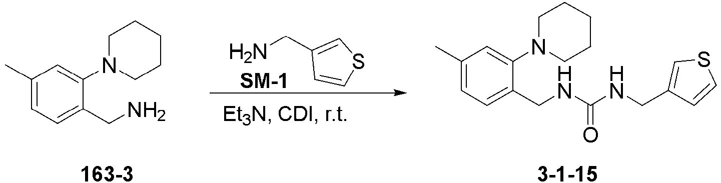

**163-3** (220 mg, 1.08 mmol) was added to a reaction flask, and then dissolved with dichloromethane (6 mL), to which were added triethylamine (436 mg, 4.32 mmol) and CDI (262 mg, 1.62 mmol), and the mixture was stirred at room temperature for 2 h. Subsequently, SM-1 (122 mg, 1.08 mmol) was added to the system, and the reaction solution was stirred at room temperature overnight. After completion of the reaction, the solvent was removed by evaporation under reduced pressure to obtain the crude product, which was purified by column chromatography [Eluent: dichloromethane-methanol (100:1-20:1)] and reversed-phase prep-HPLC, to obtain **3-1-15** as white solids (125.06 mg, yield: 34%). MS Calcd.: 344.2[M+H] ⁺; MS Found: 344.4 [M + H]⁺.

¹H NMR (400 MHz, DMSO-*d*₆) *δ*: 7.47 (dd, *J* = 4.8, 2.8 Hz, 1H), 7.23 (dd, *J* = 2.4, 0.8 Hz, 1H), 7.08 (d, *J* = 7.6 Hz, 1H), 7.03-7.00 (m, 1H), 6.89 (s, 1H), 6.81 (d, *J* = 8.0 Hz, 1H), 6.37 (t, *J* = *6.0* Hz, 1H), 6.23 (t, *J* = 5.6 Hz, 1H), 4.24-4.20 (m, 4H), 2.60 (s, 6H), 2.25 (s, 3H) *_{∘}*

### Example 55. Preparation of compound 3-1-16 of the present invention

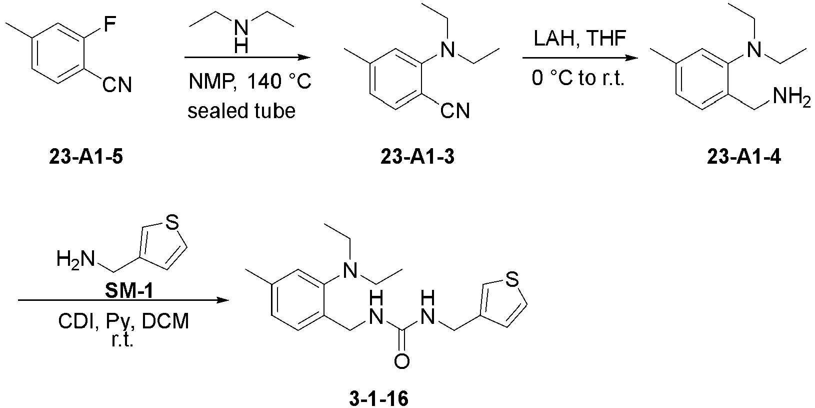

### 1. Synthesis of compound 23-A1-3

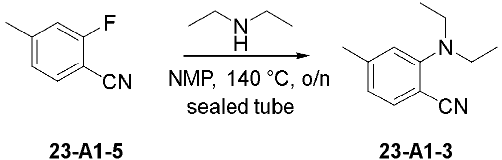

**23-A1-5** (2.0 g, 14.8 mmol) and diethylamine (4.32 g, 59.2 mmol) were added to a reaction tube, and then dissolved with methylpyrrolidone (50 mL). The reaction solution was stirred at 140 °C overnight. After completion of the reaction, the reaction solution was cooled to room temperature, to which was slowly added water (50 mL), and then extracted three times with ethyl acetate. The combined organic phases were dried over anhydrous magnesium sulfate, and filtered. The solvent was removed by evaporation under reduced pressure to obtain the crude product, which was purified by silica gel column chromatography (ethyl acetate / petroleum ether (v/v) = 12%) to obtain **23-A1-3** as a light yellow oil (1.1 g, yield: 40%). MS Calcd.: 189.1[M+H] ⁺; MS Found: 189.4 [M + H]⁺.

### 2. Synthesis of compound 23-A1-4

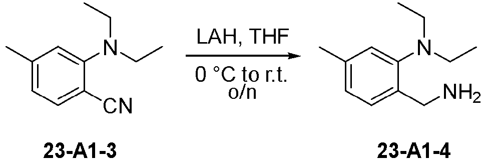

**23-A1-3** (1.1 g, 5.85 mmol) was added to a reaction flask, and dissolved with THF (50 mL), to which was then added 2.5 N lithium aluminum hydride (4.7 mL, 11.7 mmol) at 0 °C. Then, the reaction was slowly warmed from 0 °C to room temperature and stirred overnight. After completion of the reaction, water (1 mL), 15% NaOH solution (1 mL), and water (3 mL) were successively added to the reaction solution. The resultant solution was stirred at room temperature for 20 min, dried over anhydrous magnesium sulfate, and filtered. The solvent was evaporated under reduced pressure, to obtain the crude product, which was subjected to normal phase column chromatography [dichloromethane: methanol (0%-15%)] to obtain compound **23-A1-4** as a light yellow oil (400 mg, yield 36%). MS Calcd.: 193.2[M+H] ⁺; MS Found: 193.3 [M + H]⁺.

### 3. Synthesis of compound 3-1-16

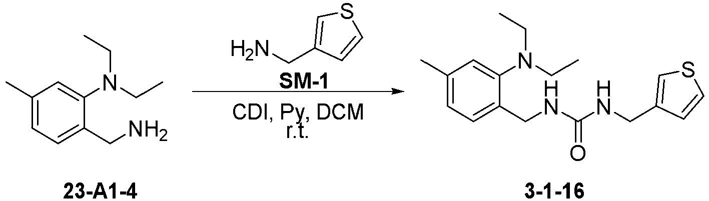

**SM-1** (237 mg, 2.08 mmol) was added to a reaction flask, and then dissolved with dichloromethane (20 mL), to which were added pyridine (822 mg, 10.4 mmol) and CDI (337 mg, 2.08 mmol), and the mixture was stirred at room temperature for 2 h. Subsequently, **23-A1-4** (400 mg, 2.08 mmol) was added to the system, and the reaction solution was stirred at room temperature overnight. After completion of the reaction, the solvent was removed by evaporation under reduced pressure to obtain the crude product, which was purified by silica gel column chromatography (methanol/dichloromethane (v/v) = 0-3%) and reversed-phase prep-HPLC, to obtain **3-1-16** as off-white solid (134.70 mg, yield: 20%). MS Calcd.: 332.2 [M+H] ⁺; MS Found: 332.2 [M + H]⁺.

¹H NMR (400 MHz, DMSO-d₆) δ 7.47 (dd, *J* = 2.8, 4.8 Hz, 1H), 7.23 (dd, *J* = 1.2, 2.8 Hz, 1H), 7.10 (d, *J* = 7.6 Hz, 1H), 7.02 (dd, *J* = 1.2, 5.2 Hz, 1H), 6.94 (s, 1H), 6.85 (dd, *J* = 0.8, 8.0 Hz, 1H), 6.35 (t, *J* = 6.0 Hz, 1H), 6.16 (t, *J* = 5.6 Hz, 1H), 4.24 (d, *J* = 5.6 Hz, 2H), 4.20 (d, *J* = 6.0 Hz, 2H), 2.93-2.87 (m, 4H), 2.26 (s, 3H), 0.90 (t, *J* = 7.2 Hz, 6H).

### Example 56. Preparation of compound 3-1-17 of the present invention

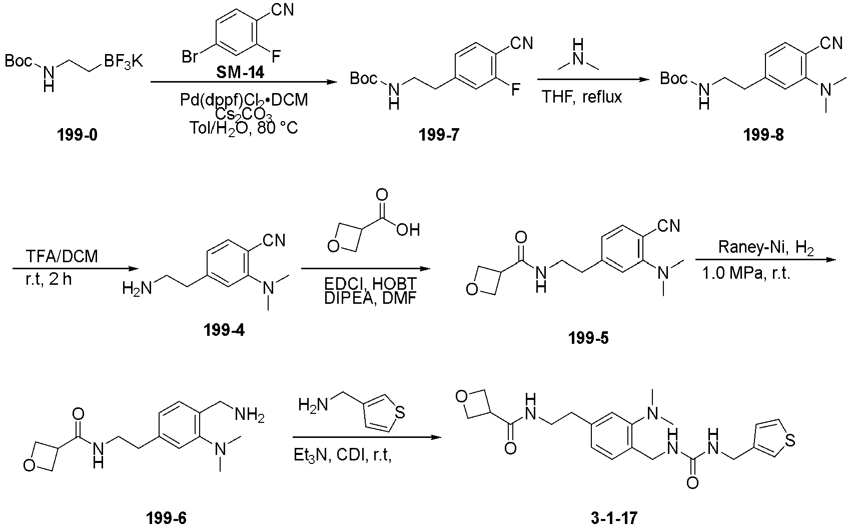

### 1. Synthesis of compound 199-7

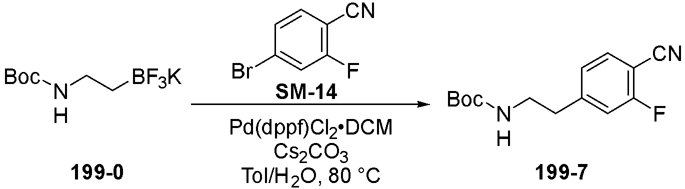

**SM-14** (2.0 g, 10.0 mmol) was added to a reaction flask, and dissolved with toluene/water (40 mL/8 mL), to which were successively added **199-0** (3.0 g, 12.0 mmol), Cs₂CO₃ (9.78 g, 30.0 mmol), and Pd(dppf)Cl₂.DCM (82 mg, 0.1 mmol), and then the system was purged with nitrogen three times. The reaction was stirred at 80 °C and allowed to react overnight under nitrogen atmosphere. After completion of the reaction, the solvent was removed by evaporation under reduced pressure. The residue was poured into water. The resultant solution was extracted three times with ethyl acetate (100 mL). The organic phase was washed once with saturated saline (100 mL), dried over anhydrous sodium sulfate, and filtered. The solvent was removed by evaporation under reduced pressure to obtain the residue, which was purified by normal phase column chromatography [petroleum ether: ethyl acetate (20:1-5:1)] to yield **199-7** as a yellow oil (1.5 g, yield: 47%). MS Calcd.: 265.1[M+H] ⁺; MS Found: 265.4[M+H] ⁺_{∘}

### 2. Synthesis of compound 199-8

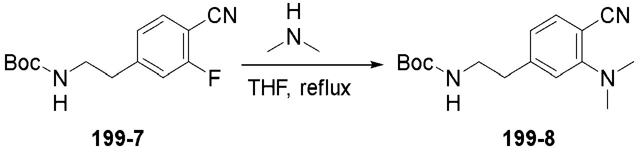

To a 250 mL sealed tube, were added **199-7** (1.5 g, 5.68 mmol) and dimethylamine tetrahydrofuran (60 mL), and the mixture was stirred and reacted at 70 °C for two days. After completion of the reaction, the solvent was removed by evaporation under reduced pressure to obtain the crude product, which was purified by normal phase column chromatography [petroleum ether: ethyl acetate (20:1-5:1)] to yield **199-8** (1.3 g, yield: 79%) as a yellow oil. MS Calcd.: 290.1 [M+H]⁺; MS Found: 290.4 [M+H]⁺.

### 3. Synthesis of compound 199-4

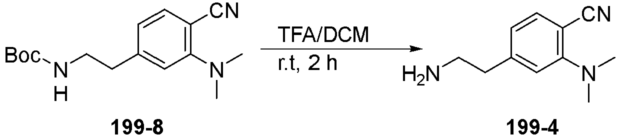

**199-8** (600 mg, 2.08 mmol) was added to a reaction flask, and then dissolved with dichloromethane (10 mL), to which were added trifluoroacetic acid (2 mL) dropwise at room temperature, and the mixture was allowed to react for 1 h at room temperature. After completion of the reaction, the solvent was removed by evaporation under reduced pressure to obtain a residue. The residue was dissolved in dichloromethane and poured into water. Then, saturated NaHCO₃ solution was added to adjust the pH to 9. The resultant solution was extracted three times with dichloromethane. The organic phase was dried over anhydrous sodium sulfate, and filtered. The solvent was removed by evaporation under reduced pressure to obtain a residue, which was purified by normal phase column chromatography [dichloromethane: methanol (100:1-5:1)] to obtain **199-4** as a yellow oil (200 mg, yield: 51%). MS Calcd.: 190.1 [M+H] ⁺; MS Found: 190.2 [M+H]⁺.

### 4. Synthesis of compound 199-5

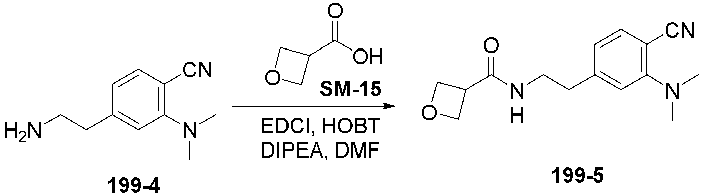

**SM-15** (200 mg, 1.06 mmol) was added to a reaction flask, and dissolved with DMF (5 mL), to which were successively added **199-4** (162 mg, 1.59 mmol), DIPEA (410 mg, 3.18 mmol), and EDCI (305 mg, 1.59 mmol). The mixture was stirred and reacted at room temperature under nitrogen atmosphere for 2 h, followed by adding HOBT (215 mg, 1.59 mmol). The mixture was allowed to react at room temperature overnight. After completion of the reaction, the reaction solution was poured into water, and extracted three times with ethyl acetate (5 mL). The organic phase was washed twice with water and once with saturated saline (5 mL), dried over anhydrous sodium sulfate, and filtered. The solvent was removed by evaporation under reduced pressure to obtain a residue, which was purified by normal phase column chromatography [petroleum ether: ethyl acetate (20:1-5:1)] to obtain **199-5** as a yellow oil (60 mg, yield: 30%). MS Calcd.: 161.1 [M+H] ⁺; MS Found: 161.4 [M+H]⁺.

### 5. Synthesis of compound 199-6

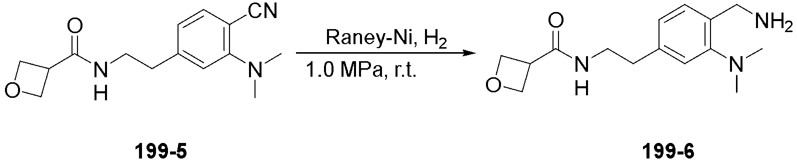

**199-5** (3.0 g, 22.73 mmol) was added to an autoclave, and dissolved with methanol (100 mL), to which were then added Raney-Ni (7.2 g, 113.65 mmol). The system was purged with hydrogen three times. The mixture was stirred and reacted at room temperature under hydrogen atmosphere overnight. After completion of the reaction, the reaction solution was filtered. The solvent was removed by evaporation under reduced pressure, to obtain the residue, which was purified by normal phase column [dichloromethane: methanol (20:1-5:1)] to obtain **199-6** as a yellow oil (3.2 g, yield: 89.9%). MS Calcd.: 278.2 [M+H] ⁺; MS Found: 278.4 [M+H]⁺.

### 6. Synthesis of compound 3-1-17

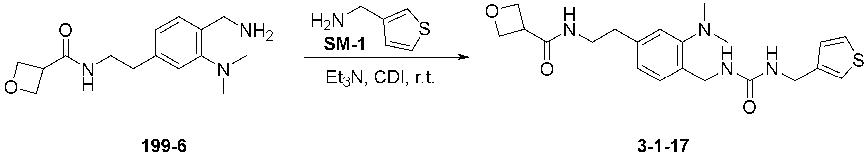

**SM-1** (25 mg, 0.22 mmol) was added to a reaction flask, and then dissolved with dichloromethane (3 mL), to which were added triethylamine (87.5 mg, 0.87 mmol) and CDI (53.5 mg, 0.33 mmol), and the mixture was stirred at room temperature for 2 h. Subsequently, **199-6** (60 mg, 0.22mmol) was added to the system, and the mixture was allowed to react at room temperature overnight. After completion of the reaction, the solvent was removed by evaporation under reduced pressure to obtain the crude product, which was purified by column chromatography [Eluent: dichloromethane-methanol (100:1-20:1)] and reversed-phase prep-HPLC, to obtain **3-1-17** as white solids (18.32 mg, 20%). MS Calcd.: 417.1 [M+H] ⁺; MS Found: 417.3 [M + H]⁺.

¹H NMR (400 MHz, DMSO-*d₆*) *δ*: 7.96 (t, J = 5.6 Hz, 1H), 7.47 (q, J = 3.2 Hz, 1H), 7.23 (d, J = 5.6 Hz, 1H), 7.11 (d, J = 8.0 Hz, 1H), 7.03 (t, J = 4.4 Hz, 1H), 6.89 (s, 1H), 6.84 (q, J = 8.0 Hz, 1H), 6.40 (t, J = 6.0 Hz, 1H), 6.28 (t, J = 6.0 Hz, 1H), 4.61-4.55(m, 4H), 4.25-4.20 (m, 4H), 3.72-3.67 (m, 1H), 3.31-3.25 (m, 2H), 2.67 (t, J = 7.2 Hz, 2H), 2.60 (s, 6 H)_{∘}

### Example 57. Preparation of compound 3-1-18 of the present invention

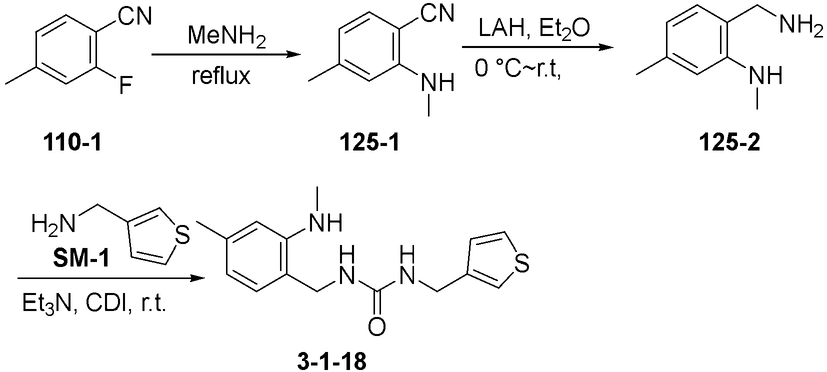

### 1. Synthesis of compound 125-1

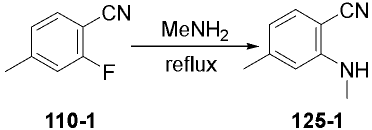

**110-1** (3.0 g, 22.2 mmol) was added to a reaction flask, and dissolved with 2N methylamine (100 mL). The reaction was stirred at 70 °C overnight. After completion of the reaction, the solvent was removed by evaporation under reduced pressure to obtain a residue, which was purified by normal phase column chromatography [petroleum ether: ethyl acetate 5:1] to yield **125-1** as yellow solid (3.0 g, yield: 94%). MS Calcd.: 147.1 [M+H] ⁺; MS Found: 147.3 [M + H]⁺.

### 2. Synthesis of compound 125-2

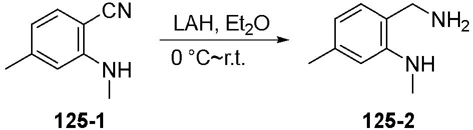

To a reaction flask, were added **125-1** (1.0 g, 6.85 mmol) and Et₂O (20 mL). Lithium aluminum hydride (13.70 mmol) was added to the system at 0 °C, and the mixture was stirred at room temperature overnight. After completion of the reaction, water (1 mL), 15% NaOH (1 mL), and water (3 mL) were gradually added. The resultant solution was stirred at room temperature for 20 min, dried over anhydrous magnesium sulfate, and filtered. The solvent was removed by evaporation under reduced pressure to obtain the crude product, which was purified by normal phase column chromatography [dichloromethane: methanol 20:1] to yield **125-2** as yellow solid (600 mg, yield 58%).

### 3. Synthesis of compound 3-1-18

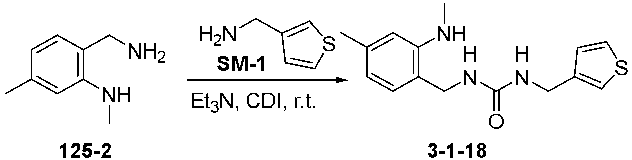

**SM-1** (220 mg, 1.95 mmol) and dichloromethane (20 mL) were added to a reaction flask, to which were then added CDI (473 mg, 2.92 mmol) and triethylamine (591 mg, 5.85 mmol), and then the mixture was stirred overnight at room temperature. After completion of the reaction, the solvent was removed by evaporation under reduced pressure to obtain the crude product, which was purified by prep-HPLC to obtain **3-1-18** as white solids (116 mg, yield 20%). MS Calcd.: 290.1[M+H] ⁺; MS Found: 290.4 [M + H]⁺.

¹H NMR (400 MHz, DMSO-*d*₆) *δ*: 7.44 (q, *J* = 2.8 Hz, 1H), 7.19 (q, *J* = 1.2 Hz, 1H), 6.98 (dd, *J* = 1.2, 4.8 Hz, 1H), 6.34-6.27 (m, 3H), 6.84 (d, *J* = 7.2 Hz, 1H), 5.52 (d, *J*= 4.8 Hz, 1H), 4.17 (d, *J* = 5.6 Hz, 2H), 4.02 (d, *J* = 6.0 Hz, 2H), 2.66 (d, *J* = 5.2 Hz, 3H), 2.18 (s, 3H).

### Example 58. Preparation of compound 3-1-19 of the present invention

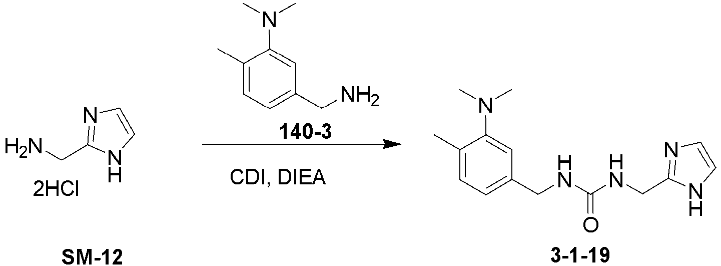

### 1. Synthesis of 140-3

Compound **140-3** was synthesized with reference to Example 18.

### 2. Synthesis of 3-1-19

To a solution of compound **SM-12** (350 mg, 3.604 mmol) in tetrahydrofuran (10 mL), was added CDI (1751.44 mg, 10.811 mmol), and the mixture was stirred and reacted at room temperature for 2 h. After that, compound **140-3** (1183.84 mg, 7.208 mmol) and 2-[ethyl(2-hydroxyethyl)amino]ethanol-1-ol (2 mL, 15.256 mmol) were added, and the reaction was stirred at 80 °C for 14 h. After completion of the reaction, water was added, and the resultant solution was extracted with ethyl acetate (20 mL x 3). The organic layer was washed with concentrated brine, dried over anhydrous Na₂SO₄, and concentrated under vacuum. The residue was purified by prep-HPLC to provide compound 3-1-19 as white solids (157.5 mg, yield 15.21%). MS Calcd.: 288.4[M+H]⁺; MS Found: 288.2 [M + H]⁺.

¹H NMR (400 MHz, DMSO-*d₆*) *δ* 11.73 (s, 1H), 7.06-7.04 (m, 1H), 6.99 (s, 1H), 6.91 (s, 1H), 6.80-6.79 (m, 2H), 6.46-6.43 (m, 1H), 6.32-6.30 (m, 1H), 4.22-4.15 (m, 4H), 2.60 (s, 6H), 2.21 (s, 3H).

### Example 59. Preparation of compound 3-1-20 of the present invention

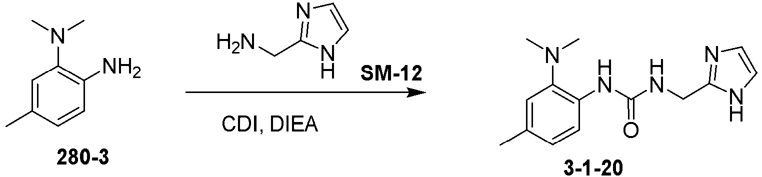

### 1. Synthesis of 280-3

Compound **280-3** was synthesized with reference to Example 13.

### 2. Synthesis of 3-1-20

To a solution of compound **280-3** (1.94 g, 19.971 mmol) in tetrahydrofuran (20 mL), was added CDI (3.24 g, 19.971 mmol), and the mixture was stirred and reacted at room temperature for 2 h. After that, compound **SM-12** (1.5 g, 9.985 mmol) and 2-[ethyl(2-hydroxyethyl)amino]ethanol-1-ol (2 mL, 15.256 mmol) were added, and the reaction was stirred at 80 °C for 14 h. After completion of the reaction, water was added, and the resultant solution was extracted with ethyl acetate (20 mL x 3). The organic layer was washed with concentrated brine, dried over anhydrous Na₂SO₄, and concentrated under vacuum. The residue was purified by prep-HPLC to provide compound **3-1-20** as white solids (269.1 mg, yield 9.86%). MS Calcd.: 274.3[M+H] ⁺; MS Found: 274.1 [M + H]⁺.

¹H NMR (400 MHz, DMSO-*d₆*) *δ* 11.82 (s, 1H), 8.04 (s, 1H), 7.95 (d, *J* = 8 Hz, 1H), 7.43-7.41 (m, 1H), 7.01 (s, 1H), 6.92 (s, 1H), 6.82 - 6.75 (m, 2H), 4.27 (d, *J* = 8 Hz, 2H), 2.55 (s, 6H), 2.21 (s, 3H).

### Example 60. Preparation of compound 3-1-21 of the present invention

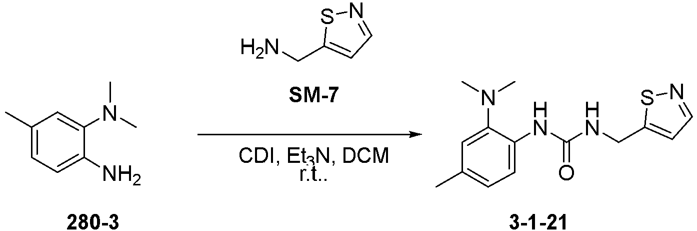

### 1. Synthesis of 280-3

Compound **280-3** was synthesized with reference to Example 13.

### 2. Synthesis of 3-1-21

**SM-7** (300 mg, 2.00 mmol) and triethylamine (606 mg, 5.99 mmol) were added to a reaction flask, and then dissolved with dichloromethane (10 mL). The reaction solution was cooled to 0 °C in an ice-water bath, to which was added CDI (356 mg, 2.20 mmol), and then the reaction was stirred for 30 min. Then, **280-3** (330 mg, 2.20 mmol) was slowly added to the reaction system, and the mixture was stirred at room temperature overnight. After completion of the reaction, the solvent was removed by evaporation under reduced pressure to obtain the crude product, which was purified by silica gel column chromatography (ethyl acetate / petroleum ether (v/v) = 50%) and reversed-phase prep-HPLC to obtain **3-1-21** as off-white solid (89 mg, yield: 15%). MS Calcd.: 291.1[M+H] ⁺; MS Found: 291.2 [M + H]⁺.

¹H NMR (400 MHz, DMSO-*d₆*) *δ* 8.44 (d, *J* = 1.6 Hz, 1H), 8.03 (s, 1H), 7.88 (d, *J* = 8.0 Hz, 1H), 7.67 (t, *J* = 5.6 Hz, 1H), 7.28 (s, 1H), 6.94 (s, 1H), 6.79 (d, *J* = 8.4 Hz, 1H), 4.59 (d, *J* = 5.6 Hz, 2H), 2.56 (s, 6H), 2.22 (s, 3H).

### Example 61. Preparation of compound 3-1-22 of the present invention

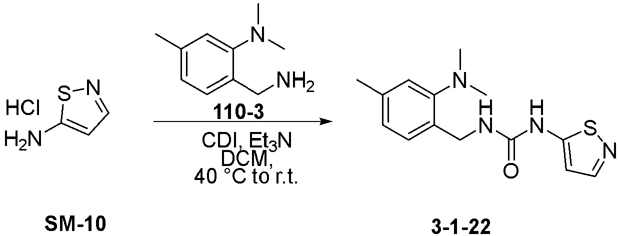

### 1. Synthesis of 110-3

Compound **110-3** was synthesized with reference to Example 1.

### 2. Synthesis of 3-1-22

**SM-10** (408 mg, 3.0 mmol) was added to a reaction flask, and then dissolved with dichloromethane (20 mL), to which were added triethylamine (1.52 g, 15.0 mmol) and CDI (486 mg, 3.0 mmol), and the mixture was stirred at room temperature for 2 h. Subsequently, **110-3** (492 mg, 3.0 mmol) was added to the system, and the mixture was allowed to react at room temperature overnight. After completion of the reaction, the solvent was removed by evaporation under reduced pressure to obtain the crude product, which was purified by column chromatography (methanol/dichloromethane (v/v) = 3%) and prep-HPLC, to obtain **3-1-22** as off-white solid (116.32 mg, yield: 13%). MS Calcd.: 291.1[M+H] ⁺; MS Found: 291.1 [M + H]⁺.

¹H NMR (400 MHz, DMSO-d₆) δ 8.10 (d, *J* = 2.0 Hz, 1H), 7.11-7.09 (m, 2H), 6.94 (s, 1H), 6.84 (d, *J* = 7.6 Hz, 1H), 6.72 (d, *J* = 1.6 Hz, 1H), 4.36 (d, *J* = 5.6 Hz, 2H), 2.63 (s, 6H), 2.26 (s, 3H).

### Example 62. Preparation of compound 3-1-23 of the present invention

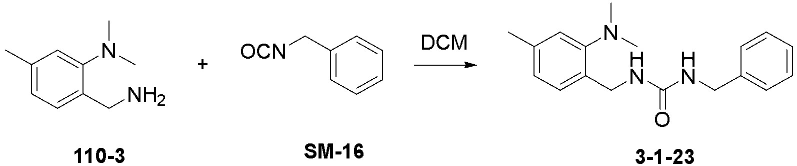

### 1. Synthesis of 110-3

Compound **110-3** was synthesized with reference to Example 1.

### 2. Synthesis of compound 3-1-23

**110-3** (820 mg, 5.0 mmol, 1.0 equiv.) and **SM-16** (765 mg, 5.0 mmol, 1.0 equiv.) were dissolved in DCM (15.0 mL), and warmed to room temperature and allowed to react for 2 h. After completion of the reaction, the reaction was subjected to column chromatography (PE/EtOAc 3:1) to provide compound **3-1-23** as white solids (289 mg, 0.97 mmol, yield 19%). HRMS (ESI-TOF) *m*/*z*: [M + H]⁺ Calcd for C₁₈H₂₃N₃OH⁺ 298.1914; Found 298.1913.

¹H NMR (400 MHz, CDCl₃): *δ* 7.31-7.18 (m, 3H), 7.14 (d, *J* = 7.6 Hz, 1H), 6.93-6.83 (m, 2H), 5.81 (s, 1H), 5.39 (s, 1H), 4.35-4.29 (m, 4H), 2.52 (s, 6H), 2.30 (s, 3H). ¹³C NMR (100 MHz, CDCl₃): *δ* 158.60, 151.63, 139.51, 138.04, 130.25, 129.77, 128.50, 127.37, 127.10, 124.81, 120.04, 45.03, 44.53, 40.72, 21.30.

### Example 63. Preparation of compound 3-1-24 of the present invention

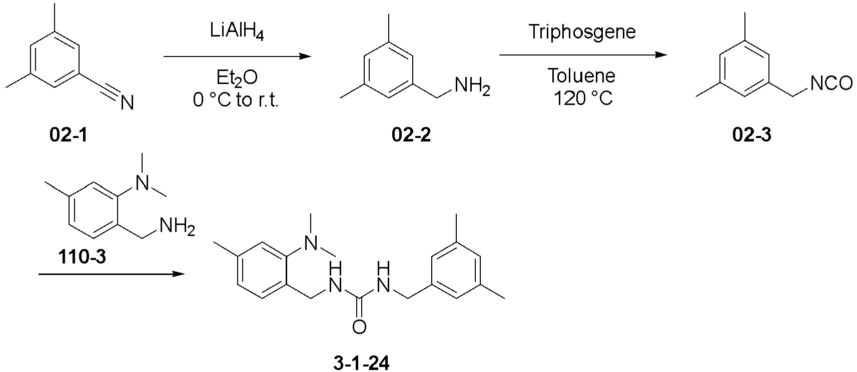

### 1. Synthesis of compound 02-2

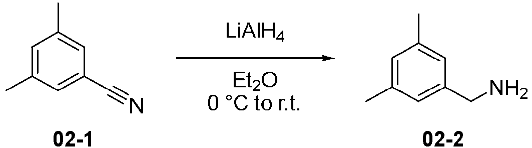

**02-1** (3.94 g, 30 mmol, 1.0 equiv.) was dissolved in Et₂O (60 mL), to which was then gradually added lithium aluminum hydride (2.85 g, 75 mmol, 2.5 equiv.) in batches at 0 °C. After that, the mixture was warmed to room temperature and allowed to react for 2 h. After completion of the reaction, 3 mL of 15% NaOH solution was first added for quenching, followed by adding 3 mL of water dropwise and then another 3 mL of 15% NaOH solution. The resultant solution was filtered through diatomaceous earth and over anhydrous Na₂SO₄, and concentrated under reduced pressure to obtain compound **02-2** as a yellow oil (3.74 g, yield 92%).

### 2. Synthesis of compound 02-3

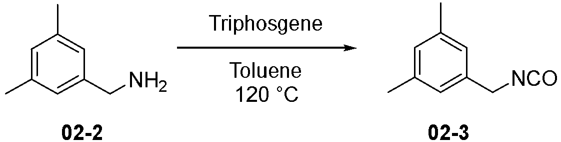

**02-2** (270 mg, 2.0 mmol) was dissolved in toluene (12 mL), to which was added triphosgene (297 mg, 1.0 mmol). After that, the mixture was heated to 120 °C and allowed to react under reflux for 4 h. Then, toluene was removed by rotatory evaporation, and the crude product **02-3** was obtained as a yellow oil, which was directly used in the next step.

### 3. Synthesis of compound 110-3

Compound **110-3** was synthesized according to Example 1.

### 4. Synthesis of compound 3-1-24

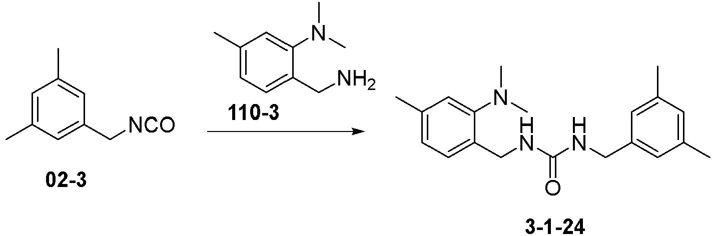

**110-3** (164 mg, 1.0 mmol) and **02-3** (161 mg, 1.0 mmol) were dissolved in DCM (6.0 mL), and then the reaction solution was warmed to room temperature and allowed to react for 1 h. After completion of the reaction, the reaction was subjected to column chromatography (PE/EtOAc = 3:1) to obtain compound **3-1-24** as yellow solid (50 mg, yield 15%). HRMS (ESI-TOF) *m*/*z:* [M + H]⁺ Calcd for C₂₀H₂₇N₃OH⁺ 326.2227; Found 326.2229.

¹H NMR (400 MHz, CDCl₃): *δ* 7.16 (d, *J* = 7.6 Hz, 1H), 6.91-6.84 (m, 3H), 6.81 (s, 2H), 5.61 (s, 1H), 5.30 (s, 1H), 4.33 (s, 2H), 4.27 (d, *J =* 5.6 Hz, 2H), 2.54 (s, 6H), 2.31 (s, 3H), 2.26 (s, 6H).

### Example 64. Preparation of compound 3-1-25 of the present invention

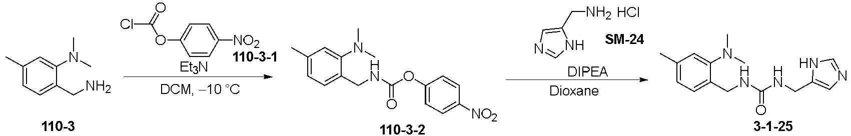

### 1. Synthesis of compound 110-3

Compound **110-3** was synthesized by referring to Example 1.

### 2. Synthesis of compound 110-3-2

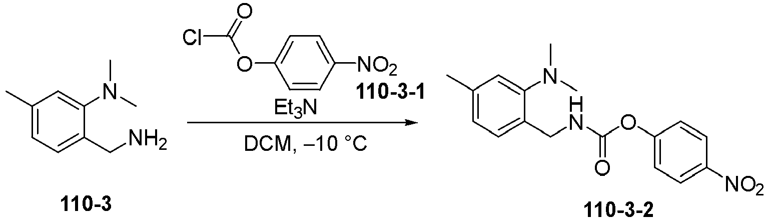

**110-3-1** (1.01 g, 5 mmol) was dissolved in DCM (30 mL), to which was slowly added **110-3** (820 mg, 5 mmol) dropwise at -10 °C. After that, the reaction was allowed to react at -10 °C for additional 16 h. After completion of the reaction, water was added to quench the reaction, followed by dilution with DCM. The resultant solution was washed twice with 20% aqueous NaHCO₃ solution, dried over anhydrous sodium sulfate, and concentrated under reduced pressure to obtain compound **110-3-2** as light yellow solid (1.06 g, yield 65%).

¹H NMR (400 MHz, CDCl₃) δ 8.21 (m, 2H), 7.30 (m, 2H), 7.18 (d, *J =* 7.6 Hz, 1H), 7.00 (d, *J =* 1.7 Hz, 1H), 6.90 (dd, *J =* 7.8, 1.7 Hz, 1H), 6.41 (t, *J =* 6.0 Hz, 1H), 4.51 (d, *J =* 5.7 Hz, 2H), 2.72 (s, 6H), 2.34 (s, 3H).

### 3. Synthesis of compound 3-1-25

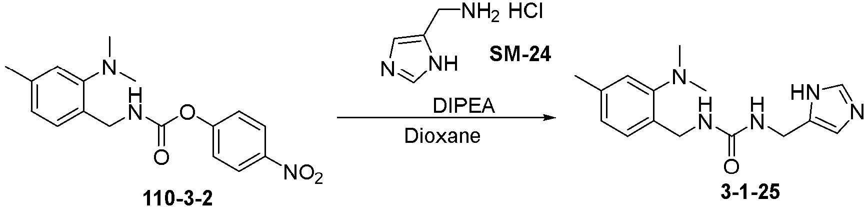

**110-3-2** (592 mg, 1.8 mmol) and **SM-24** (268 mg, 2 mmol) were dissolved in 1,4-dioxane (10 mL), to which was added DIPEA (626 µL, 3.6 mmol), and the mixture was reacted in an oil bath at 65 °C for 16 h. After completion of the reaction, the reaction was subjected to column chromatography (DCM/MeOH = 20:1), to provide compound 3-1-25 as white solids (151 mg, yield 30%). HRMS (ESI-TOF) *m*/*z:* [M + H]⁺ Calcd for C₁₅H₂₂N₅O⁺ 288.1819; Found 288.1824.

¹H NMR (400 MHz, CDCl₃) *δ* 8.32 (s, 1H), 7.40 (d, *J* = 1.9 Hz, 1H), 7.15 (d, *J* = 7.7 Hz, 1H), 6.92 (m, 3H), 6.13 (d, *J* = 1.9 Hz, 1H), 4.86 (d, *J* = 4.9 Hz, 2H), 4.30 (s, 2H), 3.70 (s, 3H), 2.42 (s, 6H), 2.32 (s, 3H).

### Example 65. Preparation of compound 3-1-26 of the present invention

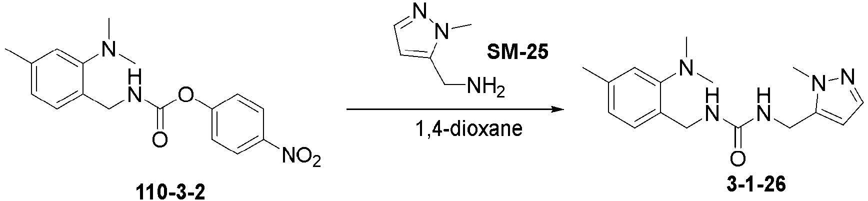

### 1. Synthesis of compound 110-3-2

Compound **110-3-2** was synthesized by referring to Example 59.

### 2. Synthesis of compound 3-1-26

**110-3-2** (201 mg, 1.5 mmol) and **SM-25** (222 mg, 2 mmol) were dissolved in 1,4-dioxane (10 mL), to which was added triethylamine (404 mg, 4 mmol), and the mixture was allowed to react at 50 °C for 4 h. After completion of the reaction, the reaction was subjected to column chromatography (DCM/MeOH = 20:1) to provide the compound as white solids (300 mg, yield 50%). HRMS (ESI-TOF) *m*/*z:* [M + H]⁺ Calcd for C₁₆H₂₄N₅O⁺ 302.1975; Found 302.1977.

¹H NMR (400 MHz, CDCl₃) *δ* 7.35 (t, *J =* 1.6 Hz, 1H), 7.14 (d, *J =* 7.7 Hz, 1H), 6.88 (d, *J* = 7.4 Hz, 2H), 6.06 (t, *J* = 1.7 Hz, 1H), 5.34 (s, 1H), 4.36 (dd, *J* = 5.5, 3.3 Hz, 2H), 4.26 (d, *J* = 5.8 Hz, 2H), 3.69 (d, *J =* 1.8 Hz, 3H), 2.53 (s, 6H), 2.31 (s, 3H).

### Example 66. Preparation of compound 3-2-1 of the present invention

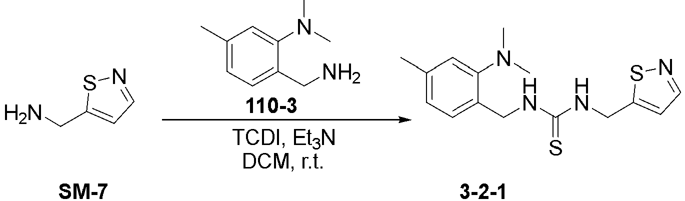

### 1. Synthesis of compound 110-3

Compound **110-3** was synthesized by referring to Example 1.

### 2. Synthesis of compound 3-2-1

**SM-7** (550 mg, 4.82 mmol) was added to a reaction flask, and then dissolved with dichloromethane (20 mL), to which were added triethylamine (1.46 g, 14.47 mmol) and TCDI (858 mg, 4.82 mmol), and the mixture was stirred at room temperature for 2 h. Subsequently, **110-3** (790 mg, 4.82 mmol) was added to the system, and the mixture was allowed to react at room temperature overnight. After completion of the reaction, the solvent was removed by evaporation under reduced pressure to obtain the crude product, which was purified by silica gel column chromatography (methanol/dichloromethane (v/v) = 3%), to obtain **3-1-1** as light yellow solid (550 mg, yield: 37%). MS Calcd.: 321.1[M+H] ⁺; MS Found: 321.4 [M + H]⁺.

¹H NMR (400 MHz, DMSO-d₆) *δ*: 8.34 (d, *J =* 1.4 Hz, 1H), 7.22-7.12 (m, 2H), 6.95 (d, *J* = 9.2 Hz, 1H), 6.90-6.85 (m, 1H), 5.10 (s, 2H), 4.83-4.72 (m, 2H), 2.67 (s, 6H), 2.29 (s, 3H).

### Example 67. Preparation of compound 3-2-2 of the present invention

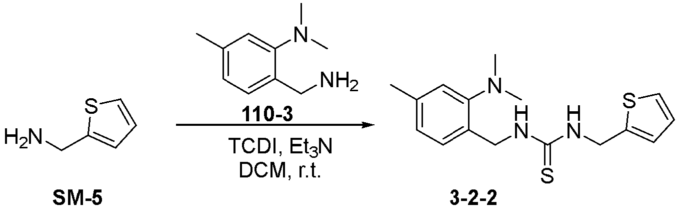

### 1. Synthesis of compound 110-3

Compound **110-3** was synthesized by referring to Example 1.

### 2. Synthesis of compound 3-2-2

**SM-5** (500 mg, 4.39 mmol) was added to a reaction flask, and then dissolved with dichloromethane (20 mL), to which were added triethylamine (1.33 g, 13.16 mmol) and TCDI (781 mg, 4.39 mmol), and the mixture was stirred at room temperature for 2 h. Subsequently, **110-3** (720 mg, 4.39 mmol) was added to the system, and the mixture was allowed to react at room temperature overnight. After completion of the reaction, the solvent was removed by evaporation under reduced pressure to obtain the crude product, which was purified by silica gel column chromatography (methanol/dichloromethane (v/v) = 3%), to obtain **3-2-2** as light yellow solid (405 mg, yield: 29%). MS Calcd.: 320.1 [M+H] ⁺; MS Found: 320.4 [M + H]⁺.

¹H NMR (400 MHz, CD₃OD) *δ* 7.35 (dd, *J* = 0.8, 4.8 Hz, 1H), 7.20 (d, *J =* 7.6 Hz, 1H), 7.06 (s, 1H), 7.02 (d, *J* = 3.2 Hz, 1H), 6.97 (q, *J =* 5.2 Hz, 2H), 4.57 (s, 2H), 4.34 (s, 2H), 2.63 (s, 6H), 2.32 (s, 1H).

### Example 68. Preparation of compound 3-2-3 of the present invention

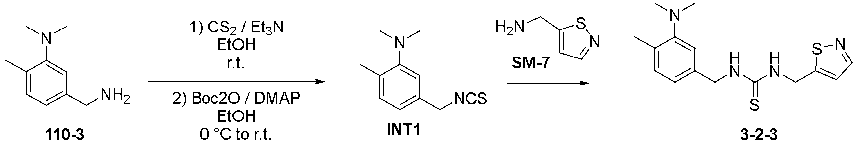

### 1. Synthesis of compound 110-3

Compound **110-3** was synthesized by referring to Example 1.

### 2. Synthesis of compound INT-1

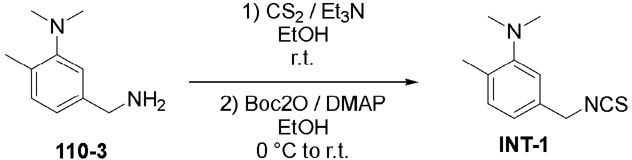

**110-3** (1.64 g, 10 mmol) was dissolved in ethanol (20 mL), to which were added carbon disulfide (1.81 mL, 30 mmol) and triethylamine (1.39 mL, 10 mmol) at room temperature, and then the mixture was allowed to react at room temperature for 1 h. Then, DMAP (37 mg, 0.3 mmol) and Boc₂O (2.3 mL, 10 mmol) were added at 0 °C, and the mixture was allowed to react overnight at room temperature. After completion of the reaction, the reaction solution was extracted three times with water and ethyl acetate. The organic phase was dried over anhydrous sodium sulfate, and concentrated under reduced pressure. The residue was purified by column chromatography (PE/EtOAc = 40:1) to obtain compound **INT-1** as a yellow oil (824 mg, yield 67%).

### 3. Synthesis of compound 3-2-3

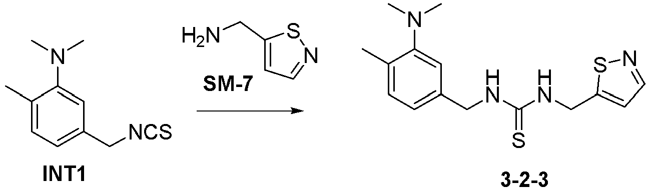

**INT1** (237 mg, 1.15 mmol) and **SM-7** (174 mg, 1.15 mmol) were dissolved in toluene (3 mL), and the mixture was reacted overnight at room temperature. After completion of the reaction, the reaction was subjected to column chromatography (PE/EtOAc = 2:1) to obtain compound **3-2-3** as a yellow oil (210 mg, 57% yield). HRMS (ESI-TOF) *m*/*z:* [M + H]⁺ Calcd for C₁₅H₂₀N₄S₂H⁺ 321.1203; Found 321.1207.

¹H NMR (400 MHz, CDCl₃) *δ* 8.32 (d, *J =* 1.2 Hz, 1H), 7.11 (d, *J =* 7.6 Hz, 1H), 7.06 (s, 1H), 6.90 (s, 1H), 6.83 (d, *J* = 7.6 Hz, 1H), 6.42 (s, 1H), 6.16 (s, 1H), 5.05 (d, *J =* 5.8 Hz, 2H), 4.48 (s, 1H), 2.66 (s, 6H), 2.29 (s, 3H).

### Example 69. Preparation of compound 3-2-4 of the present invention

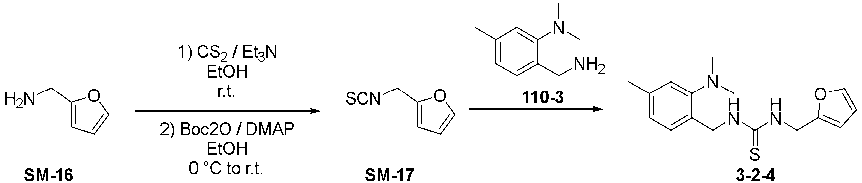

### 1. Synthesis of compound SM-17

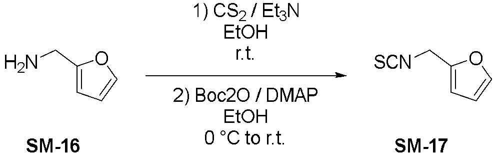

**SM-16** (1.77 mL, 20 mmol) was dissolved in EtOH (40 mL), to which were added CS₂ (3.61 mL, 60 mmol) and triethylamine (2.78 mL, 20 mmol) at room temperature, and then the mixture was allowed to react for 1 h at room temperature. Then, DMAP (73 mg, 0.6 mmol) and Boc₂O (4.6 mL, 20 mmol) were added at 0 °C, and the mixture was allowed to react overnight at room temperature. After completion of the reaction, the reaction solution was diluted by adding water (50 mL), and then extracted with EtOAc (50 mL × 3). The organic phase was dried over anhydrous Na₂SO₄, and concentrated under reduced pressure. The residue was purified by column chromatography (PE/EtOAc = 40:1) to obtain compound **SM-17** as a yellow oil (2.35 g, yield 85%).

### 2. Synthesis of compound 110-3

Compound **110-3** was synthesized by referring to Example 1.

### 3. Synthesis of compound 3-2-4

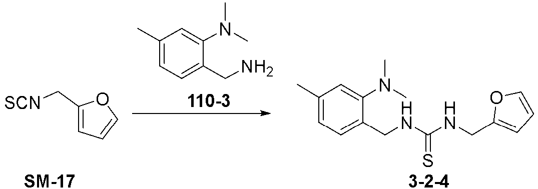

**110-3** (247 mg, 1.5 mmol) and **SM-17** (209 mg, 1.5 mmol) were dissolved in toluene (4.5 mL), and then the mixture was allowed to react at room temperature for 2 h. After completion of the reaction, the reaction was subjected to column chromatography (PE/EtOAc = 3:1), to obtain compound **3-2-4** as white solids (342 mg, yield 75%). HRMS (ESI-TOF) *m*/*z:* [M + H]⁺ Calcd for C₁₆H₂₁N₃OSH⁺ 304.1479; Found 304.1471.

¹H NMR (600 MHz, CDCl₃) *δ* 7.37 (d, *J =* 1.0 Hz, 1H), 7.13 (d, *J =* 7.8 Hz, 1H), 6.95-6.91 (m, 2H), 6.34 (dd, *J =* 3.2, 2.0 Hz, 1H), 6.24 (d, *J =* 3.0 Hz, 1H), 4.78 (s, 2H), 4.30 (s, 2H), 2.56 (s, 6H), 2.32 (s, 3H).

### Example 70. Preparation of compound 3-2-5 of the present invention

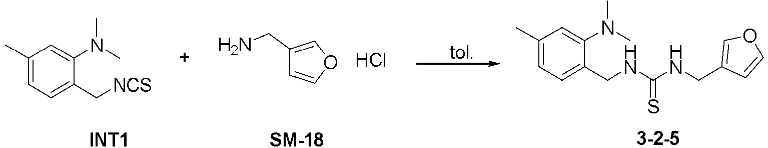

### 1. Synthesis of compound INT1

Compound **INT1** was synthesized according to Example 63.

### 2. Synthesis of compound 3-2-5

**INT-1** (309 mg, 1.5 mmol) and **SM-18** (200 mg, 1.5 mmol) were dissolved in toluene (4.5 mL), and the mixture was allowed to react at room temperature for 2 h. After completion of the reaction, the reaction was subjected to column chromatography (PE/EtOAc = 3:1), to provide compound **3-2-5** as white solids (170 mg, yield 40%). HRMS (ESI-TOF) *m*/*z:* [M + H]⁺ Calcd for C₁₆H₂₁N₃OSH⁺ 304.1479; Found 304.1473.

¹H NMR (400 MHz, CDCl₃) *δ* 7.38 (s, 1H), 7.30 (s, 1H), *7.16* (d, *J =* 7.7 Hz, 1H), 6.98-6.86 (m, 2H), 6.35 (s, 1H), 4.58 (s, 2H), 4.35 (s, 2H), 2.52 (s, 6H), 2.32 (s, 3H).

### Example 71. Preparation of compound 3-2-6 of the present invention

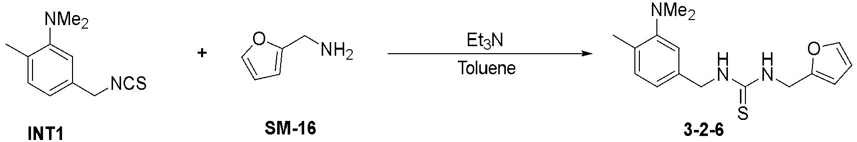

### 1. Synthesis of compound INT-1

Compound **INT1** was synthesized according to Example 63.

### 2. Synthesis of compound 3-2-6

**SM-16** (107 mg, 1.1 mmol) was placed into a 10 mL flask, to which were added toluene (3 mL) and triethylamine (0.18 mL, 1.2 mmol), and then the mixture was stirred at room temperature for 2 h, followed by adding **INT-1** (206 mg, 1.0 mmol). The mixture was stirred and allowed to react at room temperature for about 5 h, until the raw materials were completely consumed. The reaction solution was purified by silica gel column chromatography (PE: EtOAc = 10:1-3:1) to obtain **3-2-6** as white solids (287 mg, yield 95%). HRMS (ESI-TOF) *m*/*z*: [M+H]⁺ Calcd for C₁₆H₂₂N₃OS⁺ 304.1479; Found 304.1512.

¹H NMR (400 MHz, CDCl₃) *δ* 7.32 (dd, *J* = 1.9, 0.9 Hz, 1H), 7.11 (d, *J =* 7.6 Hz, 1H), 6.92 (d, *J* = 1.8 Hz, 1H), 6.84 (dd, *J* = 7.7, 1.8 Hz, 1H), 6.34-6.18 (m, 3H), 6.06 (s, 1H), 4.66 (d, *J =* 5.3 Hz, 2H), 4.58-4.42 (m, 2H), 2.67 (s, 6H), 2.29 (s, 3H).

### Example 72. Preparation of compound 3-2-7 of the present invention

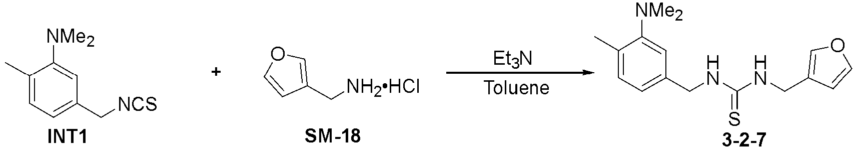

### 1. Synthesis of compound INT-1

Compound **INT-1** was synthesized according to Example 63.

### 2. Synthesis of compound 3-2-7

**SM-18** (146 mg, 1.1 mmol) was placed into a 10 mL flask, to which were added toluene (3 mL) and triethylamine (0.18 mL, 1.2 mmol), and then the mixture was stirred at room temperature for 2 h, followed by adding **INT-1** (206 mg, 1.0 mmol). The mixture was stirred and allowed to react at room temperature for about 5 h. The reaction solution was directly purified by silica gel column chromatography (eluent: PE: EtOAc = 10:1-3:1) to obtain **3-2-7** as white solids (287 mg, yield 95%). HRMS (ESI-TOF) *m*/*z*: [M+Na]⁺ Calcd for C₁₆H₂₁N₃NaOS⁺ 326.1298; Found 326.1308.

¹H NMR (400 MHz, CDCl₃) δ 7.36 (d, *J =* 1.7 Hz, 1H), 7.30 (s, 1H), 7.10 (d, *J* = 7.6 Hz, 1H), 6.90 (d, *J* = 1.8 Hz, 1H), 6.82 (dd, *J* = 7.5, 1.8 Hz, 1H), 6.38-6.14 (m, 2H), 5.95 (s, 1H), 4.48 (d, *J =* 5.8 Hz, 4H), 2.66 (s, 6H), 2.29 (s, 3H). ¹³C NMR (101 MHz, CDCl₃) δ 181.8, 153.3, 143.6, 140.2, 134.7, 131.7, 131.7, 121.4, 121.3, 117.6, 110.0, 48.5, 44.0, 39.8, 18.3.

### Example 73. Preparation of compound 3-2-8 of the present invention

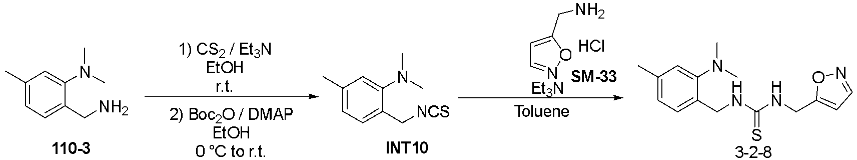

### 1. Synthesis of compound 110-3

Compound **110-3** was synthesized according to Example 1.

### 2. Synthesis of compound INT10

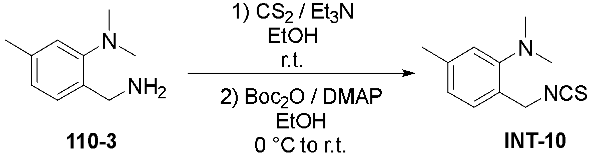

**110-3** (986 g, 6 mmol) was dissolved in absolute EtOH (12 mL), to which were added CS₂ (1.1 mL, 18 mmol) and triethylamine (0.84 mL, 6 mmol) at room temperature, and then the mixture was allowed to react for 1 h at room temperature. Then, DMAP (22 mg, 0.18 mmol) and Boc₂O (1.38 mL, 6 mmol) were added at 0 °C, and the mixture was allowed to react overnight at room temperature. After completion of the reaction, the reaction solution was extracted with water and EtOAc three times. The organic phase was dried over anhydrous Na₂SO₄, and concentrated under reduced pressure. The residue was purified by column chromatography (PE/EtOAc = 40:1) to obtain compound **INT-10** as a yellow oil (778 mg, yield 63%).

### 3. Synthesis of compound 3-2-8

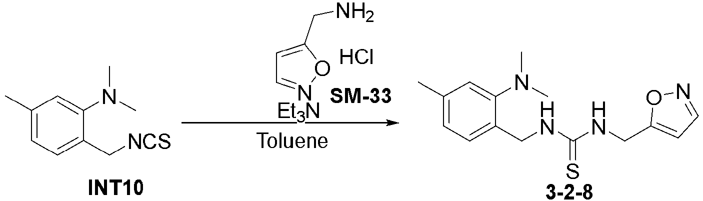

**INT-10** (412 mg, 2 mmol) and **SM-33** (268 mg, 2 mmol) were dissolved in toluene (4.5 mL), to which was added triethylamine (404 mg, 4 mmol), and then the mixture was reacted at room temperature for 4 h. After completion of the reaction, the reaction was subjected to column chromatography (DCM/MeOH = 20:1), to provide compound **3-2-8** as white solids: 150 mg, yield 25%. HRMS (ESI-TOF) *m*/*z:* [M + H]⁺ Calcd for C₁₆H₂₁N₄OS⁺ 305.1431; Found 305.1430.

¹H NMR (400 MHz, CDCl₃) *δ* 9.29 (s, 1H), 8.17 (d, *J =* 1.7 Hz, 1H), 7.26 (s, 6H), 7.14 (d, *J =* 7.7 Hz, 1H), 6.96 (d, *J=* 7.6 Hz, 2H), 6.84 (s, 1H), 6.16 (d, *J* = 1.7 Hz, 1H), 4.99 (d, *J* = 5.5 Hz, 2H), 4.31 (s, 2H), 2.66 (s, 6H), 2.33 (s, 3H).

### Example 74. Preparation of compound 3-2-9 of the present invention

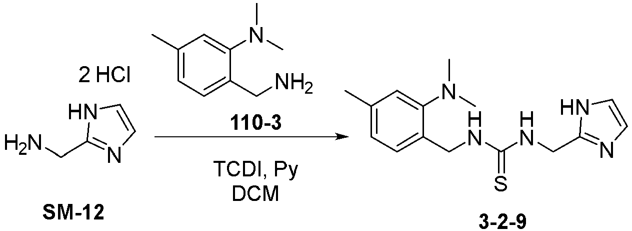

### 1. Synthesis of compound 110-3

Compound **110-3** was synthesized by referring to Example 1.

### 2. Synthesis of compound 3-2-9

**SM-12** (522 mg, 3.09 mmol) was added to a reaction flask, and then dissolved with dichloromethane (20 mL), to which were added pyridine (732 mg, 9.27 mmol) and TCDI (550 mg, 3.09 mmol), and the mixture was stirred at room temperature for 2 h. Subsequently, **110-3** (506 mg, 3.09 mmol) was added to the system, and the mixture was allowed to react at room temperature overnight. After completion of the reaction, the solvent was removed by evaporation under reduced pressure to obtain the crude product, which was purified by column chromatography [Eluent: dichloromethane-methanol: 95:5] and prep-HPLC, to obtain **3-2-9** as off-white solid (105.25 mg, yield 11%). MS Calcd.: 304.2 [M+H] ⁺; MS Found: 304.3 [M + H]⁺.

¹H NMR (400MHz, DMSO-d₆) *δ* 11.89 (s, 1H), 7.87 (br, 2H), 7.11 (d, *J =* 7.6 Hz, 1H), 7.02~6.83 (m, 4H), 4.65 (m, 4H), 2.59 (s, 6H), 2.26 (s, 3H).

### Example 75. Preparation of compound 3-2-10 of the present invention

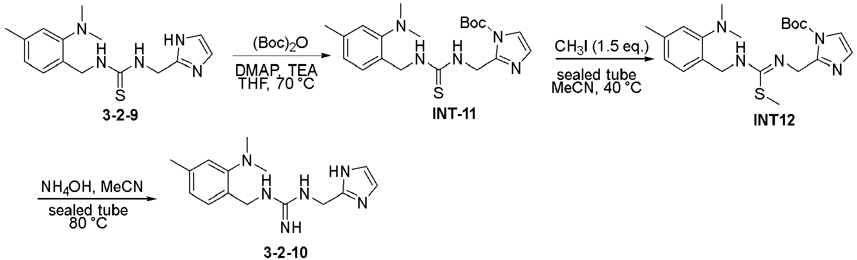

### 1. Synthesis of compound 3-2-9

Compound **3-2-9** was synthesized by referring to Example 74.

### 2. Synthesis of compound INT-11

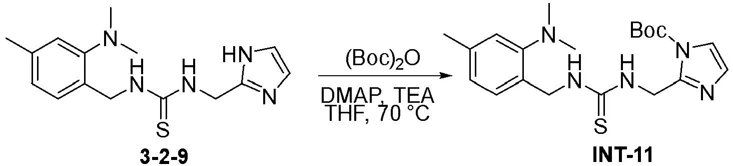

**3-2-9** (350 mg, 1.16 mmol) was added to a reaction flask, and then dissolved with tetrahydrofuran (20 mL), to which were successively added triethylamine (351 mg, 3.40 mmol), DMAP (15 mg, 0.12 mmol), and (Boc)₂O (506 mg, 2.32 mmol), and the mixture was stirred at 70 °C overnight. After completion of the reaction, the solvent was removed by evaporation under reduced pressure, to obtain the crude product, which was purified by column chromatography [eluent: dichloromethane-methanol (100:1-95:5)] to obtain **INT-11** (410 mg, yield 88%). MS Calcd.: 404.2 [M+H] ⁺; MS Found: 404.3 [M + H]⁺.

### 3. Synthesis of compound INT-12

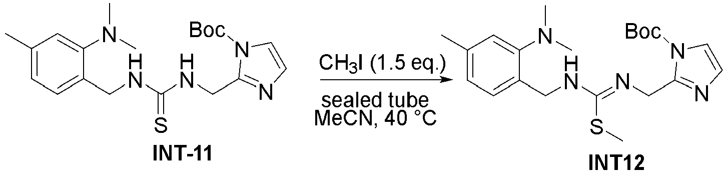

**INT-11** (403 mg, 1.0 mmol) was added to a reaction tube, and dissolved with acetonitrile (10 mL), to which was then added iodomethane (213 mg, 1.5 mmol). The tube was sealed, and the mixture was stirred and reacted at 40 °C overnight. After completion of the reaction, the solvent was removed by evaporation under reduced pressure to obtain the crude product, which was purified by column chromatography [petroleum ether: ethyl acetate (100:1-2:1)] to obtain **INT-12** (400 mg, yield: 95%) as light yellow solid. MS Calcd.: 417.2 [M+H] ⁺; MS Found: 417.8 [M + H]⁺.

### 4. Synthesis of compound 3-2-10

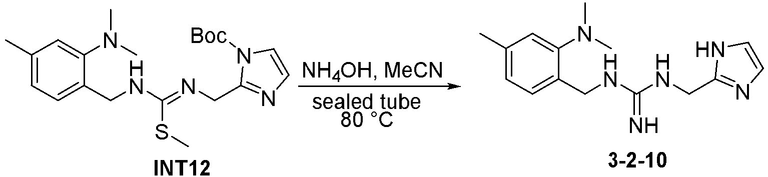

**INT-12** (400 mg, 0.96 mmol) was added to a sealed tube, and dissolved with acetonitrile (10 mL), to which was then added ammonia water (5 mL), and the mixture was stirred and reacted at 80 °C overnight. After completion of the reaction, the solvent was removed by evaporation under reduced pressure to obtain the crude product, which was subjected to column chromatography [dichloromethane: methanol (100:1-5:1)] to obtain **3-2-10** as grayish-white solid (106.86 mg, yield: 39%). MS Calcd.: 287.2 [M+H] ⁺; MS Found: 287.3 [M + H]⁺.

¹H NMR (400 MHz, CD₃OD) *δ*: 7.21 (d, *J* = 8.0 Hz, 1H), 7.06 (s, 1H), 7.01 (s, 2H), 6.96 (d, *J* = 7.6 Hz, 1H), 4.42 (s, 2H), 4.35 (s, 2H), 2.61 (s, 6H), 2.32 (s, 3H).

### Example 76. Preparation of compound 3-2-11 of the present invention (P3-2-9-2)

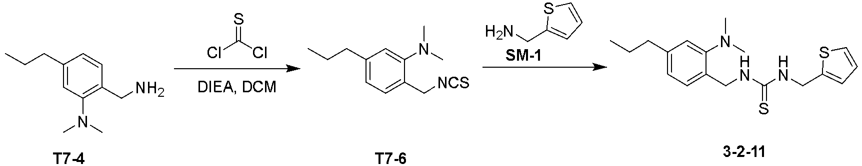

### 1. Synthesis of compound T7-4

Compound **T7-4** was synthesized by referring to Example 53.

### 2. Synthesis of compound T7-6

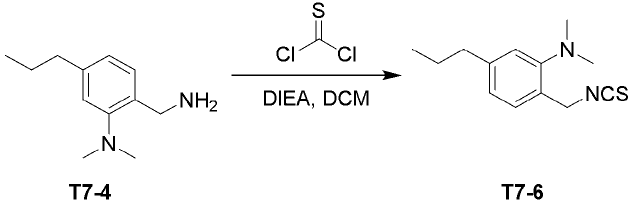

At 0 °C, to a solution of **T7-4** (1 g, 5.2 mmol) and DIEA (1.7 mL, 10.4 mmol) in dichloromethane (10 mL), was gradually added thiophosgene (0.44 mL, 5.7 mmol), and then the mixture was stirred at room temperature for 2 h. After completion of the reaction, the solvent was removed under reduced pressure to obtain **T7-6** (1.2 g, yield 98%).

### 3. Synthesis of compound 3-2-11

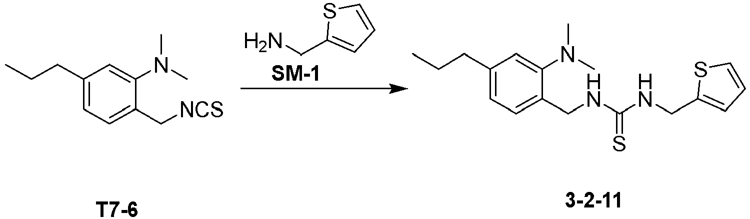

To a solution of **T7-6** (1.2 g, 5.1 mmol) in tetrahydrofuran (10 mL), were added **SM-1** (1.16 g, 10.2 mmol) and DIEA (2 mL). The mixture was stirred at 80 °C for 3 h. After completion of the reaction, water was added to quench the reaction, followed by extraction with ethyl acetate. The organic phase was dried over anhydrous sodium sulfate, and the solvent was removed by evaporation under reduced pressure to obtain the crude product, which was purified by column chromatography to provide **3-2-11** as white solids (331 mg, yield 19%). LCMS: m/z calculated for [M+H]⁺ 348.5, found 348.1.
¹H NMR (400 MHz, DMSO-*d*₆) *δ* 7.94-7.76 (m, 1H), 7.40-7.38 (m, 1H), 7.11 - 7.84 (m, 6H), 4.87-4.69 (m, 4H), 2.71-2.52 (m, 8H), 1.60-1.51 (m, 2H), 0.90-0.87 (m, 3H).

### Example 77. Preparation of compound 3-2-12 of the present invention

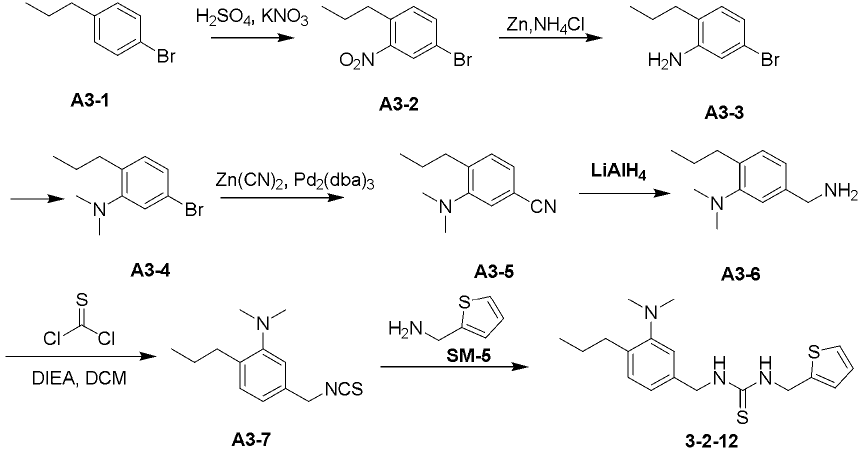

### 1. Synthesis of A3-2

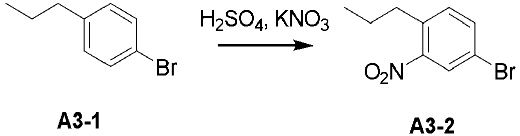

At 0 °C, to a solution of compound **A3-1** (10 g, 50.2 mmol) in sulfuric acid (10 mL), was added potassium nitrate (5.6 g, 55.3 mmol), and the mixture was stirred for 30 min. After completion of the reaction, the reaction solution was concentrated under reduced pressure, and then 200 mL of water was added. The reaction solution was extracted with ethyl acetate. The organic phase was dried over anhydrous sodium sulfate and concentrated. The crude product was purified by column chromatography to obtain **A3-2** (4 g, yield 33%).

### 2. Synthesis of A3-3

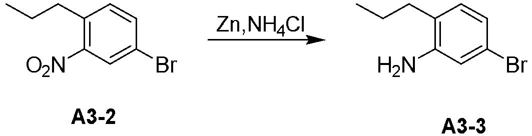

At 0 °C, to a solution of compound **A3-2** (4 g, 16.39 mmol) in water/2,6-dioxane (4/16 mL), were added zinc (5.36 g, 81.94 mmol) and ammonium chloride (5.26 g, 98.32 mmol), and the mixture was stirred for 2 h. After completion of the reaction, the reaction solution was concentrated under reduced pressure, and then 20 mL of water was added. The resultant solution was extracted with ethyl acetate. The organic phase was dried over anhydrous sodium sulfate and concentrated. The crude product was purified by column chromatography to obtain A3-3 (3.5 g, yield 99%). *m*/*z* calculated for [M+H]⁺ 151.1, found 151.1.

### 3. Synthesis of A3-4

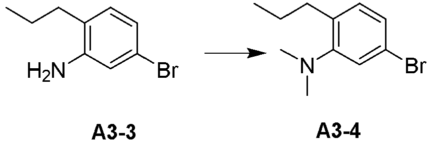

At 0 °C, to a solution (35 mL) of compound **A3-3** (3.5 g, 16.35 mmol) in methanol, acetic acid (0.94 mL, 16.35 mmol), paraformaldehyde (8.2 g, 81.74 mmol), and sodium borohydride (3.09 g, 81.74 mmol) were added, and the mixture was stirred for 16 h. After completion of the reaction, the reaction solution was concentrated under reduced pressure, and then 200 mL of water was added. The mixture was extracted with ethyl acetate. The organic phase was dried over anhydrous sodium sulfate and concentrated. The crude product was purified by column chromatography to obtain **A3-4** as a yellow oil (3.5 g, yield 88%). *m*/*z* calculated for [M+H]⁺ 224.2, found 224.0.

### 4. Synthesis of A3-5

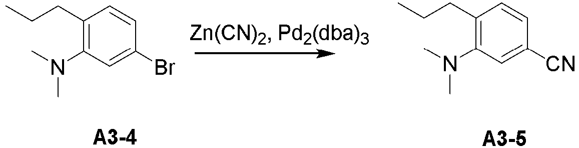

Under nitrogen protection, to a solution of compound **A3-4** (3.5 g, 14.45 mmol) in methanol (35 mL), were added Zn(CN)₂ (5 g, 42.74 mmol), zinc (2.83 g, 43.36 mmol), Pd₂(dba)₃ (0.66 g, 0.72 mmol), Pd(dppf)Cl₂ (2.12 g, 2.89 mmol), and DMA (30 mL), and then the mixture was stirred at 150 °C for 16 h. After completion of the reaction, the reaction solution was concentrated under reduced pressure, and then 200 mL of water was added. The resultant solution was extracted with ethyl acetate. The organic phase was dried over anhydrous sodium sulfate and concentrated. The crude product was purified by column chromatography to obtain **A3-5** as a yellow oil (2.7 g, yield 99%). m/z calculated for [M+H]⁺ 189.3, found 189.1.

### 5. Synthesis of A3-6

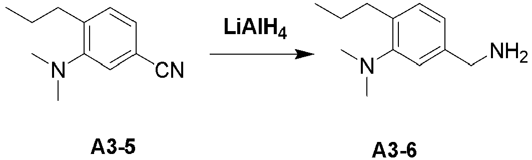

To a solution of compound **A3-5** (1.7 g, 9.03 mmol) in tetrahydrofuran (10 mL), was gradually added lithium aluminum hydride (14.45 mL), and then the mixture was stirred at 40 °C for 2 h. After completion of the reaction, the reaction solution was concentrated under reduced pressure, and then 200 mL of water was added. The resultant solution was extracted with ethyl acetate. The organic phase was dried over anhydrous sodium sulfate and concentrated. The crude product was purified by column chromatography to obtain **A3-6** as a yellow oil (1.0 g, yield 58%). m/z calculated for [M+H] ⁺ 193.3, found 193.1.

### 6. Synthesis of A3-7

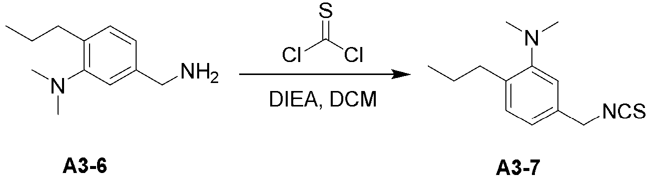

To a solution of compound **A3-6** (500 mg, 2.60 mmol) in dichloromethane (10 mL), were added DIEA (1008.16 mg, 7.80 mmol) and thiophosgene (358.7 mg, 3.12 mmol), and the mixture was stirred for half an hour. After completion of the reaction, the reaction solution was concentrated under reduced pressure to obtain **A3-7** as a black oil (0.50 g, yield 82%). m/z calculated for [M+H]⁺ 235.4, found 235.1.

### 7. Synthesis of 3-2-12

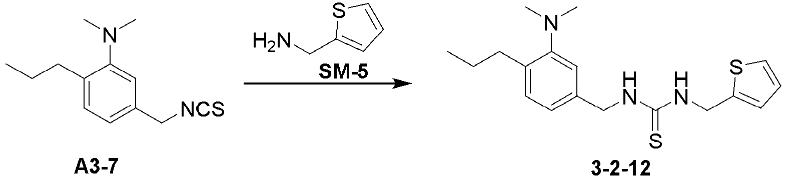

To a solution of compound **A3-7** (500 mg, 2.13 mmol) in tetrahydrofuran (10 mL), were added DIEA (827.25 mg, 6.40 mmol) and thiophen-2-ylmethanamine (289.75 mg, 2.56 mmol), and the mixture was stirred at room temperature for half an hour. After completion of the reaction, the reaction solution was concentrated under reduced pressure to obtain **3-2-12** as white solids (213 mg, yield 28%). m/z calculated for [M+H]⁺ 348.54, found 348.1.

¹HNMR (400 MHz, DMSO-*d₆*) *δ* 7.90 (s, 2H), 7.47 - 7.30 (m, 1H), 7.13 - 7.09 (m, 1H), 7.04 (s, 1H), 7.02 - 6.99 (m, 1H), 6.97 - 6.93 (m, 1H), 6.91 - 6.88 (m, 1H), 4.91 - 4.81 (m, 2H), 4.60 (s, 2H), 2.64 - 2.59 (m, 1H), 2.58 (s, 6H), 2.56 - 2.55 (m, 1H), 1.61 - 1.54 (m, 2H), 0.94 - 0.90 (m, 3H).

### Example 78. Preparation of compound 3-2-13 of the present invention

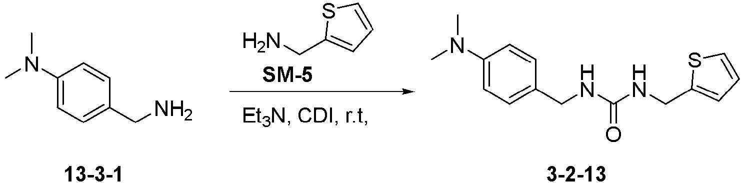

**13-3-1** (300 mg, 2.0 mmol) was added to a reaction flask, and then dissolved with dichloromethane (20 mL), to which were added triethylamine (606 mg, 6.0 mmol) and CDI (630 mg, 2.4 mmol), and the mixture was stirred at room temperature for 2 h. Subsequently, **SM-5** (226 mg, 2.0 mmol) was added to the system, and the mixture was allowed to react at room temperature overnight. After completion of the reaction, the solvent was removed by evaporation under reduced pressure to obtain the crude product, which was purified by silica gel column chromatography (DCM/MeOH=30/1) and reversed-phase prep-HPLC, to obtain **3-2-13** as white solids (155 mg, yield 27%). MS Calcd.: 290.1 [M+H] ⁺; MS Found: 290.4 [M + H]⁺.

¹H NMR (400 MHz, DMSO-*d₆*) *δ* 7.35 (dd, *J =* 4.8, 1.2 Hz, 1H), 7.08 (s, 1H), 7.06 (s, 1H), 6.95-6.93 (m, 2H), 6.66 (d, *J* = 8.8 Hz, 2H), 6.38 (d, *J* = 6.0 Hz, 1H), 6.22 (d, *J =* 5.6 Hz, 1H), 4.38 (d, *J =* 6.0 Hz, 2H), 4.09 (d, *J* = 5.6 Hz, 2H), 2.85 (s, 6H).

### Example 79. Preparation of compound 3-2-14 of the present invention

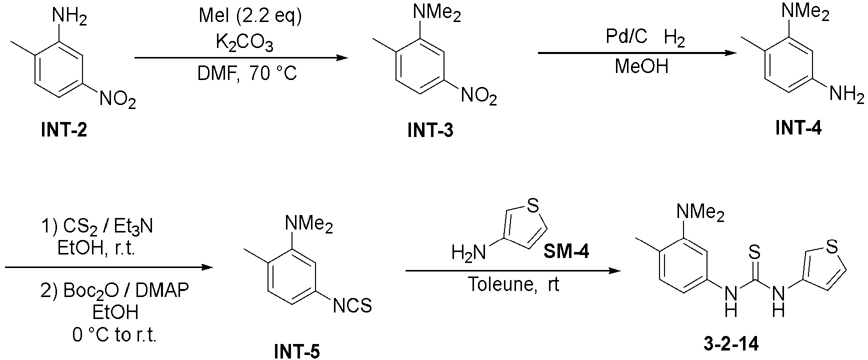

### 1. Synthesis of INT-3

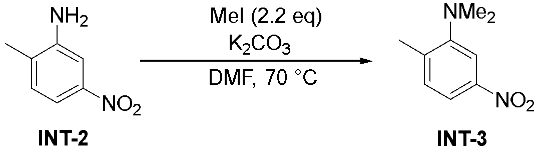

**INT-2** (3.3 g, 20 mmol) was dissolved in DMF (80 mL), to which was added potassium carbonate (6.07 g, 44 mmol). The mixture was stirred at 70 °C and allowed to react overnight, followed by cooling to room temperature. The reaction solution was diluted with water, and then extracted with ethyl acetate. The organic phase was washed twice with saturated saline, dried over anhydrous sodium sulfate, and concentrated at room temperature. The residue was purified by silica gel column chromatography to obtain **INT-3** as a yellow oil (2.5 g, yield 70%).

### 2. Synthesis of INT-4

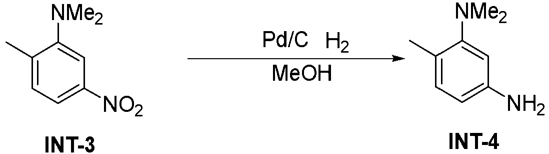

**INT-3** (2.5 g, 14 mmol) was dissolved in methanol (20 mL), to which was added Pd/C (360 mg). The reaction system was purged with argon and then with hydrogen. The reaction was stirred overnight under the pressure of a hydrogen balloon. The reaction solution was filtered through diatomaceous earth, washed with methanol, and concentrated to obtain **INT-4** as a red oil (2.09 g, crude product).

¹H NMR (400 MHz, CDCl₃) *δ* 7.13 (d, *J =* 7.6 Hz, 1H), 6.98 (d, *J* = 1.9 Hz, 1H), 6.89 (dd, *J =* 7.7, 1.8 Hz, 1H), 3.82 (s, 2H), 2.71 (s, 6H), 2.31 (s, 3H).

### 3. Synthesis of INT-5

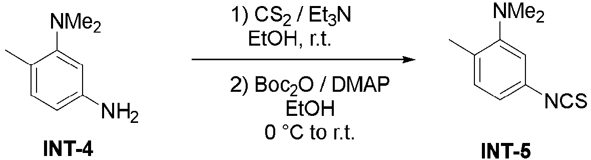

**INT-4** (750 mg, 5 mmol) was dissolved in EtOH (10 mL), to which were added CS₂ (0.9 mL, 15 mmol) and triethylamine (0.7 mL, 5 mmol) at room temperature, and then the mixture was allowed to react for 1 h at room temperature. Then, DMAP (18 mg, 0.15 mmol) and Boc₂O (1.2 mL, 5 mmol) were added at 0 °C, and the reaction solution was warmed to room temperature and allowed to react overnight. After completion of the reaction, the reaction solution was extracted with water and EtOAc three times. The organic phase was dried over anhydrous Na₂SO₄, and concentrated under reduced pressure. The residue was purified by column chromatography (PE/EtOAc = 40:1) to obtain compound **INT-5** as a yellow oil (800 mg, yield 84%).

### 4. Synthesis of 3-2-14

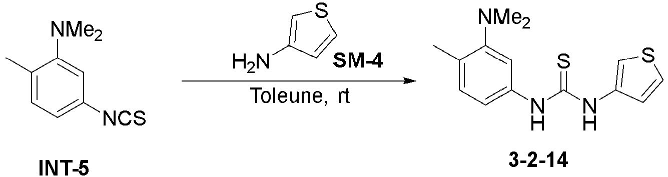

**INT-5** (288 mg, 1.5 mmol) and **SM-4** (150 mg, 1.5 mmol) were dissolved in toluene (5 mL) and reacted overnight at room temperature. After completion of the reaction, the reaction solution was purified by column chromatography (PE/EtOAc = 2:1) to provide compound **3-2-14** as a yellow oil (260 mg, yield 64%). HRMS (ESI-TOF) *m*/*z*: [M+Na]⁺ Calcd for C₁₄H₁₈N₃S⁺ 292.0937; Found 292.0937.

¹H NMR (400 MHz, CDCl₃) *δ* 7.75 (s, 2H), 7.53 (s, 1H), 7.30 (t, *J =* 4.2 Hz, 1H), 7.19 (d, *J* = 7.9 Hz, 1H), 7.06 (dd, *J =* 5.2, 1.1 Hz, 1H), 6.96 (s, 1H), 6.89 (d, *J =* 7.4 Hz, 1H), 2.71 (s, 6H), 2.32 (s, 3H).

### Example 80. Preparation of compound 3-2-15 of the present invention

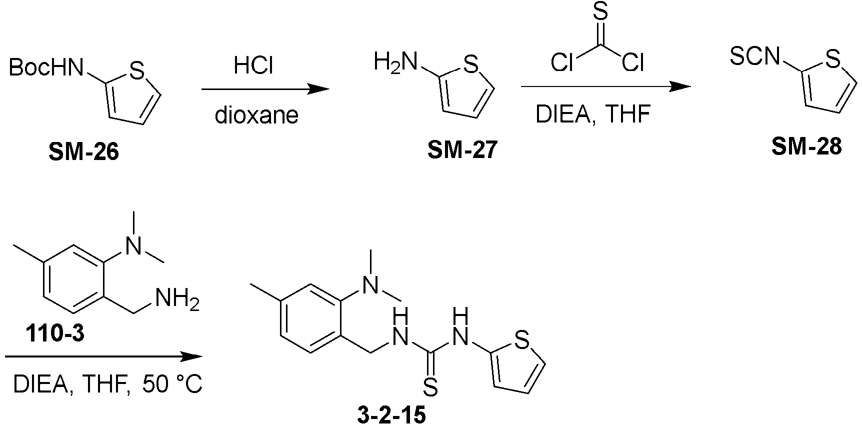

### 1. Synthesis of SM-27

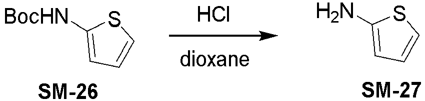

A solution of compound **SM-26** (1 g, 5.02 mmol) in 4N hydrochloric acid/dioxane (20 mL) was stirred at 25 °C for 1 h. The reaction mixture was concentrated to yield compound SM-27 as a black oil (400 mg, yield 80%).

### 2. Synthesis of SM-28

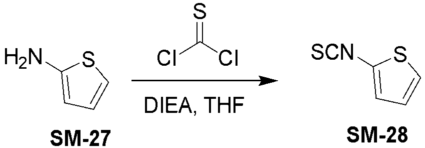

To a solution of compound **SM-27** (400 mg, 4.034 mmol) in THF (15 mL), was added CSCl₂ (463.82 mg, 4.034 mmol) at 0 °C, and then the reaction was stirred for 2 h at 25°C. After completion of the reaction, the reaction mixture was concentrated to obtain compound **SM-28** as a yellow oil (380 mg, yield 67%).

### 3. Synthesis of compound 110-3

Compound **110-3** was synthesized with reference to Example 1.

### 4. Synthesis of 3-2-15

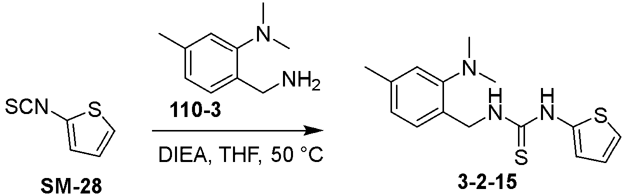

To a solution of compound **110-3** (500 mg, 1.728 mmol) in tetrahydrofuran (20 mL), were added compound **SM-28** (601.8 mg, 4.26 mmol) and DIEA (1.1 g, 8.52 mmol), and the mixture was stirred and reacted at 50 °C for 3 h. After completion of the reaction, water was added, and the resultant solution was extracted with ethyl acetate (50 mL x 3). The organic layer was washed with concentrated brine, dried over anhydrous sodium sulfate, and concentrated under vacuum. The residue was purified by silica gel column chromatography to yield compound **3-2-15** as light orange solid (170.8 mg, yield 13.12%). MS Calcd.: 360.0 [M+H] ⁺; MS Found: 360.0 [M + H]⁺.

¹HNMR (400 MHz, DMSO-*d₆*) δ 10.36 (s, 1H), 8.04 (s, 1H), 7.11 (d, J = 8.0 Hz, 1H), 7.01 (d, J = 4.0 Hz, 1H), 6.96 (s, 1H), 6.88 - 6.85 (m, 2H), 6.72 (d, J = 4.0 Hz, 1H), 4.69 (s, 2H), 2.61 (s, 6H), 2.27 (s, 3H).

### Example 81. Preparation of compound 3-2-16 of the present invention

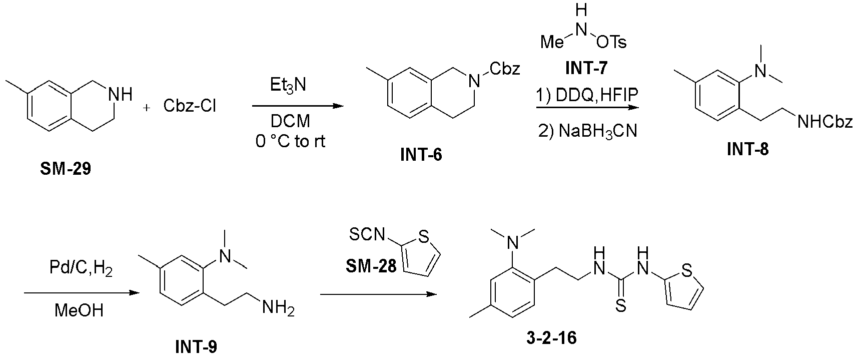

### 1. Synthesis of compound INT-6

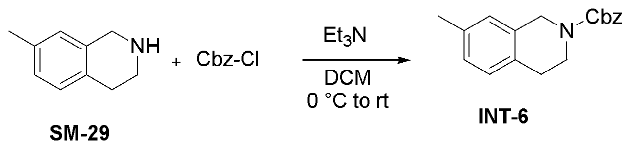

**SM-19** (0.75 mL, 5.0 mmol) was placed in a 25 mL flask, to which were added dichloromethane (14 mL) and triethylamine (0.85 mL, 6.0 mmol), and the mixture was cooled to 0 °C, followed by slowly adding benzyl chloroformate (0.85 mL, 6.0 mmol) dropwise. The reaction solution was stirred at 0 °C for 10 min, then warmed to room temperature and stirred overnight. After completion of the reaction, the reaction solution was washed with water and saturated saline, dried over anhydrous sodium sulfate, and then rotatory evaporated to dry. The residue was purified by silica gel column chromatography (DCM) to obtain **INT-6** (1.26 g, yield 87%) as a colorless oil.

### 2. Synthesis of compound INT-7

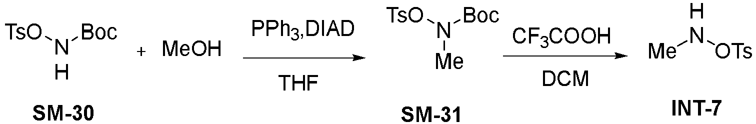

Triphenylphosphine (8.61 g, 30 mmol) was weighed and placed in a 100 mL flask. Under argon protection, tetrahydrofuran (12 mL) and diisopropyl azodicarboxylate (DIAD) (7.2 mL, 36.0 mmol) were added at 0 °C. White solid was rapidly formed. The reaction solution was further stirred at 0 °C for 30 min, to which was then added a solution of methanol (1.2 mL, 30.0 mmol) and **SM-30** (8.61 g, 30.0 mmol) in tetrahydrofuran (12 mL). The reaction was stirred at 0 °C for 2 h, and then warmed to room temperature and stirred overnight. After the reaction was complete, the reaction solution was diluted with water, and extracted with dichloromethane. The organic phases were combined, dried over anhydrous sodium sulfate, and rotatory evaporated to dry. The residue was purified by silica gel column chromatography (PE/EtOAc=10:1) to obtain the product **SM-31** as a colorless oil (5.01 g, yield 71%).

**SM-31** (5.01 g, 16.6 mmol) was dissolved in dichloromethane (17 mL), to which was added trifluoroacetic acid (25.0 mL, 332.0 mmol, 20.0 equiv) at 0 °C, and then the reaction was stirred overnight. After completion of the reaction, the reaction was quenched by adding ice-water at 0 °C. The resultant solution was adjusted to pH 9 with saturated NaHCO₃ solution, and extracted with dichloromethane. The organic phases were combined, dried over anhydrous sodium sulfate, and rotatory evaporated to dryness. The residue was purified by silica gel column chromatography (PE/EtOAc = 5:1) to obtain **INT-7** (729 mg, yield 29%) as a light yellow oil.

### 3. Synthesis of compound INT8

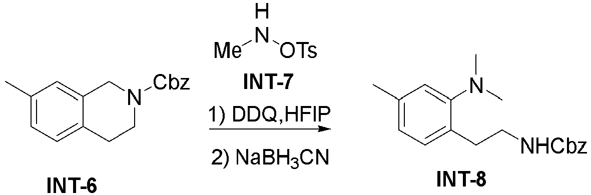

**INT-6** (843 mg, 3.0 mmol) was weighed and placed into a 100 mL flask, to which were added hexafluoroisopropanol (30 mL), 2,3-dichloro-5,6-dicyanoquinone (1.021 g, 4.5 mmol), and **INT-7** (905 mg, 4.5 mmol). Under argon protection, the reaction was stirred at room temperature. After completion of the reaction, sodium cyanoborohydride (900 mg, 15.0 mmol) was added, and the mixture was allowed to react at room temperature. After completion of the reaction, the reaction was quenched with water. The resultant solution was extracted with ethyl acetate. The organic phases were combined, dried over anhydrous sodium sulfate, and rotatory evaporated to dry. The residue was purified by silica gel column chromatography (DCM) to obtain **INT-8** (706 mg, yield 75%) as a light yellow oil.

¹H NMR (400 MHz, CDCl₃) *δ* 7.40 - 7.27 (m, 5H), 7.04 (d, *J* = 7.7 Hz, 1H), 6.94 (s, 1H), 6.86 (d, *J =* 7.5 Hz, 1H), 6.00 (s, 1H), 5.06 (s, 2H), 3.42 (q, *J* = 6.6 Hz, 2H), 2.86 (t, *J* = 6.5 Hz, 2H), 2.66 (s, 6H), 2.31 (s, 3H).

### 4. Synthesis of compound INT-9

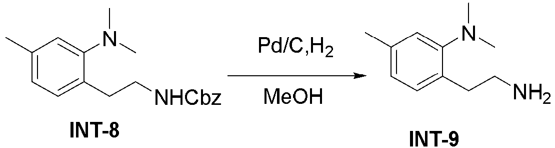

**INT-8** (706 mg, 2.2 mmol) was weighed and placed into a 10 mL test tube, to which were added methanol (3.0 mL) and Pd/C (220 mg). The reaction system was purged with hydrogen gas, and then stirred at room temperature. After completion of the reaction, the reaction solution was filtered through diatomaceous earth, and rotatory evaporated to dry under reduced pressure. The residue was purified by silica gel column chromatography to obtain **INT-9** (390 mg, yield 99%) as light yellow solid.

### 5. Synthesis of compound SM-28

Compound **SM-28** was synthesized according to Example 74.

### 6. Synthesis of compound 3-2-16

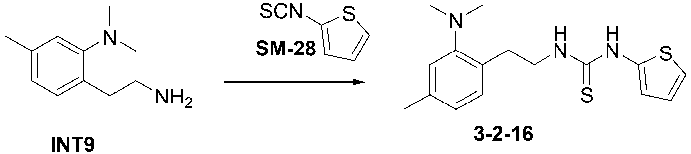

**INT-9** (178 mg, 1.0 mmol) and **SM-28** (141 mg, 1.0 mmol) were dissolved in toluene (2 mL) and allowed to react at room temperature for 2 h. After completion of the reaction, the reaction was subjected to column chromatography (PE/EtOAc = 5:1) to provide compound **3-2-16** (188 mg, yield 59%) as a yellow oil. HRMS (ESI-TOF) m/z: [M + H]⁺ Calcd for C₁₆H₂₁N₃S₂H⁺ 320.1250; Found 320.1248.

¹H NMR (400 MHz, CDCl₃) *δ* 7.55 (s, 1H), 7.14 (dd, *J =* 5.6, 1.0 Hz, 1H), 7.00 (d, *J* = 7.6 Hz, 1H), 6.93-6.86 (m, 2H), 6.82 (d, *J =* 7.4 Hz, 1H), 6.72 (s, 1H), 3.83 (s, 2H), 2.93 (t, *J* = 6.7 Hz, 2H), 2.49 (s, 6H), 2.30 (s, 3H).

### Example 82. Preparation of compound 3-2-17 of the present invention

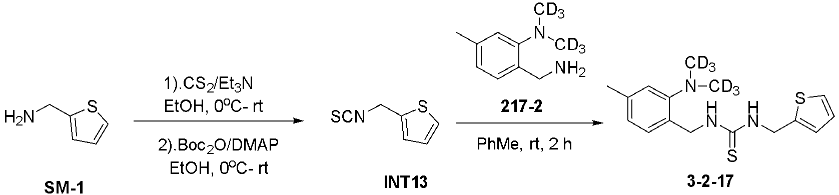

### 1. Synthesis of compound INT-13

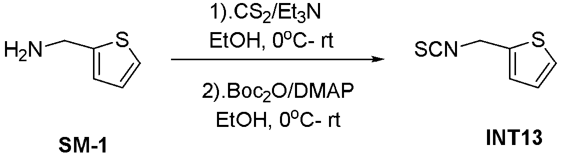

**SM-1** was dissolved in EtOH (8.8 mL), to which were added CS₂ (1.0 g, 13.2 mmol) and triethylamine (445 mg, 4.4 mmol) at room temperature, and then the mixture was allowed to react for 1 h at room temperature. Then, DMAP (16 mg, 0.13mmol) and Boc₂O (960 mg, 4.4 mmol) were added at 0 °C, and the reaction solution was warmed to room temperature and allowed to react overnight. After completion of the reaction, the reaction solution was extracted with water and EtOAc three times. The organic phase was dried over anhydrous Na₂SO₄, and the residue was purified by column chromatography, to obtain compound **INT-13** (260 mg), with a yield of 38%.

### 2. Synthesis of compound 217-2

Compound **217-2** was synthesized with reference to Example 17.

### 3. Synthesis of compound 3-2-17

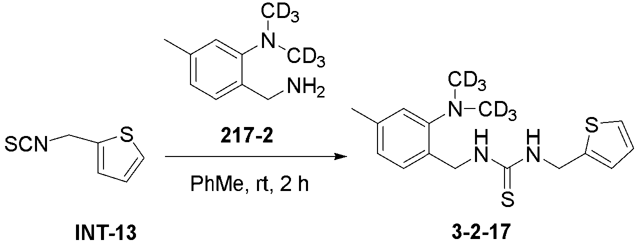

**217-2** (290 mg, 1.7 mmol) and **INT-13** (260 mg, 1.7 mmol) were dissolved in 5.0 ml of toluene, and the mixture was stirred for 2 h. The reaction was detected by TLC. After completion of the reaction, the reaction solution was rotatory evaporated to dry, and the residue was directly purified by column chromatography, to obtain 390 mg of **3-2-17,** with a yield of 70%. MS (ESI) m/z: [M + H]⁺ Calcd for: 326.1; Found: 326.1.

¹H NMR (400 MHz, CDCl₃) *δ* 7.55 (s, 1H), 7.24 - 7.12 (m, 2H), 6.97 - 6.81 (m, 4H), 4.94 (s, 2H), 4.30 (s, 2H), 2.29 (s, 3H). ¹³C NMR (100 MHz, CDCl₃) *δ* 180.5, 150.5, 146.1, 141.1, 138.8, 131.0, 128.3, 126.6, 125.6, 124.9, 120.0, 44.6, 41.8, 21.2.

### Example 83. Preparation of compound 3-2-18 of the present invention

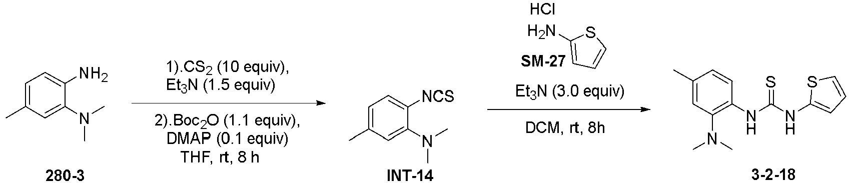

### 1. Synthesis of 280-3

Compound **280-3** was synthesized with reference to Example 13.

### 2. Synthesis of INT-14

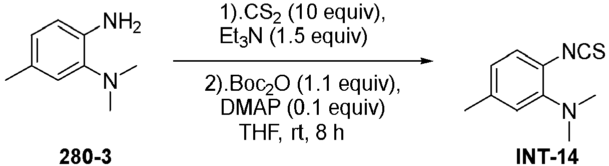

**280-3** (500 mg, 3.3 mmol) was dissolved in tetrahydrofuran (10 mL), to which were added CS₂ (2.5 g, 33 mmol) and triethylamine (506 mg, 5.0 mmol) at room temperature, and then the mixture was allowed to react for 1 h at room temperature. Then, DMAP (40 mg, 0.33 mmol, 0.1 equiv.) and Boc₂O (792 mg, 3.6 mmol) were added at 0 °C, and the reaction solution was warmed to room temperature and allowed to react overnight. After completion of the reaction, the reaction solution was extracted with water and EtOAc three times. The organic phase was dried over anhydrous Na₂SO₄, and the residue was purified by column chromatography, to obtain compound **INT-14** (340 mg), with a yield of 53%.

### 3. Synthesis of 3-2-18

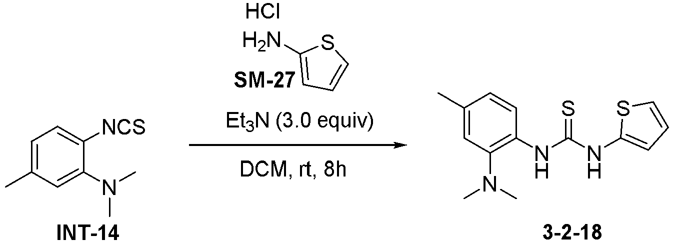

**INT-14** (100 mg, 0.52 mmol) and **SM-27** (87 mg, 0.62 mmol) were dissolved in 4 ml of dichloromethane, to which was added triethylamine (108 µL, 0.78 mmol), and then the mixture was stirred at room temperature. After completion of the reaction, the solution was rotatory evaporated to dry, and the residue was directly subjected to column chromatography, to obtain **3-2-18** (48 mg), with a yield of 31%. MS (ESI) m/z: [M + Na]⁺ Calcd for: 292.1; Found: 282.9.

¹H NMR (400 MHz, CDCl₃) *δ* 8.39 (s, 1H), 7.75 (s, 1H), 7.08 (s, 1H), 7.00 - 6.68 (m, 5H), 2.66 (s, 6H), 2.33 (s, 3H).

### Example 84. Preparation of compound 3-2-19 of the present invention

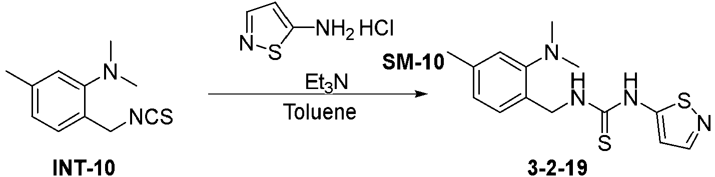

### 1. Synthesis of compound INT-10

Compound **1NT-10** was synthesized according to Example 73.

### 2. Synthesis of compound 3-2-19

**INT-10** (350 mg, 1.7 mmol) and **SM-10** (250 mg, 1.8 mmol) were dissolved in toluene (5 mL), to which was added triethylamine (343 mg, 3.4 mmol), and the mixture was allowed to react for 16 h at room temperature. After completion of the reaction, the reaction was subjected to column chromatography (DCM / MeOH = 20:1), to obtain compound **3-2-19** as white solids: 171 mg, with a yield of 33%. HRMS (ESI-TOF) m/z: [M + H]⁺ Calcd for C₁₆H₂₁N₄OS⁺ 307.1046; Found 307.1048.

¹H NMR (400 MHz, CDCl₃) *δ* 7.98 (d, *J* = 2.0 Hz, 1H), 7.19 (d, *J* = 7.7 Hz, 1H), 6.96 (s, 1H), 6.87 (d, *J* = 7.7 Hz, 1H), 6.21 (t, *J* = 1.9 Hz, 1H), 5.63 (s, 1H), 4.35 (s, 2H), 2.70 (d, *J* = 1.2 Hz, 6H), 2.33 (s, 3H), 1.69 (s, 1H).

### Example 85. Preparation of compound 3-2-20 of the present invention

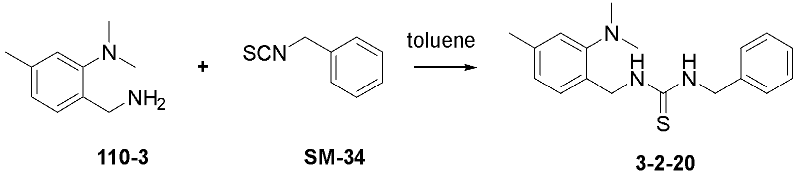

### 1. Synthesis of compound 110-3

Compound **110-3** was synthesized according to Example 1.

### 2. Synthesis of compound 3-2-20

**110-3** (164 mg, 1.0 mmol) and **SM-34** (149 mg, 1.0 mmol) were dissolved in toluene (3.0 mL), and the mixture was allowed to react for 2 h at room temperature. After completion of the reaction, the reaction was subjected to column chromatography (PE/EtOAc = 2:1), to obtain compound **3-2-20** as white solids: 220 mg, with a yield of 59%. HRMS (ESI-TOF) m/z: [M + H]⁺ Calcd for C₁₈H₂₄N₃S⁺ 314.1686; Found 314.1684.

¹H NMR (400 MHz, CDCl₃): *δ* 7.35-7.26 (m, 3H), 7.22 (d, *J* = 7.0 Hz, 2H), 7.16 (d, *J =* 7.6 Hz, 1H), 6.92 (d, *J =* 7.6 Hz, 1H), 6.83 (s, 1H), 4.75 (s, 2H), 4.39 (s, 2H), 2.37 (s, 6H), 2.31 (s, 3H).

### Example 86. Preparation of compound 3-2-21 of the present invention

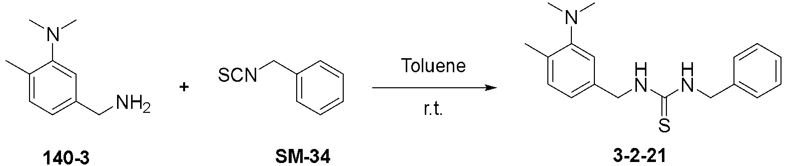

### 1. Synthesis of 140-3

Compound **140-3** was synthesized according to Example 18.

### 2. Synthesis of compound 3-2-21

**140-3** (164 mg, 1.0 mmol) and **B** (149 mg, 1.0 mmol) were dissolved in toluene (3.0 mL), and the mixture was allowed to react for 2 h at room temperature. After completion of the reaction, the reaction was subjected to column chromatography (PE/EtOAc = 2:1), to obtain compound 3-**2-21** as a yellow oil: 203 mg, with a yield of 65%. HRMS (ESI-TOF) m/z: [M + H]⁺ Calcd for C₁₈H₂₄N₃S⁺ 314.1686; Found 314.1689.

¹H NMR (400 MHz, CDCl₃): *δ* 7.36-7.25 (m, 3H), 7.21 (d, *J =* 6.6 Hz, 2H), 7.09 (d, *J =* 7.6 Hz, 1H), 6.89 (s, 1H), 6.80 (d, *J* = 7.6 Hz, 1H), 6.07 (s, 2H), 4.63 (s, 2H), 4.52 (s, 2H), 2.65 (s, 6H), 2.29 (s, 3H).

### Example 87. Preparation of compound 3-2-22 of the present invention

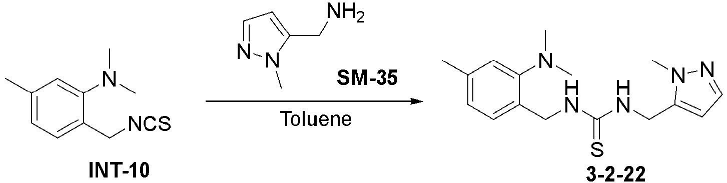

### 1. Synthesis of INT-10

Compound **INT-10** was synthesized according to Example 73.

### 2. Synthesis of compound 3-2-22

**INT-10** (350mg, 1.7 mmol) and **SM-35** (250 mg, 1.8 mmol) were dissolved in toluene (5 mL), and the mixture was allowed to react for 16 h at room temperature. After completion of the reaction, the reaction was subjected to column chromatography (DCM / MeOH = 20:1), to obtain compound **3-2-22** as white solids: 171 mg, with a yield of 32%. HRMS (ESI-TOF) m/z: [M + Na]⁺ Calcd for C₁₆H₂₃N₄OSNa⁺ 340.1566; Found 340.1563.

¹H NMR (400 MHz, CDCl₃) *δ* 7.98 (d, *J* = 2.0 Hz, 1H), 7.19 (d, *J* = 7.7 Hz, 1H), 6.96 (s, 1H), 6.87 (d, *J* = 7.7 Hz, 1H), 6.21 (t, *J* = 1.9 Hz, 1H), 5.63 (s, 1H), 4.35 (s, 2H), 2.70 (d, *J* = 1.2 Hz, 6H), 2.33 (s, 3H), 1.69 (s, 1H).

### Example 88. Preparation of compound 3-2-23 of the present invention

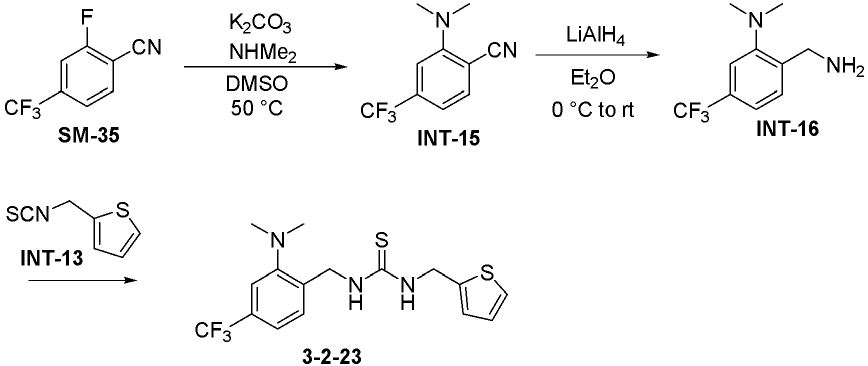

### 1. Synthesis of INT-15

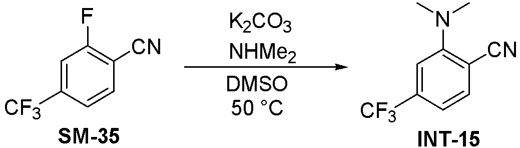

**SM-35** (1.89 g, 10.0 mmol) and potassium carbonate (5.52 g, 40.0 mmol) were weighed and placed in a 100 mL flask, to which were added dimethylamine hydrochloride (1.64 g, 20.0 mmol) and 20 mL of DMSO, and the mixture was stirred at 70 °C overnight. After completion of the reaction, the reaction solution was cooled to room temperature, and then a suitable amount of ethyl acetate was added for dilution. The resultant solution was extracted with water to remove DMSO, dried over anhydrous sodium sulfate, and then rotatory evaporated to dry. The residue was purified by silica gel column chromatography (PE:DCM=5:1), to obtain the product **INT-15** as a light yellow oil (2.04 g, 95% yield).

### 2. Synthesis of INT-16

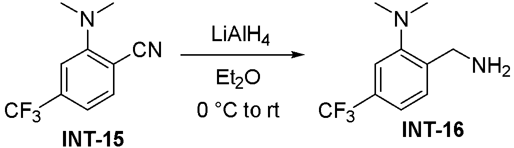

The product **INT-15** (2.04 g, 9.5 mmol) was dissolved in 24 mL of anhydrous diethyl ether, to which was gradually added lithium aluminum hydride (722 mg, 19 mmol) in batches at 0 °C, and then the reaction was stirred at room temperature. After completion of the reaction, 3 mL of water was added at 0 °C for quenching the reaction, followed by adding 3 mL of 15% NaOH solution and 3 mL of water. Then, anhydrous sodium sulfate was added for drying the reaction solution, followed by filtering. The reaction solution was washed with ethyl acetate, and rotary evaporated to dry. The residue was purified by silica gel column chromatography (DCM: MeOH = 20:1) to obtain product **C** as a light yellow oil (1.96 g, yield 95%).

### 3. Synthesis of compound INT-13

Compound **INT-13** was synthesized with reference to Example 82.

### 4. Synthesis of compound 3-2-23

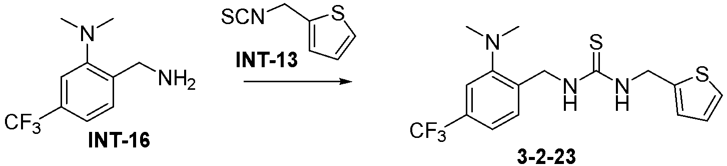

**INT-16** (240 mg, 1.1 mmol) was weighed and placed in a 50 mL flask, to which were added toluene (2.5 mL) and **INT-13** (155 mg, 1.0 mmol), and then the mixture was stirred at room temperature. After completion of the reaction, the reaction was purified by silica gel column chromatography (PE/EtOAc = 5:1) to obtain **3-2-23** as white solids: 363 mg, with a yield of 97%. HRMS (ESI-TOF) *m*/*z:* [M + H]⁺ Calcd for C₁₆H₁₉F₃N₃S₂⁺ 374.0967; Found 374.0996.

¹H NMR (400 MHz, CDCl₃) *δ* 7.43-7.22 (m, 3H), 6.95-6.91 (m, 3H), 6.98-6.88 (m, 2H), 4.91 (d, J = 5.1 Hz, 2H), 4.49 (s, 2H), 2.53 (s, 6H).

### Example 89. Preparation of compound 3-2-24 of the present invention

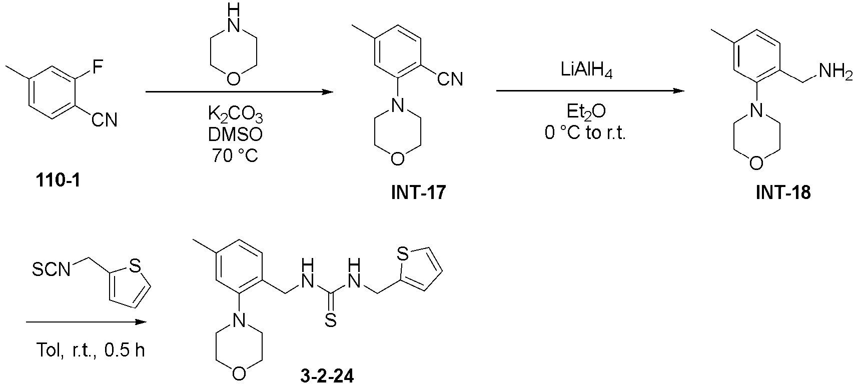

### 1. Synthesis of INT-17

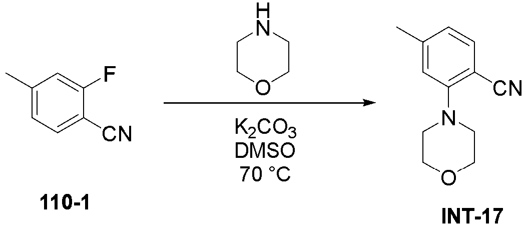

To a solution of **110-1** (1.35 g, 10 mmol) in DMSO (20 mL), were added morpholine (2.6 mL, 30 mmol) and potassium carbonate (4.14 g, 30 mmol), and then the mixture was heated to 70 °C and reacted overnight. After completion of the reaction, the solution was diluted and extracted with ethyl acetate (50 mL × 3 times). The organic layers were combined, dried over anhydrous sodium sulfate, and concentrated under reduced pressure to obtain compound **INT-17** as a yellow oil, which was directly used in the next step.

### 2. Synthesis of INT-18

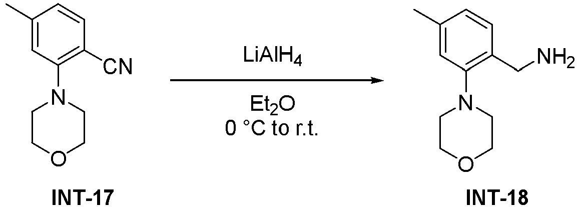

**INT-17** (515 mg, 2.5 mmol, 1.0 equiv) was dissolved in diethyl ether (7 mL), to which was gradually added lithium aluminum hydride (190 mg, 5 mmol) in batches at 0 °C, and then the mixture was allowed to react at room temperature for 2 h. After completion of the reaction, 3 mL of 15% NaOH solution was first added for quenching the reaction, to which were successively added 3 mL of water and 3 mL of 15% NaOH solution dropwise. The resultant solution was filtered through diatomaceous earth and over anhydrous sodium sulfate, and then concentrated under reduced pressure to obtain compound **INT-18** as a yellow oil (332 mg, yield 64%).

### 3. Synthesis of compound INT-13

Compound **INT-13** was synthesized by referring to Example 82.

### 4. Synthesis of 3-2-24

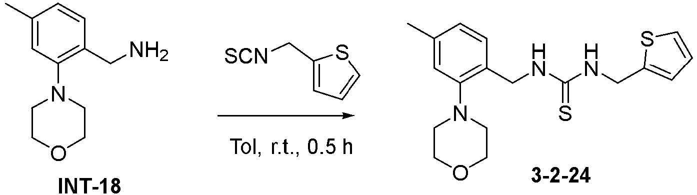

**INT-18** (248 mg, 1.2 mmol, 1.2 equiv.) was dissolved in toluene (2 mL), to which was added **SM-13** (155 mg, 1.0 mmol), and then the mixture was allowed to react for about 0.5 h at room temperature. After completion of the reaction, the reaction was directly purified by column chromatography (DCM), to obtain product **3-2-24** as white solids: 220 mg, yield 61%. HRMS (ESI-TOF) m/z: [M + Na]⁺ Calcd for C₁₈H₂₃N₃NaOS₂⁺ 384.1175; Found 384.1184.

¹H NMR (400 MHz, CDCl₃) *δ* 7.20 (dd, *J =* 9.7, 6.4 Hz, 2H), 7.09 (s, 1H), 6.95 (d, *J* = 7.8 Hz, 1H), 6.92-6.87 (m, 1H), 6.82 (d, *J* = 5.3 Hz, 2H), 4.93 (d, *J* = 5.4 Hz, 2H), 4.55-4.25 (m, 2H), 3.73 (t, *J* = 4.4 Hz, 4H), 2.76 (t, *J* = 4.5 Hz, 4H), 2.33 (s, 3H).

### Example 90. Preparation of compound 3-2-25 of the present invention

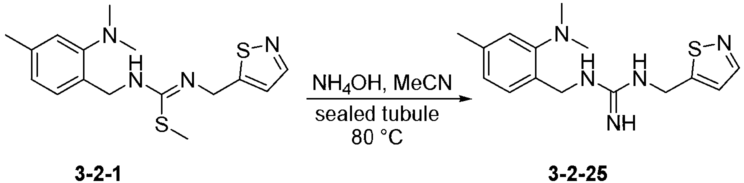

### 1. Synthesis of 3-2-14

Compound **3-2-1** was synthesized by referring to Example 61.

### 2. Synthesis of 3-2-25

**3-2-1** (280 mg, 0.84 mmol) was added to a sealed tube, and then dissolved with acetonitrile (15 mL), to which was added ammonia water (5 mL), followed by sealing the tube. The mixture was stirred and reacted at 80 °C overnight. After completion of the reaction, the solvent was removed under reduced pressure to obtain the crude product, which was purified by column chromatography [dichloromethane: methanol (100:1-5:1)] and prep-HPLC, to obtain **3-2-25** as off-white solid (162.76 mg, 64%). ESI [M+H]⁺ = 304.4.

¹H NMR (400 MHz, DMSO-d₆) *δ*: 8.52 (d, *J =* 1.4 Hz, 1H), 7.99 (s, 1H), 7.81 (s, 2H), 7.33 (d, *J = 0.8* Hz, 1H), 7.09 (d, *J =* 7.6 Hz, 1H), 7.03 (s, 1H), 6.92 (d, *J =* 7.6 Hz, 1H), 4.80 (d, *J* = 6.4 Hz, 2H), 4.34 (d, *J =* 5.6 Hz, 2H),2.61 (s, 6H), 2.29 (s, 3H).

### Example 91. Preparation of compound 3-2-26 of the present invention

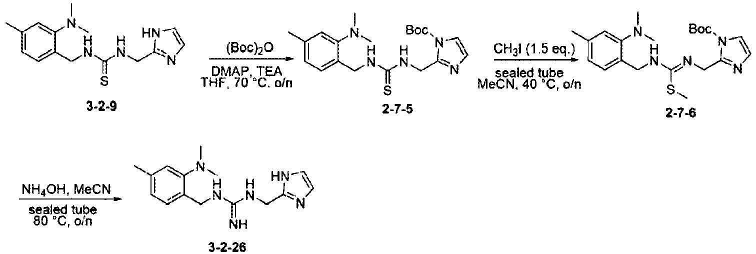

### 1. Synthesis of 3-2-9

Compound **3-2-9** was synthesized by referring to Example 74.

### 2. Synthesis of 2-7-5

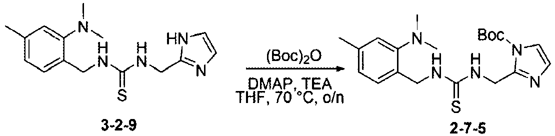

**3-2-9** (350 mg, 1.16 mmol) was added to a reaction flask, and then dissolved with tetrahydrofuran (20 mL), to which were successively added triethylamine (351 mg, 3.40 mmol), DMAP (15 mg, 0.12 mmol), and (Boc)₂O (506 mg, 2.32 mmol), and the mixture was stirred at 70 °C overnight. After completion of the reaction, the solvent was removed by evaporation under reduced pressure, to obtain the crude product, which was purified by column chromatography [eluent: dichloromethane-methanol (100:1-95:5)] to obtain **2-7-5** (410 mg, yield 88%). MS Calcd.: 404.2 [M+H] ⁺; MS Found: 404.2 [M + H]⁺.

### 3. Synthesis of 2-7-6

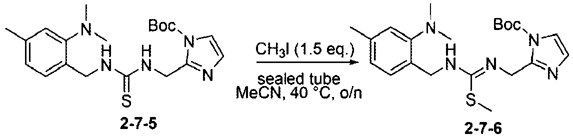

**2-7-5** (403 mg, 1.0 mmol) was added to a reaction tube, and dissolved with acetonitrile (10 mL), to which was then added iodomethane (213 mg, 1.5 mmol). The tube was sealed, and the mixture was stirred and reacted at 40 °C overnight. After completion of the reaction, the solvent was removed by evaporation under reduced pressure to obtain the crude product, which was purified by column chromatography [petroleum ether: ethyl acetate (100: 1-2: 1)] to obtain **2-7-6** (400 mg, yield: 95%) as light yellow solid. MS Calcd.: 418.2 [M+H] ⁺; MS Found: 417.8 [M + H]⁺.

### 4. Synthesis of 3-7-26

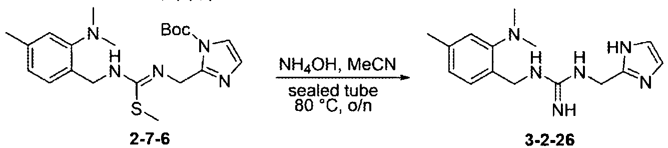

**2-7-6** (400 mg, 0.96 mmol) was added to a sealed tube, and dissolved with acetonitrile (10 mL), to which was then added ammonia water (5 mL), and the mixture was stirred and reacted at 80 °C overnight. After completion of the reaction, the solvent was removed by evaporation under reduced pressure to obtain the crude product, which was subjected to column chromatography [dichloromethane: methanol (100:1-5:1)] and SFC to obtain **3-2-26** as grayish-white solid (106.86 mg, yield: 39%). ESI MS Calcd.: 287.2 [M+H] ⁺; MS Found: 287.3 [M + H]⁺.

¹H NMR (400 MHz, CD₃OD) *δ*: 7.21 (d, *J* = 8.0 Hz, 1H), 7.06 (s, 1H), 7.01 (s, 2H), 6.96 (d, *J =* 7.6 Hz, 1H), 4.42 (s, 2H), 4.35 (s, 2H), 2.61 (s, 6H), 2.32 (s, 3H).

### Example 92. Preparation of compound 3-2-27 of the present invention

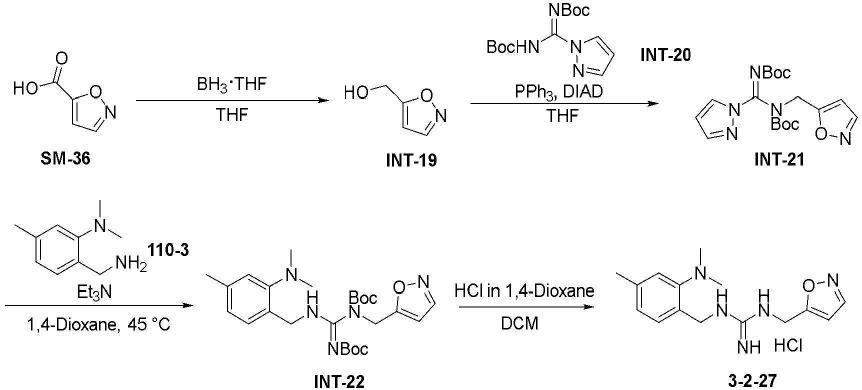

### 1. Synthesis of INT-19

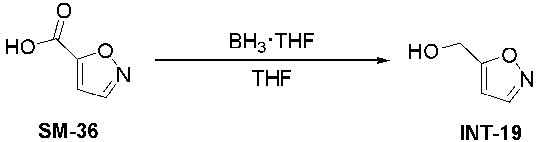

**SM-36** (2.26 g, 20 mmol) was dissolved in tetrahydrofuran (12 mL), and then the system was purged with Ar gas, to which was added BH3·THF (60 mL, 60 mmol) dropwise at 0 °C. Subsequently, the mixture was allowed to react at room temperature for 16 h. Ethanol was added at 0 °C for quenching the reaction, and the reaction solution was extracted three times with water and ethyl acetate. The organic phase was dried over anhydrous sodium sulfate, and concentrated under reduced pressure. The residue was subjected to column chromatography (PE/EtOAc = 3:1) to obtain compound **INT-19** as a colorless oil (1.25 g, yield 63%).

### 2. Synthesis of INT-21

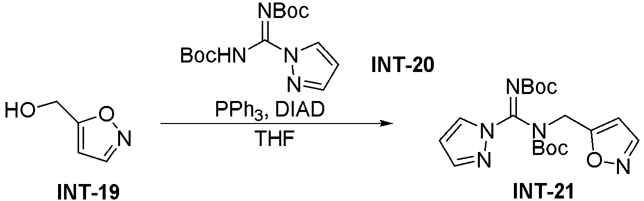

**INT-19** (416 mg, 4.2 mmol, 1.0 equiv.) and **INT-20** (1.3 g, 4.2 mmol) were dissolved in tetrahydrofuran (4.5 mL), to which were added triphenylphosphine (1.6 g, 6.3 mmol), and then DIAD (1.2 g, 6.3 mmol) was slowly added dropwise at 0 °C. The mixture was allowed to react at room temperature for 16 h. The reaction solution was concentrated under reduced pressure. The residue was subjected to column chromatography (PE/EtOAc = 20:1), to obtain compound as a colorless oil (1.11 g, yield 67%).

### 3. Synthesis of compound 110-3

Compound **110-3** was synthesized with reference to Example 1.

### 4. Synthesis of INT-22

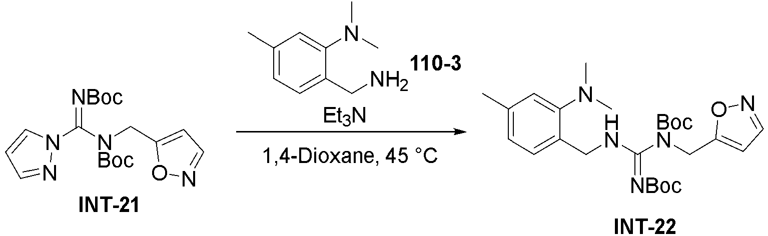

**INT-21** (1.11 g, 2.8 mmol) and **110-3** (459 mg, 2.8 mmol) were dissolved in 1,4-dioxane (5 mL), to which was added triethylamine (2.8 g, 28 mmol), and then the mixture was reacted at 45 °C for 3 h. The reaction solution was concentrated under reduced pressure, and the residue was subjected to column chromatography (PE/EtOAc = 20: 1) to obtain a colorless oily compound (925 mg, yield 68%).

### 5. Synthesis of 3-2-27

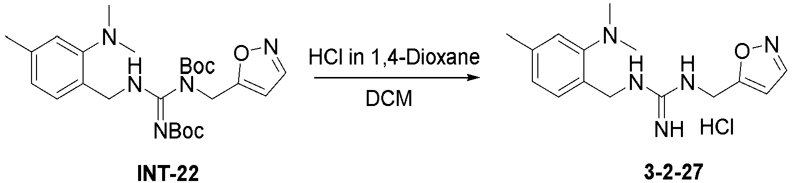

**INT-22** (487 mg, 1.0 mmol) was dissolved in dichloromethane (5 mL), to which was added a solution of hydrochloric acid in 1,4-dioxane (5 mL, 4.0 M), and then the mixture was allowed to react for 16 h at room temperature, during which white solids precipitated. After filtration and washing with ethyl acetate, the solid was recrystallized (MeOH/EA) to obtain product 3-2-27 as white solids: 82 mg, with a yield of 25%. HRMS (ESI-TOF) m/z: [M + Na]⁺ Calcd for C₁₅H₂₂N₅ONa⁺ 310.1635; Found 310.1638.

¹H NMR (400 MHz, Methanol-*d₄*) *δ* 8.39 (d, *J =* 1.8 Hz, 1H), 7.69 (s, 1H), 7.39 (s, 2H), 6.46 (s, 1H), 4.72 (d, *J* = 8.4 Hz, 4H), 3.34 (s, 11H), 2.45 (s, 3H).

### Example 93. Preparation of compound 3-2-28 of the present invention

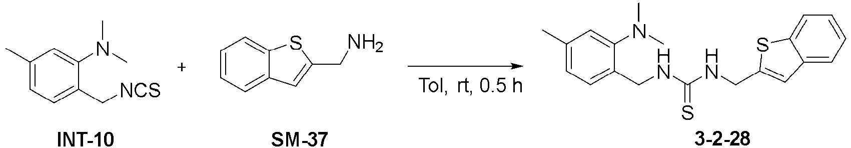

### 1. Synthesis of compound INT-10

Compound **INT-10** was synthesized by referring to Example 73.

### 2. Synthesis of compound 3-2-28

**INT-10** (180 mg, 1.1 mmol) was dissolved in toluene (2 mL), to which was added **SM-37** (206 mg, 1.0 mmol). The mixture was allowed to react at room temperature for about 0.5 h. After completion of the reaction, the reaction solution was concentrated under reduced pressure, and the resulting solid was recrystallized in PE:EtOAc to obtain product **3-2-28:** 415 mg, with a yield of 94%. HRMS (ESI-TOF) *m*/*z:* [M + H]⁺ Calcd for C₂₀H₂₄N₃S₂⁺ 370.1406; Found 370.1407.

¹H NMR (400 MHz, CDCl₃) *δ* 7.78 (d, *J =* 9.2 Hz, 1H), 7.66 (d, *J =* 7.6 Hz, 1H), 7.37 - 7.27 (m, 2H), 7.16 (d, *J* = 7.7 Hz, 1H), 7.09 (s, 1H), 6.94 (d, *J* = 8.2 Hz, 1H), 6.86 (s, 1H), 5.06 (s, 2H), 4.35 (s, 2H), 2.50 (s, 6H), 2.30 (s, 3H).

### Example 94. Preparation of compound 3-2-29 of the present invention

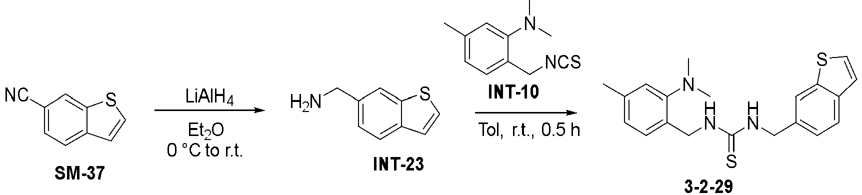

### 1. Synthesis of INT-23

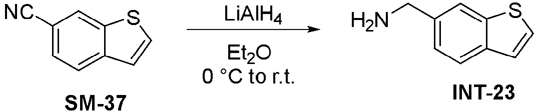

**SM-37** (509 mg, 3.2 mmol) was dissolved in diethyl ether (8 mL), to which was gradually added lithium aluminum hydride (244 mg, 6.4 mmol) in batches at 0 °C, and then the mixture was allowed to react at room temperature for 2 h. After completion of the reaction, 3 mL of 15% NaOH solution was first added for quenching the reaction, to which were successively added 3 mL of water and 3 mL of 15% NaOH solution dropwise. The resultant solution was filtered through diatomaceous earth and over anhydrous sodium sulfate, and then concentrated under reduced pressure to obtain compound **INT-23** as a yellow oil (347 mg, yield 66%).

### 2. Synthesis of compound INT10

Compound **1NT-10** was synthesized by referring to Example 73.

### 3. Synthesis of 3-2-29

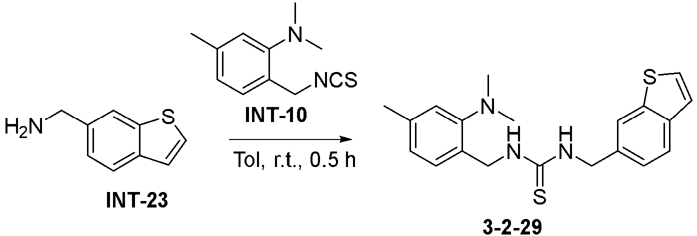

**INT-23** (309 mg, 1.9 mmol) was dissolved in toluene (2 mL), to which was added thioisocyanate **INT-10** (412 mg, 2.0 mmol), and then the mixture was allowed to react at room temperature for about 0.5 h. After completion of the reaction, the reaction solution was concentrated under reduced pressure, and the resulting solid was recrystallized to obtain product **3-2-29:** 248 mg, with a yield of 67%. HRMS (ESI-TOF) m/z: [M + H]⁺ Calcd for C₂₀H₂₄N₃S₂⁺ 370.1406; Found 370.1407.

¹H NMR (400 MHz, CDCl₃) *δ* 7.75 (d, *J* = 8.2 Hz, 1H), 7.61 (s, 1H), 7.43 (d, *J* = 5.4 Hz, 1H), 7.31 (d, *J =* 5.4 Hz, 1H), 7.23 (d, *J =* 6.7 Hz, 1H), 7.17 (d, *J =* 7.9 Hz, 1H), 6.94 (d, *J* = 7.1 Hz, 1H), 6.80 (s, 1H), 4.90 (s, 2H), 4.39 (s, 2H), 2.36 (s, 6H), 2.31 (s, 3H).

### Example 95. Preparation of compound 3-2-20 of the present invention

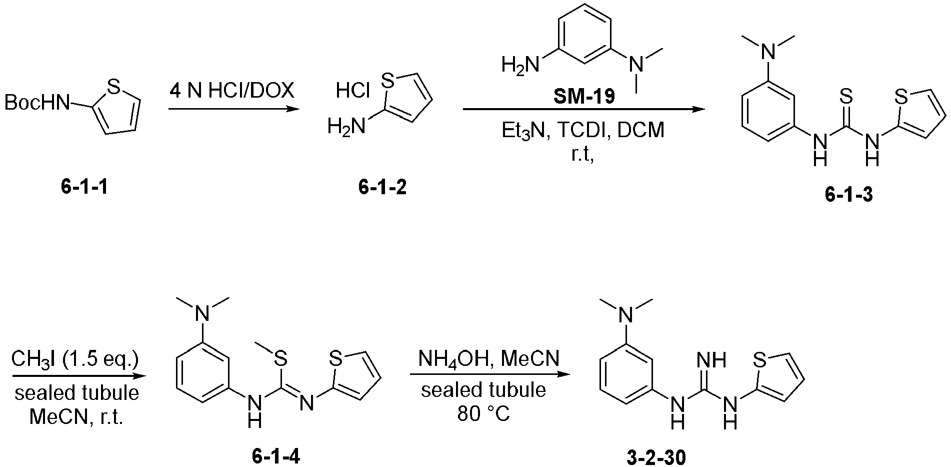

### 1. Synthesis of 6-1-2

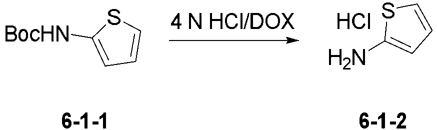

**6-1-1** (2.0 g, 10 mmol) and a solution of 4N hydrochloric acid in dioxane (20 mL) were added to a reaction flask in sequence, and the reaction system was stirred at room temperature overnight. After completion of the reaction, the solvent was removed by evaporation under reduced pressure to obtain a light yellow solid **6-1-2** (1.0 g, yield 74%).

### 2. Synthesis of 6-1-3

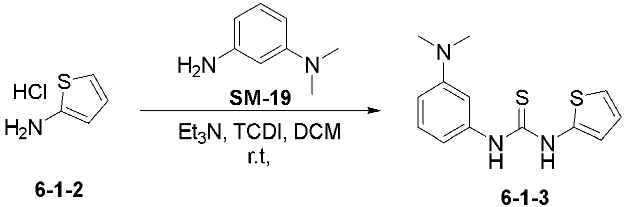

**SM-19** (680 mg, 5.0 mmol) was added to a reaction flask, and then dissolved with dichloromethane (20 mL), to which were added triethylamine (3.03 g, 30 mmol) and TCDI (1.07 g, 6.0 mmol), and the mixture was stirred at room temperature for 2 h. Subsequently, **6-1-2** (675 mg, 5.0 mmol) was added to the system, and the mixture was allowed to react at room temperature overnight. After completion of the reaction, the solvent was removed by evaporation under reduced pressure to obtain the crude product, which was purified by silica gel column chromatography (methanol/dichloromethane (v/v) = 3%), to obtain **6-1-3** as pale yellow solid (800 mg, yield 58%). MS Calcd.: 278.1 [M+H] ⁺; MS Found: 278.4 [M + H]⁺.

### 3. Synthesis of 6-1-4

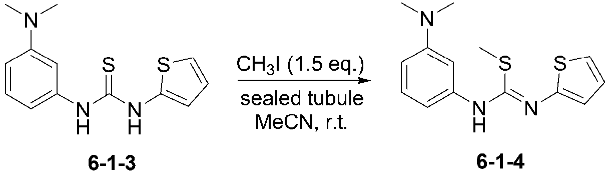

**6-1-3** (800 mg, 2.89 mmol) was added to a reaction tube, and dissolved with acetonitrile (15 mL), to which was then added iodomethane (616 mg, 4.34 mmol). The reaction was stirred at room temperature overnight. After completion of the reaction, the solvent was removed by evaporation under reduced pressure to obtain the crude product, which was purified by silica gel column chromatography (methanol/dichloromethane (v/v) = 5%) to obtain **6-1-4** (400 mg, yield: 48%) as light yellow semi-solid. MS Calcd.: 292.1 [M+H] ⁺; MS Found: 292.4 [M + H]⁺.

### 4. Synthesis of 3-2-20

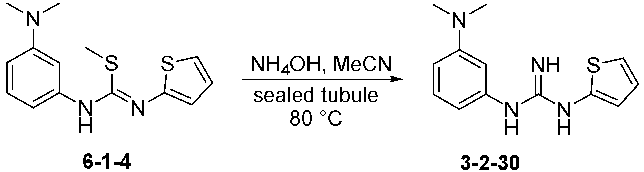

**6-1-4** (400 mg, 1.37 mmol) was added to a sealed tube, and dissolved with acetonitrile (10 mL), to which was then added 25% ammonia water (932 mg, 13.7 mmol), and the mixture was stirred at 80 °C overnight. After completion of the reaction, the solvent was removed by evaporation under reduced pressure to obtain the crude product, which was subjected to silica gel column chromatography (methanol/dichloromethane (v/v) = 5%) and reversed-phase prep-HPLC to obtain **3-2-20** as white solids (130 mg, yield 33%). MS Calcd.: 261.1[M+H] ⁺; MS Found: 261.4[M + H]⁺.

¹H NMR (400 MHz, CD₃OD) *δ* 7.12 (d, *J =* 8.0 Hz, 1H), 6.85 (dd, *J* = 1.6, 6.0 Hz, 1H), 6.82 (dd, *J* = 3.2, 5.6 Hz, 1H), 6.78 (t, *J* = 2.0 Hz, 1H), 6.63-6.60 (m, 1H), 6.50 (dd, *J* = 2.0, 8.4 Hz, 1H), 6.43 (dd, *J* = 1.2, 3.6 Hz, 1H), 2.91(s, 6H).

### Example 96. Preparation of compound 3-2-31 of the present invention

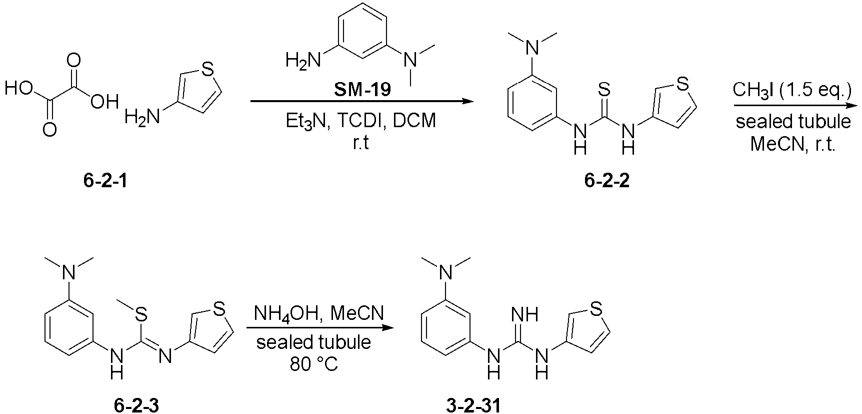

### 1. Synthesis of 6-2-2

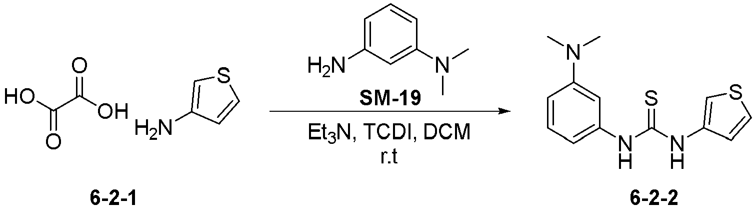

**SM-19** (680 mg, 5.0 mmol) was added to a reaction flask, and then dissolved with dichloromethane (20 mL), to which were added triethylamine (3.03 g, 30 mmol) and TCDI (1.07 g, 6.0 mmol), and the mixture was stirred at room temperature for 2 h. Subsequently, 6-2-1 (945 mg, 5.0 mmol) was added to the system, and the mixture was stirred and allowed to react at room temperature overnight. After completion of the reaction, the solvent was removed by evaporation under reduced pressure to obtain the crude product, which was purified by silica gel column chromatography (methanol/dichloromethane (v/v) = 3%), to obtain **6-2-2** as pale yellow solid (800 mg, yield 58%). MS Calcd.: 278.1 [M+H] ⁺; MS Found: 287.4 [M + H]⁺.

### 2. Synthesis of 6-2-3

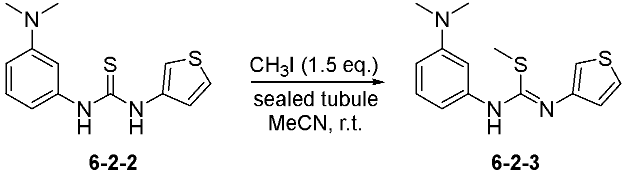

**6-2-2** (800 mg, 2.89 mmol) was added to a reaction tube, and dissolved with acetonitrile (15 mL), to which was then added iodomethane (616 mg, 4.34 mmol). The reaction was stirred at room temperature overnight. After completion of the reaction, the solvent was removed by evaporation under reduced pressure to obtain the crude product, which was purified by silica gel column chromatography (methanol/dichloromethane (v/v) = 5%) to obtain **6-2-3** (400 mg, yield: 48%) as light yellow semi-solid. MS Calcd.: 292.1 [M+H] ⁺; MS Found: 292.4 [M + H]⁺.

### 3. Synthesis of 3-2-21

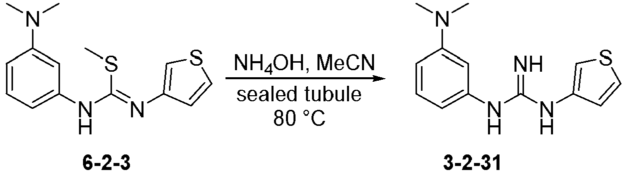

**6-2-3** (400 mg, 1.37 mmol) was added to a sealed tube, and dissolved with acetonitrile (10 mL), to which was then added 25% ammonia water (932 mg, 13.7 mmol), and the mixture was stirred at 80 °C overnight. After completion of the reaction, the solvent was removed by evaporation under reduced pressure to obtain the crude product, which was subjected to silica gel column chromatography (methanol/dichloromethane (v/v) = 5%) and reversed-phase prep-HPLC to obtain **3-2-21** as white solids (201.32 mg, yield 56%). MS Calcd.: 261.1 [M+H] ⁺; MS Found: 261.4 [M + H]⁺.

¹H NMR (400 MHz, CD₃OD) *δ* 7.31 (dd, *J =* 3.2, 5.2 Hz, 1H), 7.13 (t, *J =* 8.0 Hz, 1H), 6.94-6.91 (m, 2H), 6.63 (t, *J* = 3.6 Hz, 1H), 6.54-6.50 (m, 2H), 2.91 (s, 6H).

### Example 97. Preparation of compound 3-2-32 of the present invention

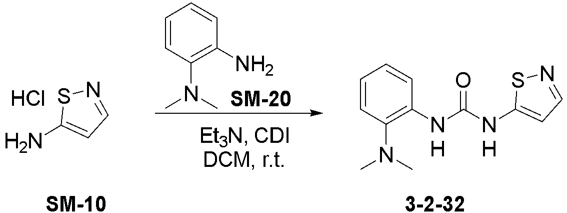

**SM-10** (408 mg, 3.0 mmol) was added to a reaction flask, and then dissolved with dichloromethane (20 mL), to which were added triethylamine (1.52 g, 15.0 mmol) and CDI (486 mg, 3.0 mmol), and the mixture was stirred at room temperature for 4 h. Subsequently, **SM-20** (408 mg, 3.0 mmol) was added to the system, and the mixture was allowed to react at 45 °C overnight. After completion of the reaction, the solvent was removed by evaporation under reduced pressure to obtain the crude product, which was purified by silica gel column chromatography (methanol/dichloromethane (v/v) = 3%) and reversed-phase prep-HPLC, to obtain **3-2-32** as off-white solid (131.88 mg, yield: 17.5%). MS Calcd.: 263.1 [M+H] ⁺; MS Found: 263.2 [M + H]⁺.

¹H NMR (400 MHz, DMSO-d₆) δ 11.17 (s, 1H), 8.73 (s, 1H), 8.18 (d, *J =* 2.0 Hz, 1H), 8.09 (dd, *J =* 8.0, 1.6 Hz, 1H), 7.23 (dd, *J* = 7.6, 1.6 Hz, 1H), 7.09-6.99 (m, 2H), 6.80 (d, *J =* 1.6 Hz, 1H), 2.62 (s, 6H).

### Example 98. Preparation of compound 3-2-33 of the present invention

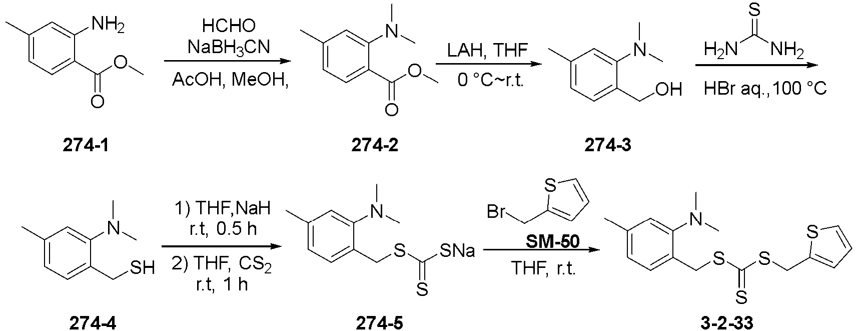

### 1. Synthesis of 274-2

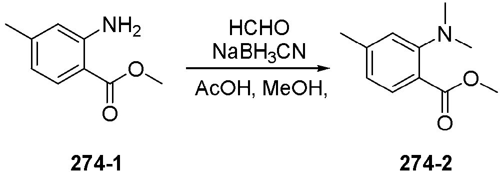

**274-1** (5.0 g, 30.3 mmol) was added to a 500 mL reaction flask, and dissolved with alcohol (150 mL), to which were then sequentially added formaldehyde aqueous solution (24.0 g, 300.3 mmol), acetic acid (9.0 g, 151.5 mmol), and sodium cyanoborohydride (9.5 g, 151.5 mmol), and the mixture was reacted at room temperature overnight. After completion of the reaction, the reaction solution was poured into ice water and extracted with dichloromethane (3 × 100 mL). The organic phase was washed with saturated saline (100 mL), dried over anhydrous Na₂SO₄, filtered under suction, and concentrated under reduced pressure. The crude product was purified by silica gel column chromatography to obtain compound **274-2** (5.0 g, yield: 85%). MS Calcd.: 194.1[M+H] ⁺; MS Found: 194.4 [M + H]⁺.

### 2. Synthesis of 274-3

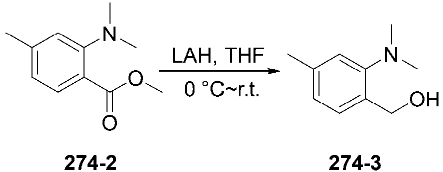

To a reaction flask, were added **274-2** (5.0 g, 25.91 mmol) and THF (120 mL), and then the system was purged with nitrogen balloon three times, and cooled to 0 °C in an ice bath. Then, lithium aluminum hydride (2.5 N in THF, 20.7 mL, 51.8 mmol) was slowly added dropwise, and the reaction was stirred at room temperature overnight. After completion of the reaction, the system was cooled to 0 °C, and then water (3 mL), 15% NaOH solution (3 mL), and water (6 mL) were slowly added. The resultant solution was stirred at room temperature for 20 min, dried over anhydrous magnesium sulfate, and filtered. The solvent was removed by evaporation under reduced pressure to obtain the crude product, which was purified by normal phase column chromatography [dichloromethane: methanol (20:1)] to obtain **274-3** as a yellow oil (4.2 g, yield 99%). MS Calcd.: 166.1 [M+H] ⁺; MS Found: 166.3 [M + H]⁺.

### 3. Synthesis of 274-4

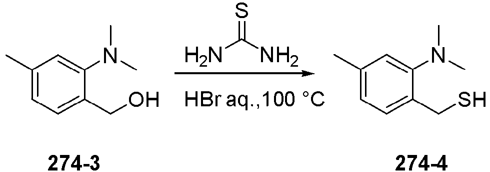

**274-3** (1.5 g, 9.09 mmol) was dissolved in concentrated HBr (20 mL), to which was then added thiourea (919 mg, 12.09 mmol), and the system was purged with nitrogen three times. The reaction mixture was stirred and allowed to react at 100 °C under nitrogen atmosphere overnight. After completion of the reaction, the reaction solution was adjusted to pH=13 by adding sodium hydroxide, and then stirred at 100 °C for 0.5 h under nitrogen atmosphere. The reaction solution was diluted with aqueous NaOH solution (1M, 30 mL), and then extracted with ethyl acetate. The extracts were combined and washed with saturated saline (20 mL), dried over anhydrous sodium sulfate, and rotatory evaporated to dry, to obtain **274-4** as yellow liquid (0.60 g, yield: 36%). MS Calcd.: 182.1[M+H] ⁺; MS Found: 182.3 [M + H]⁺.

### 4. Synthesis of compound 274-5

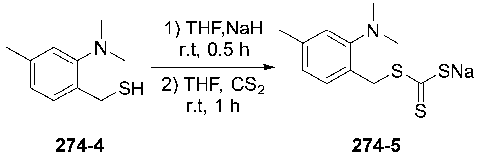

**274-4** (0.30 g, 1.65 mmol) was dissolved in tetrahydrofuran (15 mL), to which was then added sodium hydride (60%, 0.10 g, 2.48 mmol). The reaction system was purged with nitrogen three times, and stirred at room temperature under nitrogen atmosphere for 0.5 h. Then, carbon disulfide (1.26 g, 16.55 mmol) was added to the reaction solution, and then the reaction was stirred at room temperature under nitrogen atmosphere for 1 h. The reaction solution was rotatory evaporated to dry, to obtain the crude product compound **274-5** as yellow solid.

### 5. Synthesis of compound 3-2-33

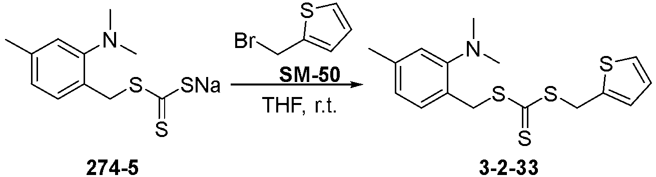

**274-5** (0.50 g, 1.79 mmol) was dissolved in tetrahydrofuran (15 mL), to which was then added **SM-50** (0.35 g, 1.97 mmol), and then the system was purged with nitrogen three times. The reaction was stirred at room temperature under nitrogen atmosphere overnight. After completion of the reaction, the reaction solution was poured into water (50 mL), and then extracted with ethyl acetate. The extracts were combined, washed with saturated saline, dried over anhydrous sodium sulfate, and rotatory evaporated to dry, to obtain the crude product, which was subjected to silica gel column chromatography to obtain **3-2-33** (0.21 g, yield: 33%) as yellow liquid. MS Calcd.: 354.1 [M+H]⁺; MS Found: 354.0 [M + H]⁺.

¹H NMR (400 MHz, DMSO-*d₆*) *δ*: 7.44 (d, *J* = 5.2, 1.2 Hz, 1H), 7.22 (d, *J* = 7.6 Hz, 1H), 7.12-7.11 (m, 1H), 7.01 (s, 1H), 6.97 (dd, *J =* 6.0, 3.4 Hz, 1H), 6.85 (d, *J =* 8.0 Hz, 1H), 4.94 (s, 2H), 4.68 (s, 2H), 2.05 (s, 2H), 2.60 (s, 6H), 2.26 (s, 3H).

### Example 99. Preparation of compound 3-2-34 of the present invention

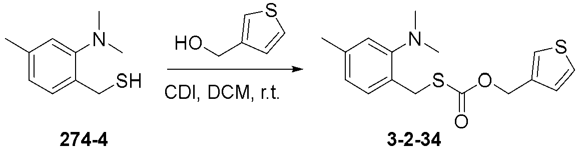

### 1. Synthesis of compound 274-4

Compound **274-4** was synthesized with reference to Example 98.

### 2. Synthesis of 3-2-34

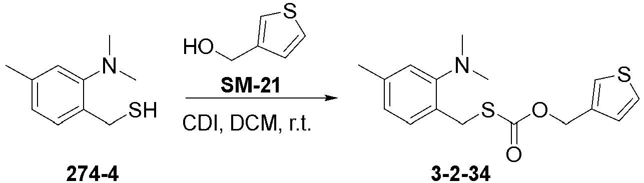

**SM-21** (189 mg, 1.66 mmol) was added to a reaction flask, and then dissolved with dichloromethane (10 mL), to which were added triethylamine (503 mg, 4.98 mmol) and CDI (295 mg, 1.66 mmol), and the mixture was stirred at room temperature for 2 h. Subsequently, **274-4** (300 mg, 1.66 mmol) was added to the system, and the mixture was allowed to react at room temperature overnight. After completion of the reaction, the solvent was removed by evaporation under reduced pressure to obtain the crude product, which was purified by column chromatography [eluent: dichloromethane-methanol (20:1)] and reversed-phase prep-HPLC, to obtain **3-2-34** as white solids (221.27 mg, yield: 42%). MS Calcd.: 321.1 [M+H] ⁺; MS Found: 321.4 [M + H]⁺.

¹H NMR (400 MHz, DMSO-*d*₆) *δ*: 7.58 (d, *J* = 1.6 Hz, 1H), 7.55 (dd, *J* = 2.8, 4.8 Hz, 1H), 7.19 (d, *J =* 7.6 Hz, 1H), 7.12 (dd, *J* = 1.2, 4.8 Hz, 1H), 6.99 (s, 1H), 6.84 (d, *J =* 7.6 Hz, 1H), 5.25 (s, 2H), 4.17 (s, 2H), 2.59 (s, 6H), 2.26 (s, 3H).

### Example 100. Preparation of compound 3-2-35 of the present invention

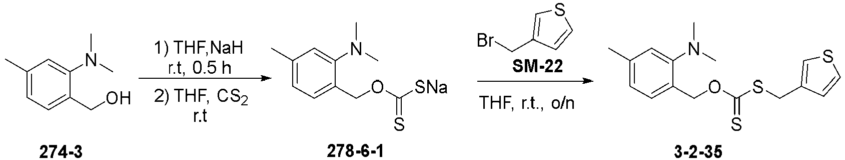

### 1. Synthesis of compound 274-3

Compound **274-3** was synthesized with reference to Example 99.

### 2. Synthesis of 278-6-1

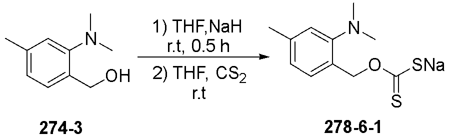

**274-3** (500 mg, 3.03 mmol) was added to a reaction flask, and dissolved with tetrahydrofuran (15 mL), to which was slowly added sodium hydride (118 mg, 3.94 mmol), and the reaction was stirred at room temperature for half an hour. Carbon disulfide (2.3 g, 30.3 mmol) was dissolved in tetrahydrofuran (10 mL) and slowly added to the above reaction system. After that, the reaction was allowed to react overnight at room temperature overnight. After completion of the reaction, the solvent was removed by evaporation under reduced pressure to obtain crude product **278-6-1,** which was directly used in the next reaction without purification.

### 3. Synthesis of compound 3-2-35

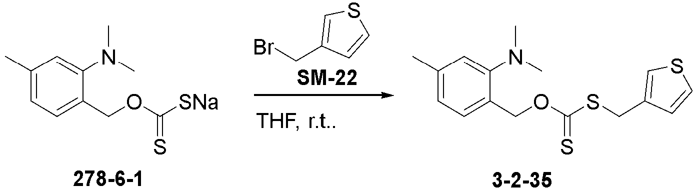

The crude product obtained above (700 mg, 2.66 mmol) was added to a reaction flask, and then dissolved with tetrahydrofuran (15 mL), to which was slowly added **SM-22** (471 mg, 2.66 mmol), and then the mixture was stirred and reacted at room temperature overnight. After the reaction was complete as determined by LCMS, the reaction solution was slowly poured into ice water (2 mL) and extracted with ethyl acetate (3x30 mL). The extracts were combined, washed with saturated saline (20 mL), dried over anhydrous sodium sulfate, and rotatory evaporated to dry to obtain the crude product, which was purified by normal-phase column and prep-HPLC to obtain **3-2-35** as a colorless oil (212.95 mg, yield: 24%). MS Calcd.: 338.1 [M+H] ⁺; MS Found: 338.4 [M + H]⁺.

¹H NMR (400 MHz, DMSO-*d*₆) *δ*: 7.49 (dd, *J* = 2.8, 4.8 Hz, 1H), 7.40-7.39 (m, 1H), 7.16 (d, *J* = 8.0 Hz, 1H), 7.03 (dd, *J* = 1.2, 5.2 Hz, 1H), 6.99 (s, 1H), 6.84 (dd, *J* = 0.8, 7.6 Hz, 1H), 4.31 (s, 2H), 4.29 (s, 2H), 2.59 (s, 6H), 2.26 (s, 3H).

### Example 101. Preparation of compound 3-2-36 of the present invention

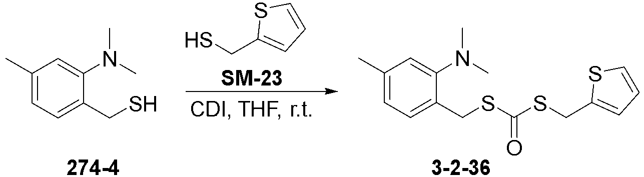

### 1. Synthesis of compound 274-4

Compound **274-4** was synthesized with reference to Example 99.

### 2. Synthesis of compound 3-2-26

**SM-23** (200 mg, 1.54 mmol) was added to a flask, and dissolved with tetrahydrofuran (10 mL), to which was then added CDI (249 mg, 1.54 mmol), and the mixture was stirred at room temperature for 2 h. **274-4** (278 mg, 1.54 mmol) was added to the system, and then the reaction was stirred at room temperature overnight. After completion of the reaction, the solvent was removed by evaporation under reduced pressure to obtain the crude product, which was purified by reversed-phase prep-HPLC to obtain **3-2-26** as a light yellow oil (155.48 mg, yield: 30%). MS Calcd.: 338.1[M+H] ⁺; MS Found: 338.1 [M + H]⁺.

¹H NMR (400 MHz, CD₃OD) *δ* 7.27 (dd, *J* = 5.2 Hz, *J* = 1.2 Hz, 1H), 7.17 (d, *J* = 7.6 Hz, 1H), 7.00-6.98 (m, 2H), 6.92-6.89 (m, 1H), 6.84 (d, *J* = 8.0 Hz, 1H), 4.47 (s, 2H), 4.35 (s, 2H), 2.63 (s, 6H), 2.28 (s, 3H).

### Example 102. Preparation of compound 3-2-27 of the present invention

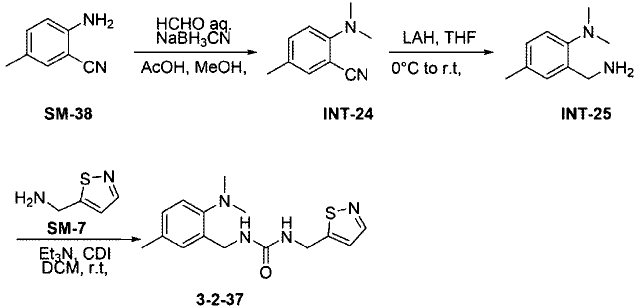

### 1. Synthesis of INT-24

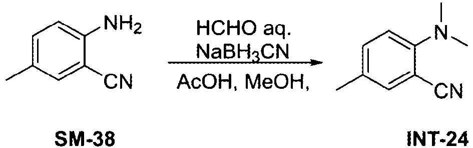

**19-1** (600 mg, 4.55 mmol) was added to a reaction flask, and dissolved with methanol (25 mL), to which were then added formaldehyde aqueous solution (3.69 g, 45.5 mmol), acetic acid (1.37 g, 22.75 mmol), and sodium cyanoborohydride (1.43 g, 22.75 mmol) in sequence, and then the mixture was stirred at room temperature under nitrogen atmosphere overnight. After completion of the reaction, the reaction was quenched by adding water (40 mL). The resultant solution was extracted with dichloromethane (40 mL) three times, dried over anhydrous sodium sulfate, and filtered. The solvent was removed by evaporation under reduced pressure to obtain a residue, which was purified by normal phase column chromatography [petroleum ether: ethyl acetate (20:1-5:1)] to obtain **19-2** as a yellow oil (560 mg, yield: 77%). MS Calcd.: 161.1[M+H] ⁺; MS Found: 161.4 [M + H]⁺.

### 2. Synthesis of INT-25

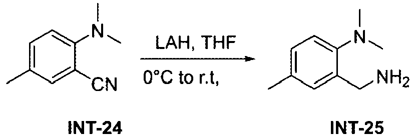

To a reaction flask, were added **INT-24** (560 mg, 3.50 mmol) and tetrahydrofuran (15 mL), and then the system was purged three times with a nitrogen ball. The reaction was cooled to 0 °C under nitrogen atmosphere, to which was then added lithium aluminum hydride (2.5 N tetrahydrofuran solution, 7.0 mmol, 2.8 mL). Then, the reaction was stirred overnight at room temperature. After completion of the reaction, the system was cooled to 0 °C, to which were slowly added water (0.6 mL), 15% aqueous NaOH solution (0.6 mL), and water (1.8 mL). The resultant solution was stirred at room temperature for 20 min, dried over anhydrous magnesium sulfate, and filtered. The solvent was evaporated under reduced pressure, to obtain the crude product, which was subjected to normal phase column chromatography [dichloromethane: methanol (100:1-20:1)] to obtain **INT-25** as a yellow oil (300 mg, yield 52%).

### 3. Synthesis of SM-7

Compound **SM-7** was synthesized with reference to Example 36.

### 4. Synthesis of INT-25

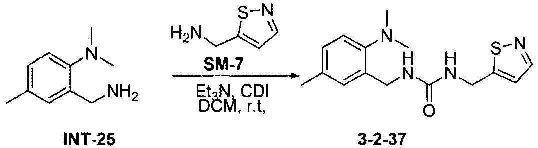

**SM-7** (209 mg, 1.83 mmol) was added to a reaction flask, and then dissolved with dichloromethane (10 mL), to which were added triethylamine (554 mg, 5.49 mmol) and CDI (297 mg, 1.83 mmol), and the mixture was stirred at room temperature for 2 h. Subsequently, **INT-25** (300 mg, 1.83 mmol) was added to the system, and the mixture was allowed to react at room temperature overnight. After completion of the reaction, the solvent was removed by evaporation under reduced pressure to obtain the crude product, which was purified by column chromatography [eluent: dichloromethane-methanol (100:1-20:1)] and reversed-phase prep-HPLC, to obtain **3-2-37** as white solids (170.6 mg, yield: 31%).

¹H NMR (400 MHz, DMSO-*d*₆) *δ*: 8.42 (d, *J* = 1.6 Hz, 1H), 7.22 (t, *J* = 0.8 Hz, 1H), 7.02 (s, 1H), 6.99 (d, *J =* 1.2 Hz, 2H), 6.99 (s, 1H), 6.76 (t, *J =* 5.6 Hz, 1H), 6.49 (t, *J* = 5.6 Hz, 1H), 4.53 (d, *J* = 6.0 Hz, 2H), 4.26 (d, *J =* 6.0 Hz, 2H), 2.58 (s, 6H), 2.22 (s, 3H).

### Example 103. Preparation of compound 3-2-28 of the present invention

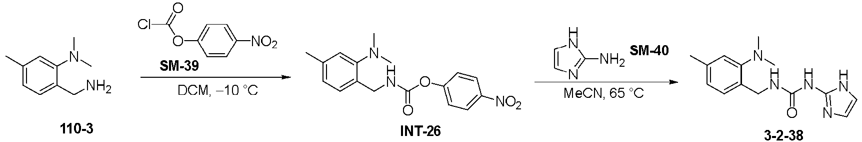

### 1. Synthesis of compound 110-3

Compound **110-3** was synthesized by referring to Example 1.

### 2. Synthesis of INT-26

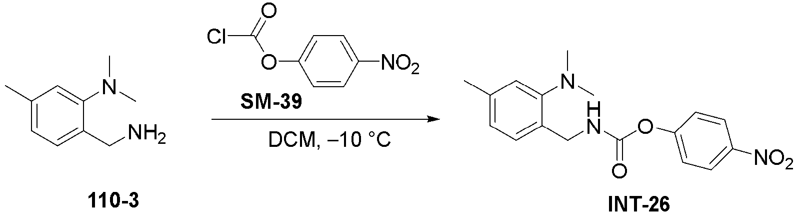

**SM-39** (1.01 g, 5 mmol) was dissolved in dichloromethane (30 mL), to which was added triethylamine (1.05 mL, 7.5 mmol, 1.5 equiv). Then, **110-3** (820 mg, 5 mmol) was slowly added dropwise at -10 °C, and the reaction was allowed to further react at -10 °C for additional 16 h. After completion of the reaction, water was added to quench the reaction. After dilution with dichloromethane, the reaction solution was washed twice with 20% aqueous NaHCO₃ solution, dried over anhydrous sodium sulfate, and concentrated under reduced pressure to obtain compound **INT-26** as light yellow solids (1.06 g, yield 65%).

### 3. Synthesis of 3-2-38

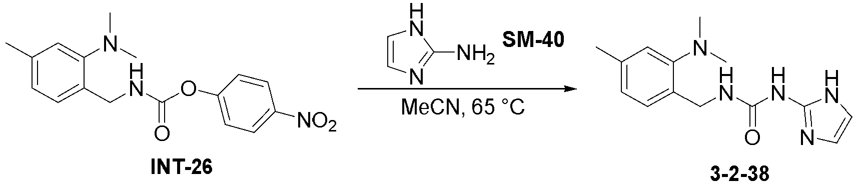

**INT-26** (2.4 g, 7.3 mmol) and **SM-40** (1.2 g, 14.6 mmol) were dissolved in acetonitrile (40 mL), and reacted at 65 °C for 4 h. After completion of the reaction, the reaction solution was concentrated under reduced pressure. The residue was subjected to column chromatography (DCM / MeOH = 10:1) to obtain the crude product, which was recrystallized in acetone to obtain the crude product as light yellow solids. Subsequently, the crude product was subjected to reversed-phase column chromatography (MeCN/H₂O = 50%, 30 min), to obtain **3-2-38** as white solids: 70 mg, with a yield of 3.5%. HRMS (ESI-TOF) m/z: [M + H]⁺ Calcd for C₁₄H₂₀N₅O⁺ 274.1662; Found 274.1667.

¹H NMR (400 MHz, CDCl₃) *δ* 7.26 (d, *J* = 1.0 Hz, 1H), 7.18 (d, *J* = 7.7 Hz, 1H), 6.95 (s, 1H), 6.87 (d, *J* = 7.7 Hz, 1H), 6.65 (s, 2H), 4.47 (d, *J* = 5.6 Hz, 2H), 2.69 (s, 6H), 2.31 (s, 3H).

### Example 104. Preparation of compound 3-2-39 of the present invention

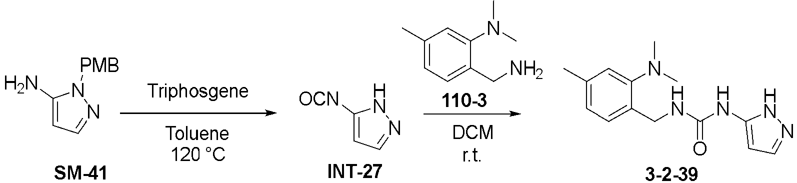

### 1. Synthesis of compound INT-27

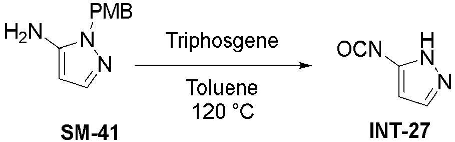

**SM-41** (812 mg, 4.0 mmol) was dissolved in toluene (12 mL), to which was added solid phosgene (594 mg, 2.0 mmol). After that, the mixture was refluxed at 120 °C for 4 h. Toluene was then removed by rotatory evaporation, to obtain crude product **INT-27** as a yellow oil, which was directly used in the next step.

### 2. Synthesis of compound 110-3

Compound **110-3** was synthesized with reference to Example 1.

### 3. Synthesis of compound 3-2-39

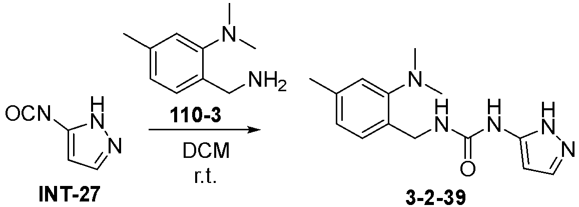

**110-3** (656 mg, 4.0 mmol) and **INT-27** (436 mg, 4.0 mmol) were dissolved in dichloromethane (6.0 mL), and then the reaction was allowed to react at room temperature for 1 h. After completion of the reaction, the reaction was subjected to column chromatography (PE/EtOAc = 2:1), to yield compound **3-2-39** as a yellow oil: 152 mg, yield 14%. HRMS (ESI-TOF) *m*/*z:* [M + Na]⁺ Calcd for C₁₄H₁₉N₅ONa⁺ 296.1482; Found 296.1481.

¹H NMR (400 MHz, CDCl₃): *δ* 8.16 (d, *J* = 2.8 Hz, 1H), 7.94 (s, 1H), 6.91 (s, 1H), 6.83 (d, *J* = 7.6 Hz, 1H), 6.48 (s, 1H), 4.61 (d, *J* = 5.8 Hz, 2H), 2.59 (s, 6H), 2.28 (s, 3H).

### Example 105. Preparation of compound 3-2-40 of the present invention

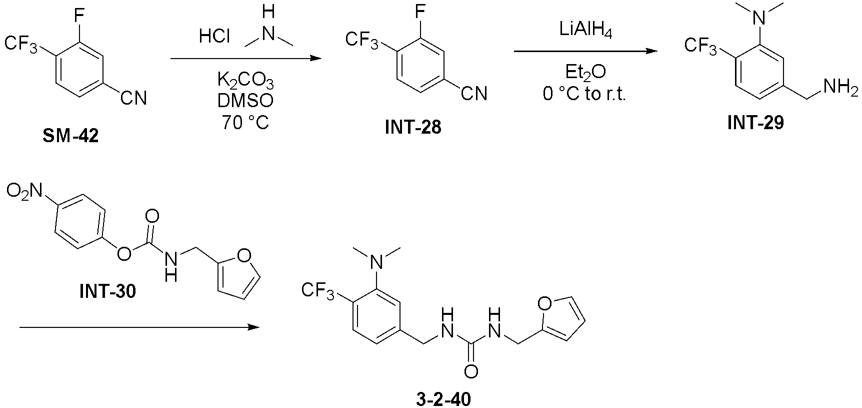

### 1. Synthesis of compound INT-28

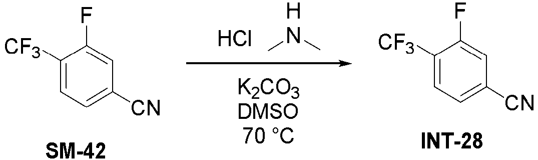

**SM-42** (1.89 g, 10 mmol) was dissolved in dimethyl sulfoxide (15 mL), to which were added dimethylamine hydrochloride (1.22 g, 15 mmol) and potassium carbonate (3.46 g, 25 mmol), and then the mixture was heated to 70 °C and reacted overnight. After completion of the reaction, water (15 mL) was added to dilute the solution, which was then extracted with ethyl acetate (15 mL × 3). The organic phase was washed three times with saturated saline, dried over anhydrous sodium sulfate, and concentrated under reduced pressure to obtain compound **INT-28** as a yellow oil.

### 2. Synthesis of compound INT-29

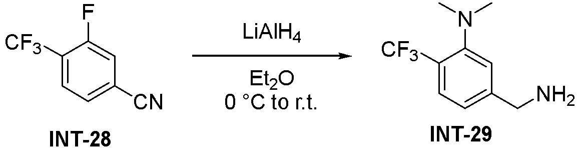

**INT-28** was dissolved in diethyl ether (20 mL), to which was gradually added lithium aluminum hydride (0.95 mg, 25 mmol) in batches at 0 °C, and then the mixture was allowed to react at room temperature for 4 h. After completion of the reaction, 3 mL of 15% NaOH solution was first added for quenching the reaction, to which were successively added 3 mL of water and 3 mL of 15% NaOH solution dropwise. The resultant solution was filtered through diatomaceous earth and over anhydrous sodium sulfate, and then concentrated under reduced pressure to obtain compound **INT-29** as a yellow oil (1.85 g, yield 85%).

### 3. Synthesis of compound INT-30

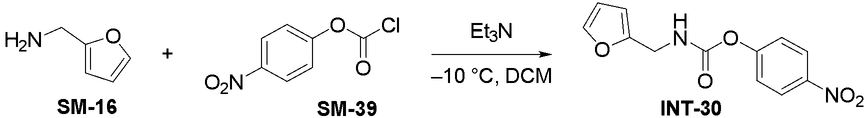

**SM-16** (4.84 g, 24 mmol) was dissolved in dichloromethane (60 mL), to which was added triethylamine (8.4 mL, 60 mmol) at -10 °C, followed by adding **SM-39** (1.8 mL, 20 mmol), and then the mixture was allowed to react at room temperature for 2 h. After completion of the reaction, water was added for quenching the reaction, and the mixture was extracted with dichloromethane (50 mL × 3). The organic phase was washed three times with saturated saline, dried over anhydrous sodium sulfate, and concentrated under reduced pressure. The residue was subjected to column chromatography (PE/EtOAc = 15:1), to obtain compound **INT-30** as white solids (1.77 g, yield 34%).

### 4. Synthesis of compound 3-2-40

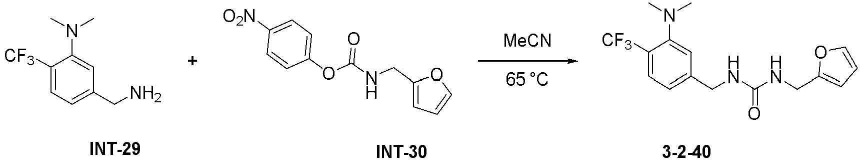

**INT-29** (327 mg, 1.5 mmol) and **INT-30** (393 mg, 1.5 mmol) were dissolved in acetonitrile (3.0 mL), and then the mixture was heated to 65 °C and allowed to react for 3 h. After completion of the reaction, the reaction was subjected to column chromatography (PE/EtOAc = 2:1), to obtain compound **3-2-40** as yellow solids: 324 mg, with a yield of 63%. HRMS (ESI-TOF) *m*/*z:* [M + H]⁺ Calcd for C₁₆H₁₉F₃N₃O₂⁺ 342.1424; Found 342.1423.

¹H NMR (400 MHz, CDCl₃): *δ* 7.49 (d, *J =* 8.0 Hz, 1H), 7.31-7.26 (m, 1H), 7.16 (s, 1H), 6.98 (d, *J=* 8.0 Hz, 1H), 6.28 (dd, *J* = 3.0, 2.0 Hz, 1H), 6.15 (d, *J =* 3.0 Hz, 1H), 5.16 (dt, *J* = 23.6, 5.4 Hz, 2H), 4.31 (d, *J* = 5.8 Hz, 4H), 2.69 (s, 6H). ¹⁹F NMR (376 MHz, CDCl₃): *δ* -59.8.

### Example 106. Preparation of compound 3-2-41 of the present invention

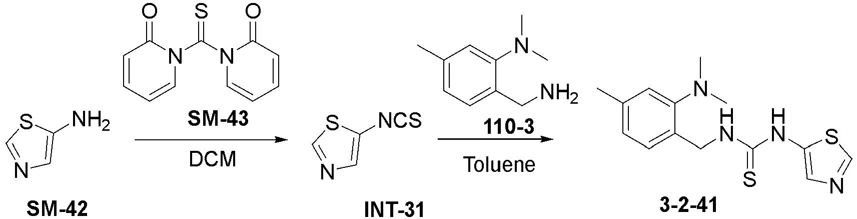

### 1. Synthesis of compound INT-31

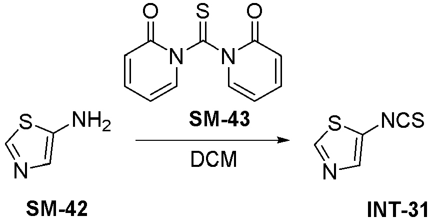

**SM-42** (250 mg, 2.5 mmol) was dissolved in dichloromethane (10 mL), to which was added **SM-43** (465 mg, 2 mmol, 1.0 equiv) in batches, and then the reaction was stirred at room temperature for 30 min. Then, the reaction solution was concentrated under reduced pressure. The residue was purified by silica gel column chromatography (PE : EtOAc = 10:1) to obtain **INT-31** (156 mg, 1.1 mmol, yield 44%) as a colorless oil.

### 2. Synthesis of compound 110-3

Compound **110-3** was synthesized with reference to Example 1.

### 3. Synthesis of compound 3-2-41

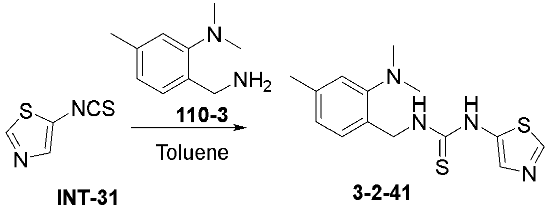

**INT-31** (156 mg, 1.1 mmol) and **110-3** (198 mg, 1.21 mmol) were dissolved in toluene (2 mL), and then the mixture was stirred at room temperature for 3 h, resulting in the precipitation of white solids. After completion of the reaction, the reaction solution was filtered, and the filter cake was washed with a mixture of PE and EA in a ratio of 10:1 to obtain the product as white solids. The white solid was dissolved in chloroform, and filtered through diatomaceous earth. The filtrate was concentrated to yield product **3-2-41** as white solids: 191 mg, with a yield of 57%. HRMS (ESI-TOF) *m*/*z:* [M + H]⁺ Calcd for C₁₄H₁₉N₄S₂⁺ 307.1046; Found 307.1041.

¹H NMR (400 MHz, DMSO-*d*₆) *δ* 10.70 (s, 1H), 8.50 (s, 1H), 8.16 (s, 1H), 7.60 (s, 1H), 7.11 (d, *J* = 7.7 Hz, 1H), 6.97 (s, 1H), 6.86 (d, *J* = 7.7 Hz, 1H), 4.71 (s, 2H), 2.63 (s, 6H), 2.27 (s, 3H).

### Example 107. Preparation of compound 3-2-42 of the present invention

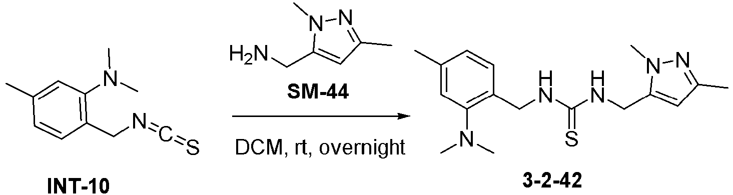

### 1. Synthesis of compound INT-10

Compound **INT-10** was synthesized by referring to Example 73.

### 2. Synthesis of compound 3-2-42

Pyrazolamine **SM-44** (101 mg, 0.81 mmol) was added to 6 ml of dichloromethane, to which was added **INT-10** (200 mg, 0.97 mmol), and the mixture was stirred at room temperature. The reaction was detected by TLC, and after completion of the reaction, the reaction solution was rotatory evaporated to dry. The residue was subjected to column chromatography to obtain 180 mg of compound **3-2-42.** Yield: 71%. MS (ESI) m/z: [M + H]⁺ Calcd for C₁₇H₂₆N₅S⁺:332.1; Found: 332.1.

¹H NMR (400 MHz, CDCl₃) *δ* 8.07 (d, *J* = 90.7 Hz, 2H), 7.12 (d, *J =* 7.6 Hz, 1H), 6.81 (d, *J* = 11.8 Hz, 2H), 5.76 (s, 1H), 4.71 (s, 2H), 4.22 (s, 2H), 3.55 (s, 3H), 2.39 (s, 6H), 2.24 (s, 3H), 2.13 (s, 3H).

### Example 108. Preparation of compound 3-2-43 of the present invention

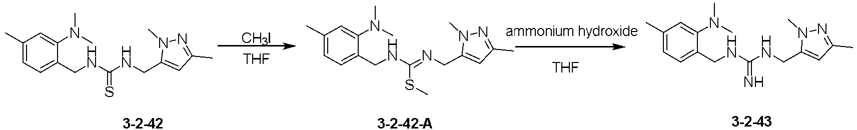

### 1. Synthesis of compound 3-2-42

Compound **3-2-42** was synthesized by referring to Example 107.

### 2. Synthesis of compound 3-2-42-A

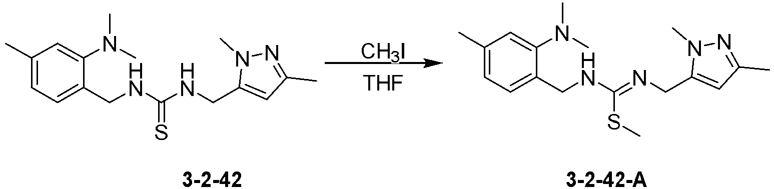

To a solution of **3-2-42** (450 mg, 1.36 mmol) in tetrahydrofuran, was added iodomethane (1.10 mL, 13.58 mmol), and then the reaction was stirred at 40 °C for 2 h. After completion of the reaction, the solvent was removed under reduced pressure to obtain crude product **3-2-42-A,** which was directly used in the next step.

### 3. Synthesis of compound 3-2-4

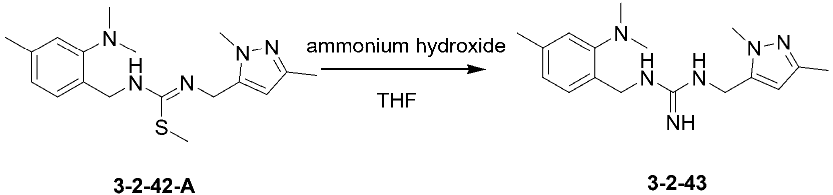

To a solution of **3-2-42-A** (480 mg, 1.39 mmol) in tetrahydrofuran (5 mL), was added ammonia water (8 mL), and then the reaction was stirred at 80 °C for 16 h. After completion of the reaction, the solvent was removed under reduced pressure to obtain the crude product, which was purified by column chromatography to yield white solid **3-2-43** (60.8 mg, yield 14%). m/z calculated for [M+H]⁺ 315.1, found 315.1.

¹H NMR (400 MHz, DMSO-*d₆*) *δ* 8.46 - 8.37 (m, 1H), 7.09 (d, *J =* 7.7 Hz, 1H), 7.00 (s, 1H), 6.91 (d, *J* = 7.7 Hz, 1H), 5.91 (s, 1H), 4.36 (s, 2H), 4.29 (s, 2H), 3.66 (s, 3H), 2.60 (s, 6H), 2.28 (s, 3H), 2.08 (s, 3H).

### Example 109. Preparation of compound 3-2-44 of the present invention

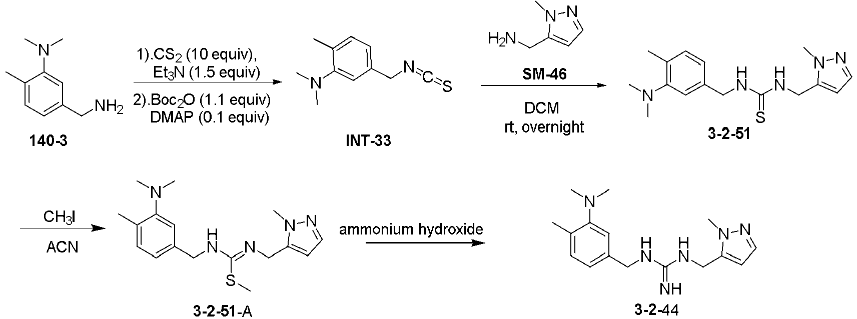

### 1. Synthesis of 140-3

Compound **140-3** was synthesized by referring to Example 18.

### 2. Synthesis of INT-33

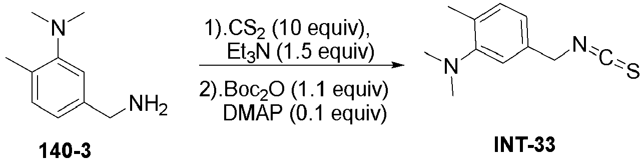

**140-3** (1.0 g, 6.0 mmol) was dissolved in tetrahydrofuran (20 mL), to which were added CS₂ (4.6 g, 60 mmol) and triethylamine (909 mg, 9 mmol) at room temperature, and then the mixture was stirred for 1 h at room temperature. Then, DMAP (74 mg, 0.6 mmol) and Boc₂O (1.44 g, 6.6 mmol) were added at 0 °C, and the reaction solution was warmed to room temperature and allowed to react overnight. The reaction was detected by TLC. After completion of the reaction, the reaction solution was extracted with water and EtOAc three times. The organic phase was dried over anhydrous Na₂SO₄, and the residue was purified by column chromatography, to obtain the target compound **INT-33** (655 mg), with a yield of 53%.

### 3. Synthesis of compound 3-2-51

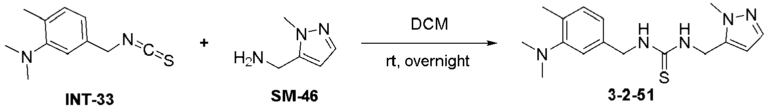

Pyrazolamine **SM-46** (101 mg, 0.81 mmol) was added to 6 ml of dichloromethane, to which was added **INT-33** (200 mg, 0.97 mmol), and the mixture was stirred overnight at room temperature. The reaction was detected by TLC, and after completion of the reaction, the reaction solution was rotatory evaporated to dry. The residue was subjected to column chromatography to obtain 185 mg of target compound **3-2-51.** Yield: 71%. MS (ESI) m/z: [M + H]⁺ Calcd for C₁₆H₂₄N₅S⁺:318.1; Found: 318.1.

### 4. Synthesis of compound 3-2-51-A

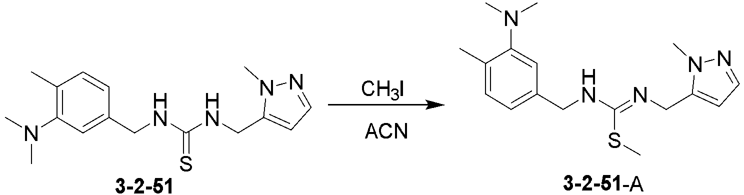

To a solution of **3-2-51** (500 mg, 1.58 mmol) in acetonitrile (10 mL), was added potassium iodide (1.28 mL, 15.75 mmol), and the mixture was stirred at 40 °C for 1 h. After the reaction was complete, the solvent was removed under reduced pressure to obtain the crude product **3-2-51-A,** which was directly used in the next step.

### 5. Synthesis of compound 3-2-44

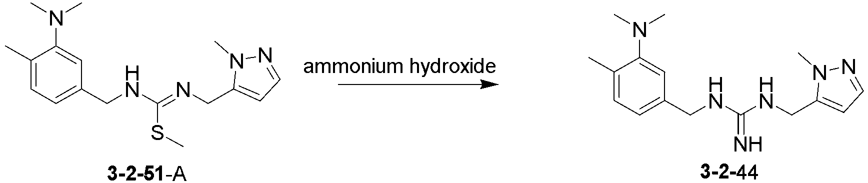

To a solution of **3-2-51-A** (500 mg, 1.51 mmol) in tetrahydrofuran (3 mL), was added ammonia water (8 mL), and then the reaction was stirred at 80 °C for 14 h. After completion of the reaction, the solvent was removed under reduced pressure, and the residue was purified by prep-HPLC to yield white solid **3-2-44** (163 mg, yield 36%). m/z calculated for [M+H]⁺ 301.4, found 301.1.

¹H NMR (400 MHz, DMSO-*d₆*) *δ* 7.26 (s, 1H), 7.07-7.05 (m, 1H), 6.96 (s, 1H), 6.83-6.81 (m, 1H), 6.11 (s, 1H), 4.33-4.29 (m, 2H), 4.24-4.21 (m, 2H), 3.72 (s, 3H), 2.60 (s, 6H), 2.21 (s, 3H).

### Example 110. Preparation of compound 3-2-45 of the present invention

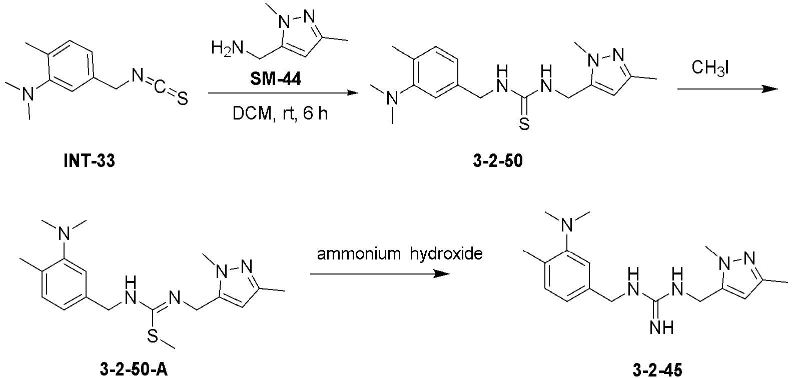

### 1. Synthesis of INT-33

Compound **INT-33** was synthesized by referring to Example 109.

### 2. Synthesis of 3-2-50

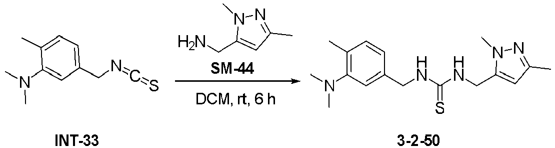

Pyrazolamine **SM-44** (80 mg, 0.64 mmol) was added to 5 ml of dichloromethane, to which was added **INT-33** (160 mg, 0.78 mmol), and the mixture was stirred at room temperature. The reaction was detected by TLC, and after completion of the reaction, the reaction solution was rotatory evaporated to dry. The residue was subjected to column chromatography to obtain 152 mg of target compound **3-2-50,** yield 75%. HRMS (ESI) m/z: [M + H]⁺ Calcd for C₁₇H₂₆N₅S⁺: 332.2; Found: 332.2.

### 3. Synthesis of 3-2-50-A

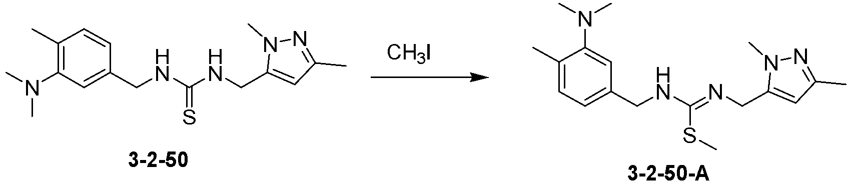

To a solution of **3-2-50** (700 mg, 2.11 mmol) in acetonitrile (10 mL), was added potassium iodide (1.71 mL, 21.12 mmol), and the mixture was stirred at 40 °C for 1 h. After the reaction was complete, the solvent was removed under reduced pressure to obtain the crude product **3-2-50-A,** which was directly used in the next step.

### 4. Synthesis of 3-2-45

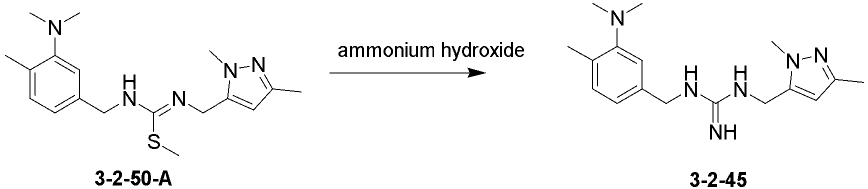

To a solution of **3-2-50-A** (700 mg, 2.03 mmol) in tetrahydrofuran (3 mL), was added ammonia water (10 mL), and then the reaction was stirred at 80 °C for 14 h. After completion of the reaction, the solvent was removed under reduced pressure, and the residue was purified by prep-HPLC to yield white solid **3-2-45** (130 mg, yield 20%). m/z calculated for [M+H] ⁺315.4, found 315.1.

¹H NMR (400 MHz, DMSO-*d₆*) *δ* 7.08-7.06 (m, 1H), 6.99 (s, 1H), 6.85-6.84 (m, 1H), 5.86 (s, 1H), 4.19 (s, 4H), 3.64 (s, 3H), 2.62 (s, 6H), 2.23 (s, 3H), 2.08 (s, 3H).

### Example 111. Preparation of compound 3-2-46 of the present invention

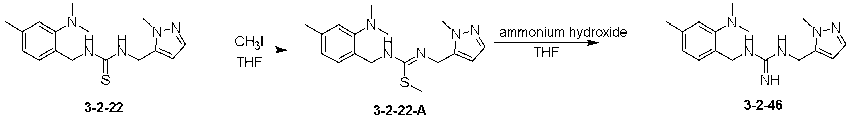

### 1. Synthesis of compound 3-2-22

Compound **3-2-22** was synthesized by referring to Example 87.

### 2. Synthesis of compound 3-2-22-A

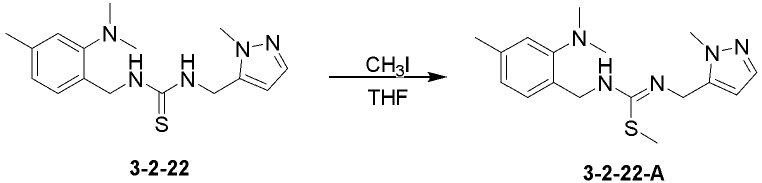

To a solution of **3-2-22** (450 mg, 1.42 mmol) in tetrahydrofuran (10 mL), was added potassium iodide (1.15 mL, 14.2 mmol), and the mixture was stirred at 40 °C for 2 h. After the reaction was complete, the solvent was removed under reduced pressure to obtain the crude product **3-2-22-A,** which was directly used in the next step.

### 3. Synthesis of compound 3-2-46

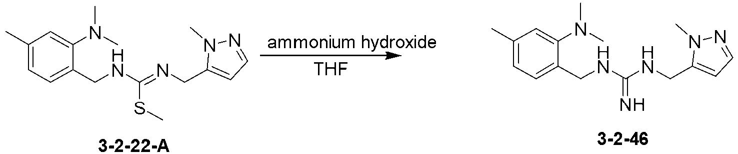

To a solution of **3-2-22-A** (450 mg, 1.36 mmol) in tetrahydrofuran (5 mL), was added ammonia water (8 mL), and then the reaction was stirred at 80 °C for 16 h. After completion of the reaction, the solvent was removed under reduced pressure, and the residue was purified by prep-HPLC to yield white solid **3-2-45** (82.7 mg, yield 21%). m/z calculated for [M+H] ⁺301.1, found 301.1.

¹H NMR (400 MHz, DMSO-*d₆*) *δ* 8.40 (s, 1H), 7.33 (d, *J =* 1.8 Hz, 1H), 7.09 (d, *J =* 7.7 Hz, 1H), 7.00 (s, 1H), 6.91 (d, *J =* 7.7 Hz, 1H), 6.17 (d, *J =* 1.5 Hz, 1H), 4.44 (s, 2H), 4.30 (s, 2H), 3.76 (s, 3H), 2.59 (s, 6H), 2.28 (s, 3H).

### Example 112. Preparation of compound 3-2-47 of the present invention

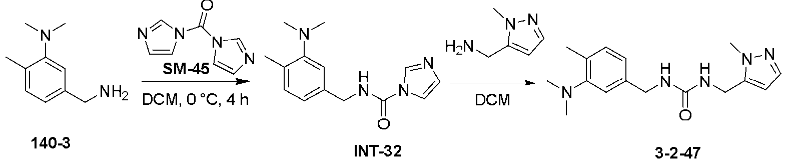

### 1. Synthesis of compound 140-3

Compound **140-3** was synthesized by referring to Example 18.

### 2. Synthesis of compound INT-32

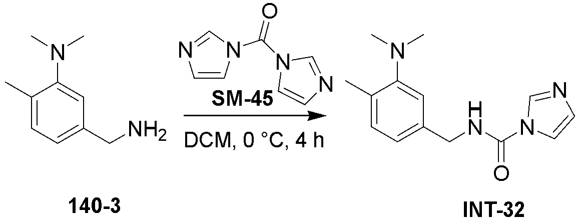

Benzylamine **110-3** (1.0 g, 6.0 mmol) was dissolved in dichloromethane and placed at 0 °C, to which was added carbonyldiimidazole **SM-45** (1.46 g, 9 mmol), and the mixture was stirred at 0 °C for 4 h. The reaction solution was rotatory evaporated to dry, and the residue was subjected to column chromatography, to obtain 990 mg of the target product **INT-32,** with a yield of 64%.

### 2. Synthesis of compound 3-2-47

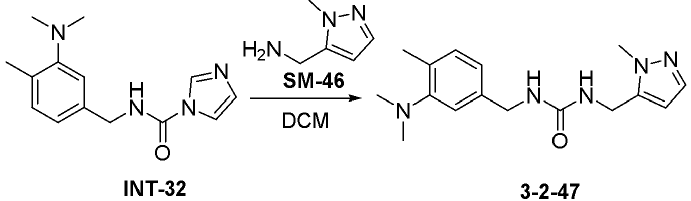

Pyrazolamine **SM-46** (64 mg, 0.58 mmol) was added to 3 ml of dichloromethane, to which was added **INT-32** (180 mg, 0.70 mmol), and the mixture was stirred at room temperature overnight. The reaction was detected by TLC, and after completion of the reaction, the reaction solution was rotatory evaporated to dry. The residue was subjected to column chromatography to obtain 154 mg of target compound **3-2-47,** yield 82%. MS (ESI) m/z: [M + H]⁺ Calcd for C₁₆H₂₄N₅O: 302.1; Found: 302.0.

¹H NMR (400 MHz, CDCl₃) *δ* 7.36 (d, *J=* 1.6 Hz, 1H), 7.10 (d, *J =* 7.6 Hz, 1H), 6.92 (s, 1H), 6.84 (d, *J* = 7.5 Hz, 1H), 6.08 (d, *J=* 1.5 Hz, 1H), 4.71 (s, 1H), 4.54 (s, 1H), 4.42 (d, *J =* 5.6 Hz, 2H), 4.30 (d, *J=* 5.6 Hz, 2H), 3.81 (s, 3H), 2.67 (s, 6H), 2.29 (s, 3H).

### Example 113. Preparation of compound 3-2-48 of the present invention

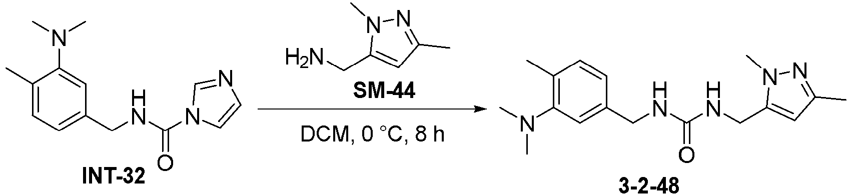

### 1. Synthesis of INT-32

Compound **INT-32** was synthesized by referring to Example 112.

### 2. Synthesis of 3-2-48

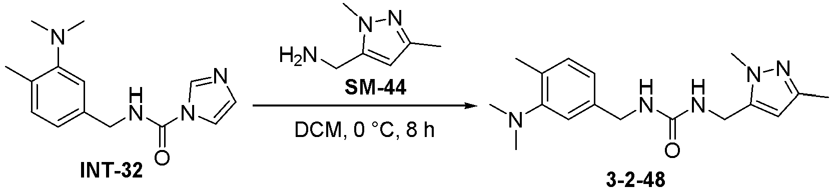

Pyrazolamine **INT-32** (72 mg, 0.58 mmol) was added to 3 ml of dichloromethane, to which was added **SM-44** (180 mg, 0.70 mmol), and the mixture was stirred at room temperature overnight. The reaction was detected by TLC, and after completion of the reaction, the reaction solution was rotatory evaporated to dry. The residue was subjected to column chromatography to obtain 150 mg of compound **3-2-48,** yield 82%. MS (ESI) m/z: [M + H]⁺ Calcd for C₁₇H₂₆N₅₀:316.2; Found: 316.1.

¹H NMR (400 MHz, CDCl₃) *δ* 7.10 (d, *J=* 7.4 Hz, 1H), 6.92 (s, 1H), 6.84 (d, *J =* 7.5 Hz, 1H), 5.85 (s, 1H), 4.76 (s, 1H), 4.58 (s, 1H), 4.34 (d, *J=* 5.4 Hz, 4H), 3.71 (s, 3H), 2.67 (s, 7H), 2.29 (s, 3H), 2.18 (s, 3H).

### Example 114. Preparation of compound 3-2-49 of the present invention

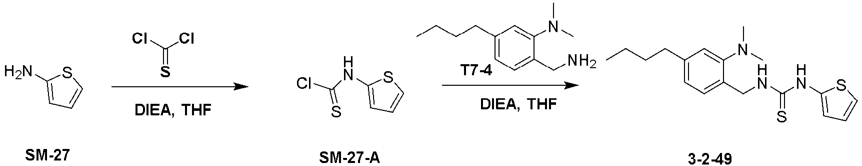

### 1. Synthesis of SM-27

Compound **SM-27** was synthesized by referring to Example 80.

### 2. Synthesis of SM-27-A

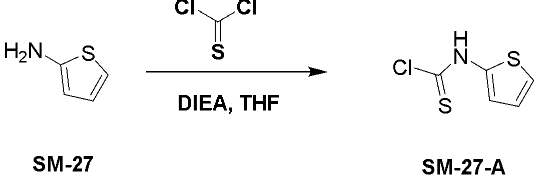

At 0 °C, to a solution of **SM-27** (180 mg, 2.01 mmol) in tetrahydrofuran (5 mL), were added DIEA (1042.85 mg, 8.07 mmol) and thiophosgene (463.82 mg, 4.03 mmol). The mixture was stirred at room temperature for 30 min, and the solvent was removed under reduced pressure to obtain **SM-27-A** as a brown oil, which was directly used in the next step.

### 3. Synthesis of T7-4

Compound **T7-4** was synthesized by referring to Example 53.

### 4. Synthesis of 3-2-49

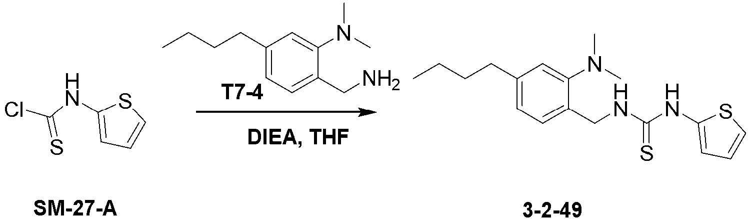

At 0 °C, to a solution of **SM-27-A** (300 mg, 1.69 mmol) in tetrahydrofuran (10 mL), were added DIEA (873 mg, 6.75 mmol) and **T7-4** (1045.23 mg, 5.07 mmol), and the mixed solution was stirred for 30 min. After completion of the reaction, water was added, and the resultant solution was extracted with ethyl acetate. The organic phase was dried over anhydrous sodium sulfate and concentrated. The crude product was purified by HPLC to obtain white solid **3-2-49** (196 mg, yield 33.4%). m/z calculated for [M+H] ⁺ 348.54, found 384.0.

¹HNMR (400 MHz, DMSO-*d₆*) *δ* 10.35 (s, 1H), 8.03 (s, 1H), 7.15 - 7.10 (m, 1H), 7.02 - 6.99 (m, 1H), 6.95 (s, 1H), 6.89 - 6.84 (m, 2H), 6.73 - 6.69 (m, 1H), 4.74 - 4.63 (m, 2H), 2.61 (s, 6H), 2.56 - 2.52 (m, 2H), 1.57 - 1.48 (m, 2H), 1.36 - 1.25 (m, 2H), 0.93 - 0.86 (m, 3H).

### Example 115. Preparation of compound 3-2-50 of the present invention

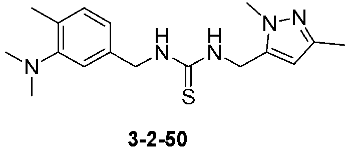

Compound **3-2-50** was synthesized by referring to Example 110.

¹H NMR (400 MHz, CDCl₃) *δ* 7.05 (d, *J =* 7.6 Hz, 1H), 6.88 (s, 2H), 6.79 (d, *J =* 7.5 Hz, 1H), 6.40 (s, 1H), 5.76 (s, 1H), 4.67 - 4.38 (m, 4H), 3.51 (s, 3H), 2.61 (s, 6H), 2.24 (s, 3H), 2.07 (s, 3H).

### Example 116. Preparation of compound 3-2-51 of the present invention

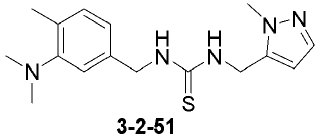

Compound **3-2-51** was synthesized by referring to Example 109.

¹H NMR (400 MHz, CDCl₃) *δ* 7.29 (d, *J =* 1.6 Hz, 1H), 7.09 (d, *J =* 7.6 Hz, 1H), 6.89 (s, 1H), 6.82 (d, *J =* 7.5 Hz, 1H), 6.62 (s, 1H), 6.04 (s, 2H), 4.73 (d, *J =* 4.9 Hz, 2H), 4.49 (s, 2H), 3.66 (s, 3H), 2.64 (s, 6H), 2.27 (s, 3H).

### Example 117. Preparation of compound 3-2-52 of the present invention

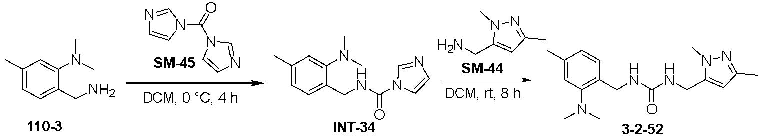

### 1. Synthesis of compound 110-3

Compound **110-3** was synthesized by referring to Example 1.

### 2. Synthesis of compound INT-34

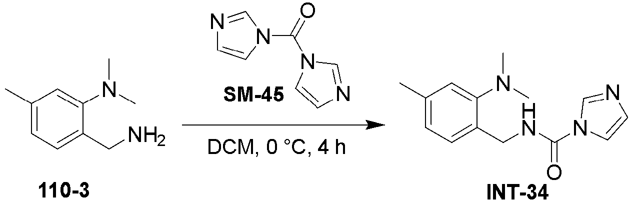

**110-3** (1.12 g, 7.1 mmol) was dissolved in dichloromethane and placed at 0 °C, to which was added **SM-45** (1.72 g, 10.6 mmol), and the mixture was stirred at 0 °C for 4 h. The reaction solution was rotatory evaporated to dry, and the residue was subjected to column chromatography, to obtain the target product **INT-34,** with a yield of 77%.

### 3. Synthesis of compound 3-2-52

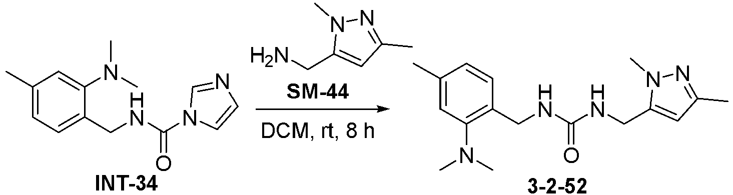

**SM-44** (100mg, 0.8 mmol) was added to 3 ml of dichloromethane, to which was added **INT-34** (248 mg, 0.96 mmol), and the mixture was stirred at room temperature overnight. The reaction was detected by TLC, and after completion of the reaction, the reaction solution was rotatory evaporated to dry. The residue was subjected to column chromatography to obtain 140 mg of compound **3-2-52,** yield 56%. MS (ESI) m/z: [M + H]⁺ Calcd for C₁₇H₂₆N₅O:316.2; Found: 316.2.

¹H NMR (400 MHz, CDCl₃) *δ* 7.14 (d, *J =* 8.1 Hz, 1H), 6.89 (d, *J =* 4.8 Hz, 2H), 5.82 (s, 1H), 5.27 (s, 1H), 4.30 (dd, *J=* 17.6, 4.7 Hz, 4H), 3.64 (s, 3H), 2.55 (s, 6H), 2.32 (s, 3H), 2.19 (s, 3H).

### Example 118. Preparation of compound 3-2-53 of the present invention

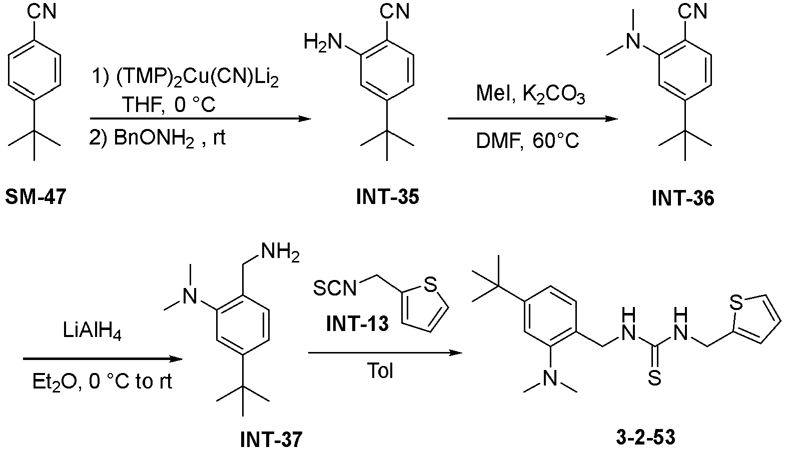

### 1. Synthesis of compound INT-35

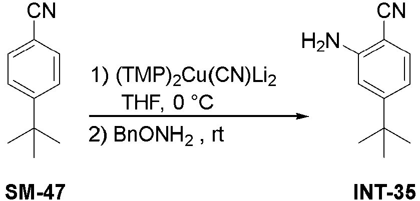

Under argon protection, 4-tert-butylbenzonitrile **SM-47** (847 µL, 5.0 mmol) was dissolved in tetrahydrofuran (13 mL) and cooled to -78 °C. The reaction solution (TMP)₂Cu(CN)Li₂ was gradually added, and then, the reaction solution was allowed to react at 0 °C for 2 h. The reaction solution was then cooled to -78°C again, to which was added benzylhydroxylamine (1.17 mL, 10.0 mmol), followed by reaction at room temperature. After the reaction was complete, the reaction was quenched by adding saturated NH₄Cl solution and saturated sodium thiosulfate solution. The resultant solution was extracted with ethyl acetate, and the organic phase was dried over anhydrous sodium sulfate. After concentration under reduced pressure, the residue was subjected to column chromatography (PE/EtOAc = 3:1) to obtain compound **INT-35** as a brown oil (720 mg, yield 83%).

### 2. Synthesis of compound INT-36

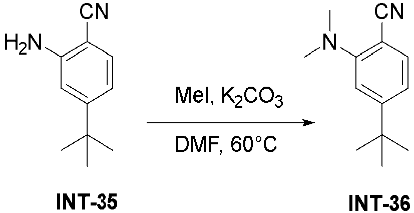

**INT-35** (700 mg, 4.0 mmol) was dissolved in dimethyl sulfoxide (15 mL), to which were added potassium carbonate (1.66 g, 12 mmol) and iodomethane (2.49 mL, 40.0 mmol). The mixture was reacted at 50 °C for 48 h. The reaction solution was extracted with ethyl acetate/water. The organic phase was dried over anhydrous sodium sulfate and concentrated under reduced pressure. The residue was subjected to column chromatography to yield **INT-36** as a yellow oil (456 mg, yield 56%).

### 3. Synthesis of compound INT-37

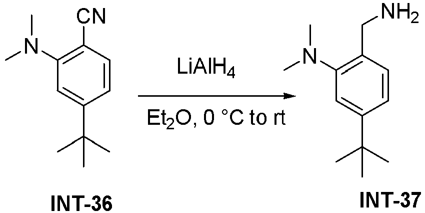

**INT-36** (456 mg, 2.2 mmol) was dissolved in diethyl ether (6 mL), to which was gradually added lithium aluminum hydride (168 mg, 4.4 mmol, 2.0 equiv) in batches at 0 °C, and then the mixture was allowed to react at room temperature for 2 h. After completion of the reaction, 3 mL of 15% NaOH solution was first added for quenching the reaction, to which were successively added 3 mL of water and 3 mL of 15% NaOH solution dropwise. The resultant solution was filtered through diatomaceous earth and over anhydrous sodium sulfate, and then concentrated under reduced pressure to obtain compound **INT-37** as a yellow oil (450 mg, yield 99%).

### 4. Synthesis of compound INT-13

Compound **INT-13** was synthesized by referring to Example 82.

### 5. Synthesis of compound 3-2-53

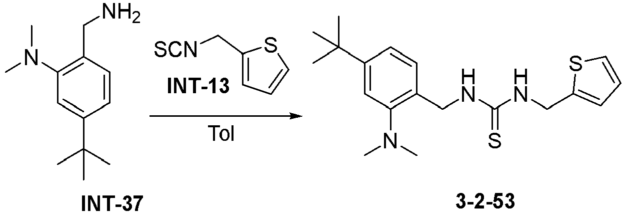

**INT-37** (248 mg, 1.2 mmol) was dissolved in toluene (2 mL), to which was added thioisocyanate **INT-13** (155 mg, 1.0 mmol). After completion of the reaction, the reaction was subjected to column chromatography (DCM/MeOH = 20:1) to provide **3-2-53:** 360 mg, with a yield of 99%. HRMS (ESI-TOF) *m*/*z:* [M +H]⁺ Calcd for C₁₉H₂₈N₃S₂⁺ 362.1719; Found 362.1721.

¹H NMR (400 MHz, CDCl₃) *δ* 7.24 -7.12 (m, 3H), 7.09 (d, *J=* 1.8 Hz, 1H), 6.94 (d, *J=* 3.4 Hz, 2H), 4.95 (s, 2H), 4.35 (s, 2H), 2.50 (s, 6H), 1.30 (s, 9H).

### Example 119. Preparation of compound 3-2-54 of the present invention

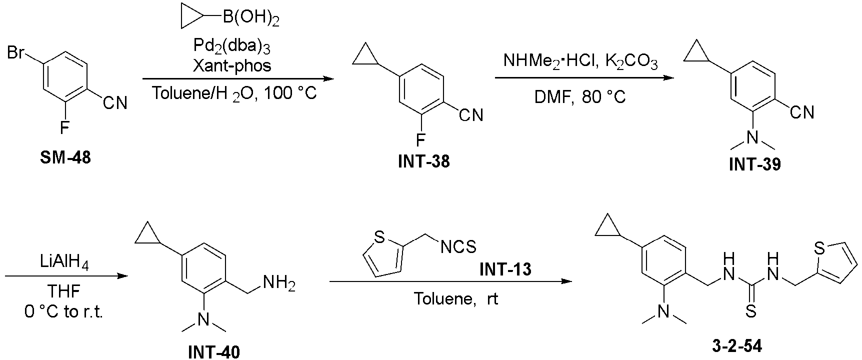

### 1. Synthesis of compound INT-38

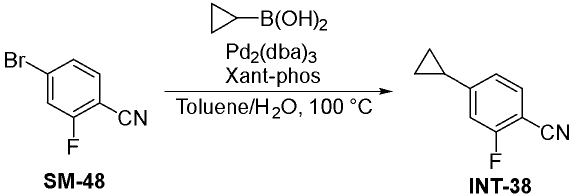

**SM-48** (990 mg, 5 mmol) and cyclopropylboronic acid (645 mg, 7.5 mmol) were dissolved in toluene (20 mL) and water (5 mL), to which were added Pd₂(dba)₃ (458 mg, 0.5 mmol) and Xant-Phos (262 mg, 0.55 mmol), and then the mixture was reacted at 100 °C. After completion of the reaction, water was added to dilute the solution, which was then extracted with ethyl acetate. The combined organic phases were dried over anhydrous sodium sulfate, and concentrated under reduced pressure. The residue was purified by column chromatography to obtain **INT-38** (790 mg, yield 98.7%) as a yellow oil.

### 2. Synthesis of compound INT-39

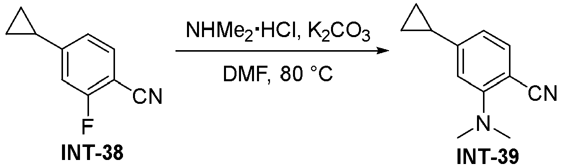

**INT-38** (790 mg, 4.93 mmol) was dissolved in DMF (15 mL), to which were added potassium carbonate (1.0 g, 17.1 mmol) and dimethylamine hydrochloride (1.4 g, 17.1 mmol). The mixture was stirred at 80 °C. After cooling to room temperature, the reaction solution was diluted with ethyl acetate and washed twice with saturated saline. The organic phase was dried over anhydrous sodium sulfate and concentrated at room temperature to yield **INT-39** as a yellow oil, which was directly used in the next step.

### 3. Synthesis of compound INT-40

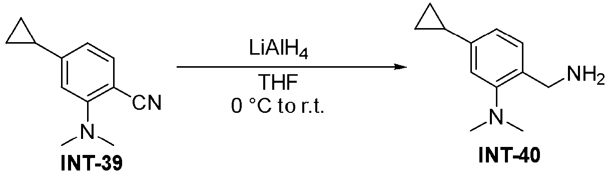

**INT-39** was dissolved in tetrahydrofuran (10 mL), to which was gradually added lithium aluminum hydride (182 mg, 4.8 mmol) in batches at 0 °C, and then the mixture was allowed to react at room temperature for 2 h. Then, ethyl acetate (20 mL) was first added to dilute the reaction solution, and then 15% NaOH solution (3 mL) was added for quenching the reaction. The resultant solution was stirred at room temperature for 15 min, and then mixed thoroughly with diatomaceous earth, and filtered through diatomaceous earth. The filtrate was washed with water, dried, and concentrated under reduced pressure to obtain compound **INT-40** as a yellow oil (330 mg, yield 35%).

### 4. Synthesis of compound INT-13

Compound **INT-13** was synthesized with reference to Example 82.

### 5. Synthesis of compound 3-2-54

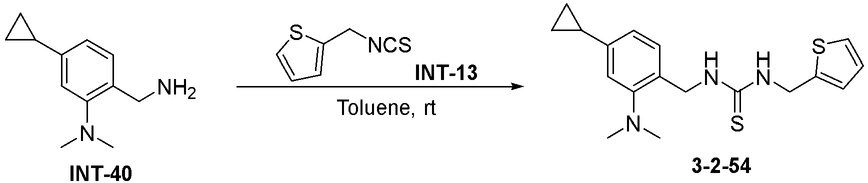

**INT-40** (175 mg, 0.85 mmol) and **INT-13** (267 mg, 1 mmol) were dissolved in toluene (10 mL) and reacted at room temperature for 16 h. The reaction was subjected to column chromatography to obtain compound **3-2-54** as white solids (yield 76%). HRMS (ESI-TOF) m/z: [M + H]⁺ Calcd for C₁₈H₂₄N₃S₂⁺ 346.1406; Found 346.1409

¹H NMR (400 MHz, CDCl₃) *δ* 7.32 (dd, *J=* 1.8, 0.9 Hz, 1H), 7.14 (d, *J =* 7.7 Hz, 1H), 6.98 (d, *J* = 1.9 Hz, 1H), 6.91 (dd, *J* = 7.7, 1.8 Hz, 1H), 6.29 (dd, *J=* 3.3, 1.9 Hz, 1H), 6.21 - 6.13 (m, 1H), 4.69 (d, *J=* 5.9 Hz, 2H), 4.37 (d, *J=* 5.7 Hz, 2H), 4.31 (d, *J=* 5.6 Hz, 2H), 2.65 (s, 8H), 1.59 (m, *J* = 7.9, 5.0 Hz, 2H), 1.38 (dt, *J=* 14.6, 7.3 Hz, 2H), 0.94 (t, *J=* 7.3 Hz, 3H).

### Example 120. Preparation of compound 3-2-55 of the present invention

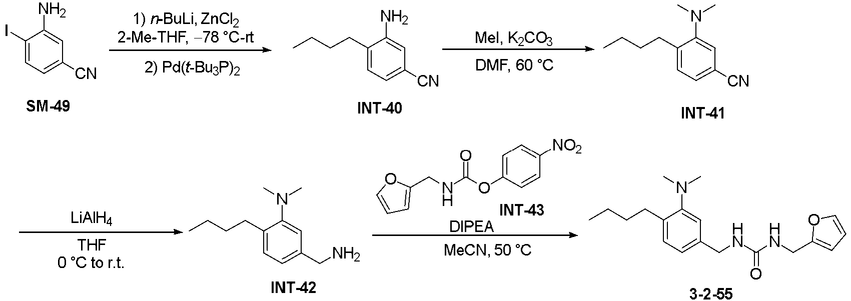

### 1. Synthesis of compound INT-40

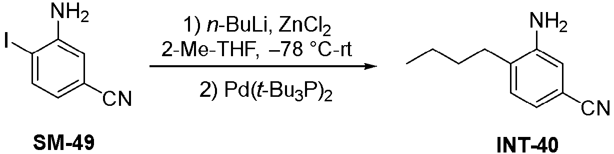

A solution of ZnCl₂ (1.22 g, 9 mmol) in 2-methyltetrahydrofuran (10 mL) was cooled to - 78 °C, to which was added n-butyllithium (5.8 mL, 14.4 mmol, 2.5 M in tetrahydrofuran) dropwise, and then the reaction solution was warmed to room temperature and stirred for 20 min. The above solution was added to a stirring mixture of **SM-49** (732 mg, 3 mmol) and Pd(*t*-Bu₃P)₂ (153 mg, 0.3 mmol), and reacted at room temperature for 2 h. The solution was diluted with water, and extracted with ethyl acetate. The organic phases were combined, dried over anhydrous sodium sulfate, and concentrated under reduced pressure. The residue was subjected to column chromatography, to obtain compound **INT-40** as white solids (417 mg, yield 80%).

### 2. Synthesis of compound INT-41

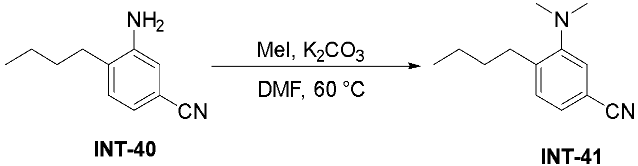

**INT-40** (417 mg, 2.4 mmol) was dissolved in DMF (15 mL), to which were added potassium carbonate (1.0 g, 7.3 mmol) and KI (0.9 mL, 14.5 mmol). The mixture was stirred at 60 °C, and reacted overnight. After cooling to room temperature, the reaction solution was diluted with ethyl acetate. The organic phase was dried over anhydrous sodium sulfate and concentrated at room temperature to yield **INT-41** as a yellow oil, which was directly used in the next step.

### 3. Synthesis of compound INT-42

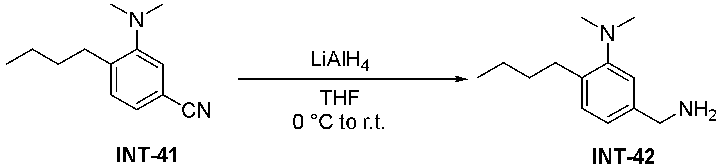

**INT-41** was dissolved in tetrahydrofuran (10 mL), to which was gradually added lithium aluminum hydride (182 mg, 4.8 mmol, 2 equiv.) in batches at 0 °C, and then the mixture was allowed to react at room temperature for 2 h. Then, ethyl acetate (20 mL) was first added to dilute the reaction solution, and then 15% NaOH solution (3 mL) was added for quenching the reaction. The resultant solution was stirred at room temperature for 15 min, and then mixed thoroughly with diatomaceous earth, and filtered through diatomaceous earth. The filtrate was dried, and concentrated under reduced pressure to obtain compound **INT-42** as a yellow oil (175 mg, yield 35%).

### 4. Synthesis of compound 3-2-5

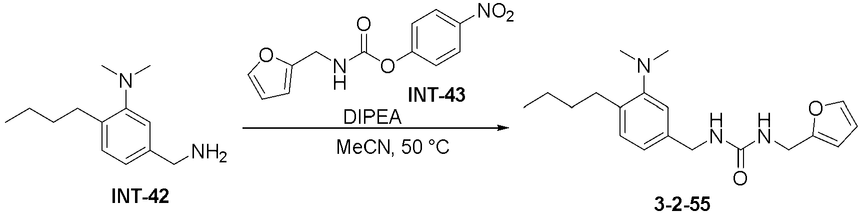

**INT-42** (175 mg, 0.85 mmol) and **INT-43** (267 mg, 1 mmol) were dissolved in acetonitrile (10 mL), to which was added DIPEA (177 *µ*L, 1 mmol), and the mixture was allowed to react at 50 °C for 16 h. The reaction was concentrated and subjected to column chromatography to obtain compound **3-2-55** as white solids: 175 mg, yield 63%. HRMS (ESI-TOF) *m*/*z:* [M + H]⁺ Calcd for C₁₉H₂₈N₃O₂⁺ 330.2176; Found 330.2184.

¹H NMR (400 MHz, CDCl₃) *δ* 7.32 (dd, *J=* 1.8, 0.9 Hz, 1H), 7.14 (d, *J =* 7.7 Hz, 1H), 6.98 (d, *J* = 1.9 Hz, 1H), 6.91 (dd, *J* = 7.7, 1.8 Hz, 1H), 6.29 (dd, *J=* 3.3, 1.9 Hz, 1H), 6.18 (m, 1H), 4.69 (d, *J=* 5.9 Hz, 2H), 4.37 (d, *J=* 5.7 Hz, 2H), 4.31 (d, *J =* 5.6 Hz, 2H), 2.65 (m, 8H), 1.59 (m, *J* = 7.9, 5.0 Hz, 2H), 1.38 (dt, *J=* 14.6, 7.3 Hz, 2H), 0.94 (t, *J=* 7.3 Hz, 3H).

### Example 121. Preparation of compound 3-2-56 of the present invention (P3-5-2-2)

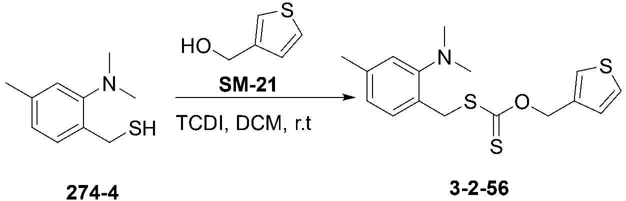

### 1. Synthesis of compound 274-4

Compound **274-4** was synthesized with reference to Example 98.

### 2. Synthesis of compound 3-2-56

**SM-21** (202 mg, 1.77 mmol) was added to a reaction flask, and then dissolved with dichloromethane (20 mL), to which were added triethylamine (536 mg, 5.31 mmol) and TCDI (315 mg, 1.77 mmol), and the mixture was stirred at room temperature for 2 h. Subsequently, **274-4** (320 mg, 1.77 mmol) was added to the system, and the mixture was allowed to react at room temperature overnight. After completion of the reaction, the solvent was removed by evaporation under reduced pressure to obtain the crude product, which was purified by silica gel column chromatography and reversed-phase prep-HPLC, to obtain **3-2-56** as white solids (170.72 mg, yield 28.7%). ESI [M + H]⁺= 337.4 [M+H]⁺.

¹H NMR (400 MHz, DMSO-*d₆*) *δ*: 7.68 (d, *J=* 2.0 Hz, 1H), 7.59 (dd, *J* = 2.8, 4.8 Hz, 1H), 7.20 (dd, *J =* 1.6, 5.2 Hz, 1H), 7.15 (d, *J =* 7.6 Hz, 1H), 6.99 (s, 1H), 6.81 (dd, *J =* 0.8, 7.6 Hz, 1H), 5.65 (s, 2H), 4.41 (s, 2H), 2.59 (s, 6H), 2.26 (s, 3H).

### Example 122. Preparation of compound 3-2-57 of the present invention (P3-2-1-5)

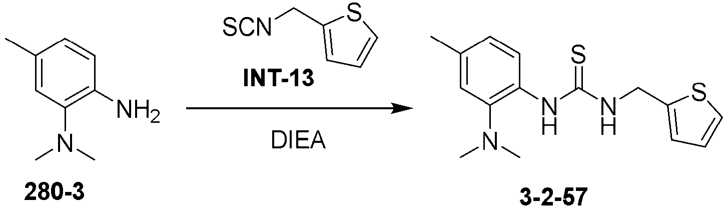

### 1. Synthesis of 280-3

Compound **280-3** was synthesized with reference to Example 13.

### 2. Synthesis of compound INT-13

Compound **INT-13** was synthesized with reference to Example 82.

### 3. Synthesis of compound 3-2-57

To a solution of compound **INT-13** (500 mg, 3.22 mmol) in tetrahydrofuran (10 mL), were added compound **280-3** (483.86 mg, 3.22mmol) and DIEA (2 mL), and the mixture was stirred and reacted at 80 °C for 3 h. After completion of the reaction, water was added, and the resultant solution was extracted with ethyl acetate. The organic layer was washed with concentrated brine, dried over anhydrous sodium sulfate, and concentrated under vacuum. The residue was purified to yield compound **3-2-57** as light purple solids (275 mg, yield 28%). m/z calculated for [M+H] ⁺ 306.5, found 306.1.

¹H NMR (400 MHz, DMSO-*d₆*) *δ* 8.99 (s, 1H), 8.32 (s, 1H), 7.46 - 7.38 (m, 2H), 7.05-7.04 (m, 1H), 7.00 - 6.95 (m, 1H), 6.86 (s, 1H), 6.78-6.76 (m, 1H), 4.91 (d, *J =* 4 Hz, 2H), 2.58 (s, 6H), 2.27 (s, 3H).

The beneficial effects of the present invention were demonstrated by the following specific experimental example.

### Experimental example 1: Study on the potent analgesic effect of the compound according to the present invention

### 1 Experimental method

Using the loss of tail withdrawal reflex (LOTWR) in rats as an indicator, the potent analgesic effect of the compounds according to the present invention was evaluated.

**1.1 Experimental animals:** Adult male SD rats weighing between 220-300 g were selected for the experiment. The rats were housed in sawdust-bedded cages with a temperature of 25±1°C, humidity ranging from 40% to 60%, and a 12-hour light/12-hour dark cycle. Each cage contained no more than 5 rats, with free access to water and food.

### 1.2 Experimental protocol:

(1) The compound of the present invention, along with the positive control drug remifentanil and the negative control (vehicle), were administered in fixed volumes. A dose escalation approach was employed to determine the minimum effective dose at which the compound of the present invention produces potent analgesic effects, as well as the minimum dose at which severe adverse reactions first appear.
(2) LOTWR lasting for 30 seconds was used as the criterion for generating potent analgesic effects. During the experiment, drugs were administered via the tail vein of rats, with a dosage volume of 0.6 mL per rat and a dosage rate of 0.02 mL/s. Subsequently, an alligator clip was applied at a distance of 1 cm from the base of the rat's tail, clamping once longitudinally and once transversely before being left in place. Meanwhile, the distal end of the alligator clip was continuously oscillated (30 times/min) to continuously generate stimulation. When the rat exhibited escape reactions (i.e., avoidance movements, struggling, and squeaking) or when the alligator clip remained on the tail for 30 seconds (to avoid tissue damage), the clip was removed. If the rat exhibited escape reactions, that time point was recorded as "no LOTWR," and the process was repeated at a 2-minute interval. When all results were "no LOTWR" after 10 minutes of testing (5 trials), the test was stopped and recorded as "ineffective."
(3) All compounds would undergo a dose escalation for efficacy evaluation, starting from 1 mg/kg (due to the high potency of remifentanil, the lowest dose was set at 1 µg/kg), followed by 5 mg/kg, 10 mg/kg, 20 mg/kg, and so on (increasing by 20 mg/kg increments after 20 mg/kg). The dose escalation would be stopped when rats show signs of death, and the lowest effective dose and the lowest dose at which severe adverse reactions occur would be recorded throughout the process. In this study, severe adverse reactions are defined as apnea, generalized tonic rigidity, convulsions, opisthotonus, seizures, etc.

**1.3 Evaluation indicators:** the minimum effective dose; the minimum dose resulting in serious adverse reactions; the safety therapeutic index = "the minimum dose resulting in serious adverse reactions" / "the minimum effective dose".

### 2 Experimental results

**Table 1. The efficacy and safety margin (single intravenous injection) of the compounds of the present invention in rats.**

| Example No. | The minimum effective dose | The safety therapeutic index |
|---|---|---|
| Remifentanil | A (10 µg/kg) | +(2) |
| Vehicle | - | - |
| Example 1 | A | ++ |
| Example 2 | A | + |
| Example 3 | A | ++ |
| Example 4 | D | + |
| Example 5 | A | ++ |
| Example 6 | A | ++ |
| Example 7 | D | + |
| Example 8 | D | + |
| Example 9 | C | + |
| Example 10 | A | +++ |
| Example 11 | A | ++ |
| Example 12 | A | + |
| Example 13 | A | +++ |
| Example 14 | A | + |
| Example 15 | B | +++ |
| Example 16 | B | + |
| Example 17 | A | ++ |
| Example 18 | A | ++ |
| Example 19 | A | + |
| Example 20 | A | + |
| Example 21 | A | +++ |
| Example 22 | D | + |
| Example 23 | D | + |
| Example 24 | B | ++ |
| Example 26 | D | + |
| Example 27 | A | +++ |
| Example 28 | D | + |
| Example 29 | A | + |
| Example 30 | C | + |
| Example 31 | A | +++ |
| Example 32 | A | +++ |
| Example 33 | A | + |
| Example 34 | C | + |
| Example 35 | C | ++ |
| Example 36 | A | +++ |
| Example 37 | A | +++ |
| Example 38 | B | ++ |
| Example 39 | B | ++ |
| Example 40 | D | + |
| Example 41 | D | + |
| Example 42 | D | + |
| Example 43 | B | +++ |
| Example 44 | B | + |
| Example 45 | D | ++ |
| Example 46 | D | + |
| Example 47 | A | + |
| Example 48 | A | ++ |
| Example 49 | D | ++ |
| Example 50 | C | + |
| Example 51 | A | ++ |
| Example 52 | A | ++ |
| Example 53 | A | + |
| Example 54 | A | + |
| Example 55 | A | + |
| Example 56 | A | + |
| Example 57 | B | + |
| Example 58 | D | + |
| Example 59 | B | ++ |
| Example 60 | B | + |
| Example 61 | A | + |
| Example 62 | B | ++ |
| Example 63 | A | + |
| Example 64 | A | +++ |
| Example 65 | A | +++ |
| Example 66 | A | ++ |
| Example 67 | A | +++ |
| Example 68 | A | ++ |
| Example 69 | A | ++ |
| Example 70 | B | + |
| Example 71 | A | + |
| Example 72 | A | ++ |
| Example 73 | A | +++ |
| Example 74 | D | + |
| Example 75 | A | ++ |
| Example 76 | A | +++ |
| Example 77 | A | ++ |
| Example 78 | B | + |
| Example 79 | C | + |
| Example 80 | A | +++ |
| Example 81 | A | ++ |
| Example 82 | A | ++ |
| Example 83 | A | +++ |
| Example 84 | D | + |
| Example 85 | A | + |
| Example 86 | A | ++ |
| Example 87 | A | +++ |
| Example 88 | A | +++ |
| Example 89 | A | ++ |
| Example 90 | A | ++ |
| Example 92 | A | ++ |
| Example 93 | A | + |
| Example 94 | A | +++ |
| Example 95 | A | + |
| Example 96 | A | + |
| Example 97 | D | + |
| Example 98 | D | + |
| Example 99 | D | + |
| Example 100 | D | + |
| Example 101 | D | ++ |
| Example 103 | A | +++ |
| Example 104 | D | + |
| Example 105 | A | +++ |
| Example 106 | A | +++ |
| Example 107 | B | ++ |
| Example 108 | A | +++ |
| Example 109 | A | + |
| Example 110 | A | + |
| Example 111 | A | + |
| Example 112 | A | +++ |
| Example 113 | B | +++ |
| Example 114 | A | ++ |
| Example 115 | A | +++ |
| Example 116 | A | ++ |
| Example 117 | D | ++ |
| Example 118 | A | + |
| Example 119 | A | + |
| Example 120 | A | +++ |
| Example 121 | D | ++ |
| Example 122 | A | +++ |

| | | |
|---|---|---|
| Note: "-" indicates that the test has been completed, but the effect has not occurred or is not present; "A" indicates that the minimum effective dose is ≤10.00 mg/kg; "B" indicates that the minimum effective dose is between 10.00 mg/kg and 20.00 mg/kg inclusive; "C" indicates that the minimum effective dose is between 20.00 mg/kg and 30.00 mg/kg inclusive; "D" indicates that the minimum effective dose is >30.00 mg/kg. "+" indicates a safety therapeutic index of ≤2; "++" indicates that the safety therapeutic index is between 2 and 4 inclusive; "+++" indicates a safety therapeutic index of >4. | | |

As shown in Table 1, both the compounds of the present invention and the µ-type opioid receptor agonist remifentanil can produce precise and potent systemic analgesic effects. Moreover, as shown in the table, compared to the µ-type opioid receptor agonist remifentanil, most of the compounds of the present invention have significantly higher therapeutic safety indices, indicating that the compounds of the present invention are safer.

The present invention provided a compound with analgesic effect. The compound of the present invention exhibited excellent analgesic effect, good safety during use, minimal toxic side effects, and no dependency during use. Therefore, the compound of the present invention had broad application prospects in the manufacture of analgesic medicaments, providing a new option for the preparation of drugs with analgesic effect in clinical practice.

## Claims

1. Compounds represented by formula I, or stereoisomers thereof, or pharmaceutically acceptable salts thereof, or solvates thereof, or crystal forms thereof, or prodrugs thereof, or metabolites thereof, or deuterated derivatives thereof: 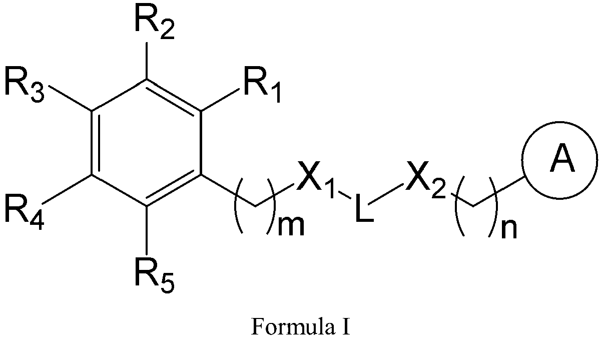 wherein,
R₁ and R₄ are each independently selected from the group consisting of H, NR₆R₇, (3-6)-membered cycloalkyl, (3-6)-membered heterocycloalkyl, substituted or unsubstituted C₁-C₆ alkyl, OR₁ₐ, and R₁ₐ is selected from the group consisting of C₁-C₆ alkyl and phenyl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0 to 5, and R_{1b} is selected from the group consisting of C₁-C₆ alkyl, (3-6)-membered cycloalkyl, and (3-6)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0 to 5; n is selected from integers of 0 to 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R_{I2}, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1 to 5;
provided that ring A is 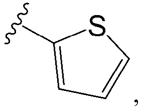 X₁ and X₂ are both NH, L is C=O, m is 0, and n is 1, 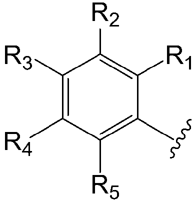 is not 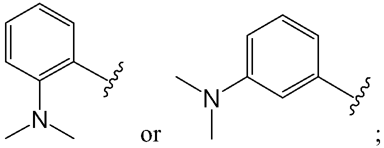
provided that ring A is 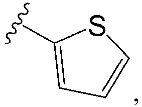 X₁ and X₂ are both NH, L is C=O, m and n are 0, 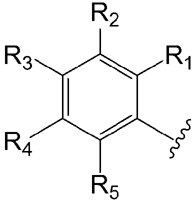 is not 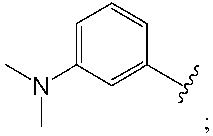
provided that ring A is 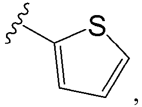 X₁ and X₂ are both NH, L is C=NH, m is 1, n is 2, 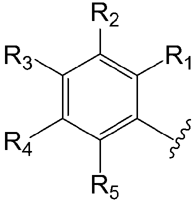 is not

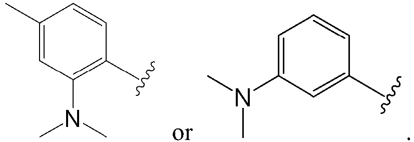

2. The compound according to claim 1, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula IIa or formula IIb: 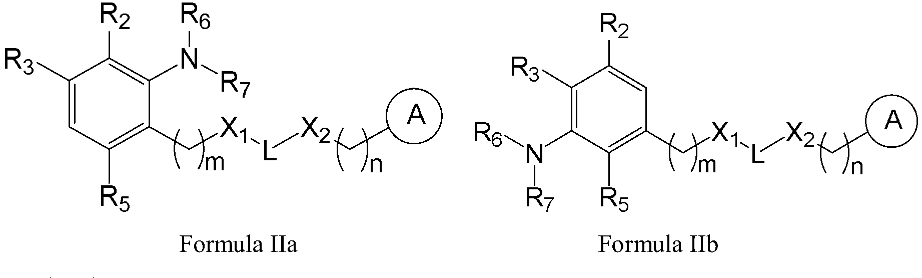 wherein,
R₂ and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0 to 5, and R_{1b} is selected from C₁-C₆ alkyl, (3-6)-membered cycloalkyl, and (3-6)-membered heterocycloalkyl;
R₃ is selected from H, substituted or unsubstituted C₁-C₆ alkyl, halogen, and hydroxyl;
R₆ and R₇ are each independently selected from the group consisting of substituted or unsubstituted C₁-C₆ alkyl, and substituted or unsubstituted C₁-C₆ alkoxy;
m is selected from 0, 1, 2, 3, 4, or 5; n is selected from 0, 1, 2, 3, 4, or 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1, 2, 3, 4, or 5;

3. The compound according to claim 2, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula IIIa or formula IIIb: 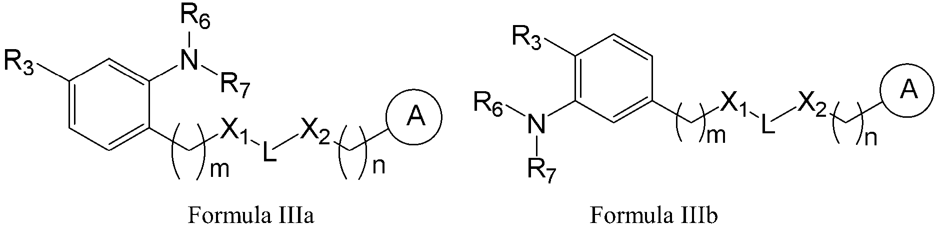 wherein,
R₃ is selected from H, substituted or unsubstituted C₁-C₆ alkyl, halogen, and hydroxyl;
R₆ and R₇ are each independently selected from the group consisting of substituted or unsubstituted C₁-C₆ alkyl, and substituted or unsubstituted C₁-C₆ alkoxy;
m is selected from 0, 1, 2, 3, 4, or 5; n is selected from 0, 1, 2, 3, 4, or 5;
X₁ and X₂ are each independently selected from the group consisting of O, S, and NR₈;
each of R₈ is independently selected from the group consisting of H and substituted or unsubstituted C₁-C₆ alkyl;
L is selected from the group consisting of C=X₃, substituted or unsubstituted (4-8)-membered cycloalkyl, substituted or unsubstituted (4-8)-membered heterocycloalkyl, substituted or unsubstituted (5-8)-membered aryl, and substituted or unsubstituted (5-8)-membered heteroaryl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy; or two substituents in the same atom form =O;
the heteroatoms in said heterocycloalkyl and heteroaryl are O, S, and/or N, with a number of 1, 2, 3, 4, or 5;

4. The compound according to any one of claims 1 to 3, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**:
X₁ and X₂ are each independently selected from the group consisting of O, S, and NH;
and/or, L is selected from the group consisting of 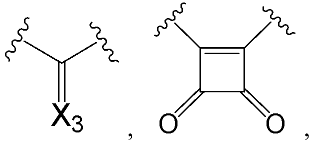 and 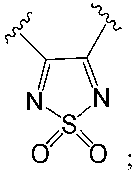
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
the number of substituents for said alkyl and alkoxy is one or two or more, and each substituent is each independently selected from the group consisting of deuterium, halogen, amino, nitro, cyano, carboxyl, hydroxyl, and mercapto.

5. The compound according to claim 4, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**: 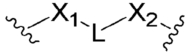 is selected from the group consisting of 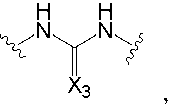 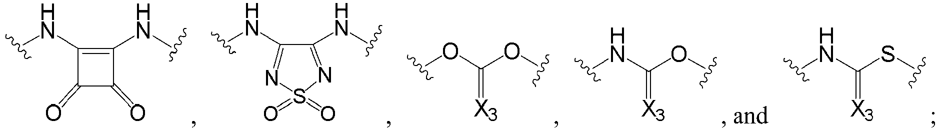
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
the number of substituents for said alkyl and alkoxy is one or two or more, and each substituent is each independently selected from the group consisting of deuterium, halogen, amino, nitro, cyano, carboxyl, hydroxyl, and mercapto.

6. The compound according to claim 1, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula IV: 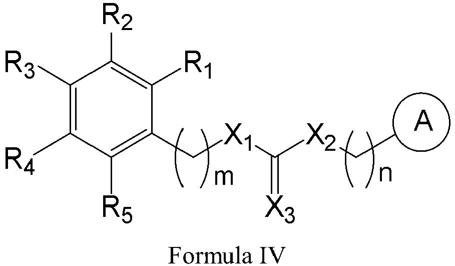 wherein,
R₁ and R₄ are each independently selected from the group consisting of H, NR₆R₇, (3-5)-membered cycloalkyl, (3-5)-membered heterocycloalkyl, substituted or unsubstituted C₁-C₆ alkyl, OR₁ₐ, and R₁ₐ is selected from the group consisting of C₁-C₆ alkyl and phenyl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-5)-membered cycloalkyl, and (3-5)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
X₁ and X₂ are each independently selected from the group consisting of O, S, and NR₈;
each of R₈ is independently selected from the group consisting of H and substituted or unsubstituted C₁-C₆ alkyl;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-8)-membered heteroaryl)-fused ((5-8)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5;

7. The compound according to claim 6, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula IVa: 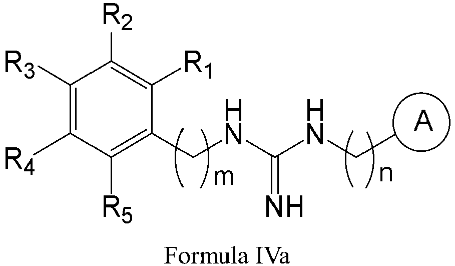 wherein,
R₁ and R₄ are each independently selected from the group consisting of H, NR₆R₇, (3-4)-membered cycloalkyl, (3-4)-membered heterocycloalkyl, substituted or unsubstituted C₁-C₆ alkyl, OR₁ₐ, and R₁ₐ is selected from the group consisting of C₁-C₆ alkyl and phenyl, with the proviso that one and only one of R₁ and R₄ is selected from NR₆R₇;
R₂, R₃, and R₅ are each independently selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused ((5-6)-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

8. The compound according to claim 6, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula Va or Vb: 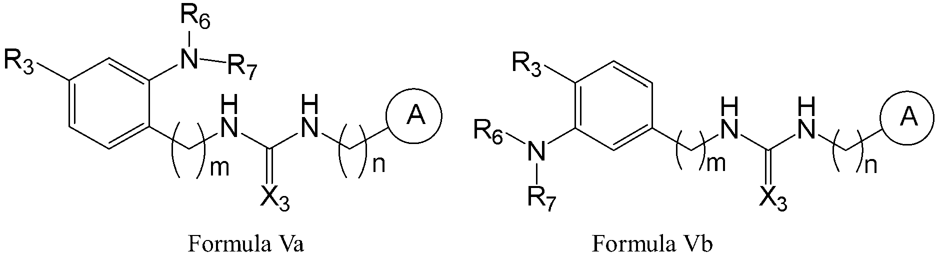 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
m is selected from integers of 0, 1, 2, 3, 4, and 5; n is selected from integers of 0, 1, 2, 3, 4, and 5;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

9. The compound according to claim 8, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula VIa or VIb: 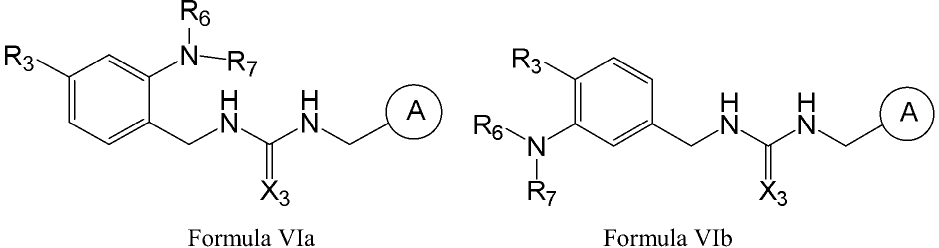 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

10. The compound according to claim 8, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** the compound is represented by formula VIIa or VIIb: 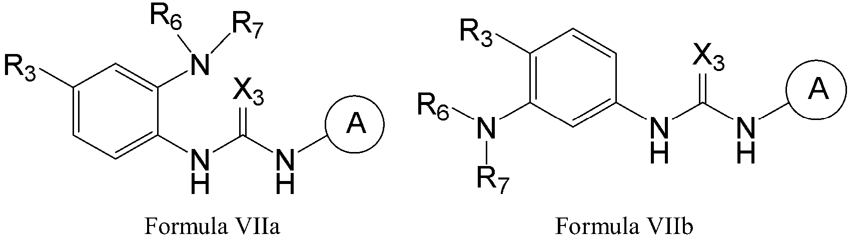 wherein,
R₃ is selected from the group consisting of H, substituted or unsubstituted C₁-C₆ alkyl, substituted or unsubstituted C₁-C₆ alkoxy, NR₆R₇, (CH₂)ₚCONHR_{1b}, halogen, and hydroxyl; p is selected from integers of 0, 1, 2, 3, 4, and 5, and R_{1b} is selected from the group consisting of C₁-C₆ alkyl, (3-4)-membered cycloalkyl, and (3-4)-membered heterocycloalkyl;
R₆ and R₇ are each independently selected from the group consisting of substituted or unsubstituted C₁-C₆ alkyl, and substituted or unsubstituted C₁-C₆ alkoxy;
X₃ is selected from the group consisting of O, S, NR₉, and CR₁₀R₁₁;
R₉ is selected from the group consisting of H, cyano, substituted or unsubstituted C₁-C₆ alkyl, and substituted or unsubstituted C₁-C₆ alkoxy,
R₁₀ and R₁₁ are each independently selected from the group consisting of H, cyano, nitro, - C(O)R₁₂, substituted or unsubstituted C₁-C₆ alkyl, and substituted or unsubstituted C₁-C₆ alkoxy;
R₁₂ is selected from the group consisting of substituted or unsubstituted C₁-C₆ alkyl, and substituted or unsubstituted C₁-C₆ alkoxy;
ring A is selected from the group consisting of substituted or unsubstituted (5-8)-membered aryl, substituted or unsubstituted (5-8)-membered heteroaryl, substituted or unsubstituted (5-8)-membered cycloalkyl, substituted or unsubstituted (5-8)-membered heterocycloalkyl, substituted or unsubstituted ((5-6)-membered heteroaryl)-fused (6-membered aryl);
the number of substituents for said alkyl and alkoxy is one or two or more, and each substituent is each independently selected from the group consisting of deuterium, halogen, amino, nitro, cyano, carboxyl, hydroxyl, and mercapto;
the number of substituents for said cycloalkyl, heterocycloalkyl, aryl, and heteroaryl is one or two or more, and each substituent is independently selected from the group consisting of deuterium, halogen, amino, nitro, cyano, carboxyl, hydroxyl, mercapto, substituted or unsubstituted C₁-C₆ alkyl, and substituted or unsubstituted C₁-C₆ alkoxy;
the heteroatoms in said heterocycloalkyl and heteroaryl are O, S, and/or N, with a number of 1, 2, 3, 4, or 5.

11. The compound according to any one of claims 1-3 or 5-10, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that**:
ring A is selected from the group consisting of substituted or unsubstituted following groups: 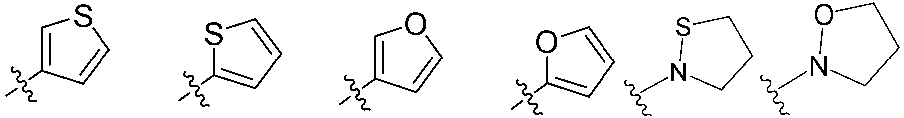 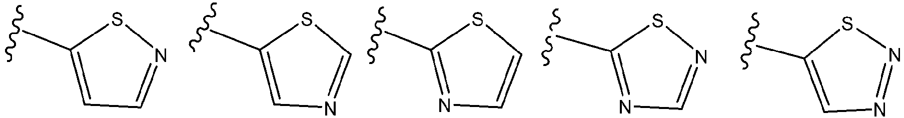 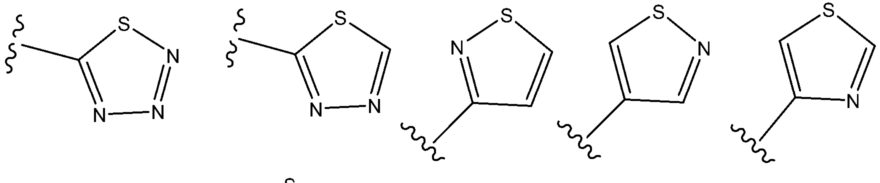 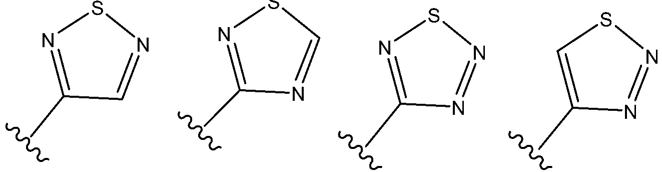 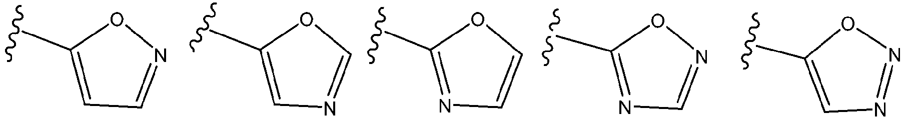 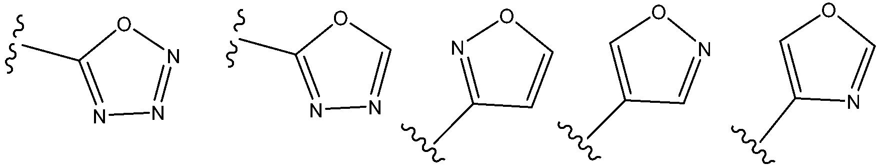 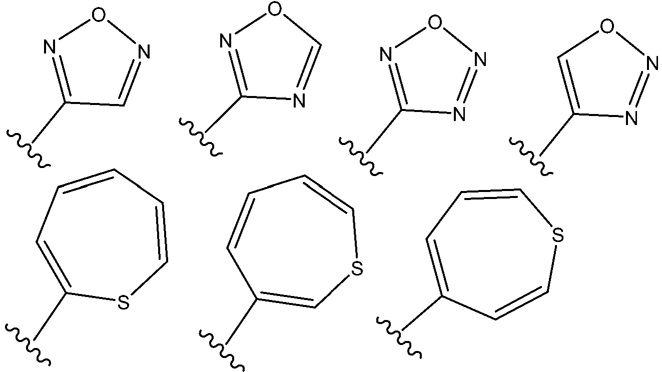 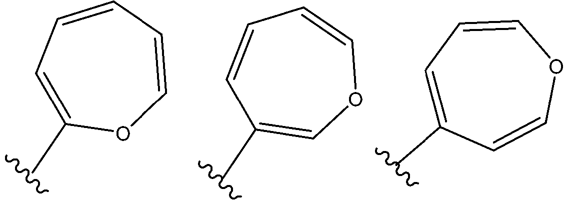 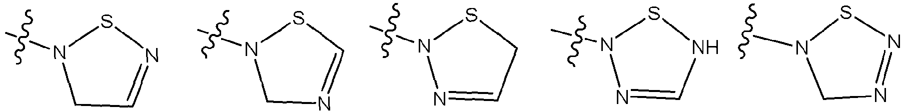 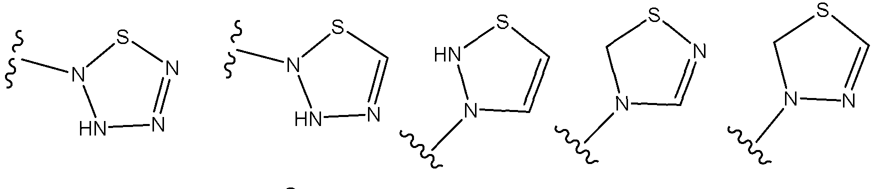 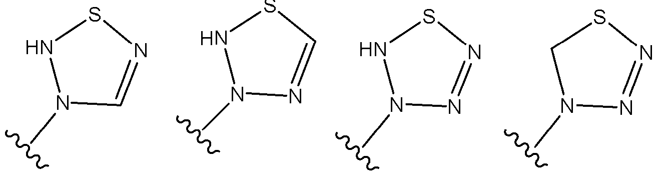 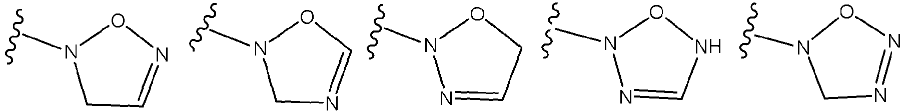 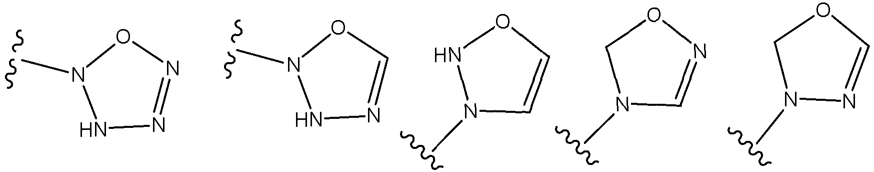 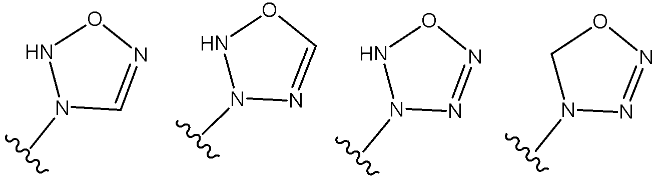 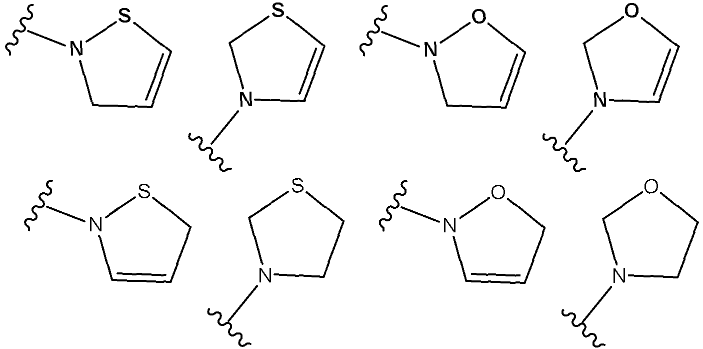 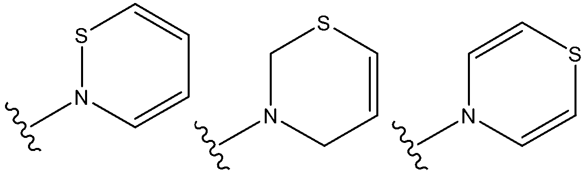 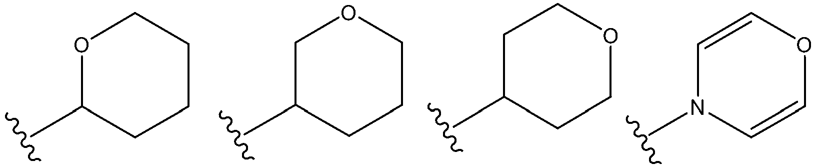 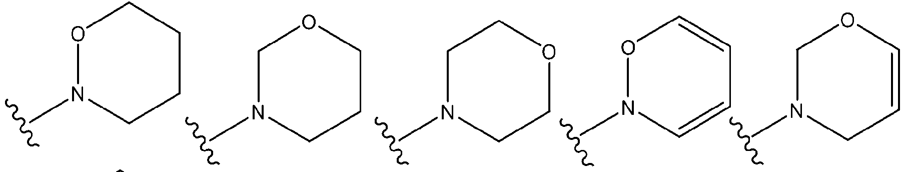 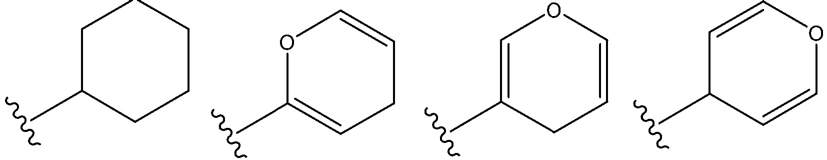 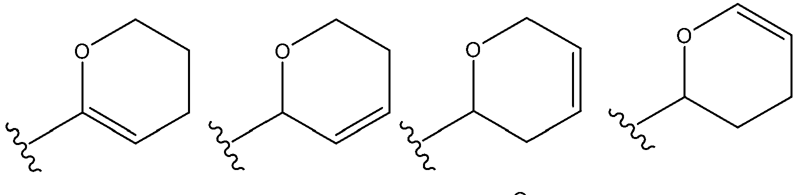 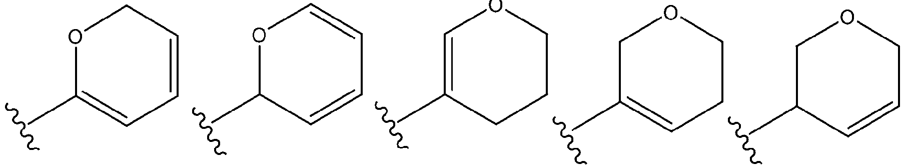 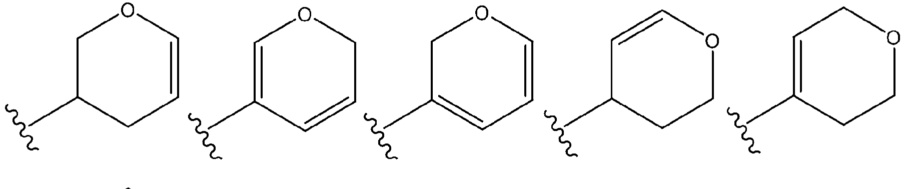 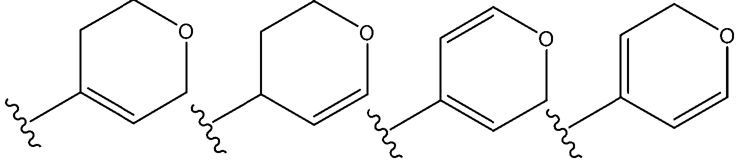 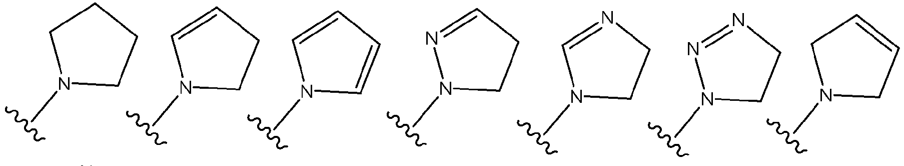 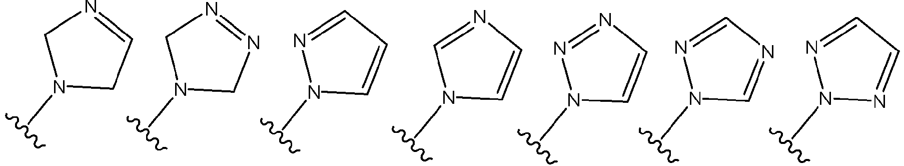 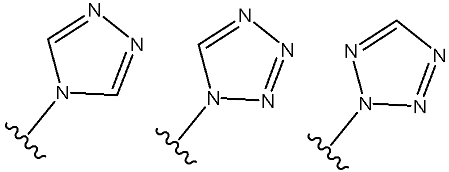 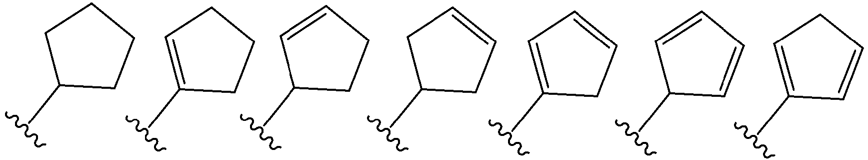 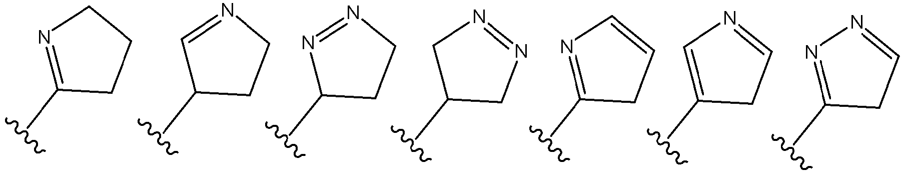 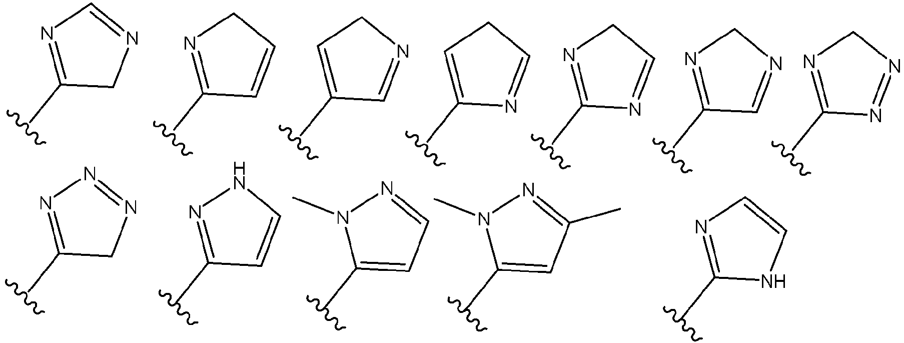 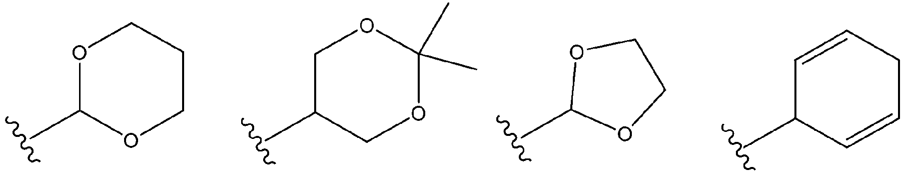 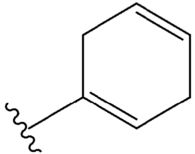 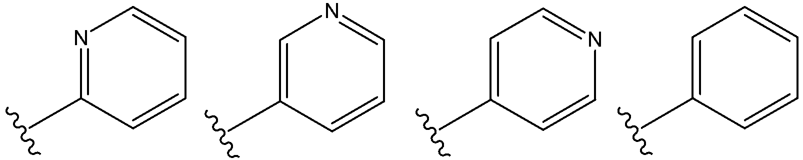 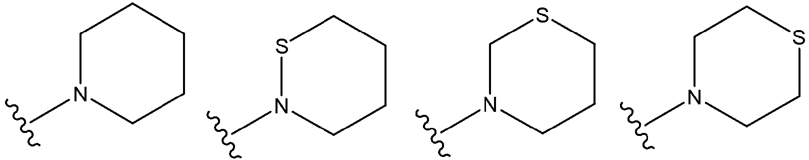 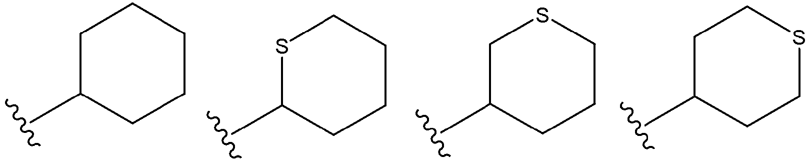 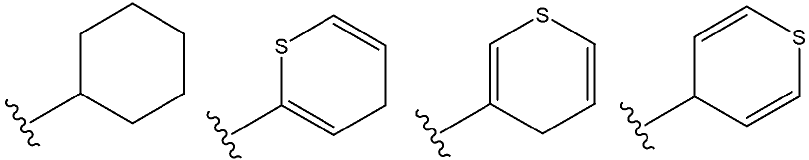 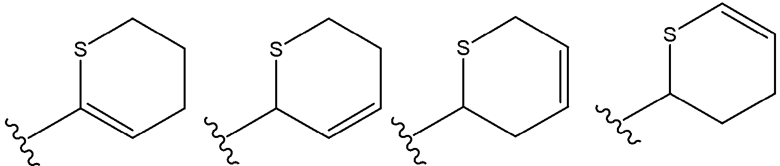 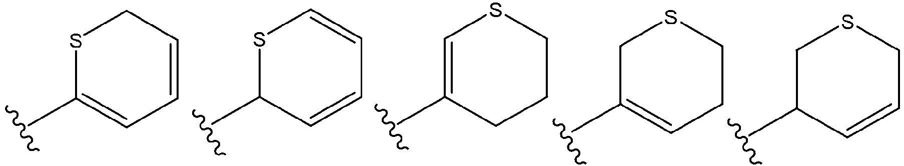 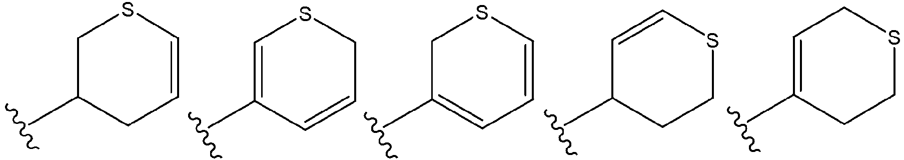 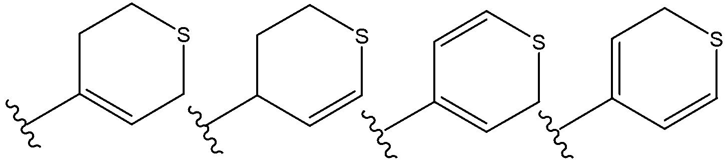 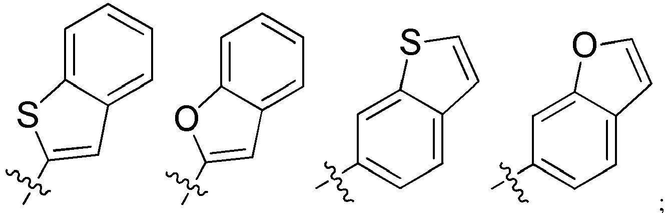
The substituent of ring A is selected from the group consisting of deuterium, halogen, amino, nitro, cyano, carboxyl, hydroxyl, mercapto, C₁-C₆ alkyl, and C₁-C₆ alkoxy;
preferably, the substituent of ring A is selected from the group consisting of deuterium, halogen, amino, nitro, cyano, carboxyl, hydroxyl, mercapto, C₁-C₄ alkyl, and C₁-C₄ alkoxy;

12. The compound according to any one of claims 1-3 or 5-10, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** it is selected from the group consisting of following compounds:

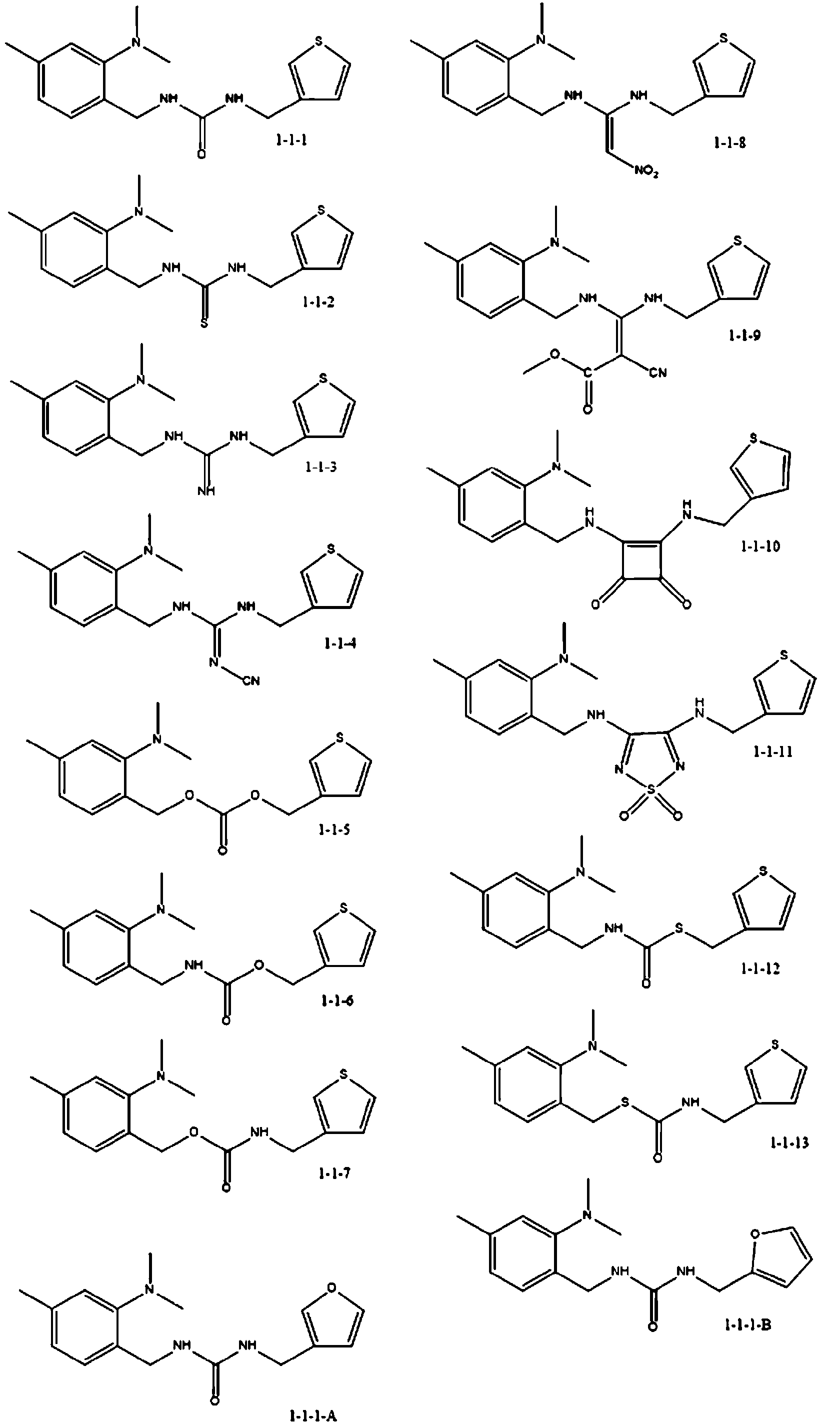

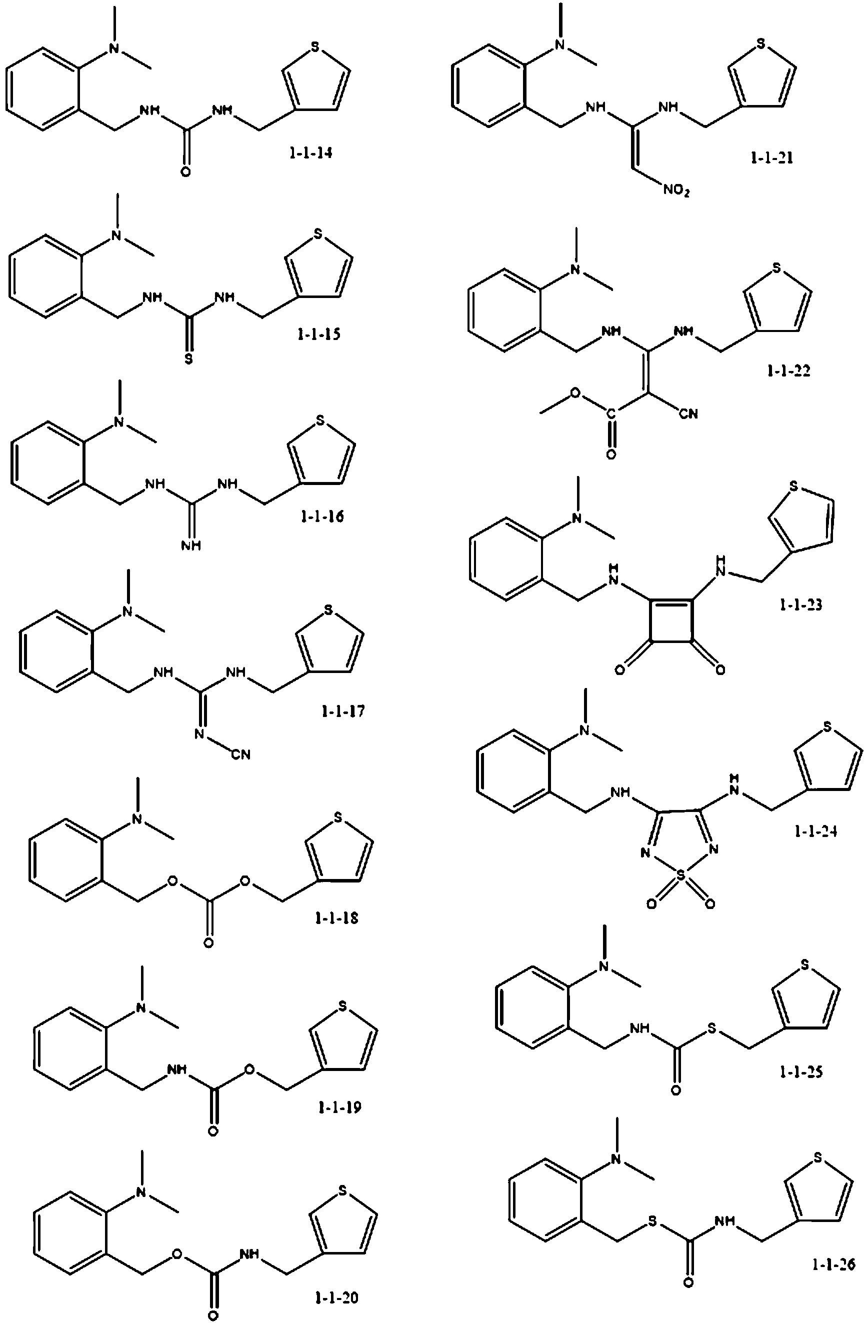

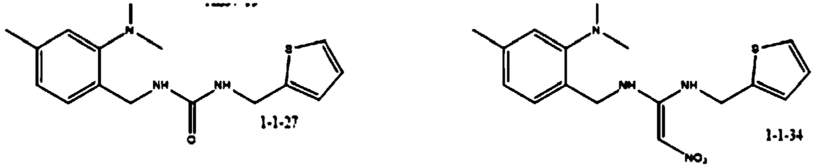

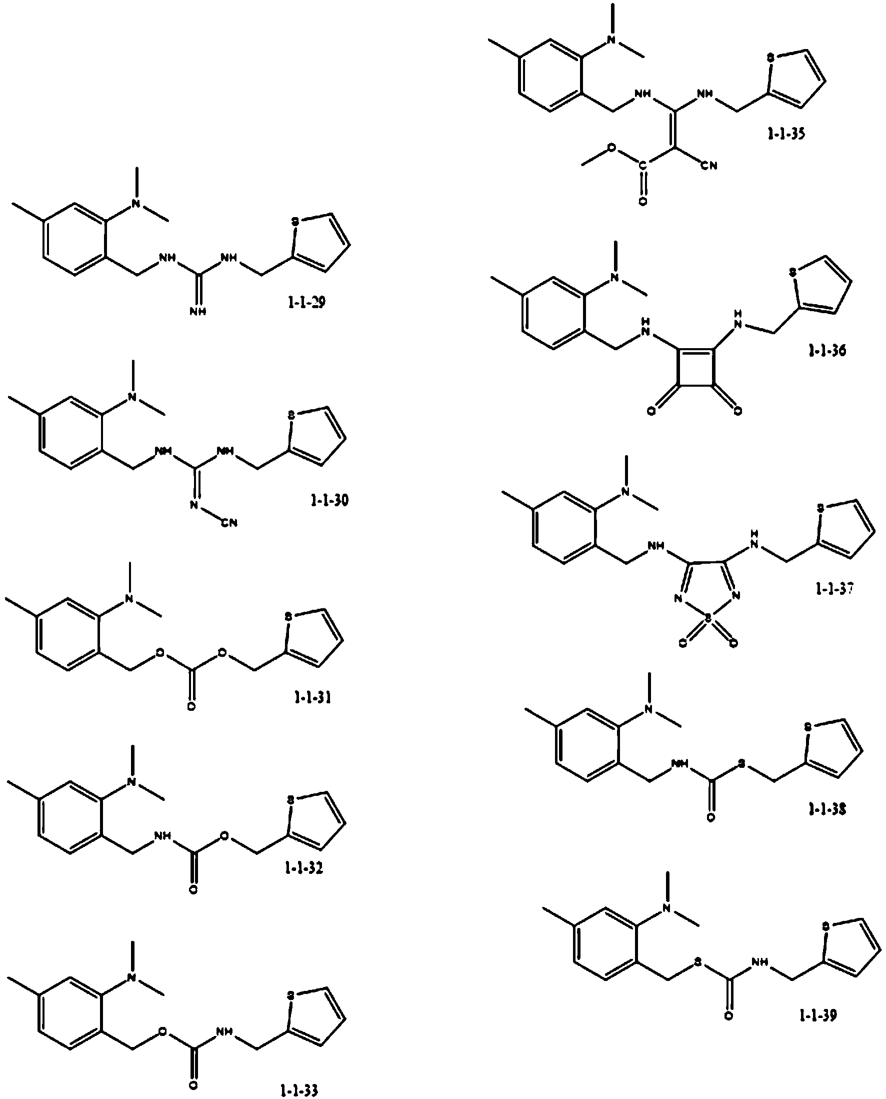

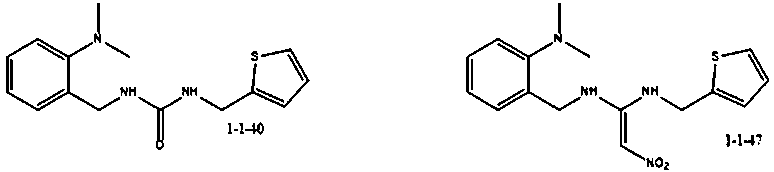

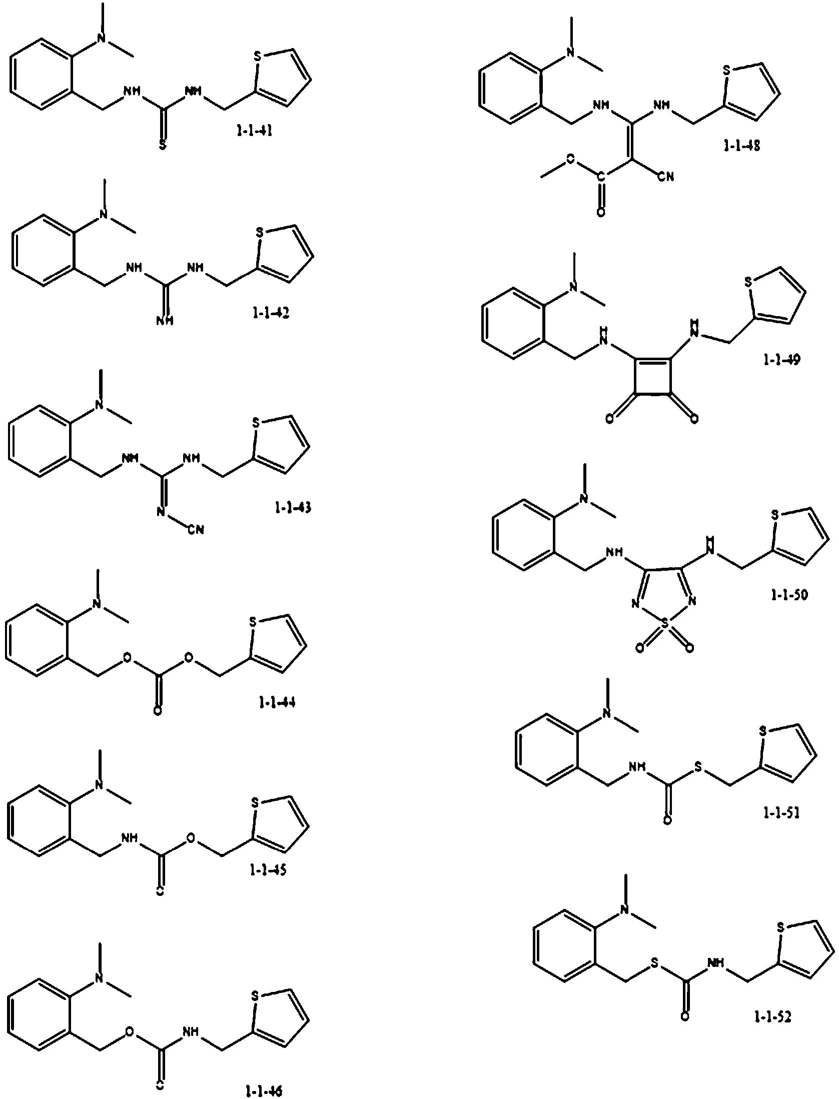

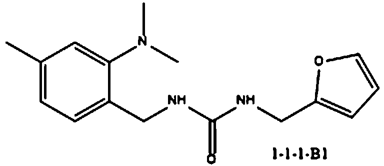

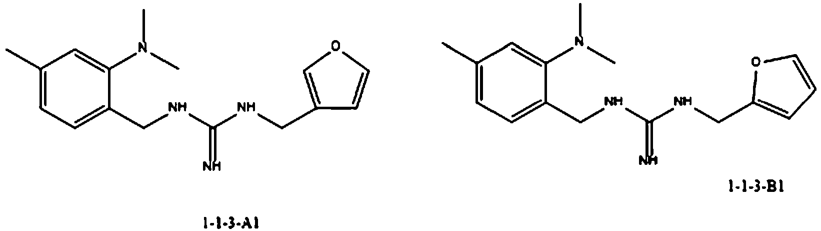

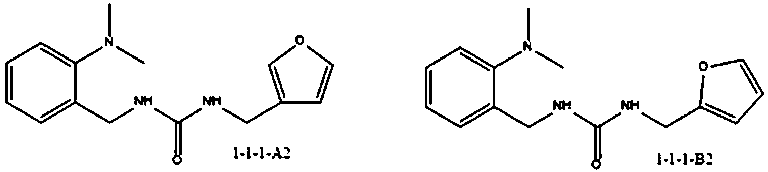

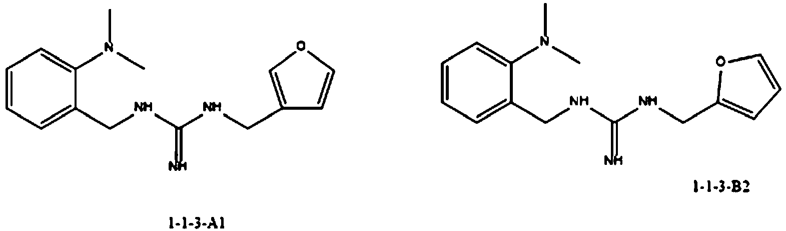

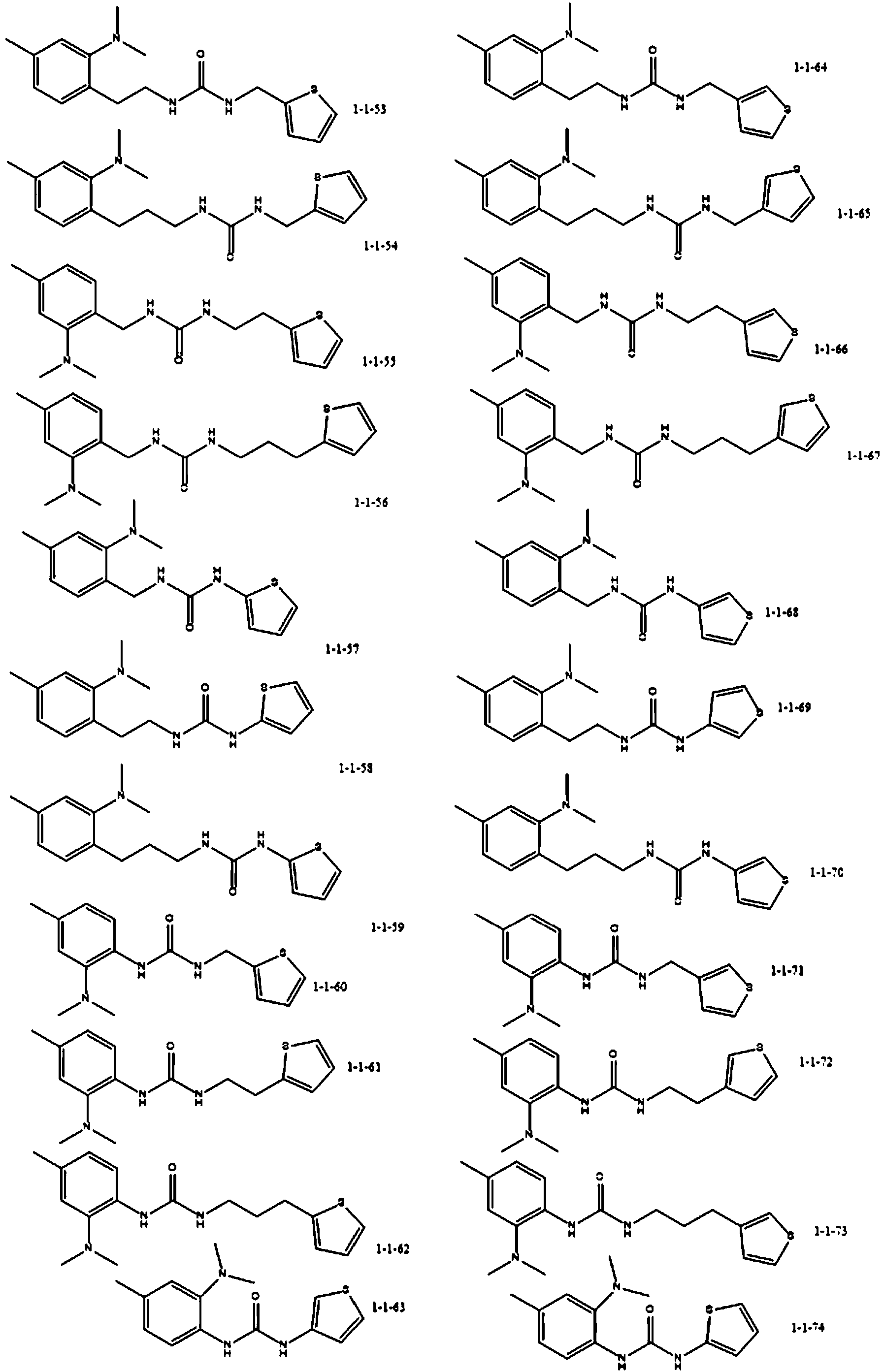

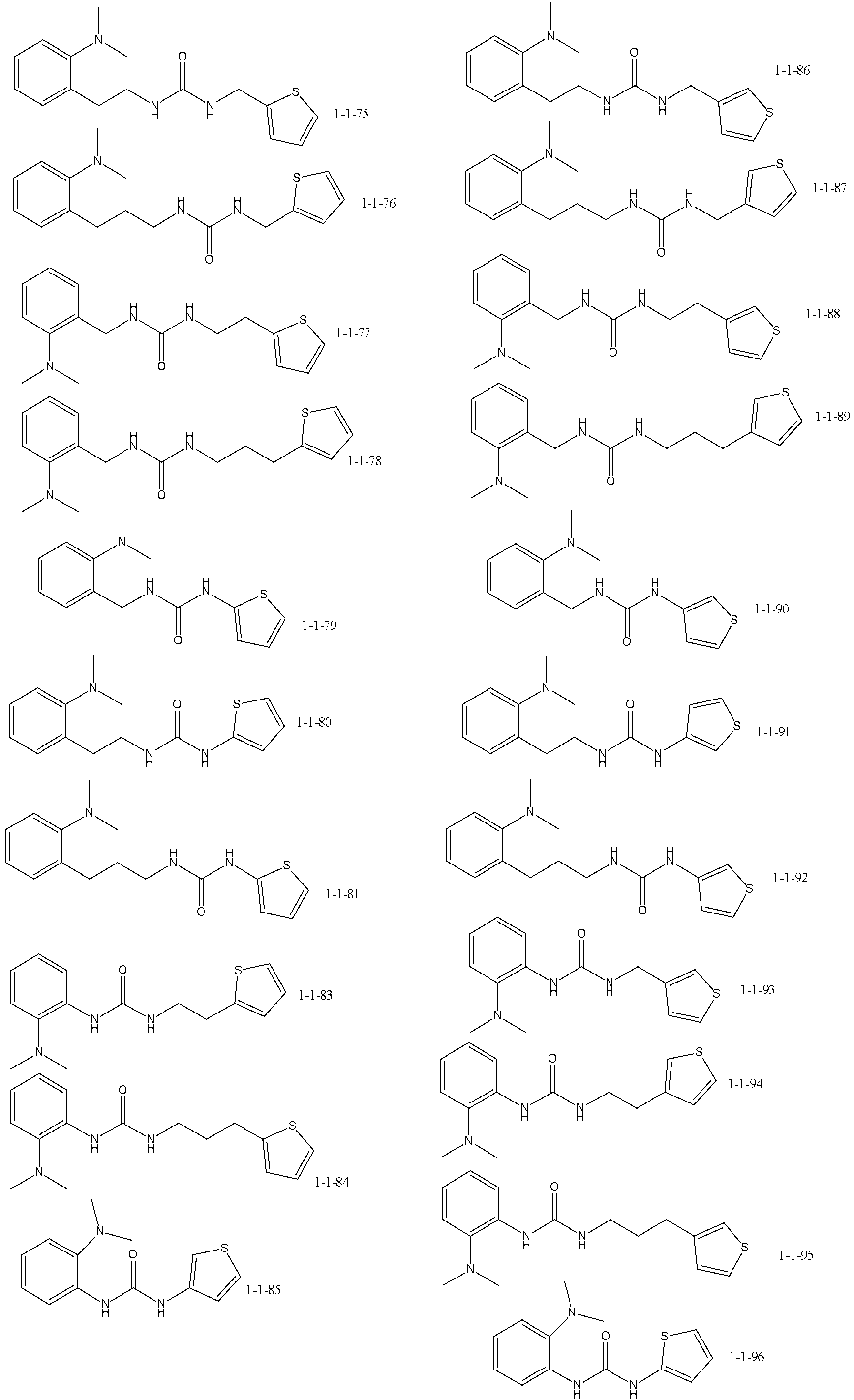

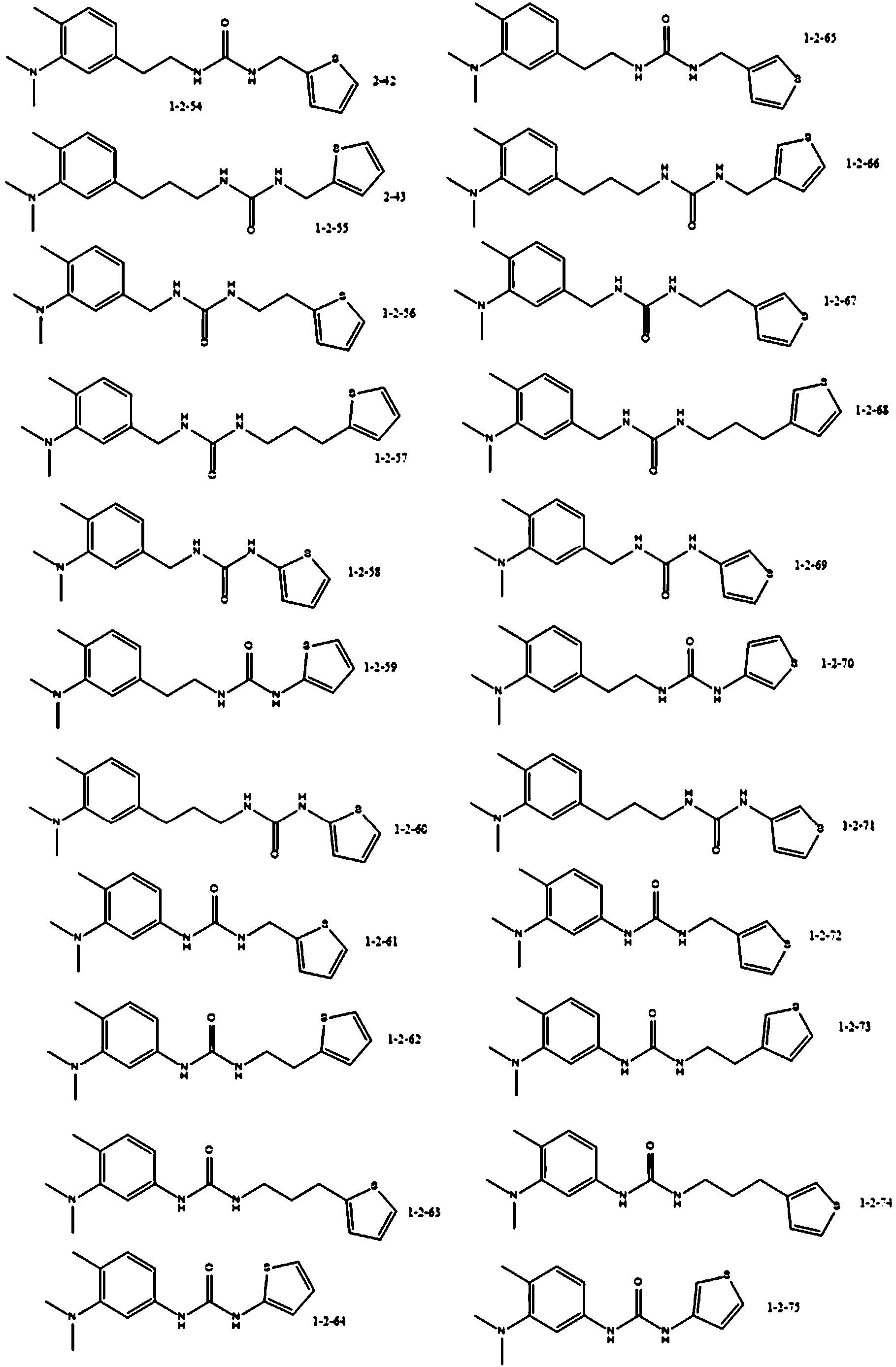

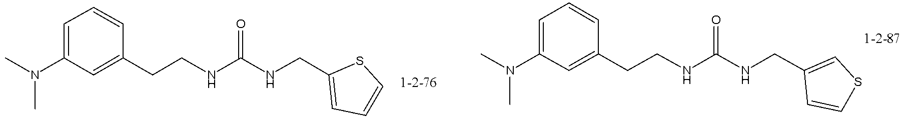

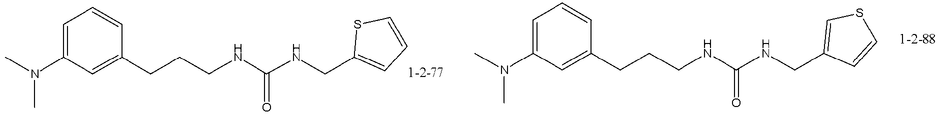

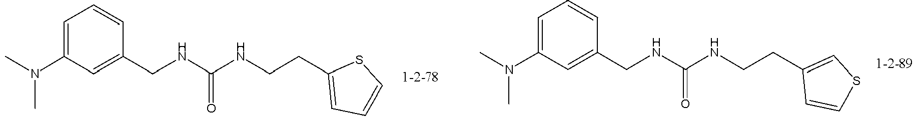

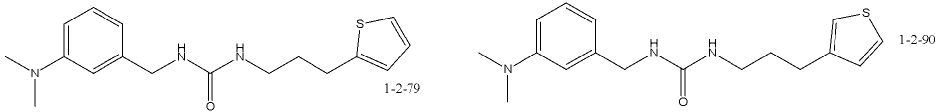

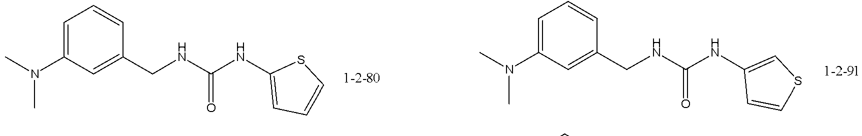

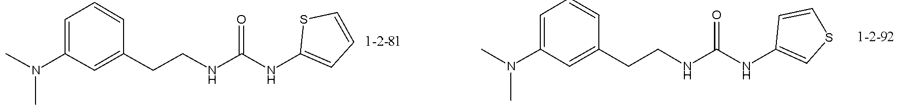

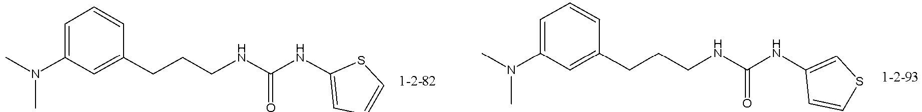

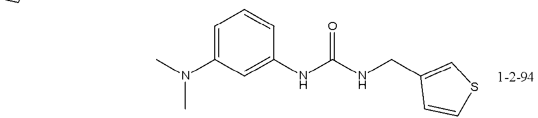

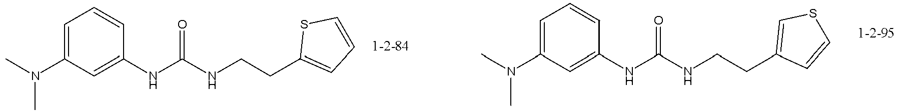

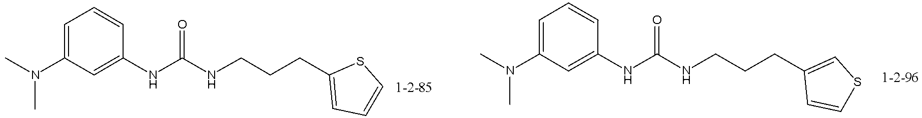

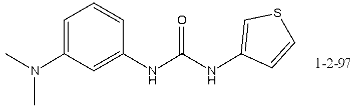

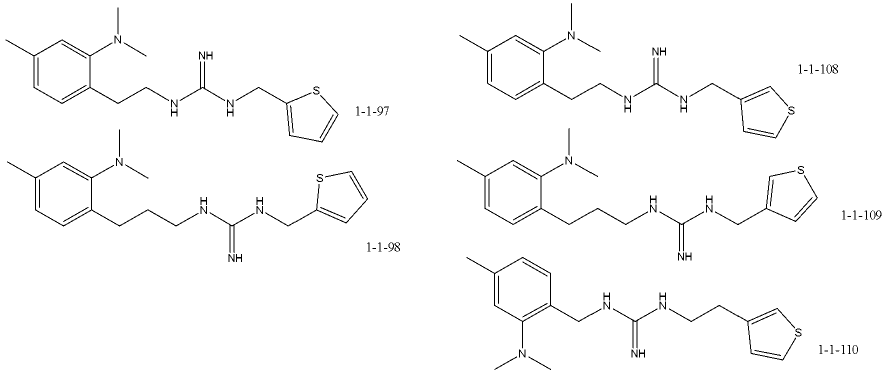

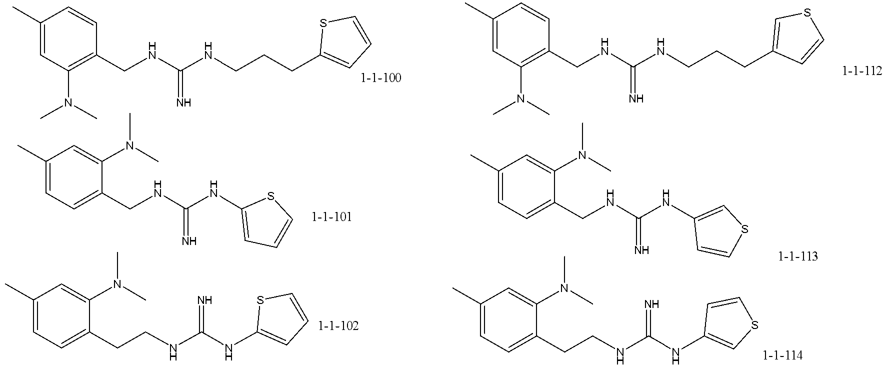

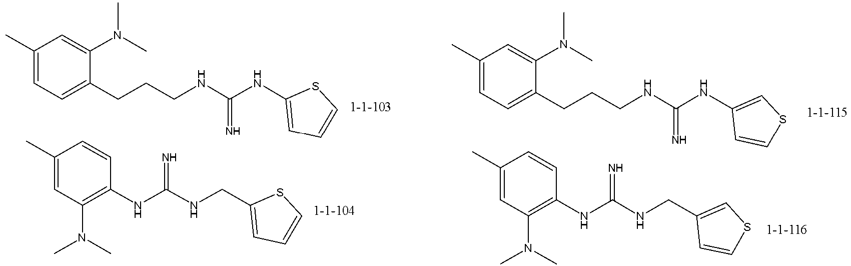

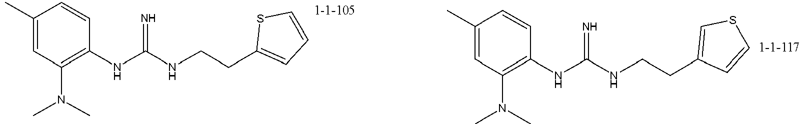

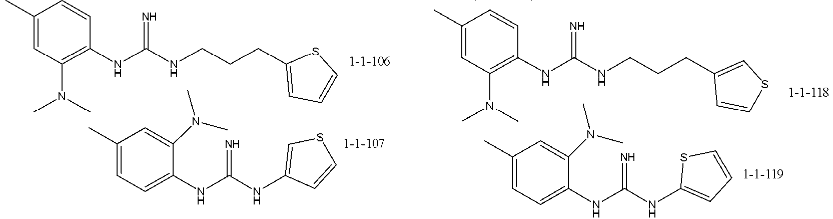

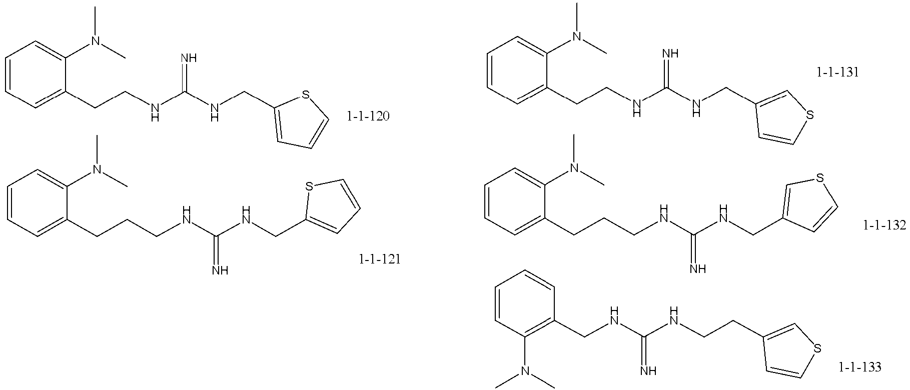

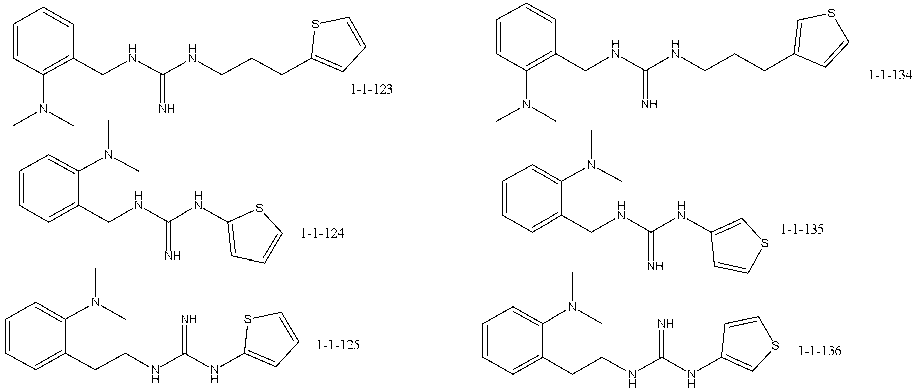

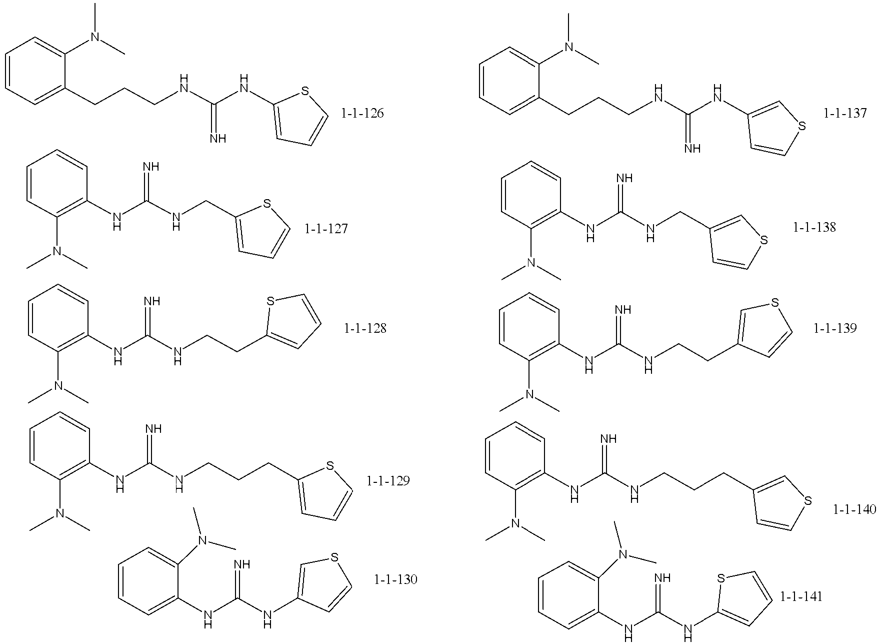

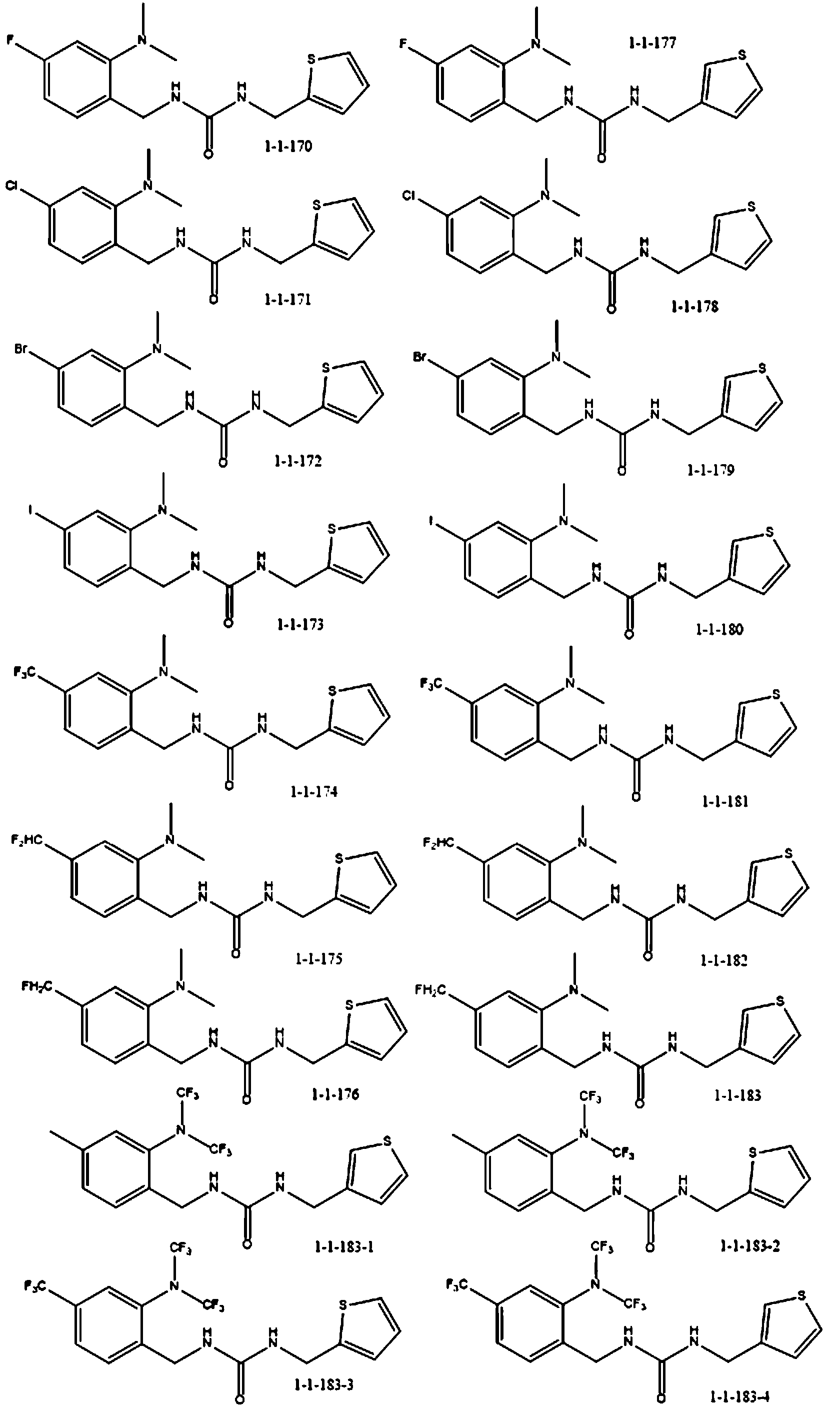

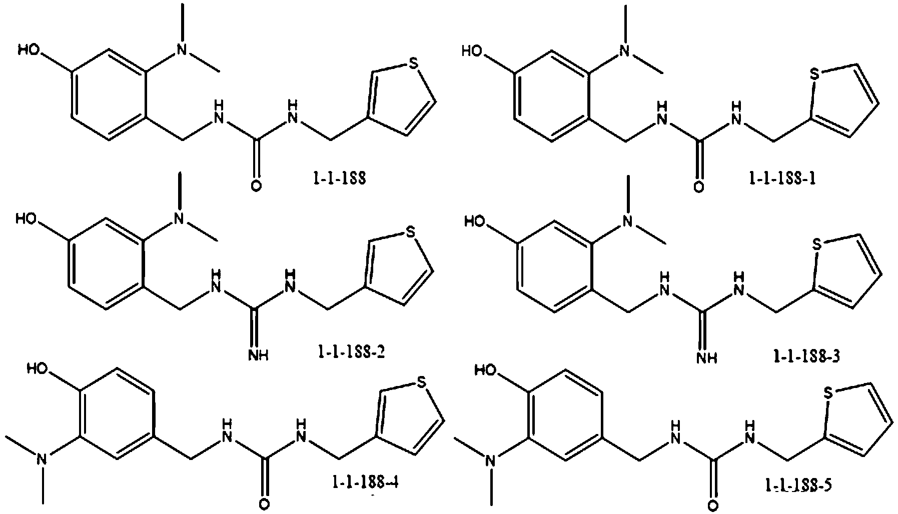

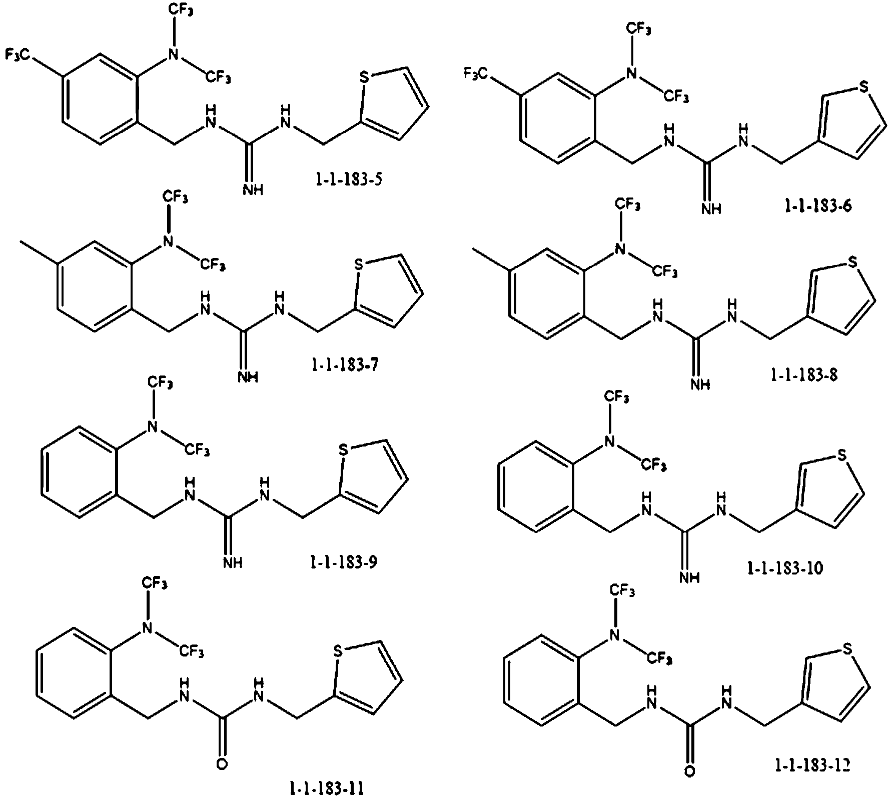

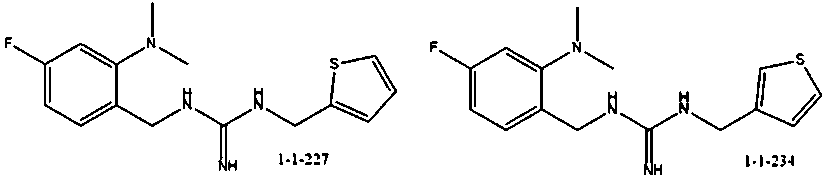

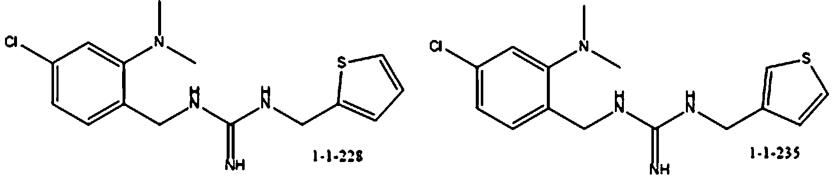

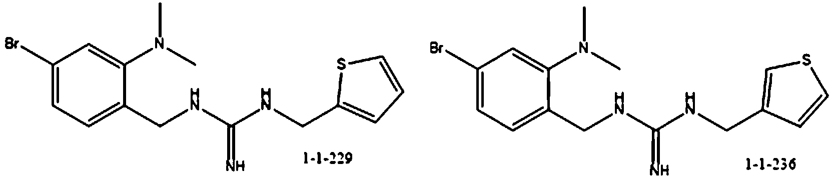

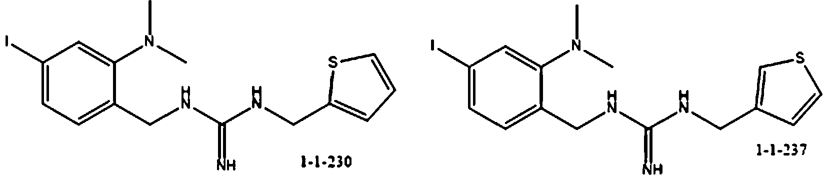

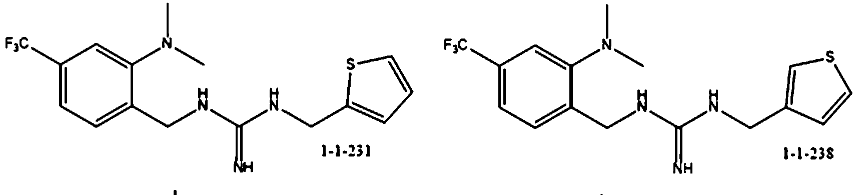

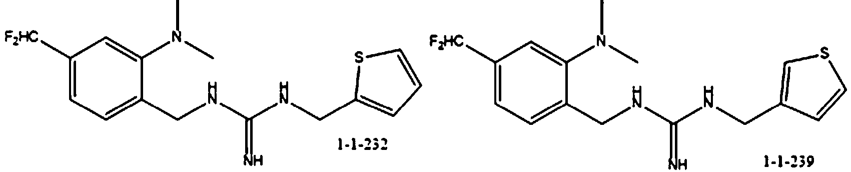

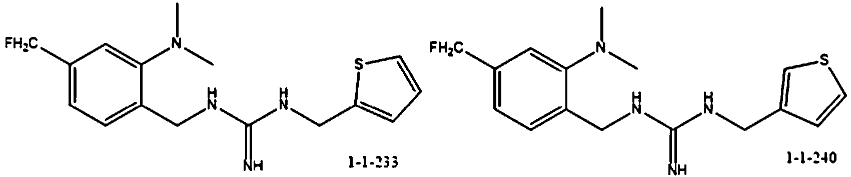

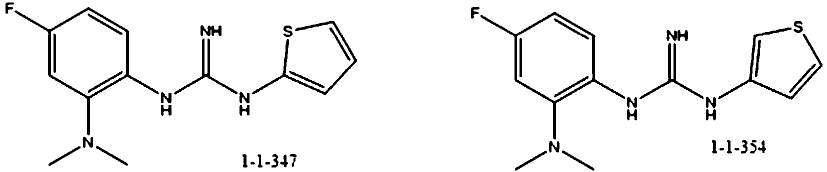

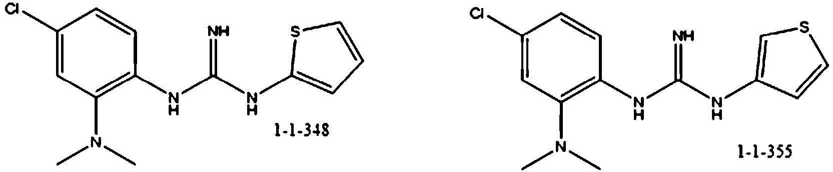

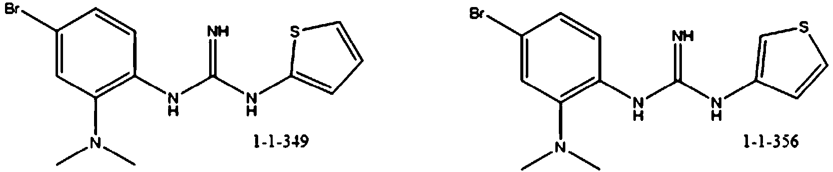

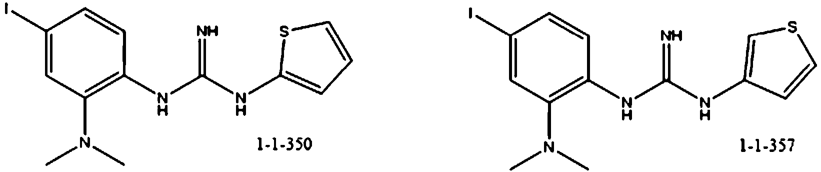

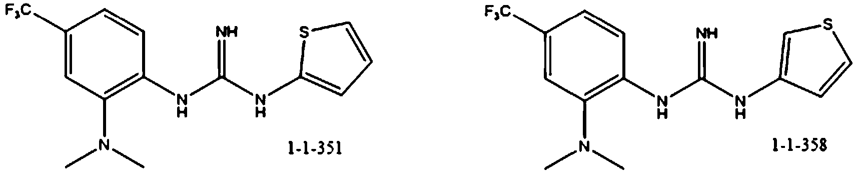

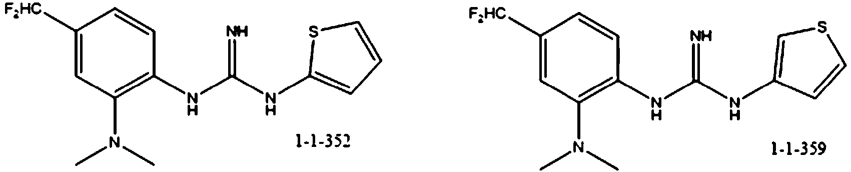

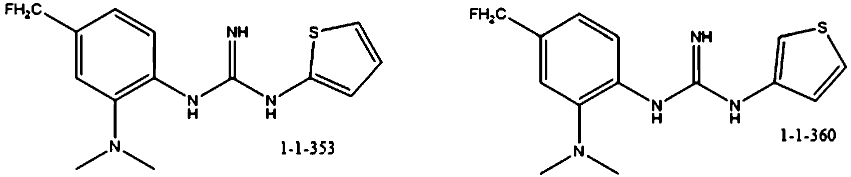

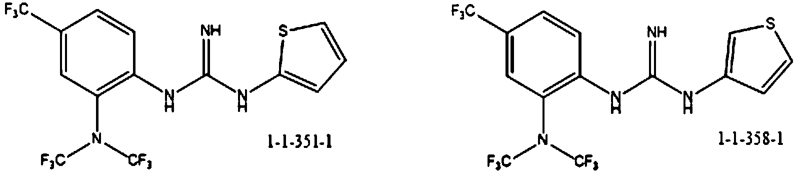

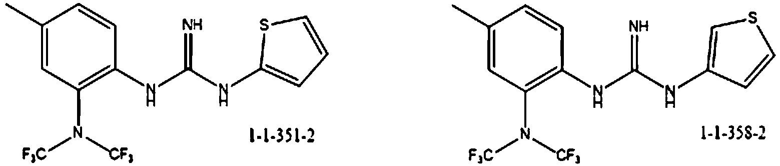

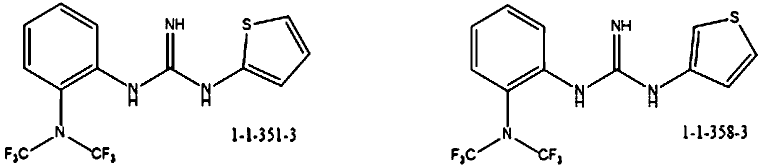

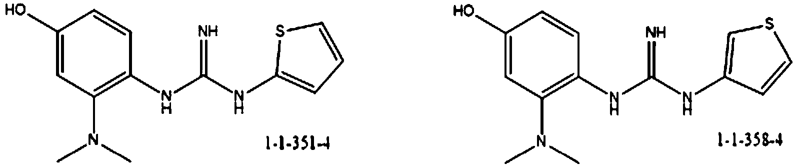

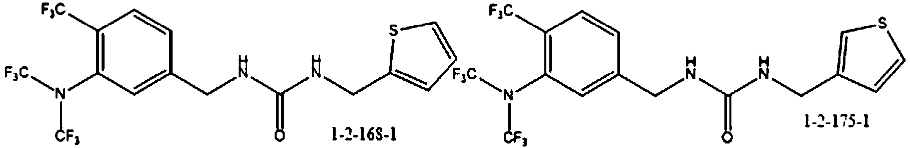

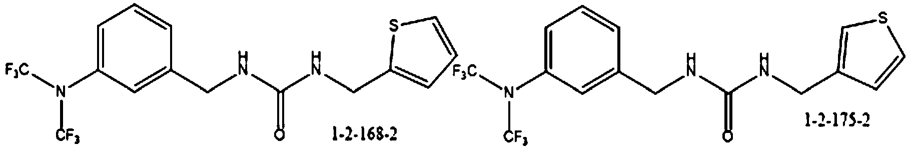

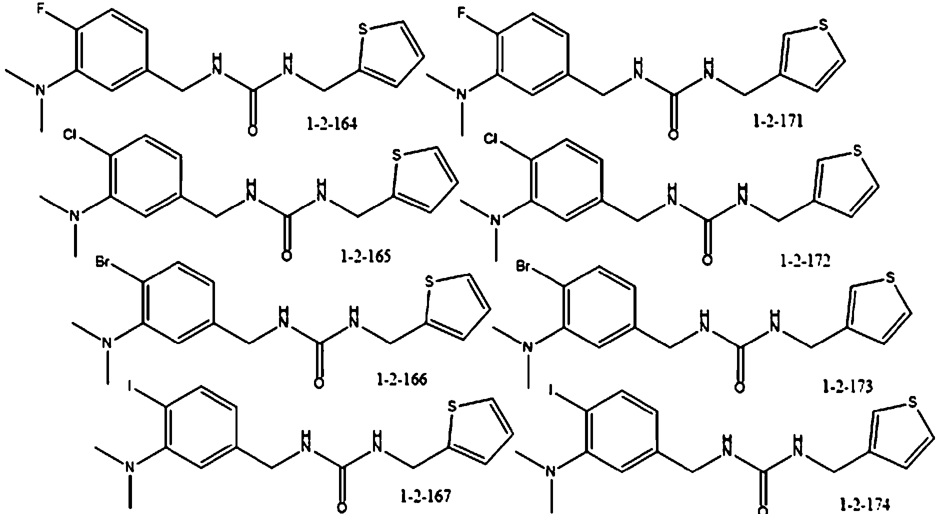

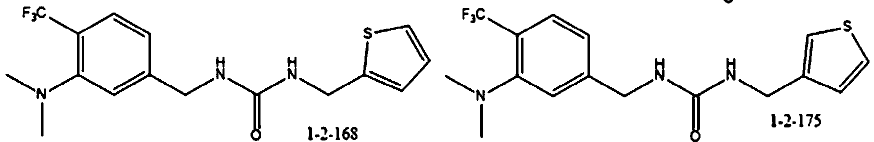

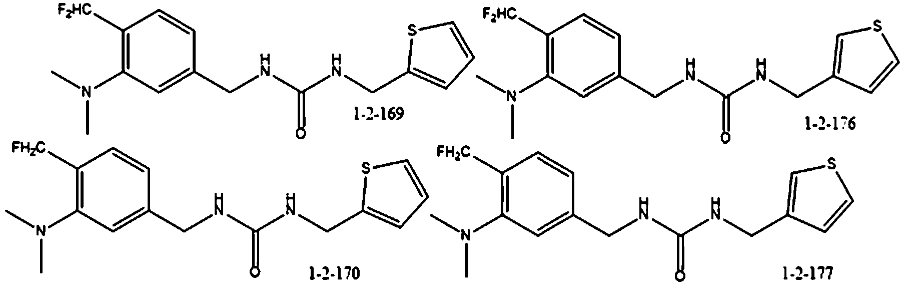

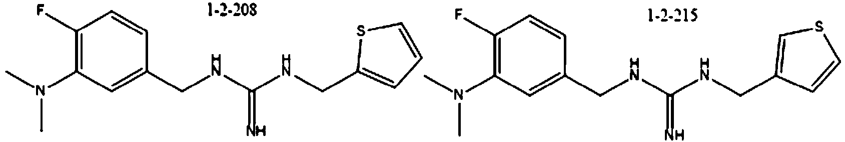

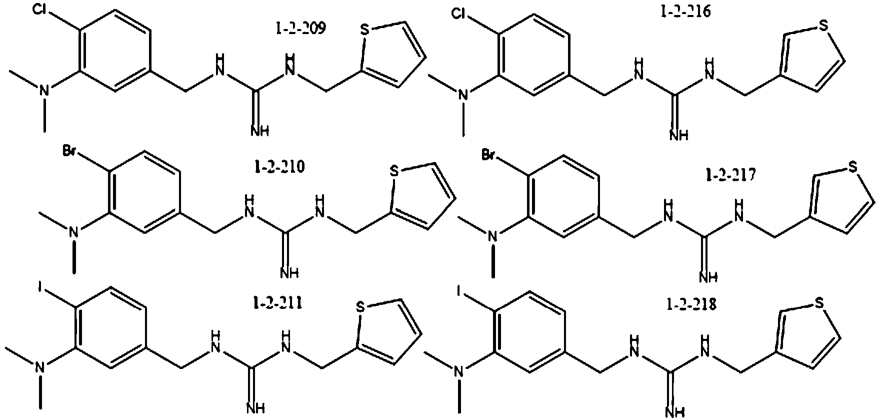

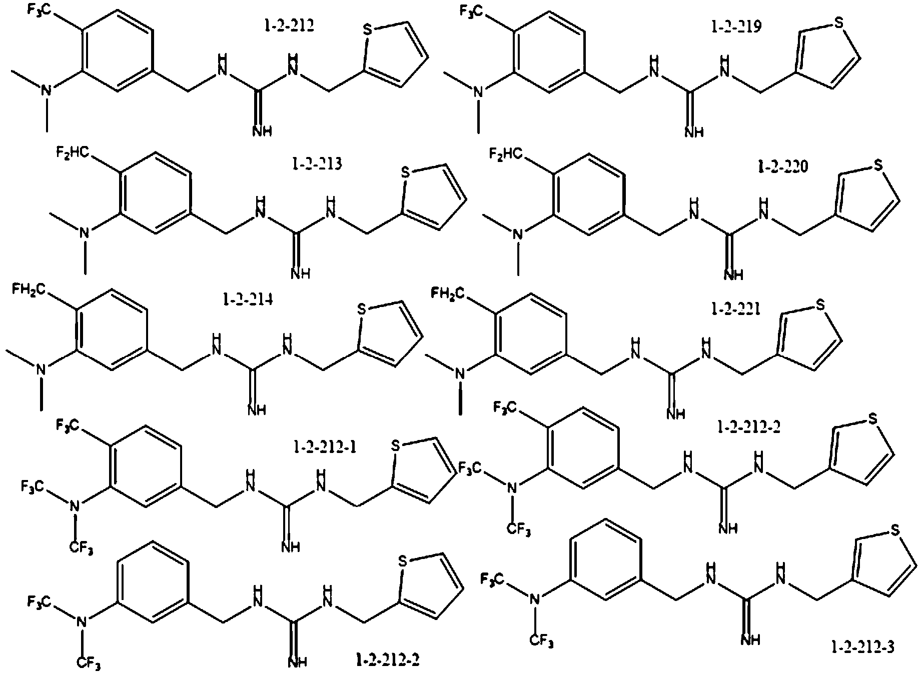

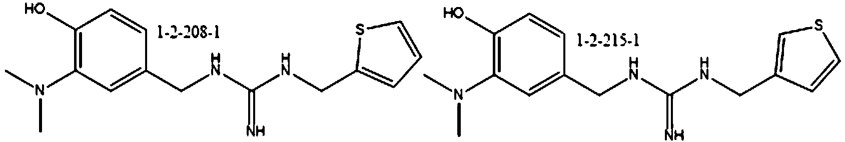

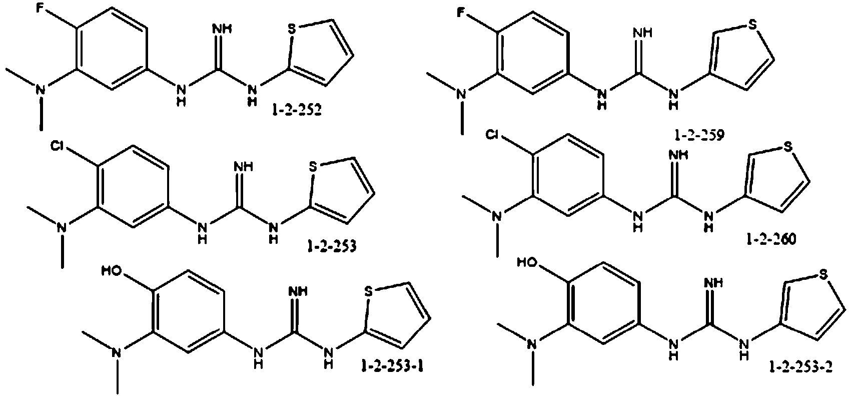

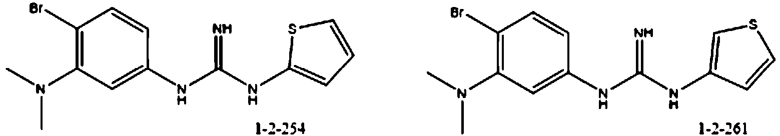

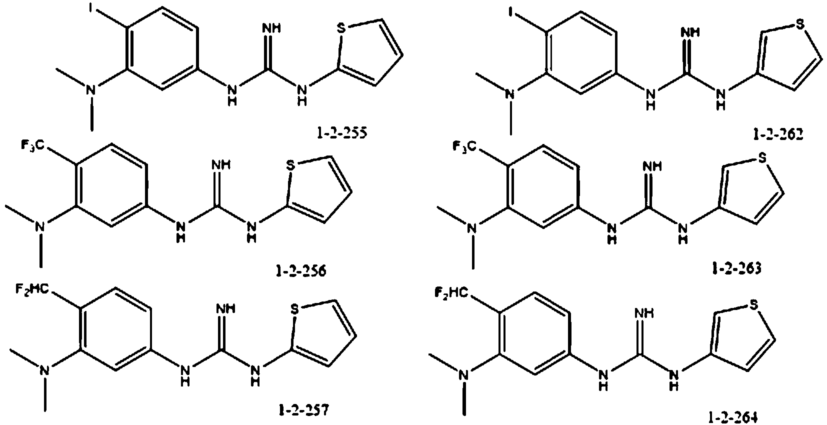

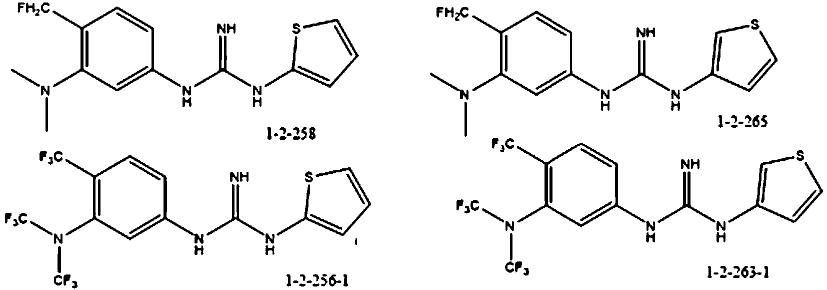

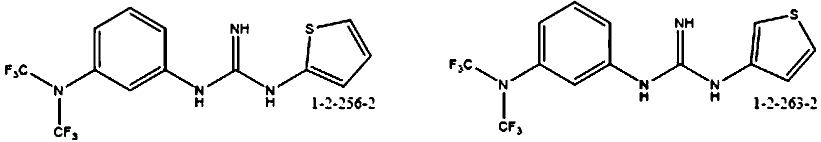

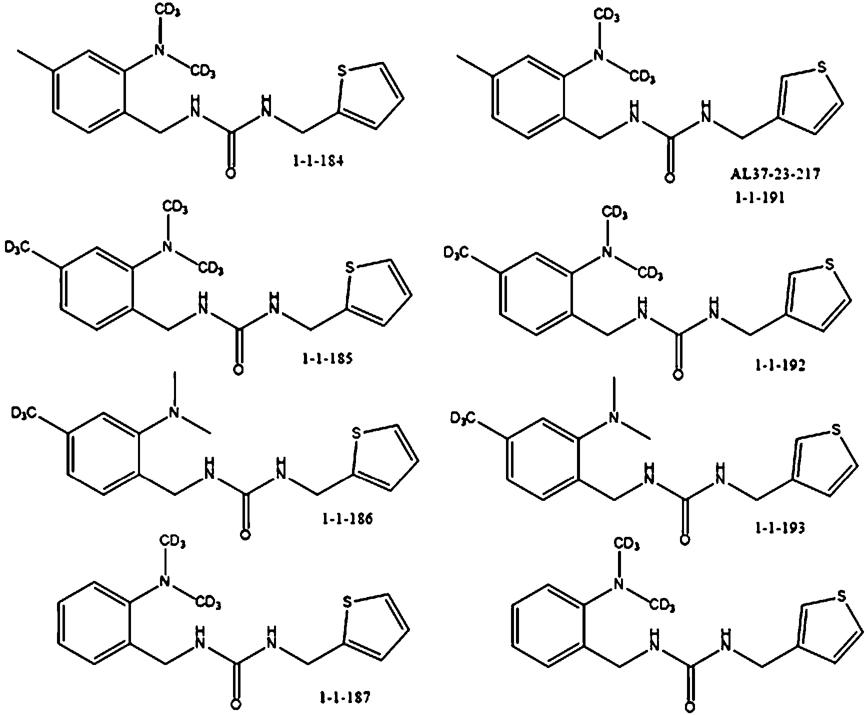

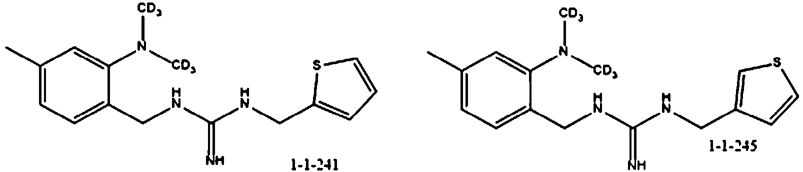

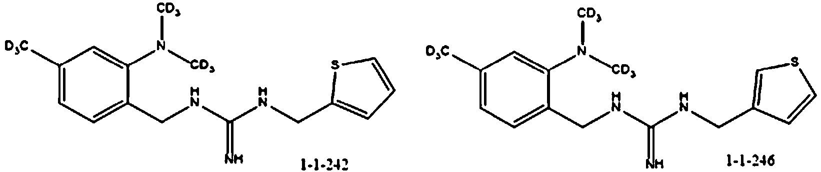

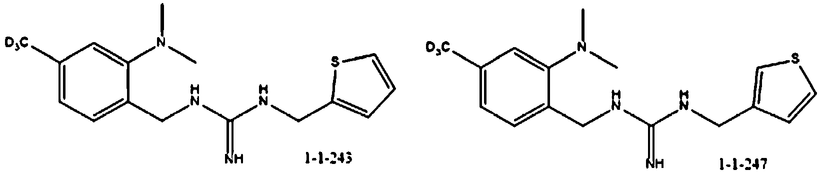

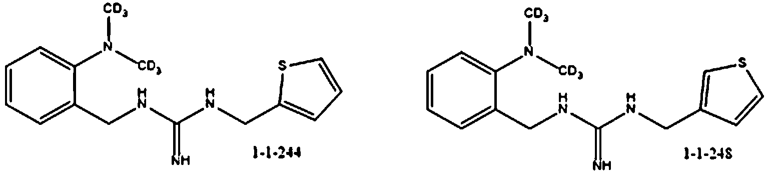

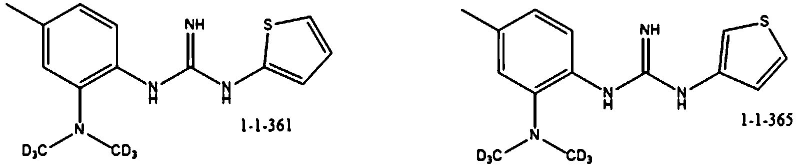

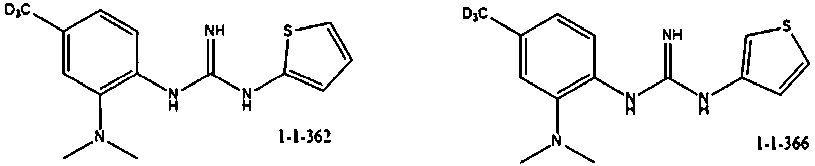

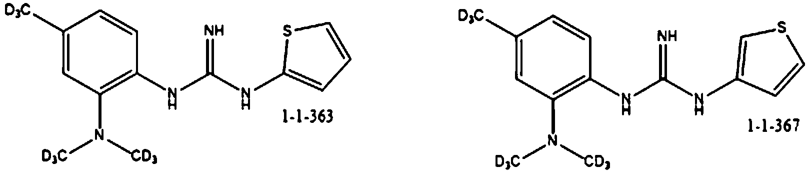

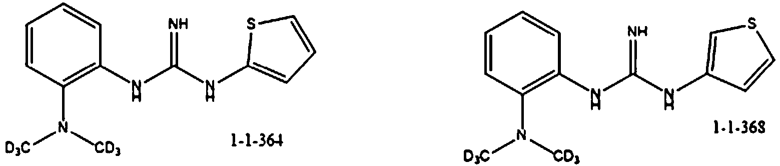

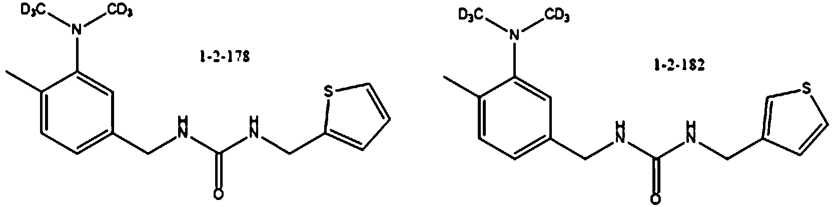

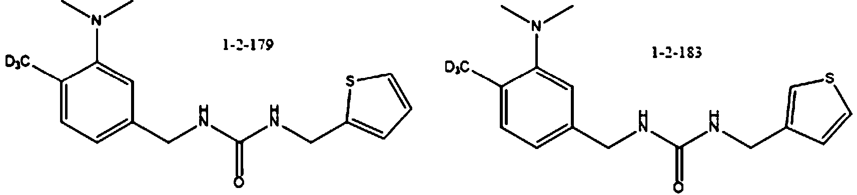

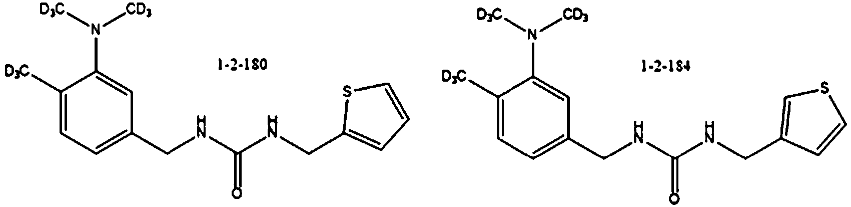

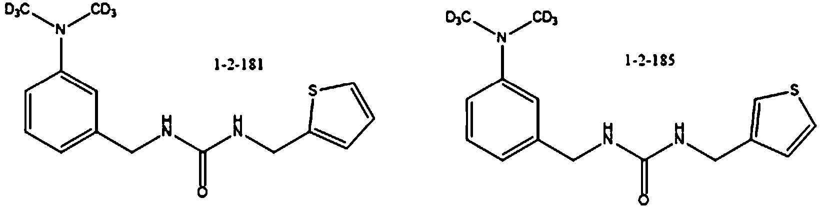

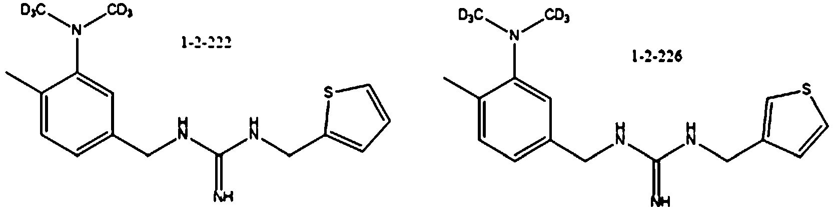

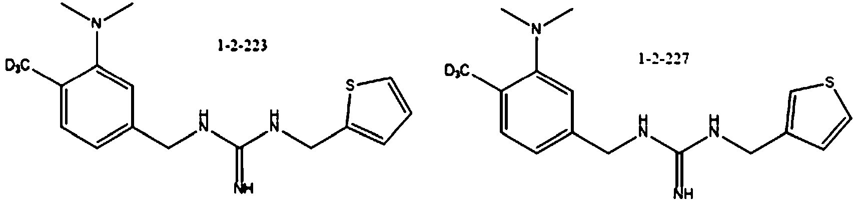

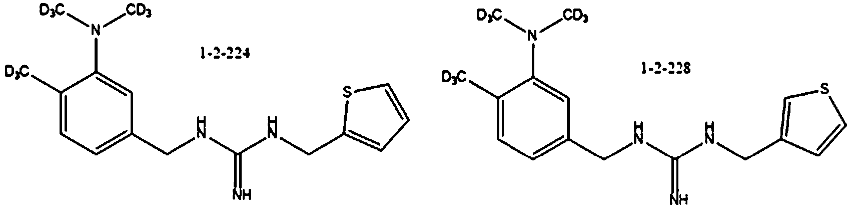

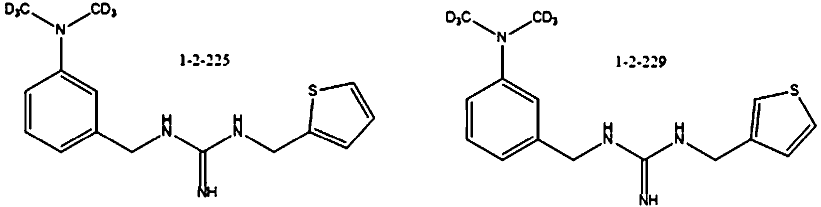

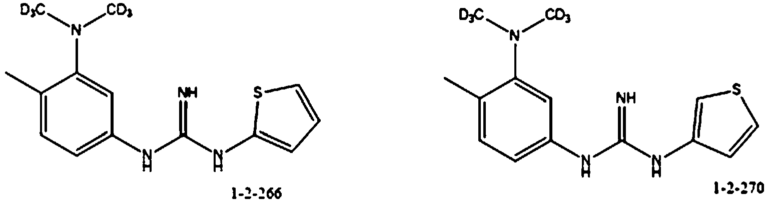

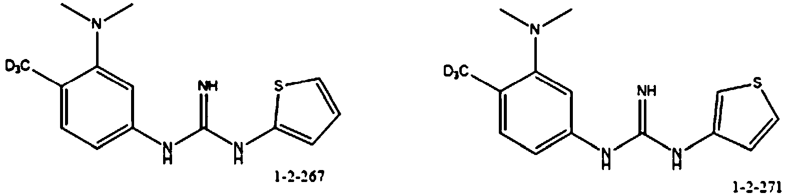

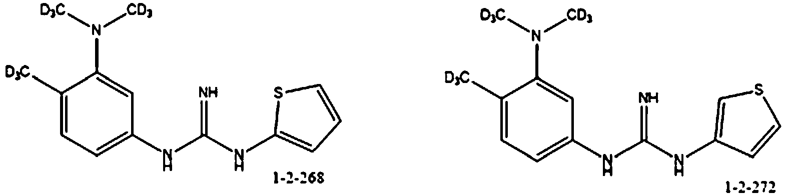

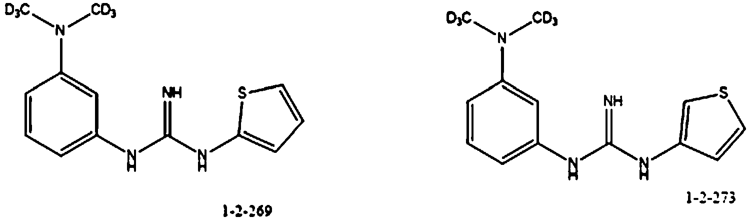

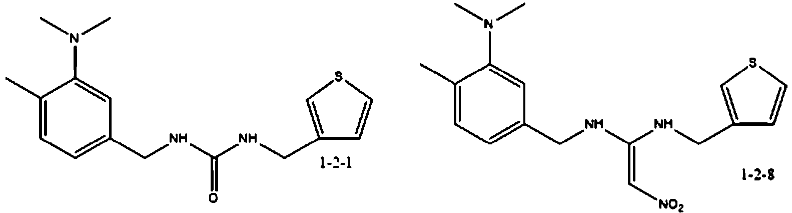

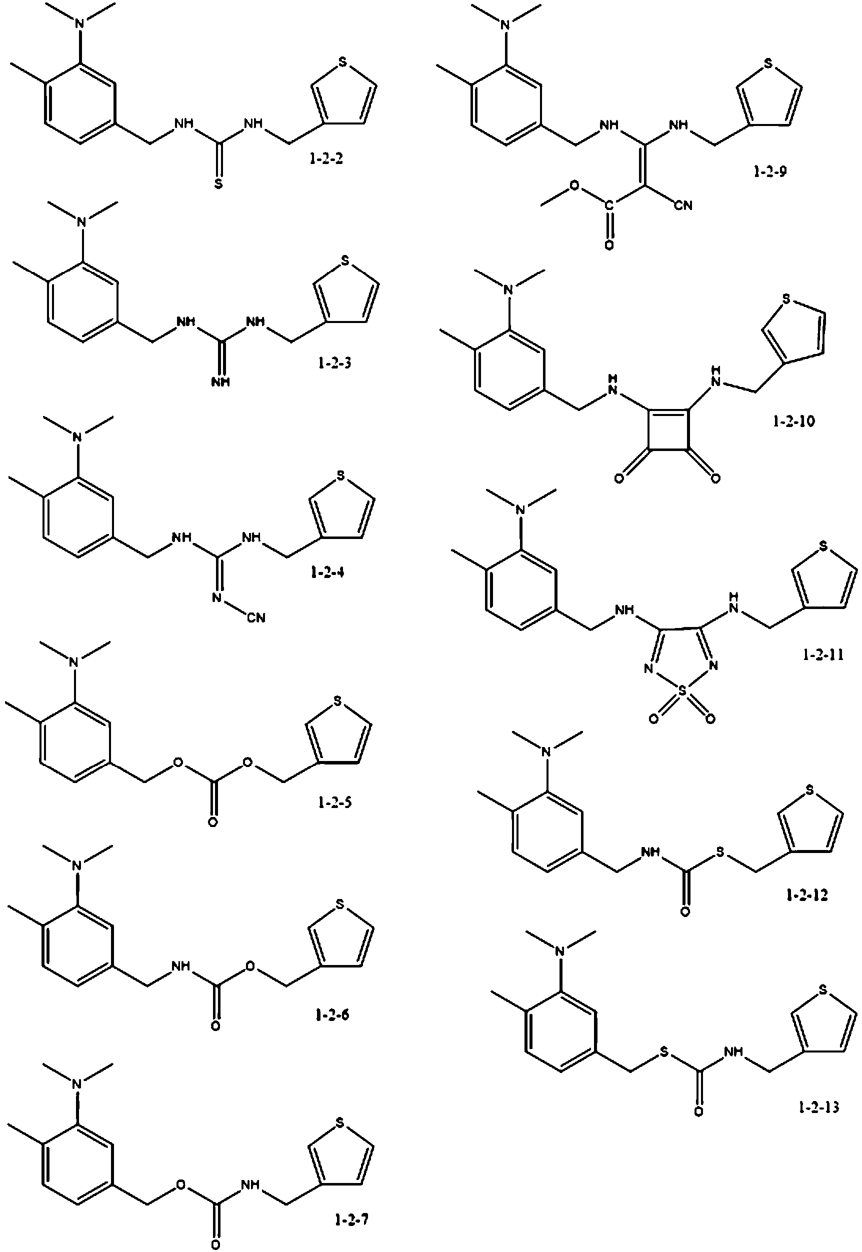

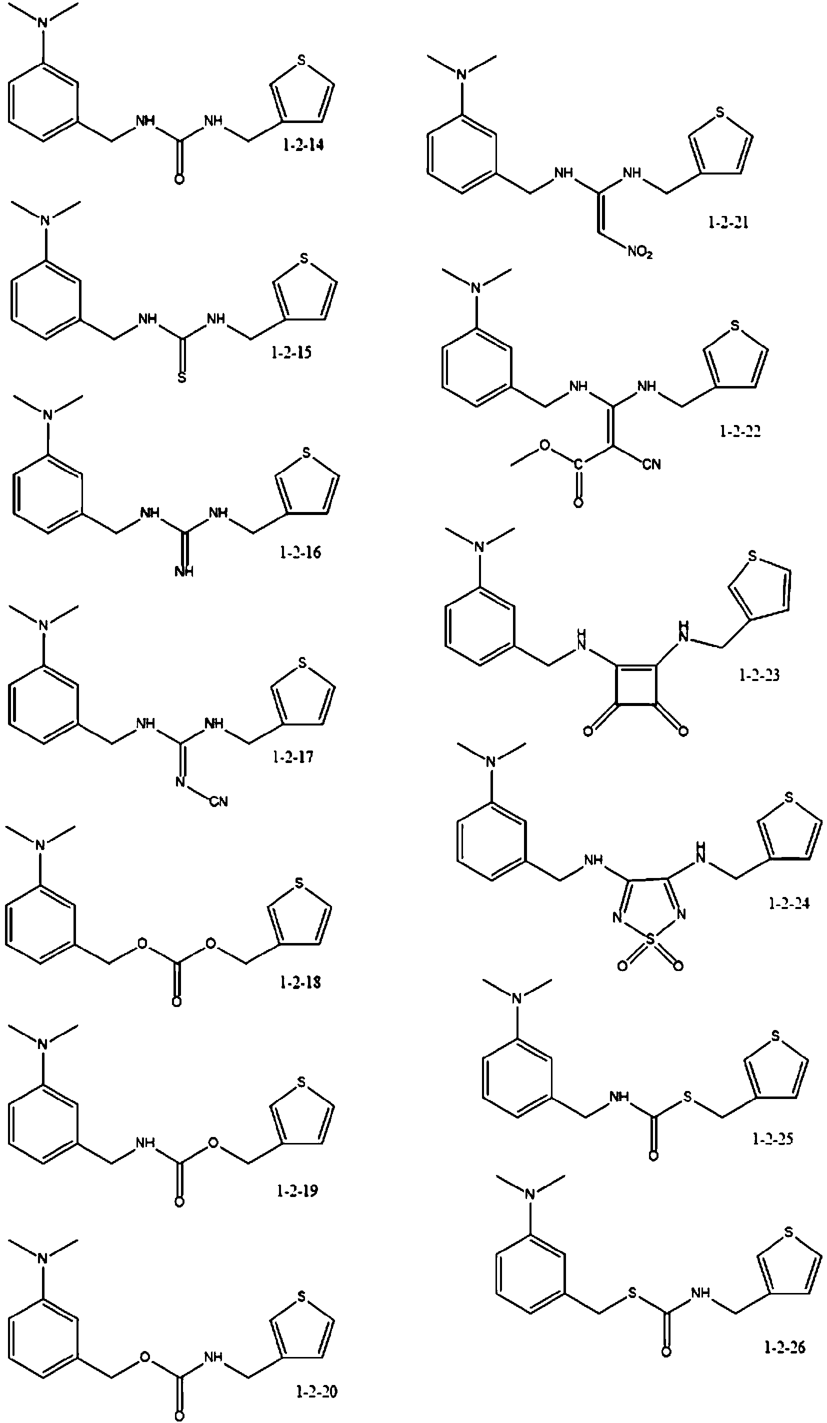

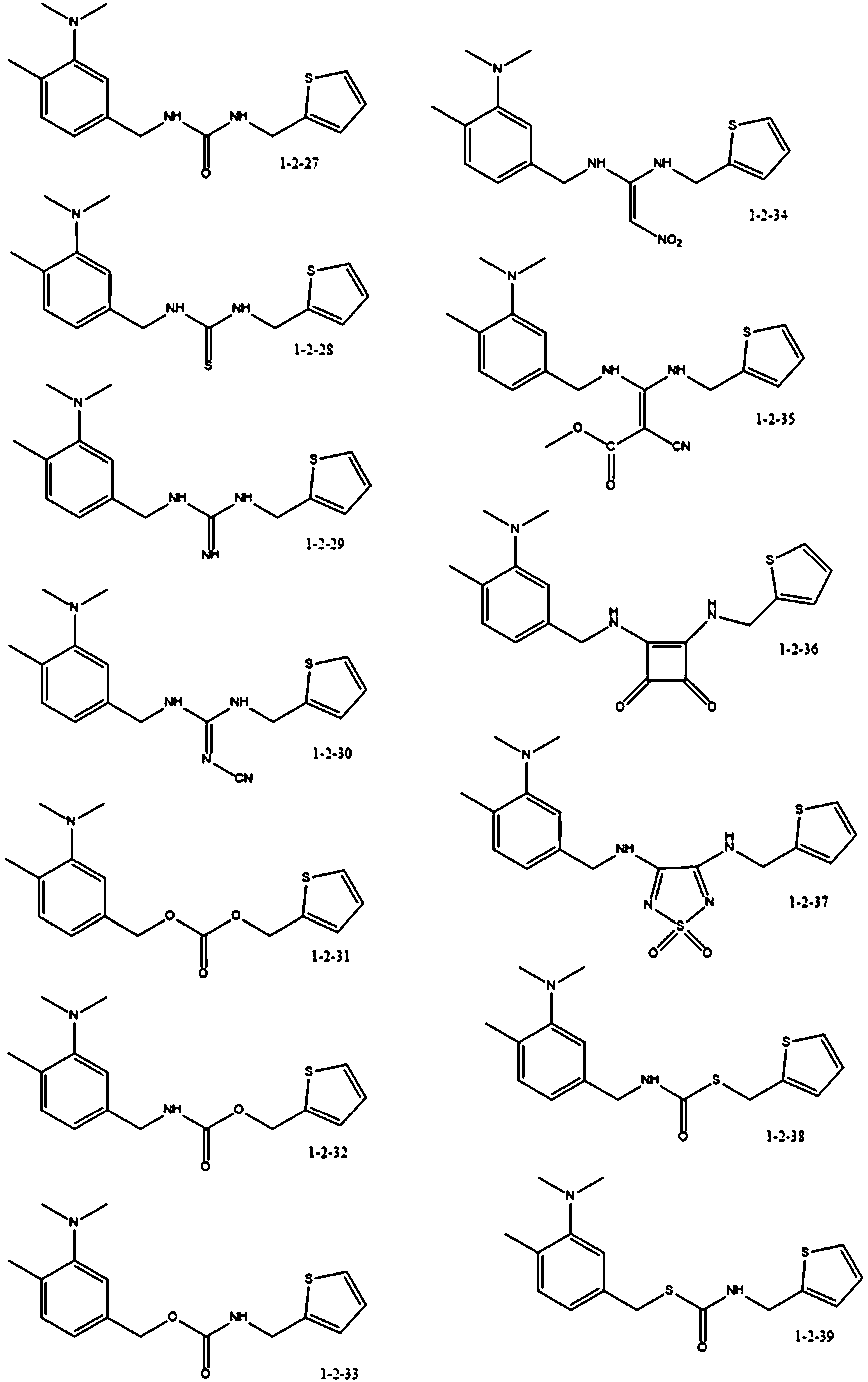

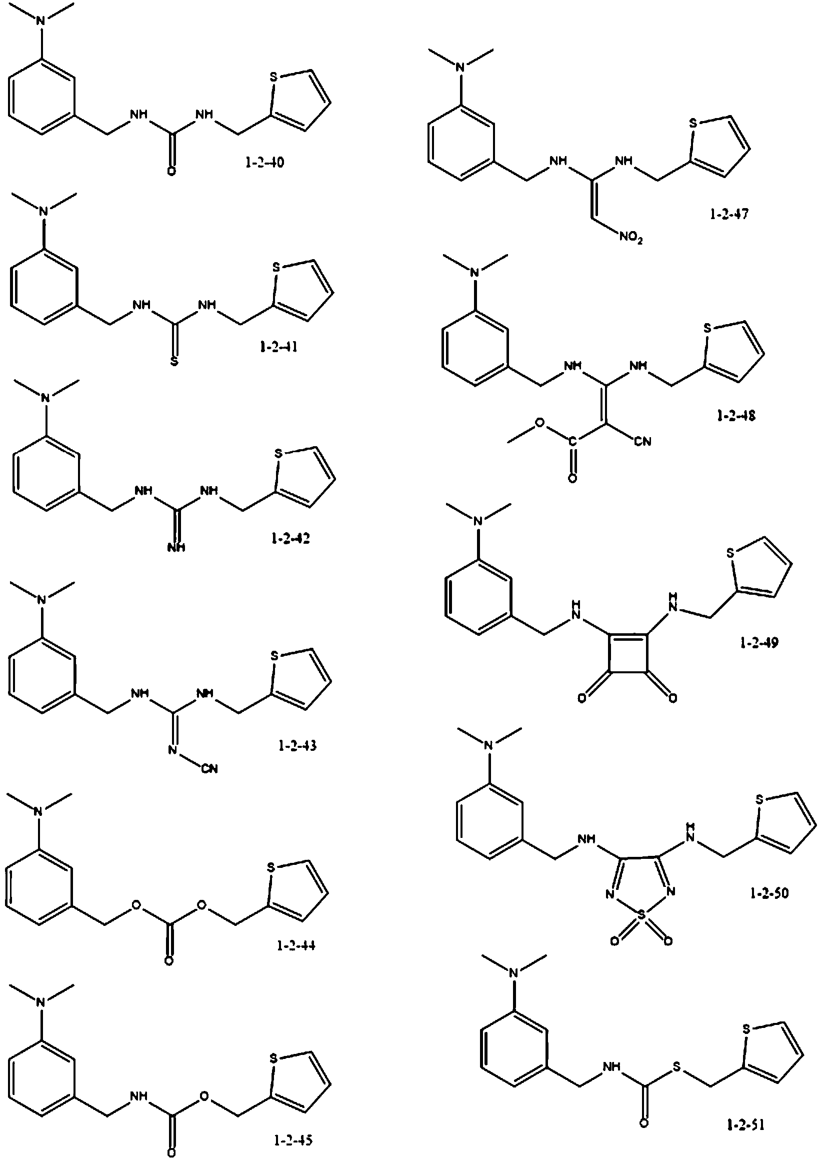

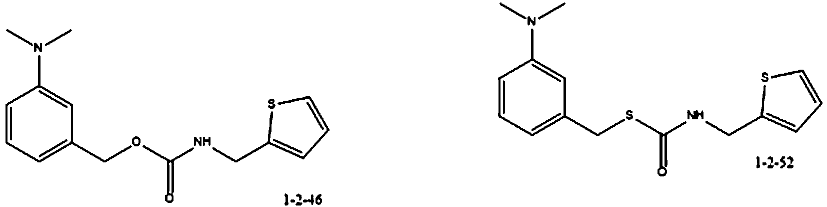

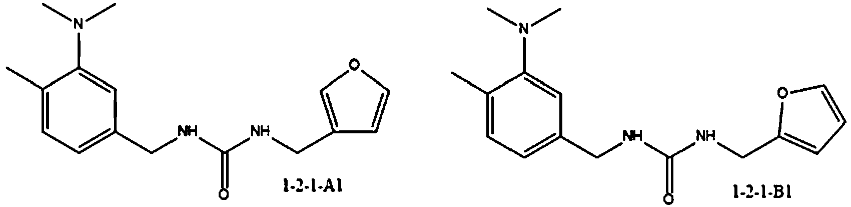

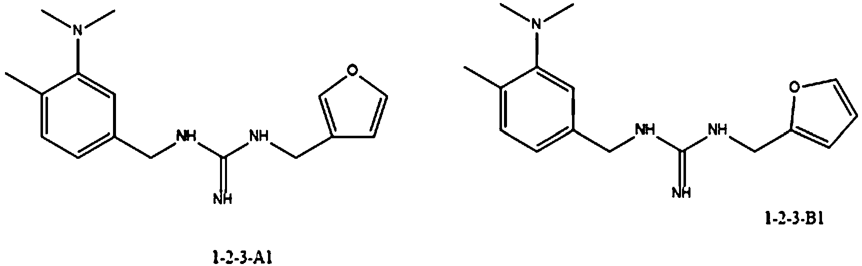

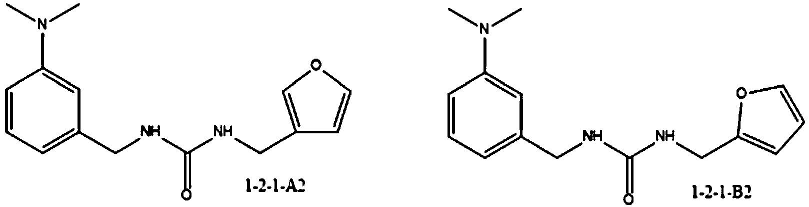

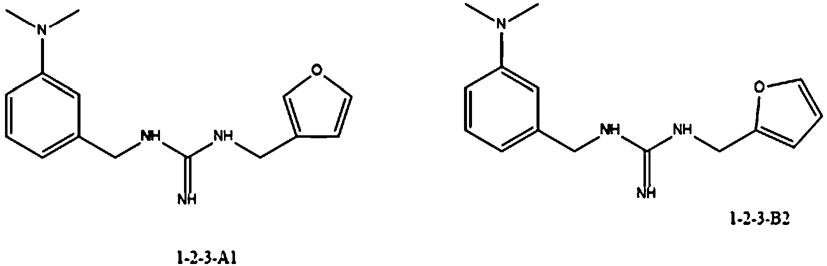

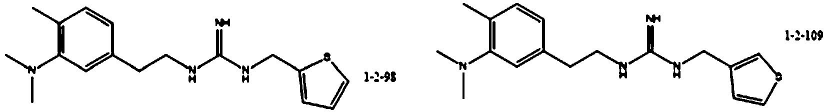

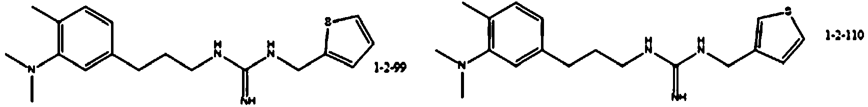

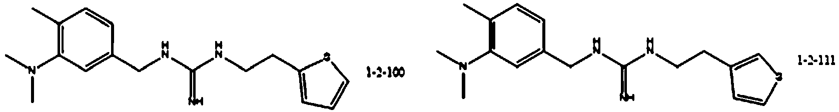

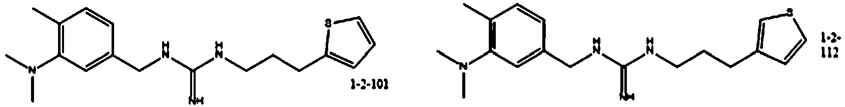

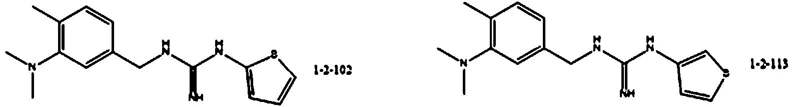

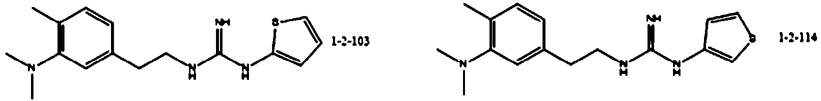

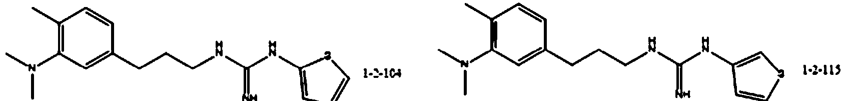

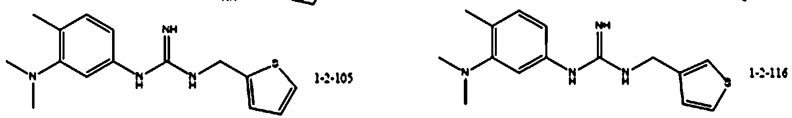

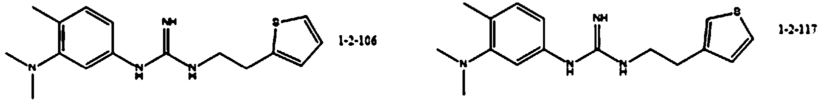

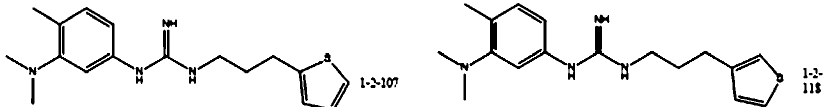

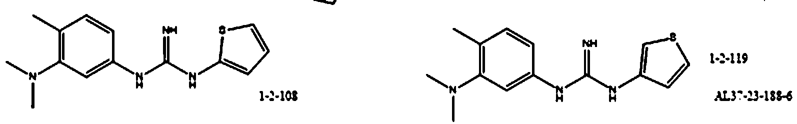

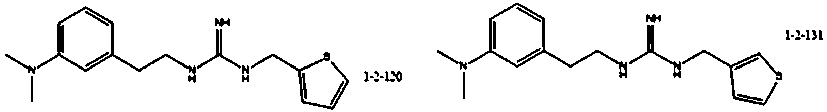

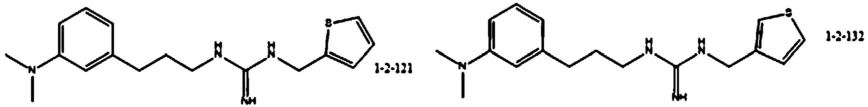

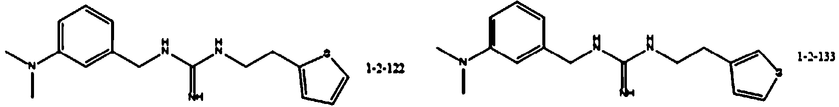

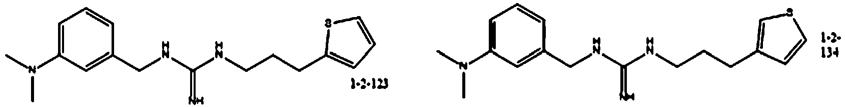

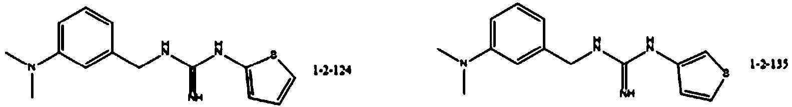

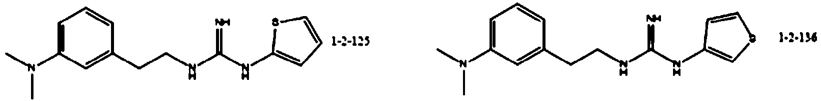

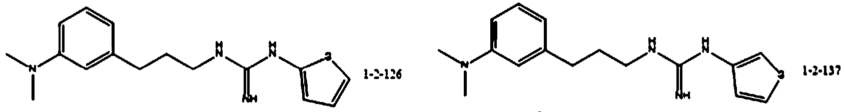

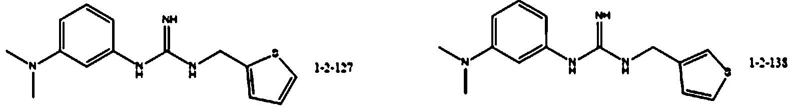

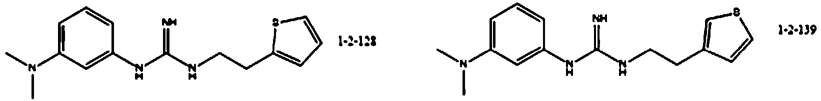

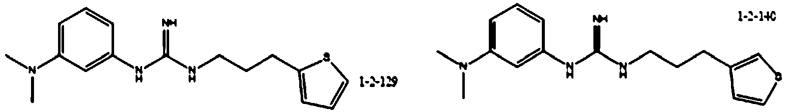

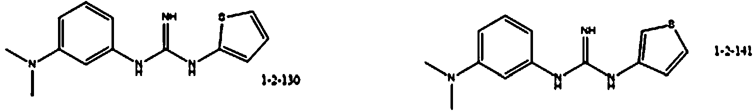

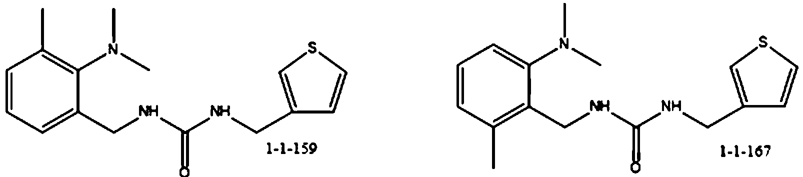

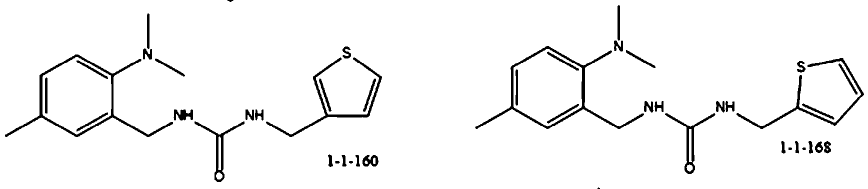

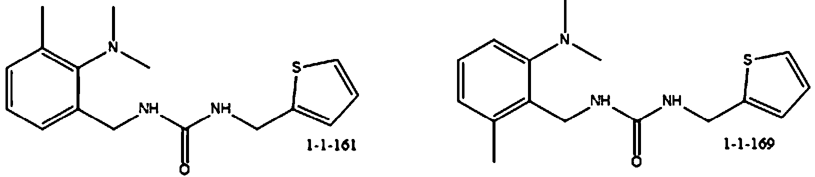

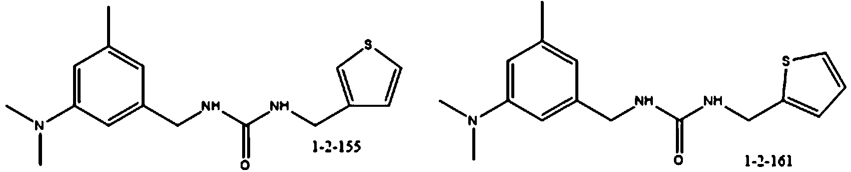

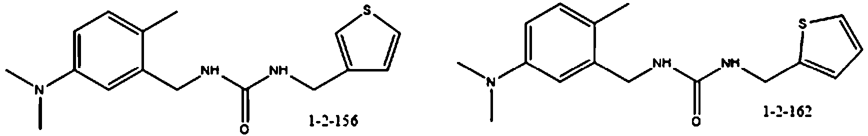

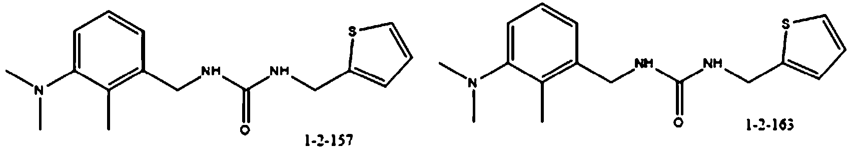

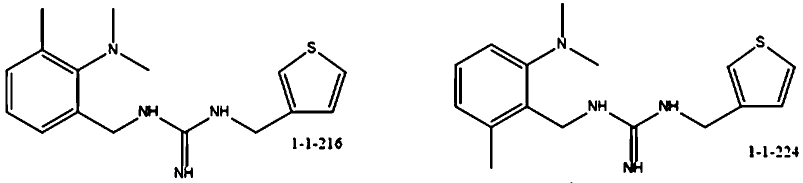

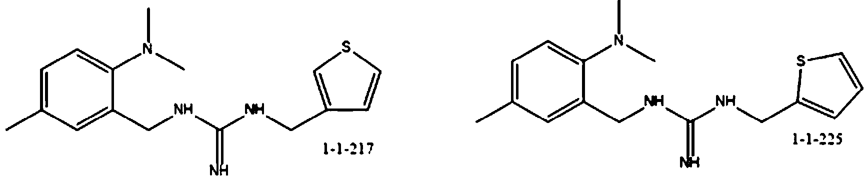

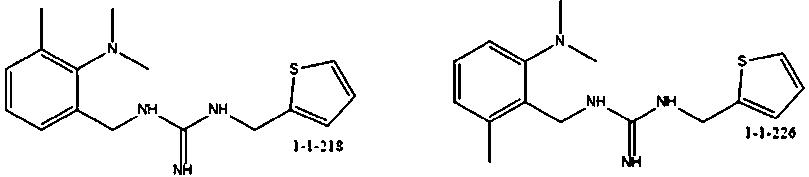

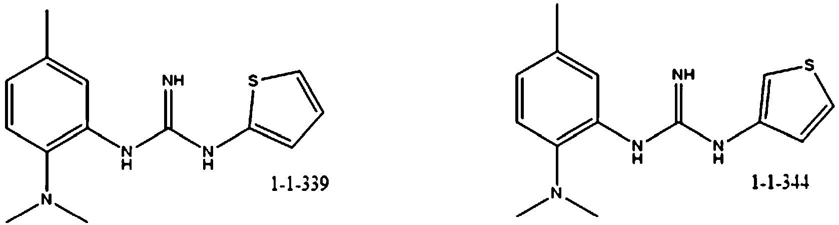

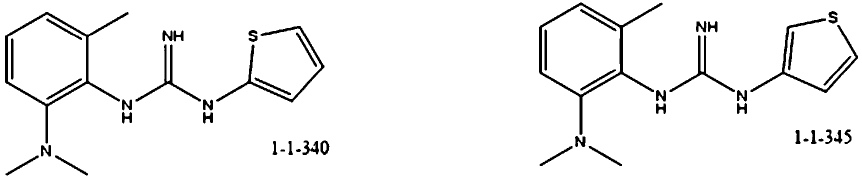

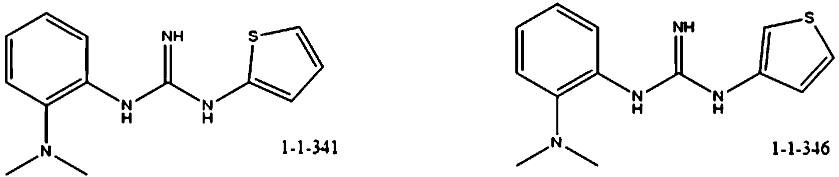

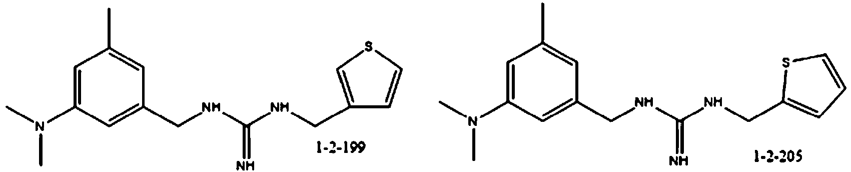

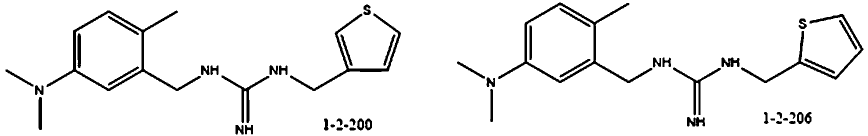

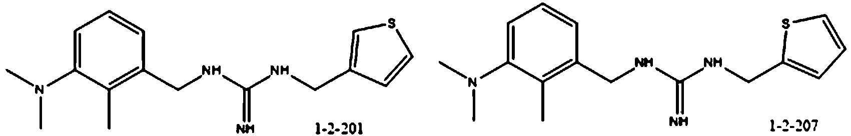

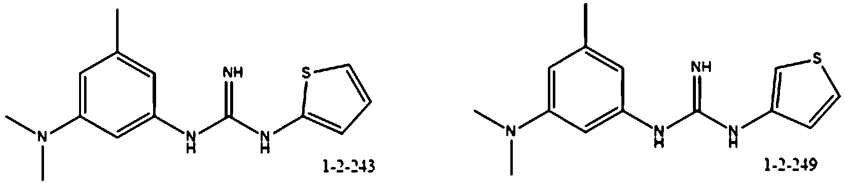

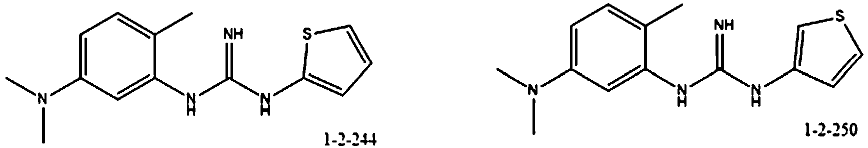

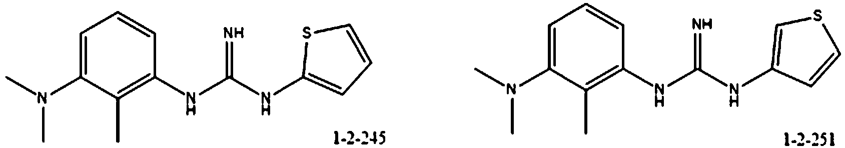

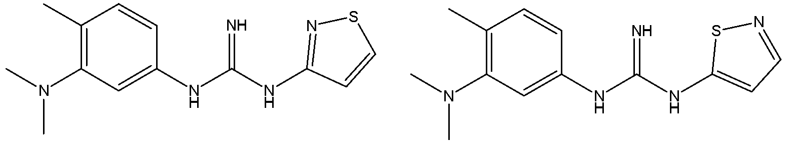

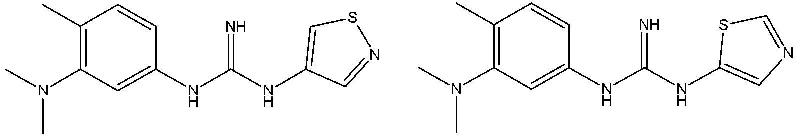

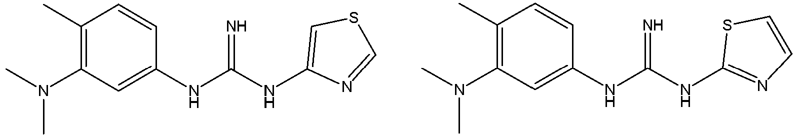

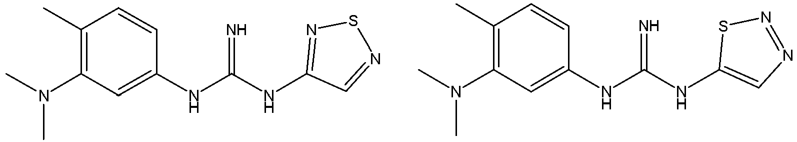

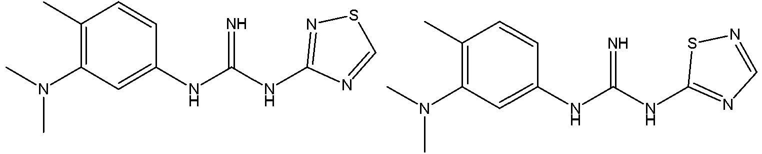

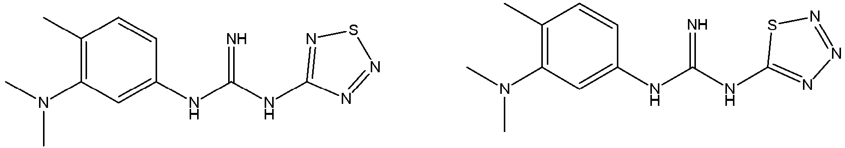

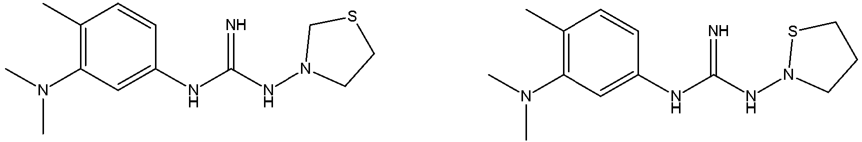

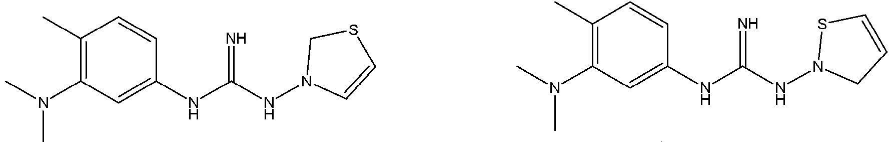

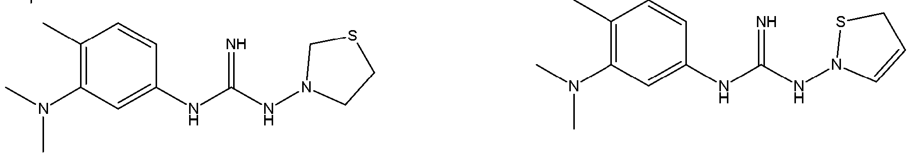

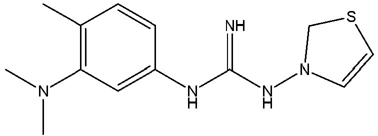

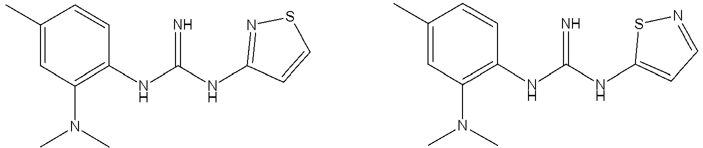

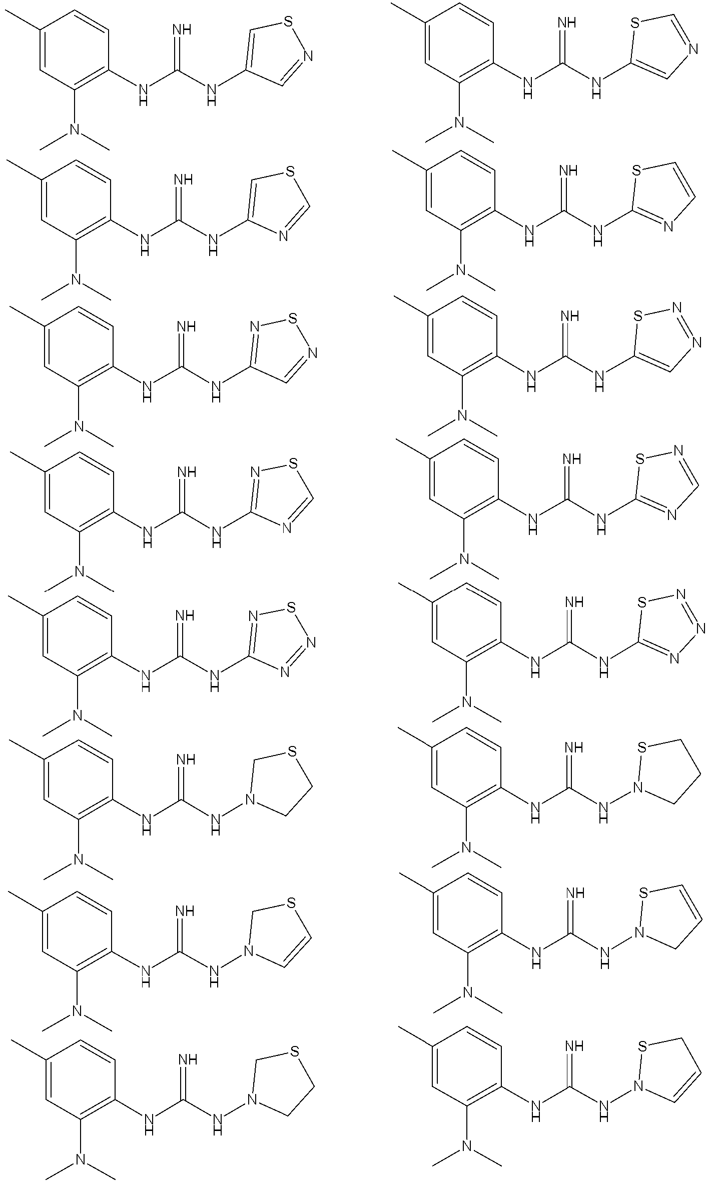

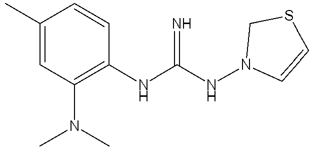

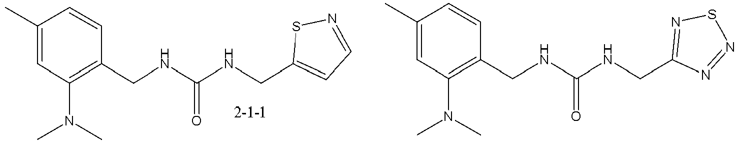

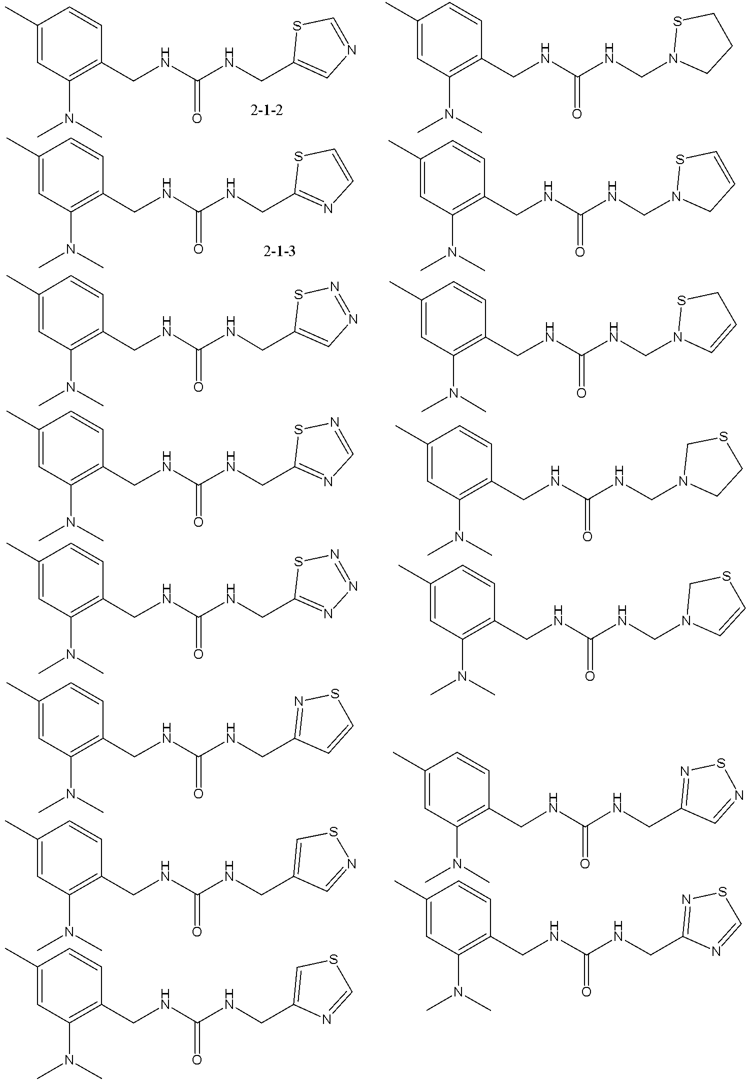

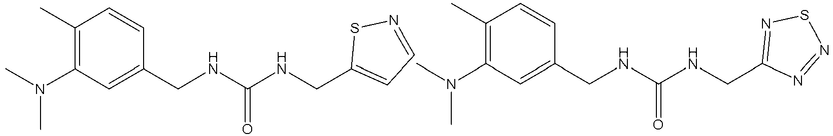

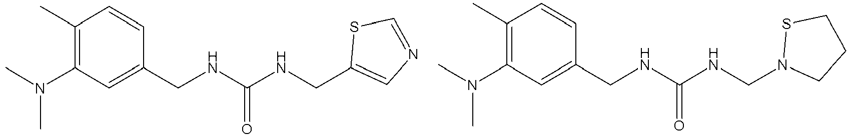

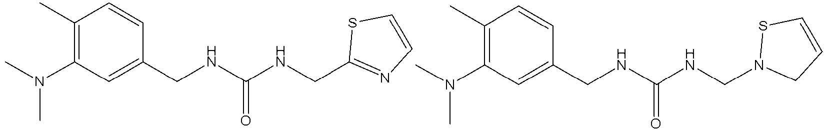

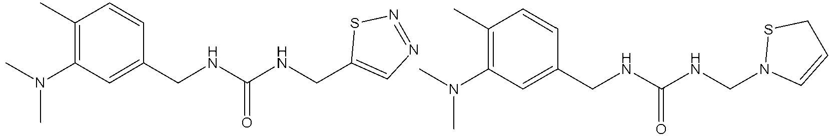

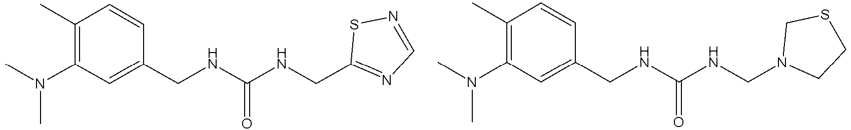

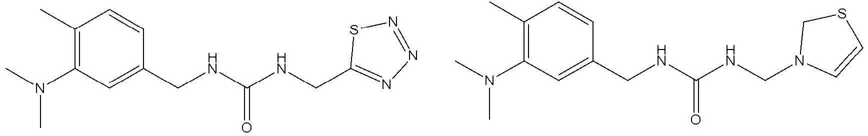

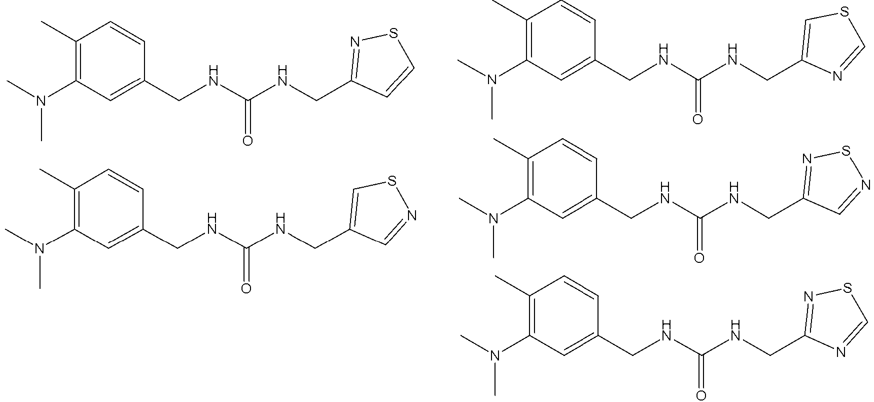

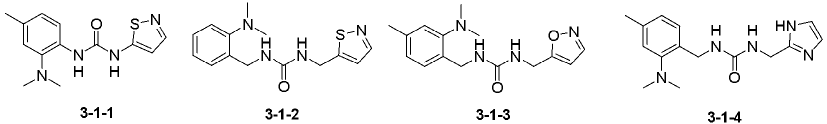

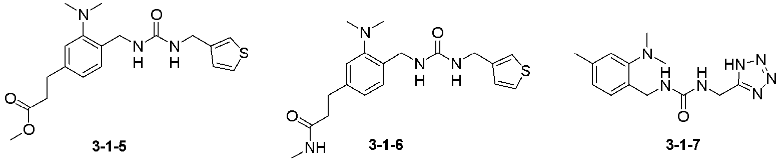

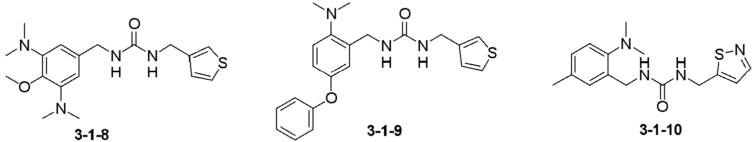

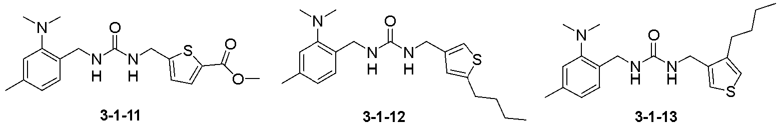

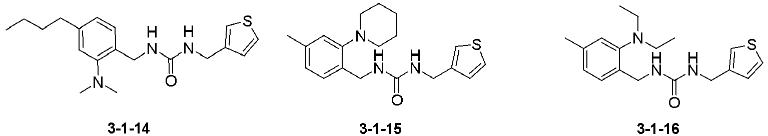

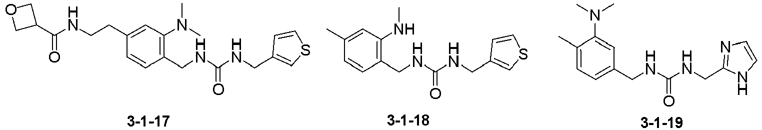

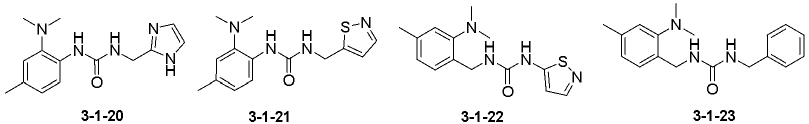

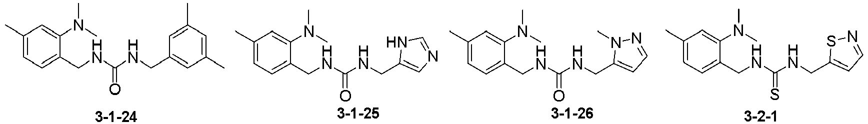

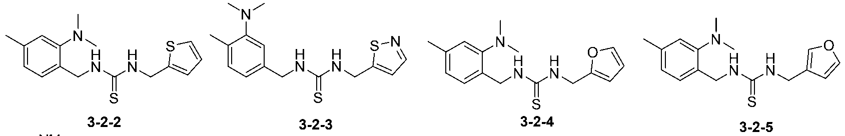

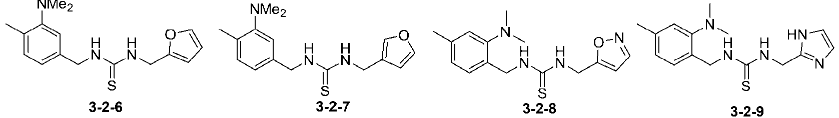

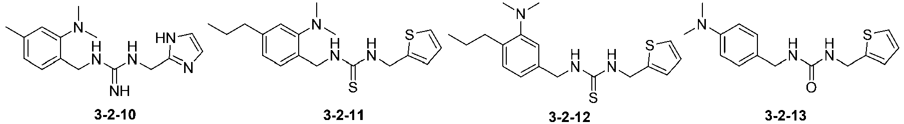

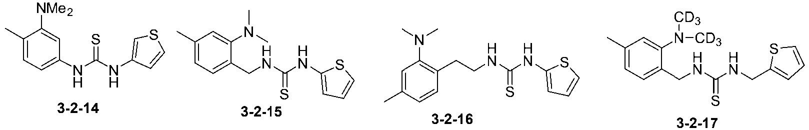

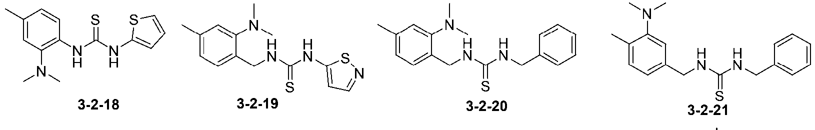

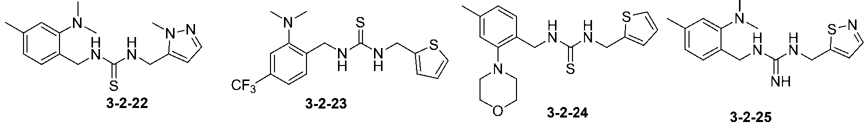

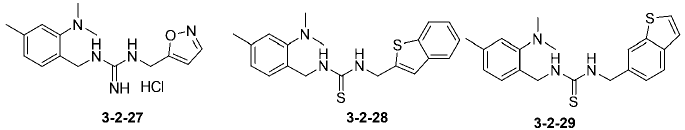

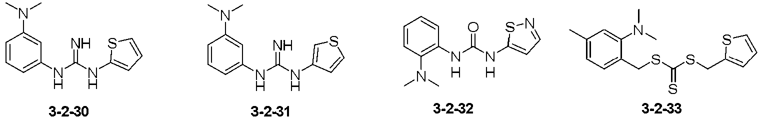

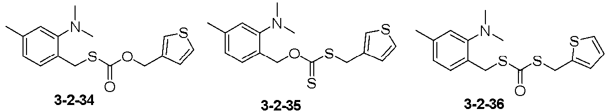

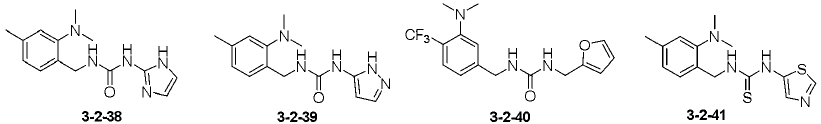

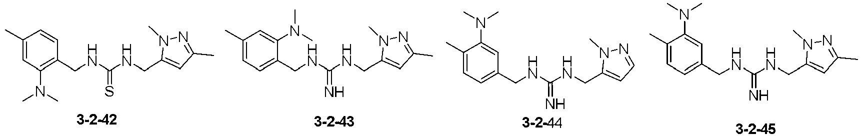

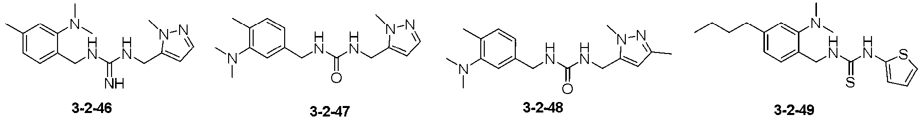

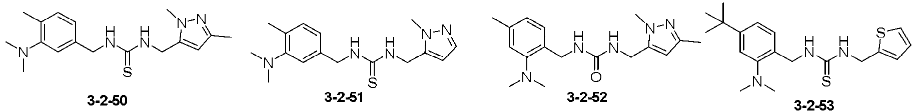

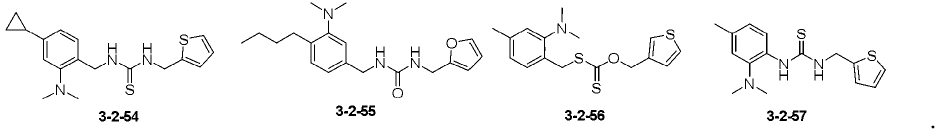

13. The method for preparation of the compound according to any one of claims 1-12, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** it comprises the following steps: 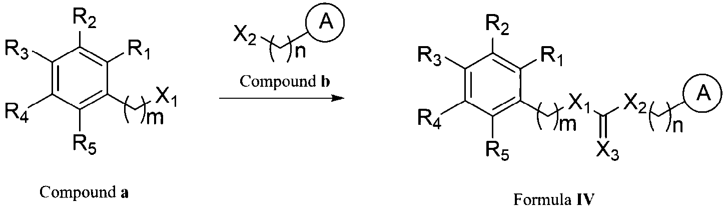
In a solvent, compound a, an organic base, TCDI or CDI, and compound b react to yield the compound represented by formula IV;
R₁, R₂, R₃, R₄, R₅, m, n, X₁, X₂, and ring A are as defined in any one of claims 1 to 12; X₃ is S or O;
preferably,
the solvent is dichloromethane;
and/or, the organic base is Et₃N;
and/or, the temperature of the reaction is 25-40 °C, and the reaction time is 10-12 h.

14. The method for preparation of the compound according to any one of claims 1-12, or a stereoisomer thereof, or a pharmaceutically acceptable salt thereof, or a solvate thereof, or a crystal form thereof, or a prodrug thereof, or a metabolite thereof, or a deuterated derivative thereof, **characterized in that** it comprises the following steps: 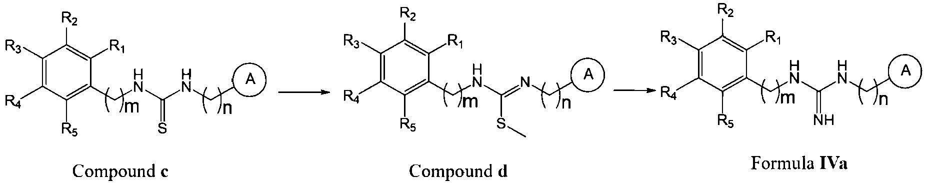
Step 1: In a solvent, compound c reacts with CH₃I to yield compound d;
Step 2: In a solvent, compound d reacts with ammonia water to obtain the compound represented by formula IVa;
R₁, R₂, R₃, R₄, R₅, m, n, and ring A are as defined in any one of claims 1 to 12;
preferably,
in step 1, the solvent is acetonitrile;
and/or, in step 1, the reaction temperature is 40-60 °C, and the reaction time is 4-10 h;
and/or, in step 2, the solvent is acetonitrile;
and/or, in step 2, the reaction temperature is 80-100 °C, and the reaction time is 10-12 h.

15. The use of the compound according to any one of claims 1-12, or a stereoisomer thereof, or a pharmaceutically acceptable salt thereof, or a solvate thereof, or a crystal form thereof, or a prodrug thereof, or a metabolite thereof, or a deuterated derivative thereof in the manufacture of medicaments with analgesic effects.

16. A medicament, **characterized in that** it is formed by using a compound according to any one of claims 1 to 12, or a stereoisomer thereof, or a pharmaceutically acceptable salt thereof, or a solvate thereof, or a crystalline form thereof, or a prodrug thereof, or a metabolite thereof, or a deuterated derivative thereof as the active ingredient, in combination with a pharmaceutically acceptable excipient.

17. A pharmaceutical composition, **characterized in that** it comprises a compound according to any one of claims 1 to 12, or a stereoisomer thereof, or a pharmaceutically acceptable salt thereof, or a solvate thereof, or a crystalline form thereof, or a prodrug thereof, or a metabolite thereof, or a deuterated derivative thereof.
